# EUROPEAN PATENT APPLICATION

(11) **EP 4 617 263 A1**
(43) Date of publication of application: **17.09.2025**
(21) Application number: 23888078.5
(22) Date of filing: 09.11.2023
(51) Int. Cl.: C07D 277/60, A61K 31/40, A61K 31/41, A61P 25/24

(54) **ARYL-CONTAINING AMINE COMPOUND, PREPARATION METHOD THEREFOR AND USE THEREOF**

(30) Priority: 09.11.2022 CN 202211401952; 04.09.2023 CN 202311134131
(71) Applicant: Shanghai Institute of Material Medica, Chinese Academy of Sciences, Shanghai 201203 (CN); Vigonvita Life Sciences Co., Ltd., Suzhou, Jiangsu 215123 (CN); XINJIANG TECHNICAL INSTITUTE OF PHYSICS AND CHEMISTRY, CHINESE ACADEMY OF SCIENCES, Urumqi, Xinjiang 830011 (CN)
(72) Inventor: SHEN, Jingshan, Shanghai 201203 (CN); AISA, Haji Akber, Beijing Road Urumqi, Xinjiang 830011 (CN); HE, Yang, Shanghai 201203 (CN); JI, Jing, Suzhou, Jiangsu 215123 (CN); ZHU, Weiliang, Shanghai 201203 (CN); ZHANG, Yong, Shanghai 201203 (CN); WU, Chunhui, Suzhou, Jiangsu 215123 (CN); TIAN, Guanghui, Suzhou, Jiangsu 215123 (CN); HU, Tianwen, Suzhou, Jiangsu 215123 (CN); OBUL, Mamateli, Beijing Road Urumqi, Xinjiang 830011 (CN)
(74) Representative: Kador & Partner Part mbB
(86) International application number: PCT/CN2023/130755
(87) International publication number: WO 2024/099393

(57) **Abstract**

Disclosed in the present invention are an aryl-containing amine compound, a preparation method therefor and a use thereof. The aryl-containing amine compound is represented by formula (I), and has the function of regulating the activity of an NMDA receptor and/or a monoamine transporter and/or a sigma receptor. Thus, the compound can be used for preparing drugs for treating and/or preventing diseases associated with an NMDA receptor and/or a monoamine transporter and/or a sigma receptor, especially central nervous system diseases.

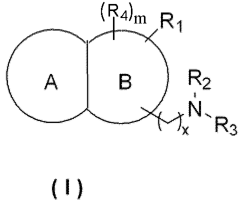

## Description

### CROSS-REFERENCE TO RELATED APPLICATION

The present application claims priority to Chinese Patent Application No. 202211401952.5, filed on November 9, 2022, and Chinese Patent Application No. 202311134131.4, filed on September 4, 2023, both entitled "AROMATIC AMINE COMPOUND, PREPARATION METHOD THEREFOR, AND USE THEREOF", which are incorporated herein by reference in their entireties as if fully set forth herein.

### TECHNICAL FIELD

The present disclosure relates to the field of pharmaceutical chemistry. Specifically, the present disclosure relates to an aryl-containing amine compound of formula (I), a preparation method thereof, and a pharmaceutical composition thereof, and use thereof in preparing a medicament for preventing or treating a disease associated with an NMDA receptor and/or a monoamine transporter and/or a sigma receptor, particularly a central nervous system disease.

### BACKGROUND

Depressive disorder is a serious psychiatric disease, mainly manifested as low mood, self-abasement, depression, and even suicidal tendencies in patients. Currently, the main drugs for treating the depressive disorder are selective serotonin/norepinephrine reuptake inhibitors (SSRI/SSNI). However, these drugs are ineffective in nearly one-third of patients and generally have drawbacks such as slow onset of action and even increased suicidal tendencies in patients. There remains a huge unmet clinical need in the art.

N-Methyl-D-aspartic acid (NMDA)-type glutamate receptor (abbreviated as NMDA receptor or NMDAR) is a ligand-gated ion channel. This receptor can be activated by glutamic acid, the most important excitatory neurotransmitter in the central nerve, thereby mediating the signaling of excitatory signals between synapses. When the ion channel of NMDAR opens, it enhances the permeability to cations such as Ca²⁺, K⁺, and Na⁺, generating excitatory postsynaptic potentials and triggering a series of physiological and biochemical reactions. The molecular structure of NMDAR is complex, with various subtypes showing specificity in spatiotemporal distribution and pharmacological characteristics. Their quantity, composition, and distribution dynamically change during different developmental stages and brain regions, participating in numerous physiological activities and providing a molecular basis for complex neural activities, thereby ensuring normal neural network function. The integration, localization, recycling, and intra- and extrasynaptic distribution of NMDAR depend on the regulation of neural activity. The disruption of its functional homeostasis is highly associated with various brain disorders such as depressive disorder, epilepsy, and schizophrenia.

Monoamine transporters are a class of proteins found on neuronal cell membranes in the central and peripheral nervous systems that are responsible for transporting commonly used neurotransmitters in synaptic transmission. Monoamine transporter inhibitors are typically used as antidepressants to treat depressive and anxiety disorders, such as bupropion which inhibits norepinephrine and dopamine transporters, and fluoxetine and citalopram which inhibit 5-HT transporters. Sigma receptors are mainly located in the central nervous system but also exist in the endocrine and immune systems, closely related to psychiatric diseases, depressive disorder, cognition, neuroprotection, and motor functions in the central nervous system. Sigma1 receptor agonists may have antidepressant and memory-enhancing effects, while antagonists may have antipsychotic and analgesic effects. sigma2 receptor is an ideal molecular target for treating central nervous system diseases such as Alzheimer's disease, schizophrenia, and traumatic brain injury, with both sigma2 receptor agonists and antagonists being potential therapies for neurodegenerative diseases.

The NMDA receptor antagonist ketamine has been used as a good anesthetic in medicine for over 50 years. Studies have found that intravenous administration of sub-anesthetic doses of the NMDAR antagonist ketamine can rapidly alleviate depressive symptoms within hours, with effects lasting at least one week. Esketamine, developed by Johnson & Johnson, was approved by the FDA in 2019 for treating refractory depressive disorder. Given that ketamine's primary molecular target is NMDAR, many have proposed that inhibition of this target is also responsible for ketamine's antidepressant effects. This mechanism suggests that ketamine's antidepressant effects and dissociative side effects may be mechanistically inseparable. However, numerous pieces of evidence challenge this hypothesis (J Psychiatry Neurosci. 2017, 42(4), 222.). First, the R-enantiomer of ketamine (R-ket) has been found to be more effective and longer-lasting as an antidepressant in rodent models compared with the S-enantiomer (S-ket), despite R-ket having significantly weaker affinity for NMDAR (Pharmacol Biochem Behav. 2014, 116, 137.). Similarly, the ketamine metabolite (2R,6R)-hydroxynorketamine (HNK) has been shown to induce antidepressant effects in rodent models but does not bind to NMDAR *in vivo* at dose levels that induce antidepressant effects (Nature. 2016, 533(7604), 481.; Proc Natl Acad Sci USA. 2019, 116(11), 5160.; Org Lett. 2017, 19(17), 4572.). Therefore, both R-ket and HNK can induce antidepressant effects while limiting ketamine's dissociative effects. However, other strategies proposed to reduce ketamine's dissociative side effects, such as targeting the NR2B subunit of NMDAR or using compounds with low trapping characteristics, have yielded poor results (Nature. 2016, 533(7604), 481.; Sci Rep. 2017, 7(1), 15725.; Int J Neuropsychopharmacol. 2019, 22(2), 119.; J Psychiatr Res. 2017, 86, 55.; Psychiatry Res. 2016, 239, 281.). Therefore, the precise molecular mechanisms underlying ketamine's antidepressant effects remain poorly understood and may involve other unidentified targets. Additionally, the magnitude of the antidepressant effects of NMDAR modulators and their accompanying dissociative effects are often highly unpredictable. These findings raise the exciting possibility that ketamine's antidepressant effects may actually be separable from its dissociative side effects.

The dissociative side effects and poor oral bioavailability of ketamine and esketamine significantly limit their clinical application. Although other orally available NMDAR antagonists have been developed, none have demonstrated rapid antidepressant clinical efficacy comparable with ketamine. Therefore, there remains an urgent need for novel antidepressants with robust clinical efficacy, low or no dissociative side effects, and good oral bioavailability. A drug that retains rapid antidepressant activity similar to ketamine while having reduced dissociative side effects and good oral bioavailability would provide a new treatment option. Due to its reduced dissociative effects and consequently lower abuse potential, this treatment option could be more manageable and potentially feasible for home use.

### SUMMARY

### Purpose of the Invention

The object of the present disclosure is to provide a novel aryl-containing amine compound with an NMDA receptor and/or monoamine transporter and/or sigma receptor regulating effect, a preparation method therefor, and use thereof.

One object of the present disclosure is to provide an aryl-containing amine compound of formula (I), or a stereoisomer thereof, a geometric isomer thereof, a conformational isomer thereof, a tautomer thereof, a pharmaceutically acceptable salt thereof, a polymorph thereof, a solvate thereof, a hydrate thereof, or an isotopically labeled compound thereof.

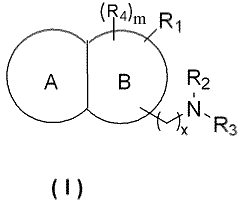

Another object of the present disclosure is to provide a method for preparing the aryl-containing amine compound of formula (I).

A further object of the present disclosure is to provide a pharmaceutical composition comprising a therapeutically effective amount of one or more selected from the compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof, and optionally one or more pharmaceutically acceptable carriers, diluents, or excipients.

A further object of the present disclosure is to provide use of one or more selected from the compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof, or the pharmaceutical composition described above, in preparing a medicament for regulating activity of an NMDA receptor and/or a monoamine transporter and/or a sigma receptor.

A still further object of the present disclosure is to provide use of one or more selected from the compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof, or the pharmaceutical composition described above, in preparing a medicament for preventing and/or treating a disease associated with an NMDA receptor and/or a monoamine transporter and/or a sigma receptor, particularly a central nervous system disease.

### Technical Schemes

According to a first aspect of the present disclosure, provided is a compound of formula (I) or a stereoisomer thereof, a geometric isomer thereof, a conformational isomer thereof, or a tautomer thereof, or a pharmaceutically acceptable salt thereof, a polymorph thereof, a solvate thereof, a hydrate thereof, or an isotopically labeled compound thereof: 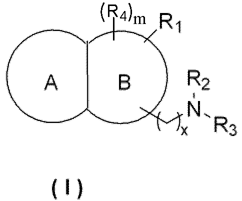 wherein:
ring A is selected from a 4- to 10-membered heterocyclic ring and a C6-C10 aromatic ring fused with a 4- to 10-membered heterocyclic ring comprising 1-3 heteroatoms (e.g., 1, 2, or 3) selected from N, O, and S, wherein the 4- to 10-membered heterocyclic ring is, for example, a 5-, 6-, 7-, 8-, or 9-membered heterocyclic ring, preferably a 5- to 8-membered heterocyclic ring; preferably, the heterocyclic ring in ring A comprises one N atom and one S atom, or two N atoms, or one S atom, or two N atoms and one S atom, or one N atom and one O atom;
ring A is optionally substituted with one or more R₅, wherein each R₅ is independently selected from halogen, hydroxy, amino, cyano, carboxy, oxo, C1-C6 alkyl, halogenated C1-C6 alkyl, C1-C6 alkoxy, C1-C6 alkanoyl, carbamoyl (-CONH₂), carbamoyl substituted with C1-C6 alkyl, amino substituted with one or two C1-C6 alkyl, amino substituted with one or two halogenated C1-C6 alkyl, amino substituted with one or two C1-C6 alkanoyl, C1-C6 alkoxycarbonyl, C3-C6 cycloalkyl, 4- to 10-membered heterocycloalkyl, C6-14 aryl, 5- to 10-membered heteroaryl, C6-C14 aryl C1-C6 alkyloxy, and 5- to 10-membered heteroaryl C1-C6 alkyloxy; preferably, each R₅ is independently selected from halogen, hydroxy, amino, cyano, carboxy, oxo, C1-C4 alkyl, halogenated C1-C4 alkyl, C1-C4 alkoxy, C1-C4 alkanoyl, carbamoyl (-CONH₂), carbamoyl substituted with C1-C4 alkyl, amino substituted with one or two C1-C4 alkyl, amino substituted with one or two halogenated C1-C4 alkyl, amino substituted with one or two C1-C4 alkanoyl, C1-C4 alkoxycarbonyl, C3-C6 cycloalkyl, 4- to 8-membered heterocycloalkyl, C6-10 aryl, 5- to 10-membered heteroaryl, C6-C10 aryl C1-C4 alkyloxy, and 5- to 10-membered heteroaryl C1-C4 alkyloxy; more preferably, each R₅ is independently selected from halogen (particularly bromine), amino, hydroxy, cyano, carboxy, C1-C3 alkyl (particularly methyl, ethyl, and isopropyl), halogenated C1-C3 alkyl (particularly trifluoromethyl), C1-C3 alkoxy (particularly methoxy and ethoxy), C1-C3 alkanoyl (particularly formyl and acetyl), carbamoyl (-CONH₂), formylamino, acetylamino, methylamino, ethylamino, N,N-dimethylamino, 2,2,2-trifluoroethylamino, C1-C3 alkoxycarbonyl (particularly methoxycarbonyl and ethoxycarbonyl), C3-C5 cycloalkyl (particularly cyclopropyl), phenyl, pyridinyl, pyrrolidinyl, piperidinyl, morpholinyl, and benzyloxy;
ring B is a 3- to 10-membered carbocyclic ring, preferably a 5- to 8-membered carbocyclic ring, such as a 5-, 6-, 7-, or 8-membered carbocyclic ring, and more preferably a 5-, 6-, or 7-membered carbocyclic ring;
R₁ and 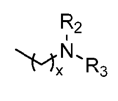 are attached to the same ring carbon atom in ring B;
x is an integer of 0-2, such as 0, 1, or 2;
R₂ and R₃ are each independently selected from hydrogen, C1-C6 alkyl, halogenated C1-C6 alkyl, C1-C6 alkoxy C1-C6 alkyl, hydroxy C1-C6 alkyl, C6-C14 aryl C1-C6 alkyl, C3-C6 cycloalkyl, C1-C6 alkanoyl, halogenated C1-C6 alkanoyl, C3-C6 cycloalkyl C1-C6 alkanoyl, C6-C14 aryl C1-C6 alkanoyl, C1-C6 alkylsulfonyl, C1-C6 alkylsulfinyl, and C3-C6 cycloalkyl C1-C6 alkyl; preferably, R₂ and R₃ are each independently selected from hydrogen, C1-C4 alkyl, halogenated C1-C4 alkyl, C1-C4 alkoxy C1-C4 alkyl, hydroxy C1-C4 alkyl, C6-C14 aryl C1-C4 alkyl, C3-C6 cycloalkyl, C1-C4 alkanoyl, halogenated C1-C4 alkanoyl, C3-C6 cycloalkyl C1-C4 alkanoyl, C6-14 aryl C1-C4 alkanoyl, C1-C4 alkylsulfonyl, C1-C4 alkylsulfinyl, and C3-C6 cycloalkyl C1-C4 alkyl; preferably, R₂ and R₃ are selected from hydrogen, C1-C4 alkyl (particularly methyl, ethyl, propyl, isopropyl, and tert-butyl), halogenated C1-C4 alkyl (particularly 1,1,1-trifluoroethyl), methoxyethyl, hydroxymethyl, hydroxyethyl, C3-C6 cycloalkylmethyl (particularly cyclopropylmethyl and cyclobutylmethyl), benzyl, C3-C6 cycloalkyl (particularly cyclopropyl), C1-C3 alkanoyl (particularly acetyl and propionyl), cyclopropylformyl, benzoyl, and *tert*-butylsulfinyl;
or, R₂ and R₃, together with the nitrogen atom to which they are attached, form a 3- to 9-membered heterocycloalkyl, wherein the 3- to 9-membered heterocycloalkyl ring optionally comprises one or more additional nitrogen atoms or oxygen atoms, and the 3- to 9-membered heterocycloalkyl is optionally substituted with one or more C1-C6 alkyl, preferably C1-C4 alkyl; preferably, R₂ and R₃, together with the nitrogen atom to which they are attached, form azetidinyl, pyrrolidinyl, piperidinyl, azepanyl, or morpholinyl;
R₁ is selected from C6-C14 aryl, 5- to 10-membered heteroaryl, 4- to 10-membered heterocyclyl, C6-C10 aryl fused with a 4- to 10-membered carbocyclic ring (e.g., indanyl), and C6-C10 aryl fused with a 4- to 10-membered heterocyclic ring (e.g., 1,2-methylenedioxyphenyl and 2,3-dihydrobenzofuranyl); preferably, R₁ is selected from C6-C14 aryl and 5- to 10-membered heteroaryl; more preferably, R₁ is phenyl, naphthyl, quinolyl, isoquinolyl, pyridinyl, pyrimidinyl, pyrazinyl, pyridazinyl, thienyl, or thiazolyl; still preferably, R₁ is phenyl, naphthyl, pyrimidinyl, pyridinyl, pyrazinyl, pyridazinyl, or quinolyl; further preferably, R₁ is phenyl;
the C6-C14 aryl, 5- to 10-membered heteroaryl, 4- to 10-membered heterocyclyl, C6-C10 aryl fused with the 4- to 10-membered carbocyclic ring, and C6-C10 aryl fused with the 4- to 10-membered heterocyclic ring are optionally substituted with one or more R₆;
R₆ are each independently selected from halogen, hydroxy, sulfhydryl, cyano, carbamoyl (NH₂CO-), sulfamoyl (NH₂SO₂-), C1-C6 alkyl, halogenated C1-C6 alkyl, hydroxy C1-C6 alkyl, C3-C6 cycloalkyl, C3-C6 cycloalkoxy, C3-C6 cycloalkyl substituted with C1-C3 alkyl, C3-C6 cycloalkoxy substituted with C1-C3 alkyl, C1-C6 alkoxy, C1-C6 alkylthio, halogenated C1-6 alkoxy, C3-C6 cycloalkyl C1-C6 alkoxy, C1-C6 alkanoyloxy, C6-C14 aryl, 5- to 10-membered heteroaryl, C6-C14 aryl C1-C6 alkoxy, and 5- to 10-membered heteroaryl C1-C6 alkoxy; preferably, R₆ are each independently selected from halogen, cyano, hydroxy, carbamoyl (NH₂CO-), sulfamoyl (NH₂SO₂-), C1-C4 alkyl, halogenated C1-C4 alkyl, hydroxy C1-C4 alkyl, C3-C6 cycloalkyl, C3-C6 cycloalkoxy, C3-C6 cycloalkyl substituted with C1-C3 alkyl, C3-C6 cycloalkoxy substituted with C1-C3 alkyl, C1-C4 alkoxy, C1-C4 alkylthio, halogenated C1-C4 alkoxy, C3-C6 cycloalkyl C1-C3 alkoxy, C1-C3 alkanoyloxy, C6-C10 aryl, 5- to 6-membered heteroaryl, C6-C10 aryl C1-C4 alkoxy, and 5- to 6-membered heteroaryl C1-C4 alkoxy; more preferably, R₆ are each independently selected from fluorine, chlorine, bromine, cyano, hydroxy, methyl, isopropyl, difluoromethyl, trifluoromethyl, hydroxymethyl, methylcyclopropyloxy, methoxy, ethoxy, isopropoxy, isobutoxy, methylthio, cyclopropyl, cyclopropoxy, carbamoyl (NH₂CO-), sulfamoyl (NH₂SO₂-), difluoromethoxy, trifluoromethoxy, 1,1,1-trifluoroethoxy, cyano, cyclopropylmethoxy, acetoxy, phenyl, pyridinyl, and benzyloxy;
m is an integer of 0-3, such as 0, 1, 2, or 3;
R₄ is attached to any carbon atom of ring B other than the carbon atom to which both R₁ and 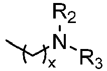 are attached, and R₄ are each independently selected from hydroxy, halogen, and C1-C6 alkyl.

In some embodiments, the compound of formula (I) is selected from compounds of formulas (I-A)-(I-D): 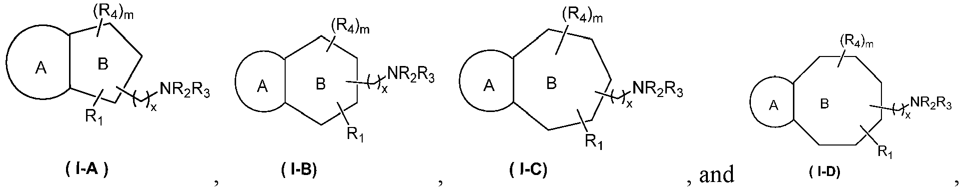 wherein ring A, R₁, m, R₄, 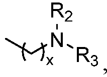 R₂, R₃, and x are as defined above.

In some embodiments, ring A is selected from a 5- or 6-membered heterocyclic ring and a benzo 5- or 6-membered heterocyclic ring, preferably 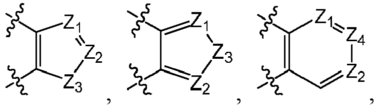 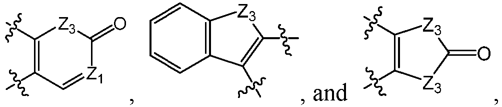 wherein Z₁, Z₂, and Z₄ are independently selected from N and CR₇, and each Z₃ is independently selected from NR₇, O, and S; Z₁, Z₂, and Z₄ are not simultaneously CR₇, wherein each R₇ is independently selected from hydrogen, halogen, cyano, hydroxy, amino, carbamoyl (-CONH₂), carbamoyl substituted with C1-C6 alkyl, carboxy, C1-C6 alkyl, C1-C6 alkoxy, halogenated C1-C6 alkyl, amino substituted with C1-C6 alkyl, amino substituted with halogenated C1-C6 alkyl, amino substituted with C1-C6 alkanoyl, C1-C6 alkanoyl, C1-C6 alkoxycarbonyl, C3-C6 cycloalkyl, 4- to 8-membered heterocycloalkyl, C6-10 aryl, 5- to 10-membered heteroaryl, C6-C10 aryl C1-C4 alkyloxy, and 5- to 10-membered heteroaryl C1-C4 alkyloxy; preferably, each R₇ is independently selected from hydrogen, halogen (particularly bromine), amino, hydroxy, cyano, carboxy, C1-C3 alkyl (particularly methyl, ethyl, and isopropyl), halogenated C1-C3 alkyl (particularly trifluoromethyl), C1-C3 alkoxy (particularly methoxy and ethoxy), C1-C3 alkanoyl (particularly formyl and acetyl), carbamoyl (-CONH₂), formylamino, acetylamino, methylamino, ethylamino, *N,N*-dimethylamino, 2,2,2-trifluoroethylamino, C1-C3 alkoxycarbonyl (particularly methoxycarbonyl and ethoxycarbonyl), C3-C5 cycloalkyl (particularly cyclopropyl), phenyl, pyridinyl, pyrrolidinyl, piperidinyl, morpholinyl, and benzyloxy; and more preferably, ring A is selected from: 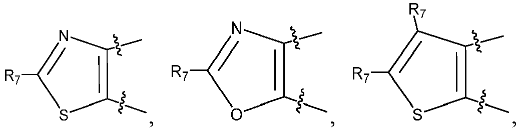 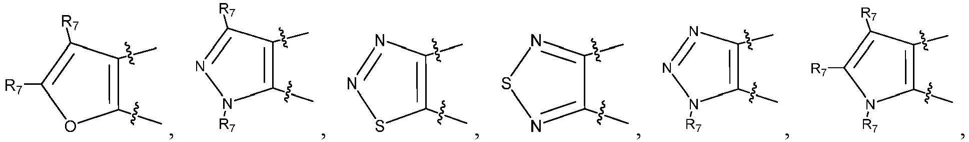 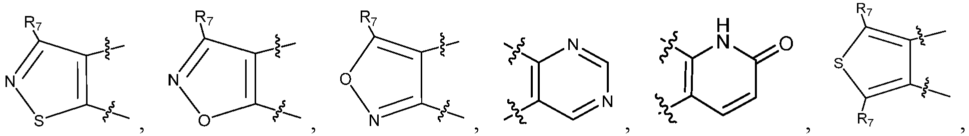 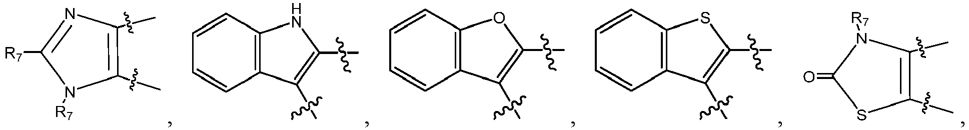 and 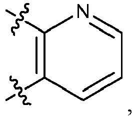 wherein each R₇ is independently selected from hydrogen, halogen, cyano, hydroxy, amino, carbamoyl (-CONH₂), carbamoyl substituted with C1-C6 alkyl, carboxy, C1-C6 alkyl, C1-C6 alkoxy, halogenated C1-C6 alkyl, amino substituted with C1-C6 alkyl, amino substituted with halogenated C1-C6 alkyl, amino substituted with C1-C6 alkanoyl, C1-C6 alkanoyl, C1-C6 alkoxycarbonyl, C3-C6 cycloalkyl, C3-C6 cycloalkyl, 4- to 8-membered heterocycloalkyl, C6-10 aryl, 5- to 10-membered heteroaryl, C6-C10 aryl C1-C4 alkyloxy, and 5- to 10-membered heteroaryl C1-C4 alkyloxy; preferably, each R₇ is independently selected from hydrogen, halogen (particularly bromine), amino, hydroxy, cyano, carboxy, C1-C3 alkyl (particularly methyl, ethyl, and isopropyl), halogenated C1-C3 alkyl (particularly trifluoromethyl), C1-C3 alkoxy (particularly methoxy and ethoxy), C1-C3 alkanoyl (particularly formyl and acetyl), carbamoyl (-CONH₂), formylamino, acetylamino, methylamino, ethylamino, *N,N*-dimethylamino, 2,2,2-trifluoroethylamino, C1-C3 alkoxycarbonyl (particularly methoxycarbonyl and ethoxycarbonyl), C3-C5 cycloalkyl (particularly cyclopropyl), morpholinyl, phenyl, pyridinyl, pyrrolidinyl, piperidinyl, and benzyloxy.

In some embodiments, R₁ is phenyl optionally substituted with one or more R₆, wherein R₆ is as defined above.

In some embodiments, R₁ is naphthyl optionally substituted with one or more R₆, wherein R₆ is as defined above.

In some embodiments, one of R₂ and R₃ is hydrogen, or both R₂ and R₃ are hydrogen.

In some embodiments, R₁ is phenyl optionally substituted with one or more R₆, and one of R₂ and R₃ is hydrogen, or both R₂ and R₃ are hydrogen.

In some embodiments, R₁ is naphthyl optionally substituted with one or more R₆, and one of R₂ and R₃ is hydrogen, or both R₂ and R₃ are hydrogen.

In some embodiments, ring B is a 6-membered carbocyclic ring; R₁ is phenyl optionally substituted with one or more R₆; one of R₂ and R₃ is hydrogen, or both R₂ and R₃ are hydrogen. In some embodiments, ring B is a 6-membered carbocyclic ring; R₁ is naphthyl optionally substituted with one or more R₆; one of R₂ and R₃ is hydrogen, or both R₂ and R₃ are hydrogen. In some embodiments, ring B is a 5-membered carbocyclic ring; R₁ is phenyl optionally substituted with one or more R₆; both R₂ and R₃ are hydrogen.

In some embodiments, ring B is a 7-membered carbocyclic ring; R₁ is phenyl optionally substituted with one or more R₆; both R₂ and R₃ are hydrogen.

In some embodiments, the compound of formula (I) is selected from the following compounds: 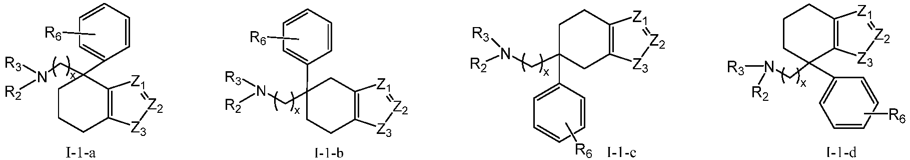 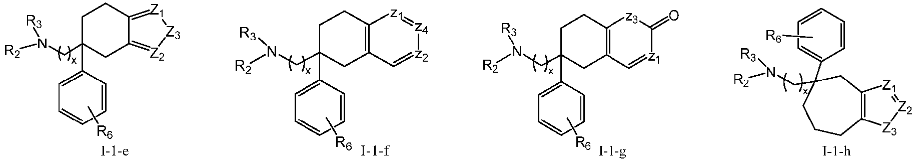 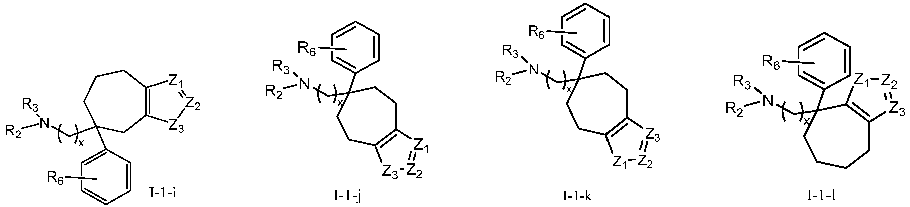 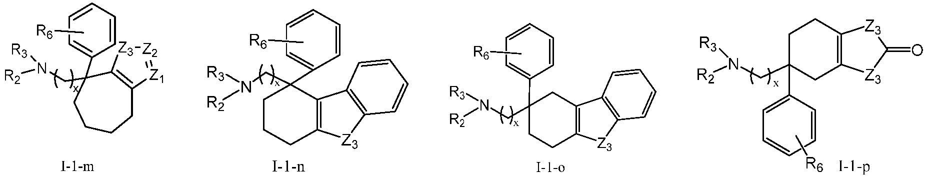 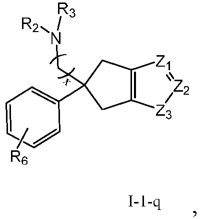 wherein x, Z₁, Z₂, Z₃, Z₄, R₂, R₃, and R₆ are as defined above.

In some embodiments, the compound of formula (I) is selected from the following compounds: 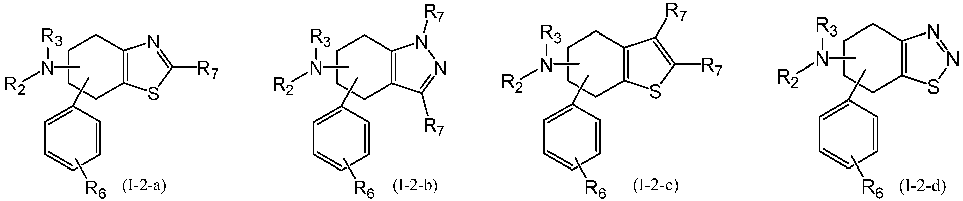 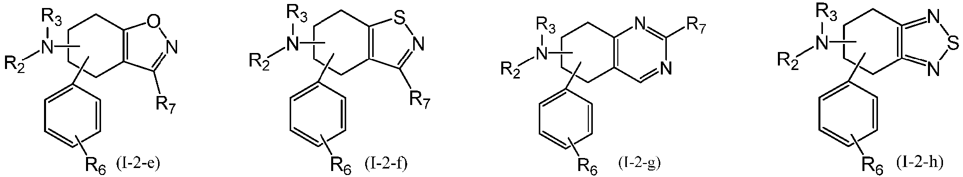 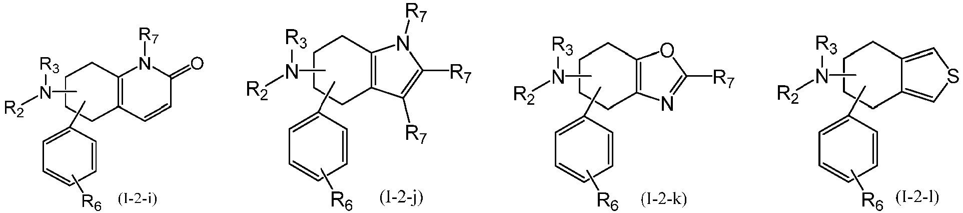 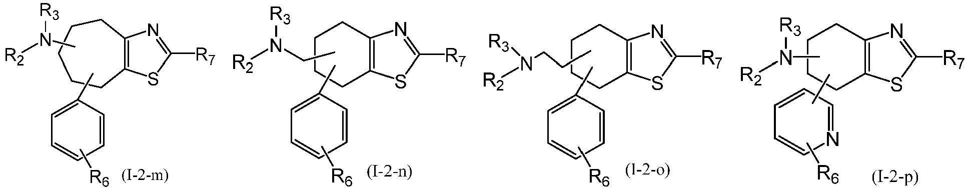 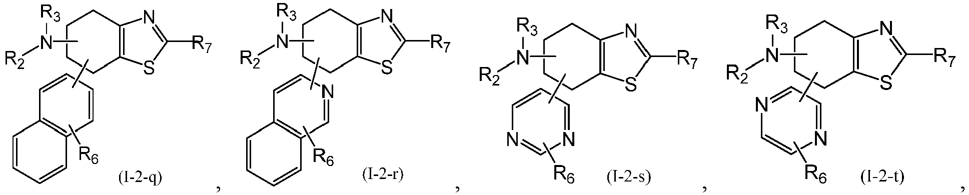 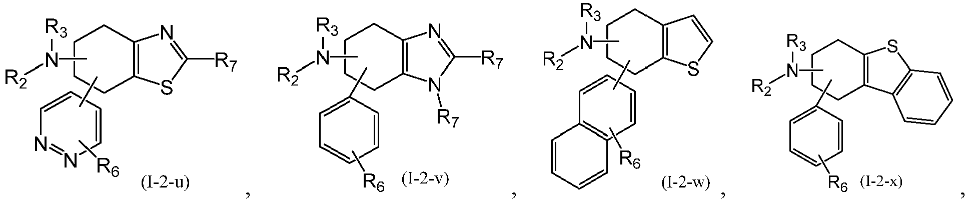 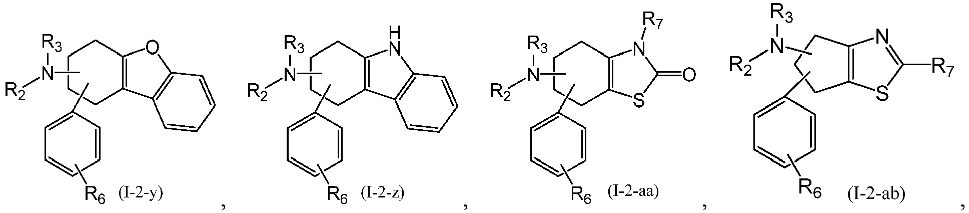 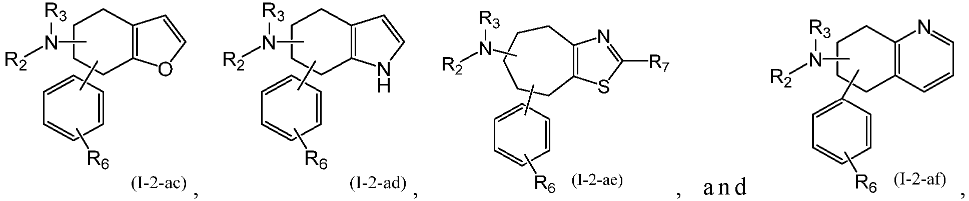
wherein R₂, R₃, R₆, and R₇ are as defined above;
and phenyl, pyridinyl, naphthyl, quinolyl, pyrimidinyl, pyrazinyl, or pyridazinyl, and -NR₂R₃, 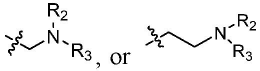 are attached to the same ring carbon atom of the carbocyclic ring to which they are attached.

In some embodiments, the compound of formula (I) is selected from the following compounds: 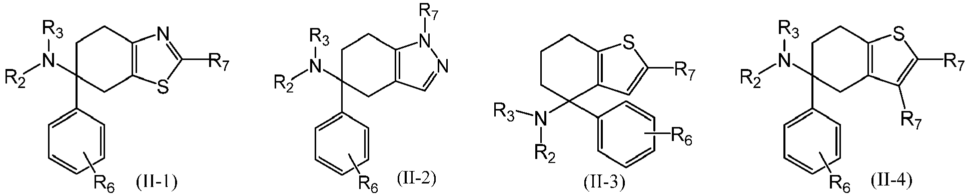 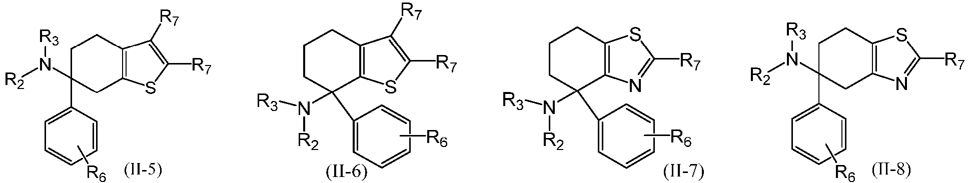 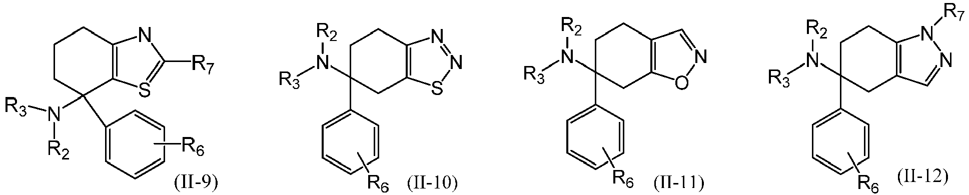 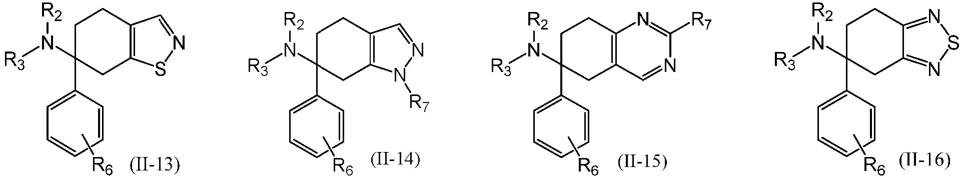 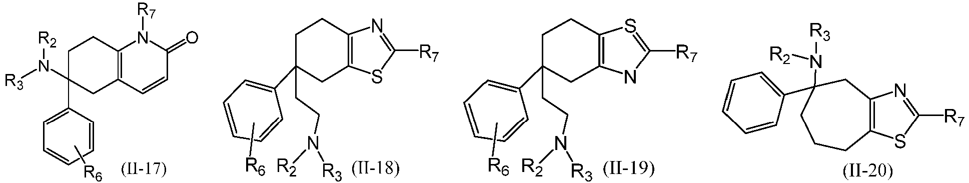 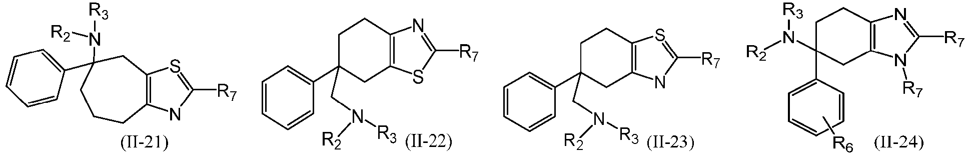 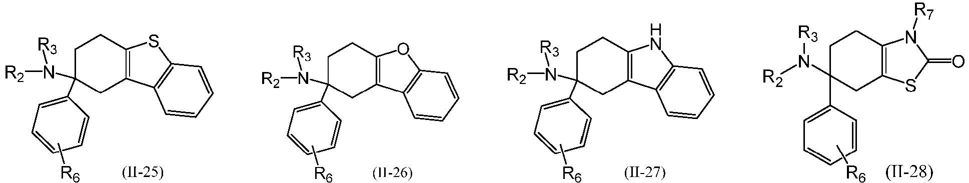 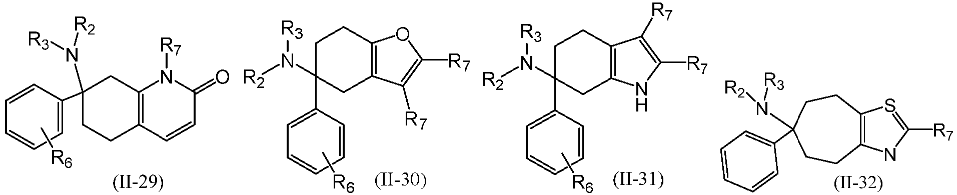 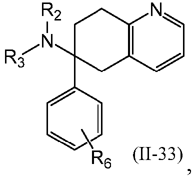 wherein R₂, R₃, R₆, and R₇ are as defined above.

Unless otherwise indicated, the definitions of the groups herein are as follows:
As used herein, the term "halogen" generally refers to fluorine, chlorine, bromine, and iodine; preferably fluorine, chlorine, or bromine, and more preferably fluorine or chlorine.

As used herein, "alkyl" refers to linear or branched saturated hydrocarbyl, such as C1-C6 alkyl refers to linear or branched saturated hydrocarbyl containing 1-6 carbon atoms, such as methyl, ethyl, *n*-propyl, isopropyl, *n-*butyl, isobutyl, *tert*-butyl, *sec*-butyl, *n*-pentyl, 1-ethylpropyl, isopentyl, neopentyl, isohexyl, 3-methylpentyl, or n-hexyl; preferably, alkyl is methyl, ethyl, *n*-propyl, isopropyl, butyl, or isobutyl.

As used herein, "halogenated C1-C6 alkyl" refers to linear or branched saturated hydrocarbyl containing 1-6 carbon atoms with hydrogen atoms substituted with one or more identical or different halogen atoms, and "halogenated C1-C4 alkyl" is defined by analogy, such as trifluoromethyl, fluoromethyl, difluoromethyl, chloromethyl, bromomethyl, dichlorofluoromethyl, chloroethyl, bromopropyl, 2-chlorobutyl, or pentafluoroethyl.

As used herein, "C1-C6 alkoxy" refers to linear or branched alkoxy containing 1-6 carbon atoms, and "C1-C4 alkoxy" and "C1-C3 alkoxy" are defined by analogy, such as methoxy, ethoxy, *n*-propoxy, isopropoxy, *n*-butoxy, isobutoxy, *tert*-butoxy, *sec*-butoxy, *n*-pentyloxy, isopentyloxy, neopentyloxy, isohexyloxy, 3-methylpentyloxy, or *n*-hexyloxy; preferably, C1-C6 alkoxy is methoxy, ethoxy, *n*-propoxy, isopropoxy, *n*-butoxy, or isobutoxy.

As used herein, "halogenated C1-C6 alkoxy" refers to linear or branched alkoxy containing 1-6 carbon atoms with hydrogen atoms substituted with one or more identical or different halogen atoms, and "halogenated C1-C4 alkoxy" is defined by analogy, such as -OCF₃, -OCH₂CH₂Cl, -OCHBrCH₂Cl, or -OCF₂CF₃.

As used herein, "C1-C6 alkanoyl" refers to linear or branched alkanoyl containing 1-6 carbon atoms, and "C1-C4 alkanoyl" and "C1-C3 alkanoyl" are defined by analogy, such as formyl, acetyl, propionyl, butyryl, isobutyryl, and valeryl; preferably, C1-C6 alkanoyl is formyl, acetyl, or propionyl.

As used herein, "halogenated C1-C6 alkanoyl" refers to linear or branched alkanoyl containing 1-6 carbon atoms with hydrogen atoms substituted with one or more identical or different halogen atoms, and "halogenated C1-C4 alkanoyl" and "halogenated C1-C3 alkanoyl" are defined by analogy.

As used herein, "oxo" refers to "=O", i.e. an oxygen atom is attached to other atoms via a double bond.

As used herein, "amino substituted with C1-C6 alkyl" refers to amino with hydrogen atoms substituted with 1 or 2 identical or different C1-C16 alkyl, such as -NHMe, -NHEt, -N(Me)Et, or -NEt₂.

As used herein, "amino substituted with C1-C6 alkanoyl" refers to amino with hydrogen atoms substituted with 1 or 2 identical or different C1-C6 alkanoyl, such as -NHCHO, -NHCOCH₃, or -NHCOCH₂CH₃.

As used herein, "C1-C6 alkoxy C1-C6 alkyl" refers to C1-C6 alkoxy with an oxygen atom attached to C1-C6 alkyl, such as -CH₂OCH₂CH₃, -CH₂CH₂OCH₂CH₃, or -CH₂CH₂OCH₃.

As used herein, "C1-C6 alkoxycarbonyl" refers to C1-C6 alkoxy with an oxygen atom attached to carbonyl, such as -C=OOCH₂CH₃, -C=OOCH₂CH₂CH₃, and -C=OOCH₂CH(CH₃)₂.

As used herein, "C3-C6 cycloalkyl" refers to saturated cyclohydrocarbyl containing 3-6 carbon atoms, such as cyclopropyl, cyclobutyl, cyclopentyl, or cyclohexyl.

As used herein, "hydroxy C1-C6 alkyl" refers to linear or branched alkyl containing 1-6 carbon atoms with one carbon atom attached to hydroxy, such as -CH₂OH, -CH₂CH₂OH, -CH(OH)CH₃, -CH₂CH₂CH₂OH, -CH₂CH₂CH₂CH₂OH, or -CH₂CH(CH₃)CH₂OH.

As used herein, "C6-C14 aryl" refers to a monocyclic or polycyclic aromatic ring group containing 6-14 ring atoms but no heteroatoms in the ring atoms, and "C6-C12 aryl" is defined by analogy, such as phenyl and naphthyl.

As used herein, "3- to 10-membered carbocyclic ring" refers to a saturated or unsaturated monocyclic hydrocarbon group containing 3-10 carbon atoms, and the carbocyclic ring preferably has 5-8 ring carbon atoms, more preferably 5-6 carbon atoms, such as cyclopropane, cyclobutane, cyclopentane, cyclopentene, cyclohexane, cyclohexene, cycloheptane, and cycloheptene.

As used herein, "C6-C14 aryl C1-C6 alkyl" refers to C6-C14 aryl attached to C1-C6 alkyl, such as benzyl, phenethyl, and phenylpropyl.

As used herein, "C3-C6 cycloalkyl C1-C6 alkanoyl" refers to C3-C6 cycloalkyl with a ring carbon atom attached to an alkyl carbon atom of C1-C6 alkanoyl, such as cyclopropylformyl, cyclopropylacetyl, cyclobutylformyl, and cyclopentylformyl.

As used herein, "C6-C14 aryl C1-C6 alkanoyl" refers to C6-C14 aryl attached to an alkyl carbon atom of C1-C6 alkanoyl, such as benzoyl and phenylacetyl.

As used herein, "C1-C6 alkylsulfonyl" refers to C1-C6 alkyl attached to sulfonyl (-S(=O)₂-), such as methylsulfonyl and ethylsulfonyl.

As used herein, "C1-C6 alkylsulfinyl" refers to C1-C6 alkyl attached to sulfinyl (> S=O), such as methylsulfinyl, ethylsulfinyl, propylsulfinyl, butylsulfinyl, and *tert*-butylsulfinyl.

As used herein, "C3-C6 cycloalkyl C1-C6 alkyl" refers to C3-C6 cycloalkyl attached to C1-C6 alkyl, such as cyclopropylmethyl and cyclobutylmethyl.

As used herein, "heterocyclic ring" refers to a monocyclic or polycyclic group containing at least one heteroatom selected from N, O, and S as a ring member, which may be an aromatic or non-aromatic group, preferably a monocyclic group herein; "4- to 10-membered heterocyclic ring" refers to heterocyclyl containing 4 to 10 ring atoms, such as pyridinyl, piperidinyl, morpholinyl, furanyl, thienyl, thiazolyl, imidazolyl, pyrrolyl, pyrazinyl, pyridazinyl, and pyrimidinyl.

As used herein, "heterocycloalkyl" refers to a saturated monocyclic or polycyclic group containing at least one heteroatom selected from N, O, and S as a ring member, and 3- to 9-membered heterocycloalkyl includes azetidinyl, pyrrolidinyl, piperidinyl, azepanyl, and morpholinyl.

As used herein, "heteroaryl" refers to a monocyclic or bicyclic aromatic ring group containing at least one heteroatom selected from nitrogen, oxygen, and sulfur as a ring member; 5- to 10-membered heteroaryl includes, but is not limited to, the following groups: pyrrolyl, imidazolyl, pyrazolyl, 1,2,3-triazolyl, pyridinyl, pyridonyl, pyridazinyl, pyrimidinyl, pyrazinyl, triazinyl, oxazolyl, isoxazolyl, oxadiazolyl, thiazolyl, isothiazolyl, thiadiazolyl, quinolyl, and the like.

As used herein, "optional" or "optionally" means that the subsequently described event may or may not occur, and the description includes both the situation where the event occurs and the situation where the event does not occur. For example, "optionally substituted alkyl" includes "unsubstituted alkyl" and "substituted alkyl" as defined herein. "Optionally substituted with halogen" includes both the situation of being "substituted with halogen" and "unsubstituted with halogen", such as being substituted with 0-3 halogen atoms. Those skilled in the art will appreciate that, for any group containing one or more substituents, the group excludes any substitution patterns that are spatially impractical, chemically incorrect, synthetically infeasible, and/or inherently unstable.

When reference is made herein to formula (I), such designation also includes its sub-formulas, such as formula (I-1-a), (I-1-b), (I-2-a), (I-2-b), (II-1), or (II-2).

The aryl-containing amine compound of general formula (I), and the geometric isomer thereof, the conformational isomer thereof, and the tautomer thereof of the present disclosure may also exist in solvate forms such as hydrates and alcoholates, and the solvates are also encompassed within the scope of the present disclosure. The pharmaceutically acceptable salt of the heterocyclic compound of general formula (I), and the geometric isomer thereof, the conformational isomer thereof, and the tautomer thereof of the present disclosure refers to a therapeutically active, non-toxic acid addition salt formed by treating the aryl-containing amine compound of general formula (I) or the stereoisomer thereof with a suitable acid. The salt is hydrochloride, hydrobromide, hydroiodide, sulfate or bisulfate, nitrate, phosphate or acid phosphate, perchlorate, formate, acetate, trifluoroacetate, propionate, pyruvate, glycolate, oxalate, malonate, succinate, glutarate, maleate, fumarate, lactate, malate, citrate, tartrate, picrate, glutamate, benzoate, mesylate, ethanesulfonate, benzenesulfonate, p-toluenesulfonate, salicylate, ascorbate, camphorate, camphorsulfonate, or the like. Conversely, the salt form may also be converted to the free base form by treatment with a base.

The term "pharmaceutically acceptable salt" as used above also includes solvates thereof, and the solvates are encompassed within the scope of the present disclosure. Examples of solvates include, for example, hydrates and alcoholates.

Those skilled in the art will recognize that the compounds of the present disclosure may contain chiral centers and thus may exist in different isomeric forms. As used herein, "isomer" refers to different compounds having the same molecular formula but differing in the arrangement or configuration of atoms.

"Stereoisomer" refers to isomers produced by different spatial arrangements of atoms in molecules, and can be classified into cis-trans isomers and enantiomers and also into enantiomers and diastereomers.

"Enantiomers" are a pair of stereoisomers that are non-superimposable mirror images of each other. A 1:1 mixture of a pair of enantiomers is a "racemic" mixture. The term is used to designate a racemic mixture where appropriate. When indicating the stereochemistry of the compounds of the present disclosure, the conventional RS system is used to designate a single stereoisomer with known relative and absolute configurations for compounds having two chiral centers (e.g., (1S,2S)); a single stereoisomer with known relative configuration but unknown absolute configuration is marked with an asterisk (e.g., (1R*,2R*)); a racemate with two letters (e.g., (1RS,2RS) refers to a racemic mixture of (1R,2R) and (1S,2S); (1RS,2SR) refers to a racemic mixture of (1R,2S) and (1S,2R)). "Diastereoisomers" are stereoisomers that have at least two asymmetric atoms, but are not mirror images of each other. Absolute stereochemistry is specified according to the Cahn-Ingold-Prelog R-S system. When the compound is a pure enantiomer, the stereochemistry at each chiral carbon may be specified by either R or S. A resolved compound with unknown absolute configuration may be designated as (+) or (-) based on the direction (dextrorotatory or levorotatory) of its rotation of plane-polarized light at the sodium D line wavelength. Alternatively, resolved compounds may be defined by their respective retention times via chiral HPLC relative to corresponding enantiomers/diastereomers.

Geometric isomerism may occur when the compound contains a double bond or other structural features conferring a degree of rigidity to the molecule. If the compound contains a double bond, the substituents may be in an E or Z conformation. If the compound contains disubstituted cycloalkyl, the substituents of the cycloalkyl may have a cis- or trans-configuration.

"Conformational isomers" are isomers differing by rotation about one or more single bonds.

"Tautomers" are isomers formed by proton transfer from one atom to another within the same molecule. All tautomeric forms of the compounds of the present disclosure are encompassed within the scope of the present disclosure.

"Polymorphs" refer to crystalline forms having the same chemical structure/composition but different spatial arrangements of the molecules and/or ions that make up the crystal. The compounds of the present disclosure may be provided as amorphous solids or crystalline solids. Lyophilization may be used to provide solid forms of the compounds of the present disclosure.

"Solvate" refers to a physical association of the compound of the present disclosure with one or more organic or inorganic solvent molecules, including hydrogen bonding. In certain cases, the solvate can be isolated, e.g., when one or more solvent molecules are incorporated into the crystal lattice of the crystalline solid. Solvent molecules in the solvate may be present in an ordered or disordered arrangement. The solvate may contain a stoichiometric or non-stoichiometric amount of solvent molecules. "Solvates" include both solution phases and isolatable solvates. Exemplary solvates include, but are not limited to, hydrates, ethanolates, methanolates, and isopropanolates. Solvation methods are well known in the art.

The present disclosure also encompasses all suitable isotopic variants of the compounds or the pharmaceutically acceptable salts thereof. Isotopic variants of the compounds or the pharmaceutically acceptable salts thereof of the present disclosure are defined as those wherein at least one atom is replaced by an atom of the same atomic number but with an atomic mass different from the atomic mass predominantly found in nature. The isotopes that may be incorporated into the compounds and the pharmaceutically acceptable salts thereof of the present disclosure include, but are not limited to, isotopes of H, C, N, and O, such as ²H, ³H, ¹¹C, ¹³C, ¹⁴C, ¹⁵N, ¹⁷O, ¹⁸O, ³⁵S, ¹⁸F, ³⁶Cl, and ¹²⁵I. The isotopic variants of the compounds and the pharmaceutically acceptable salts thereof of the present disclosure can be prepared by conventional techniques using appropriate isotopic variants of suitable reagents.

According to the present disclosure, the aryl-containing amine compound of formula (I) is selected from the following structures:

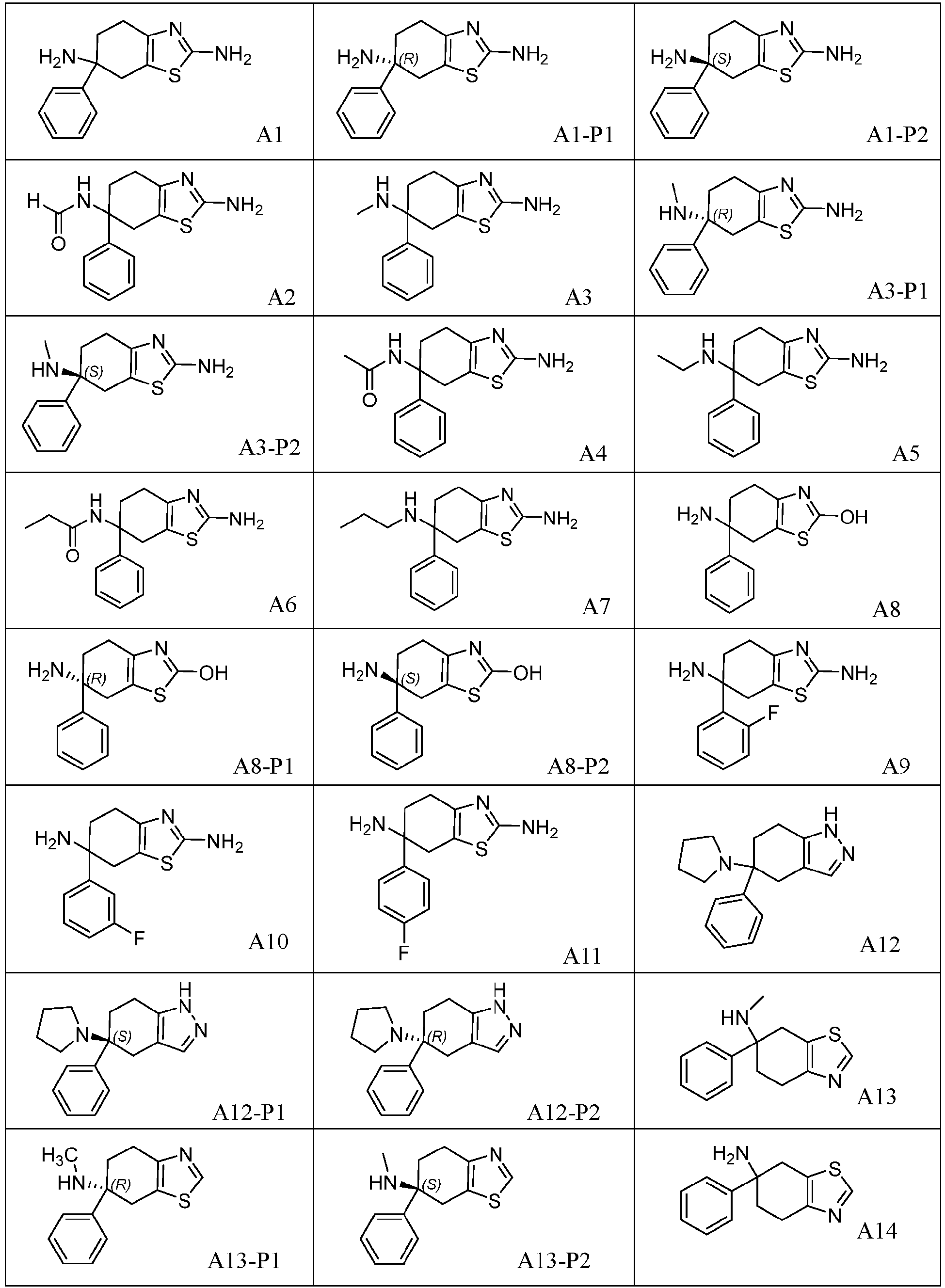

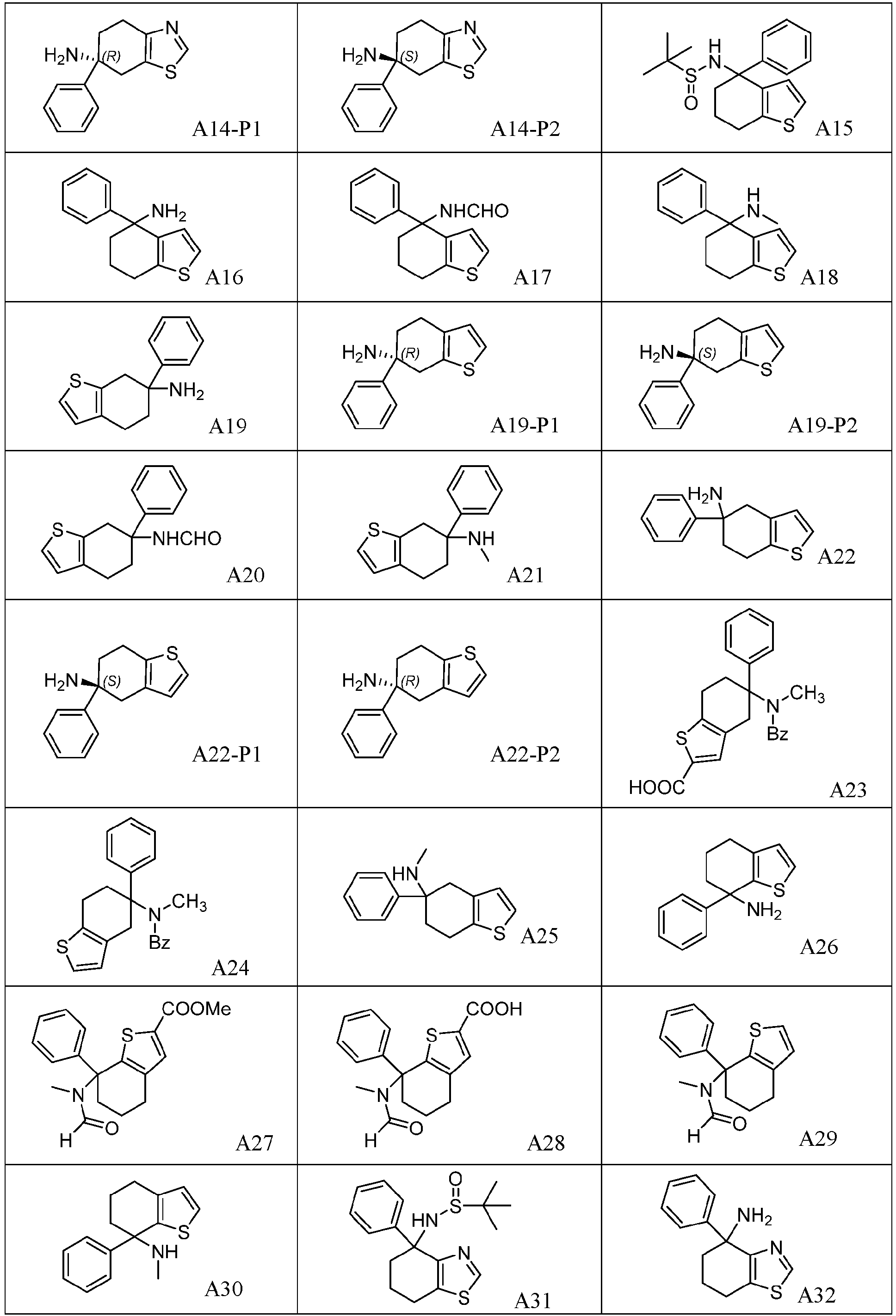

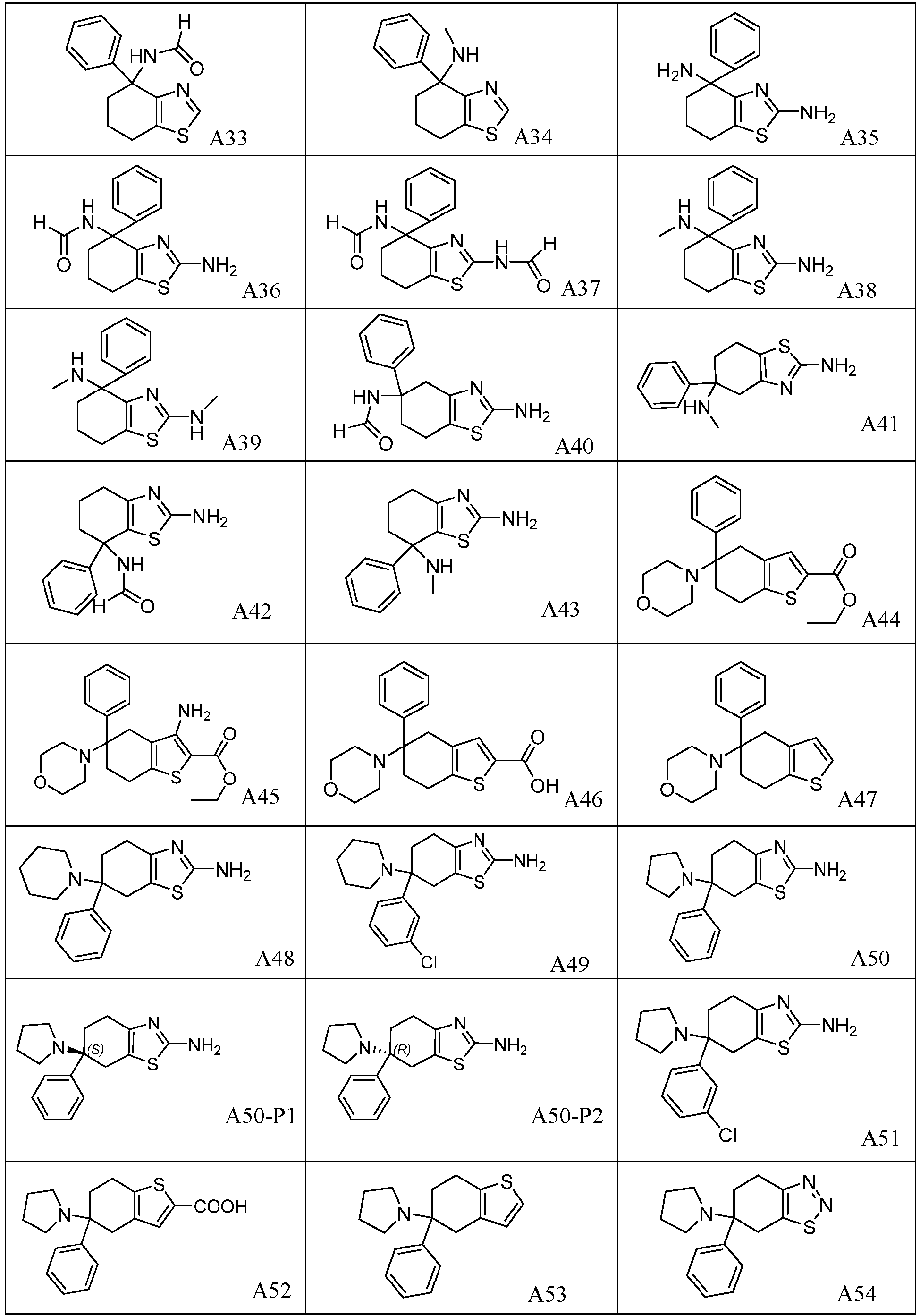

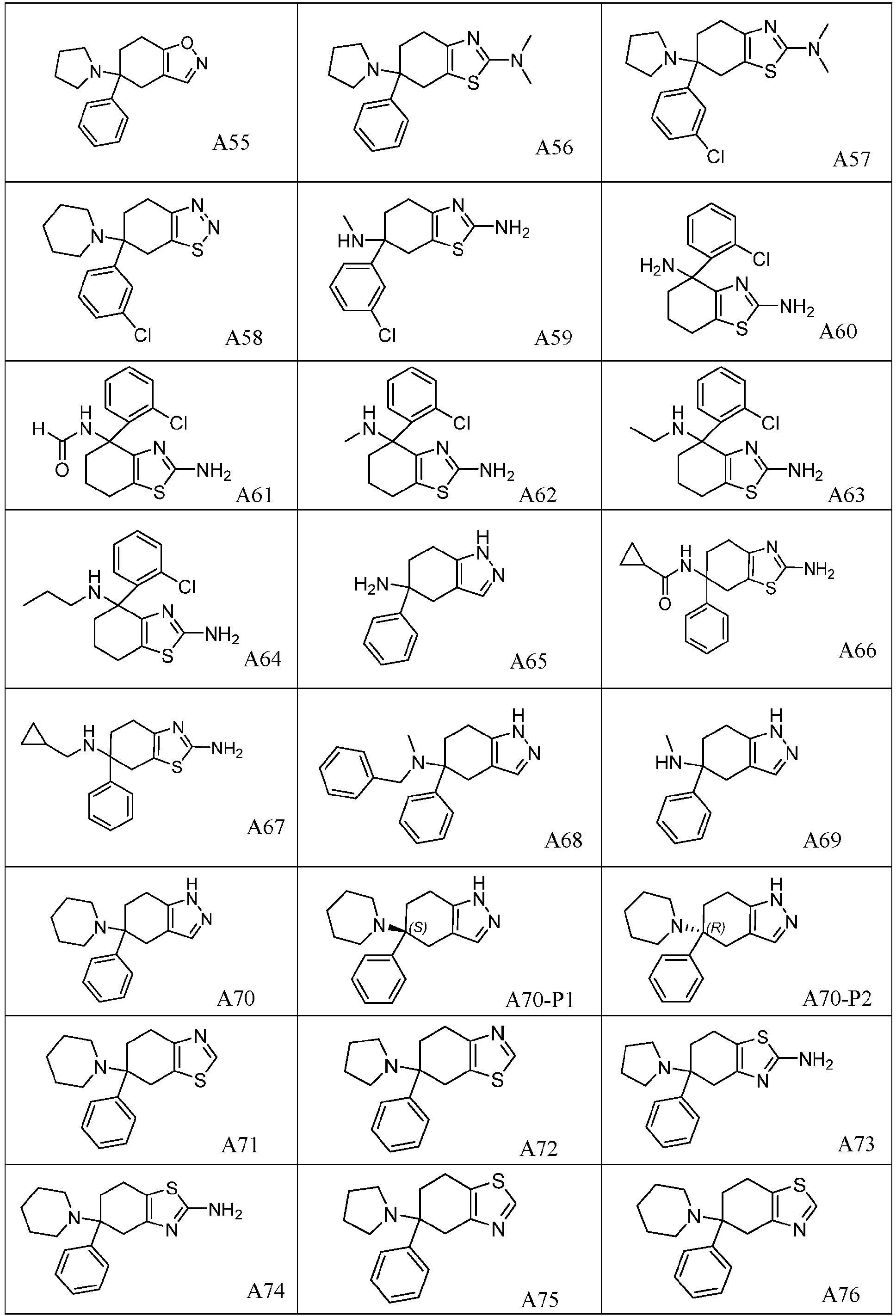

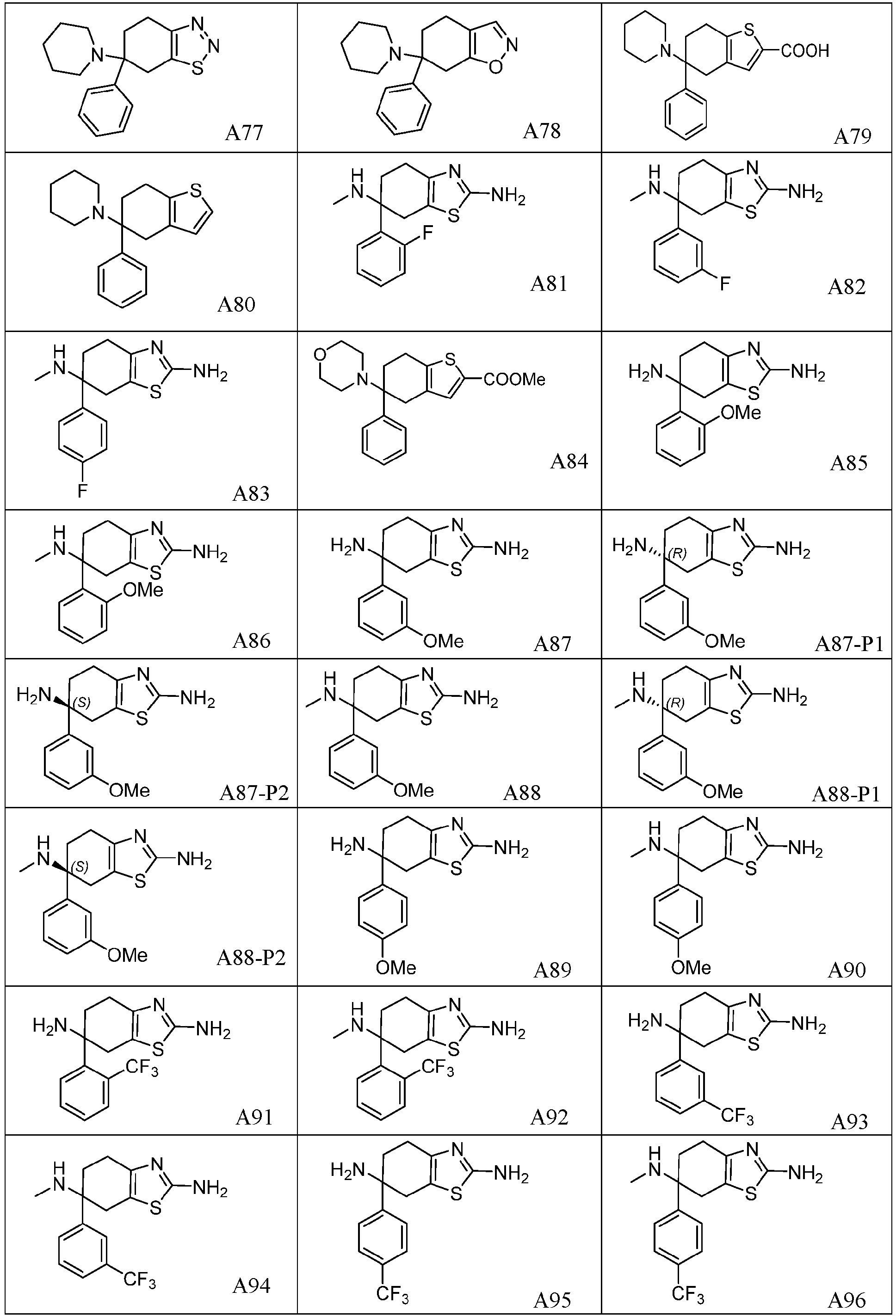

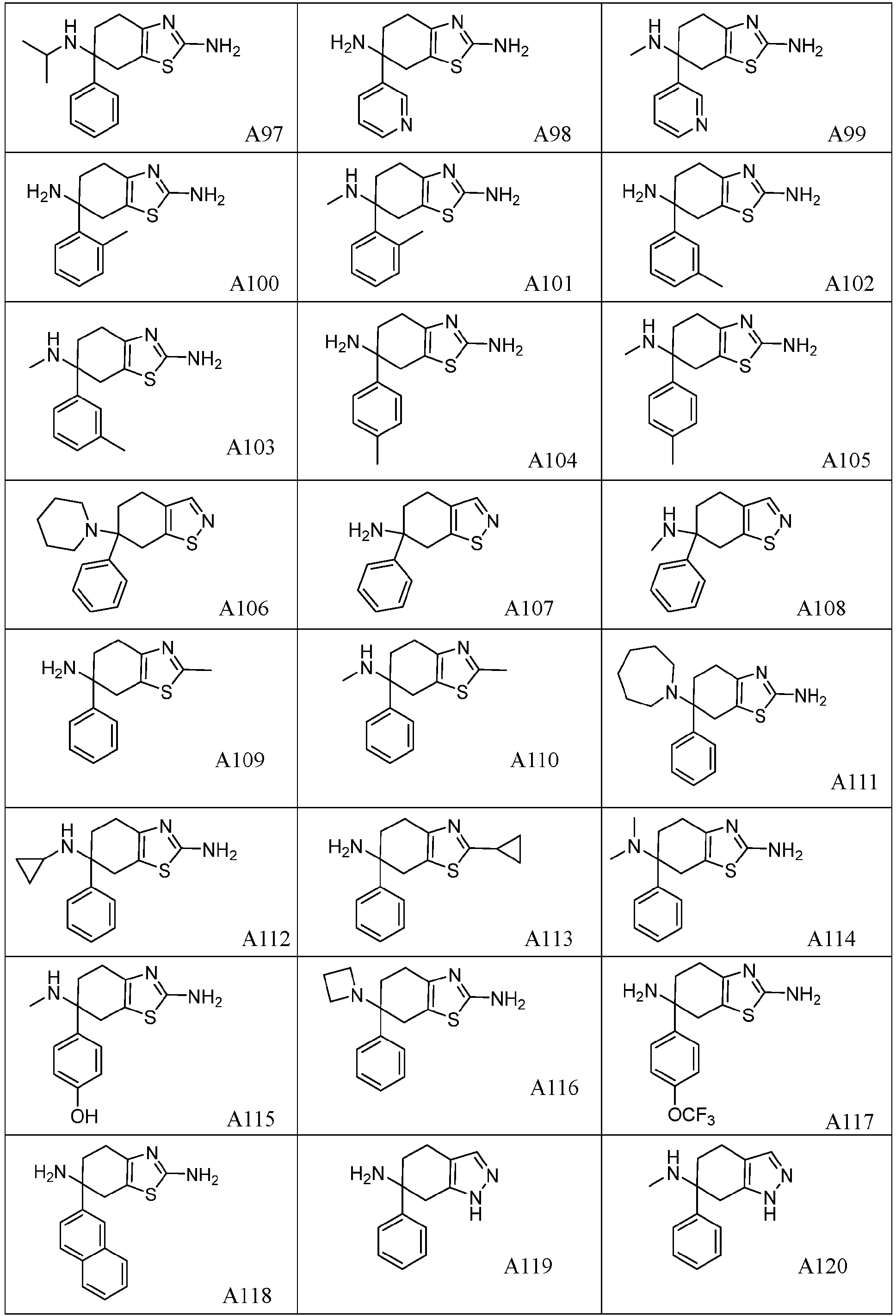

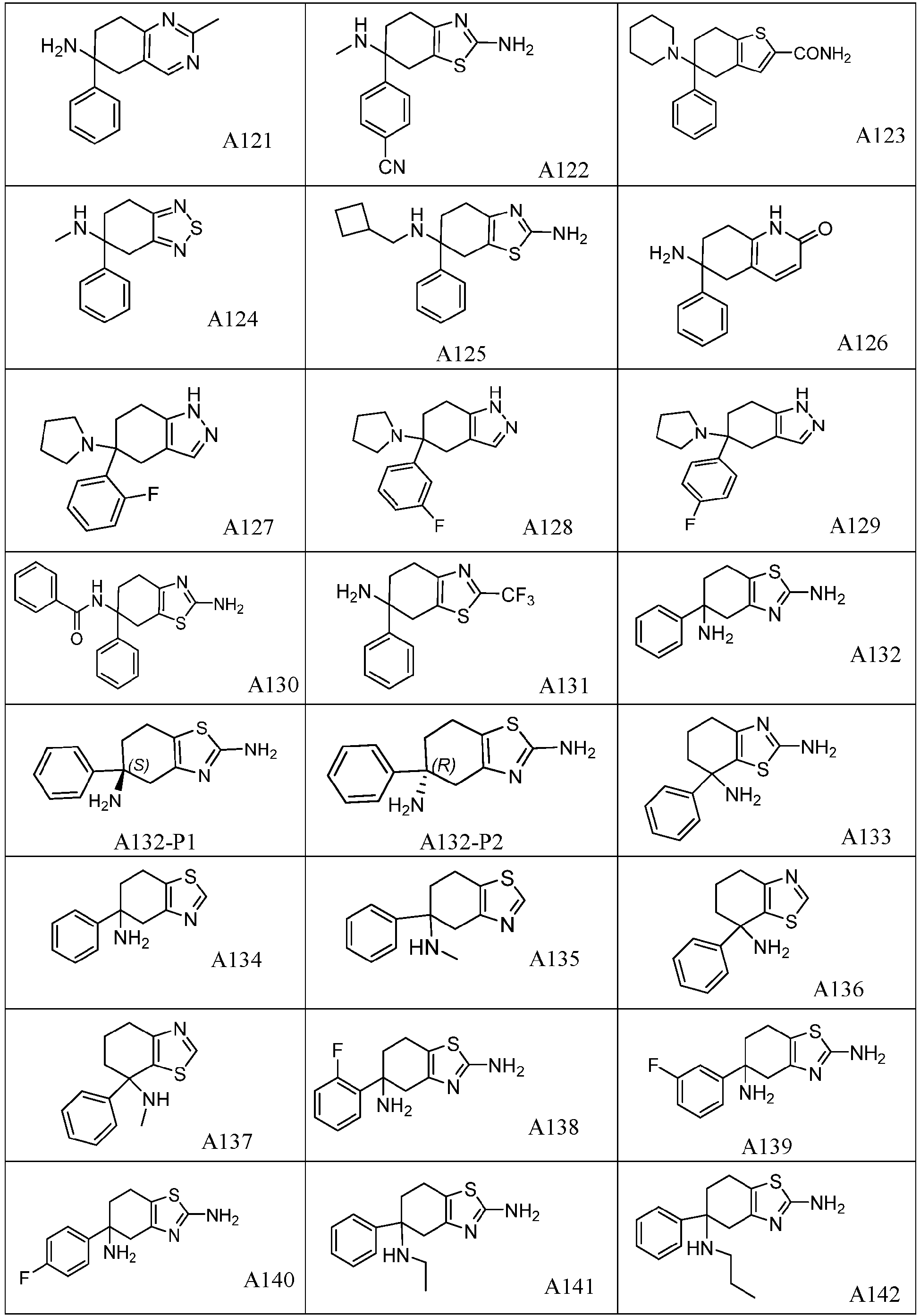

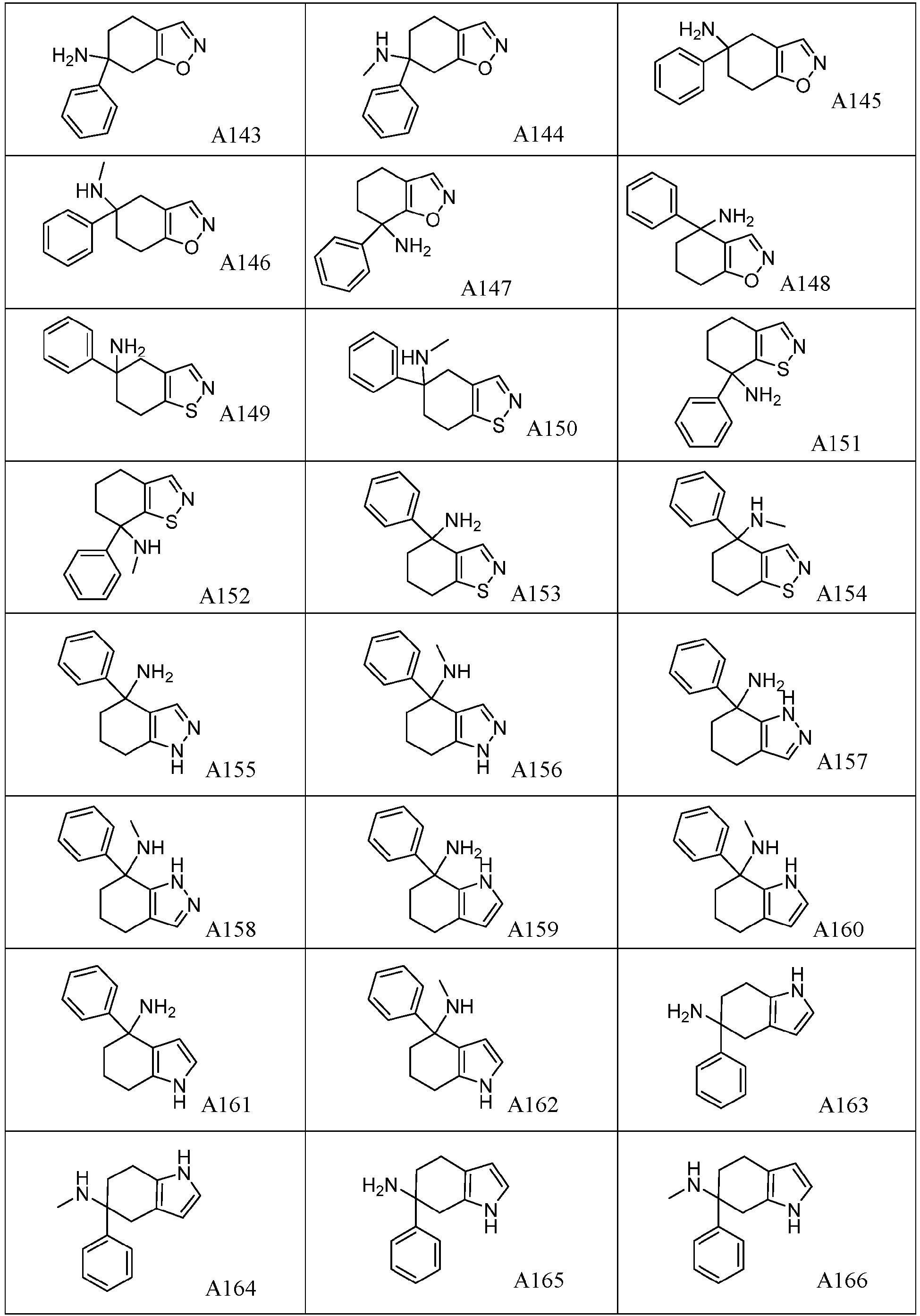

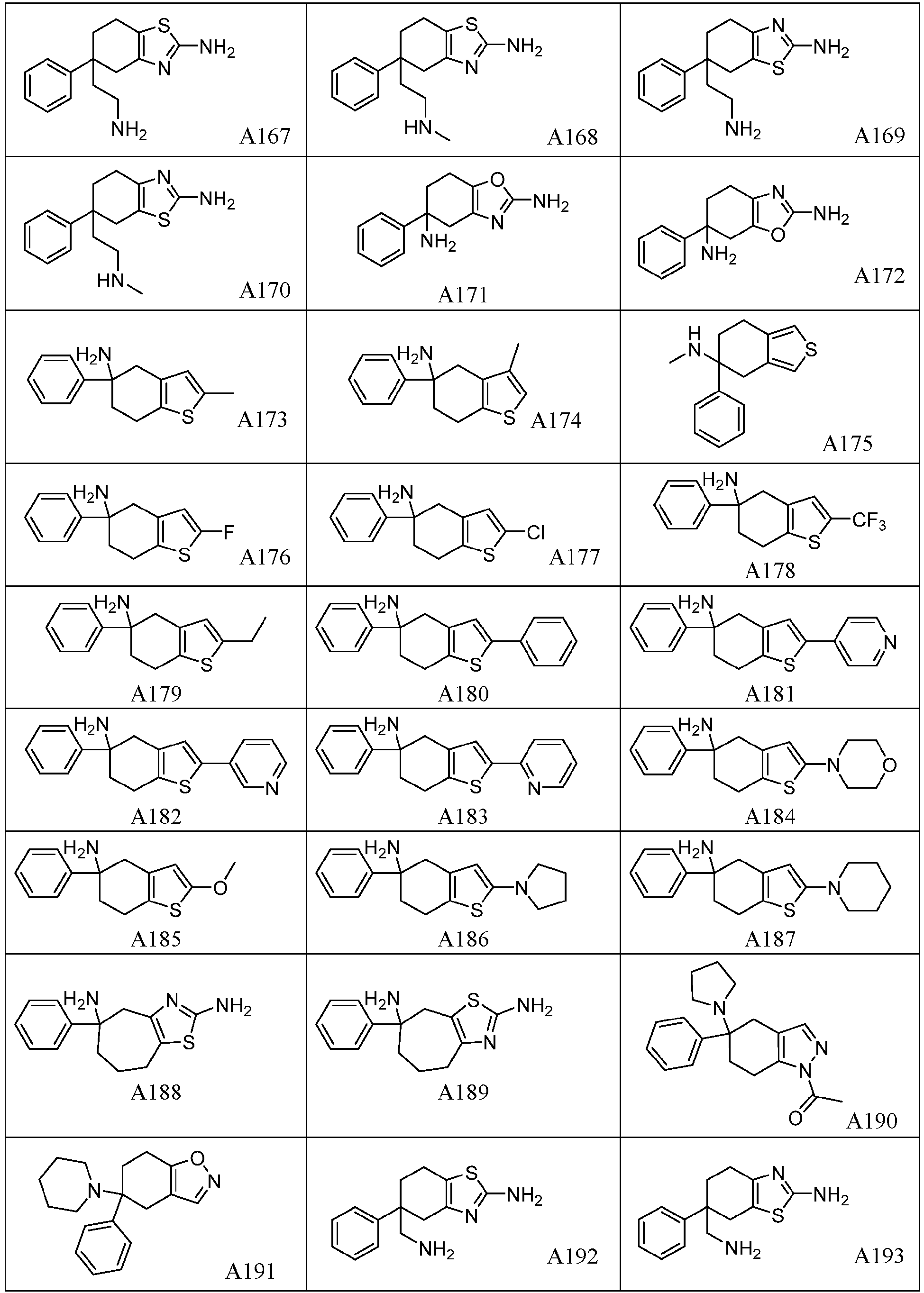

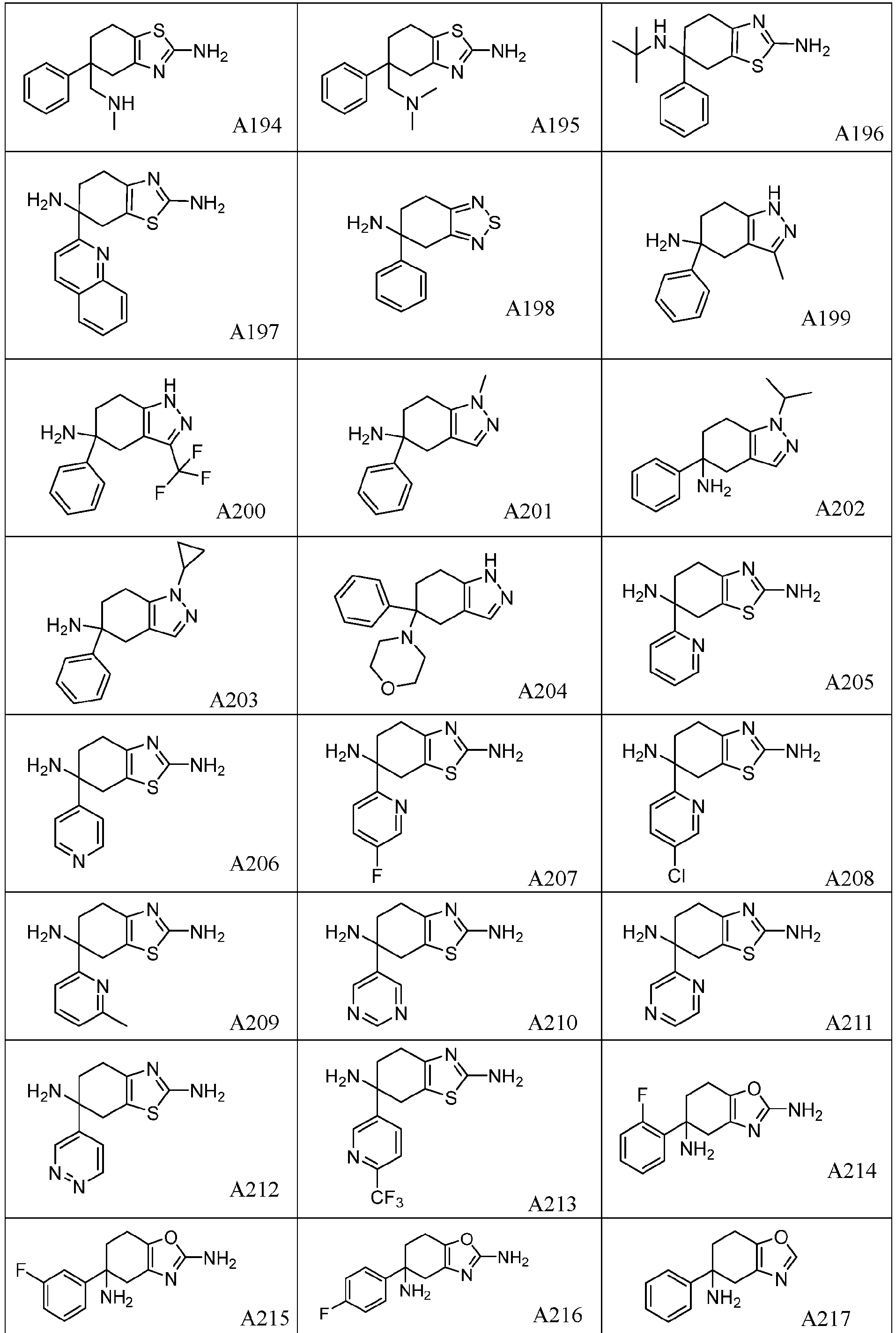

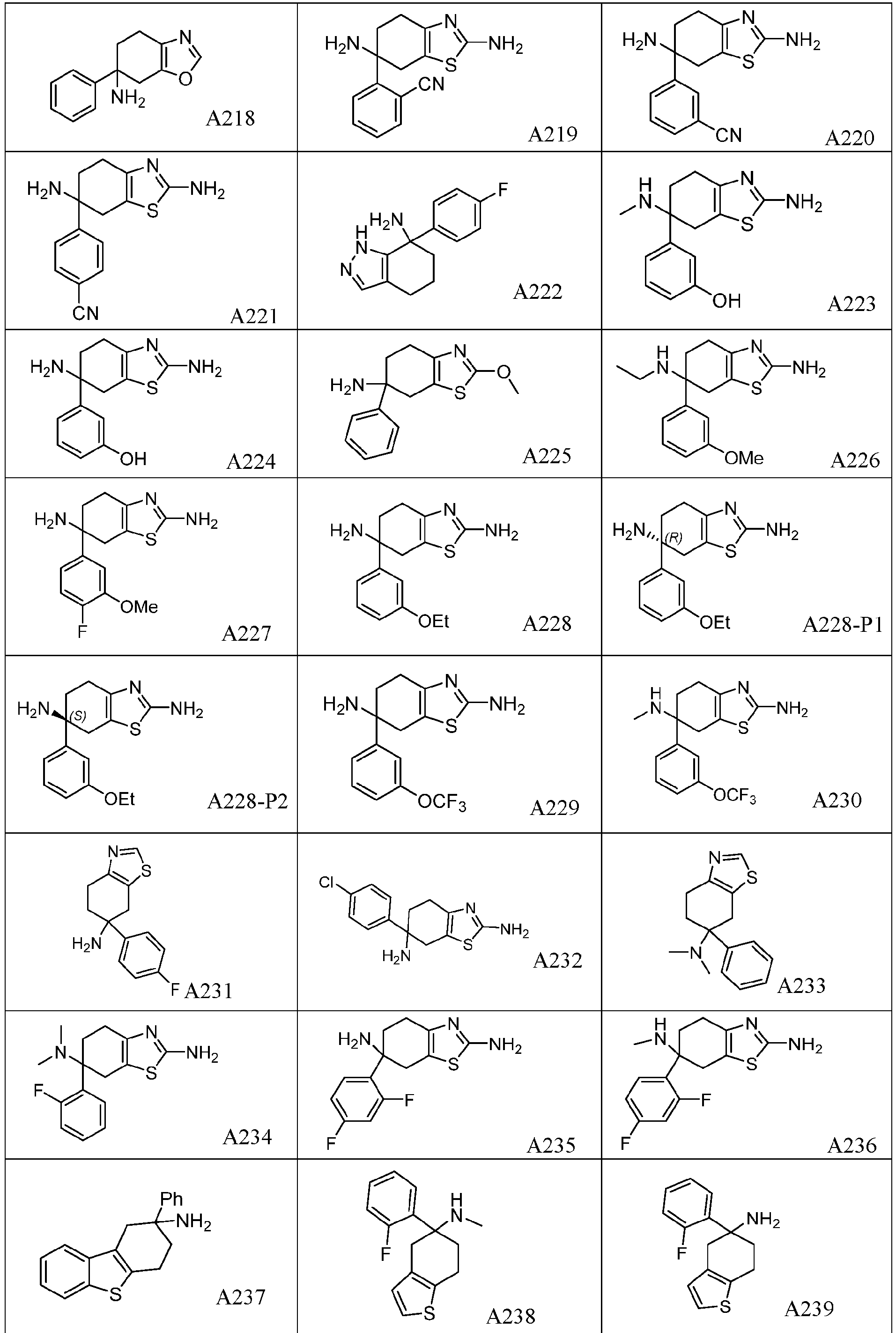

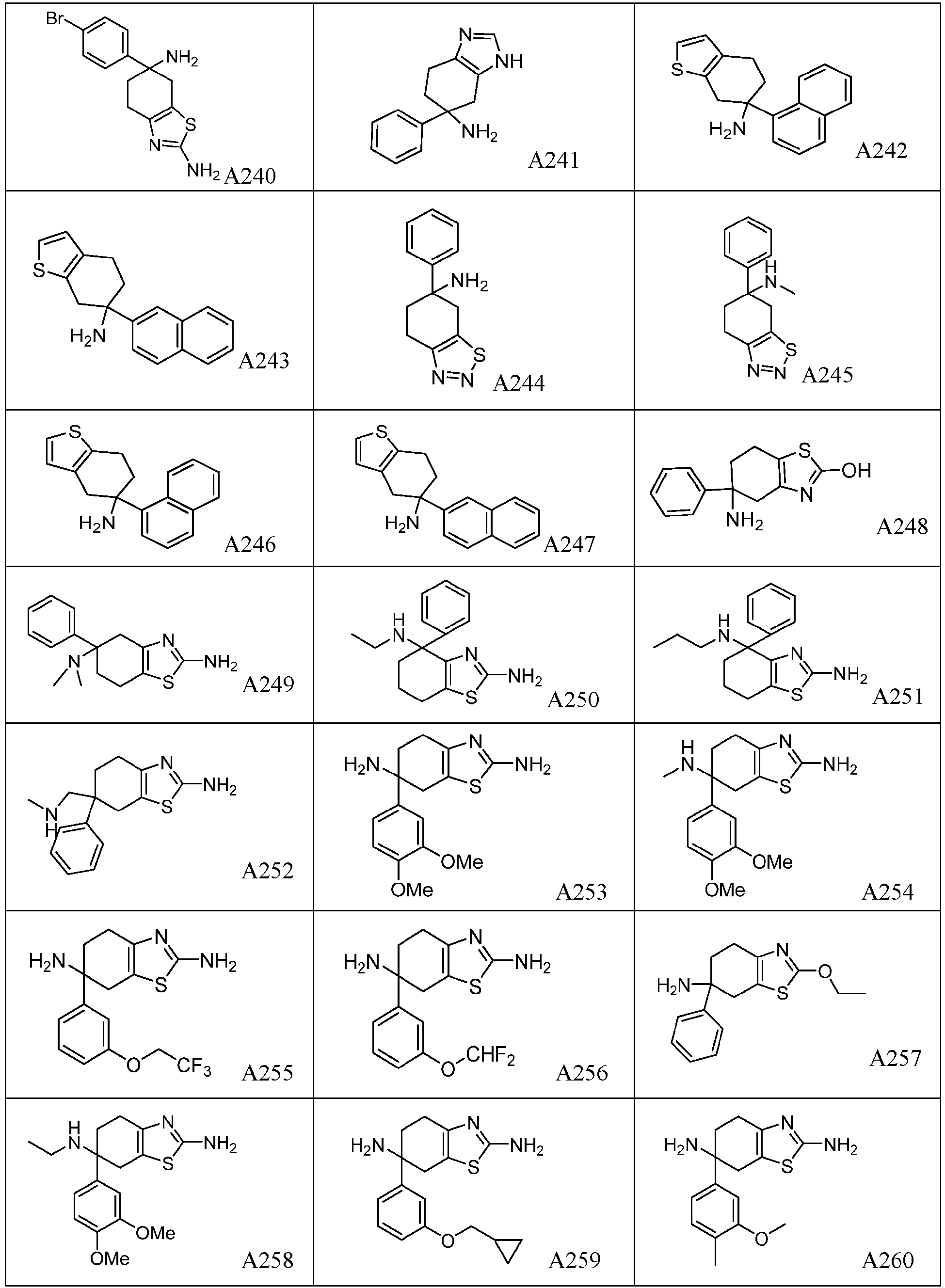

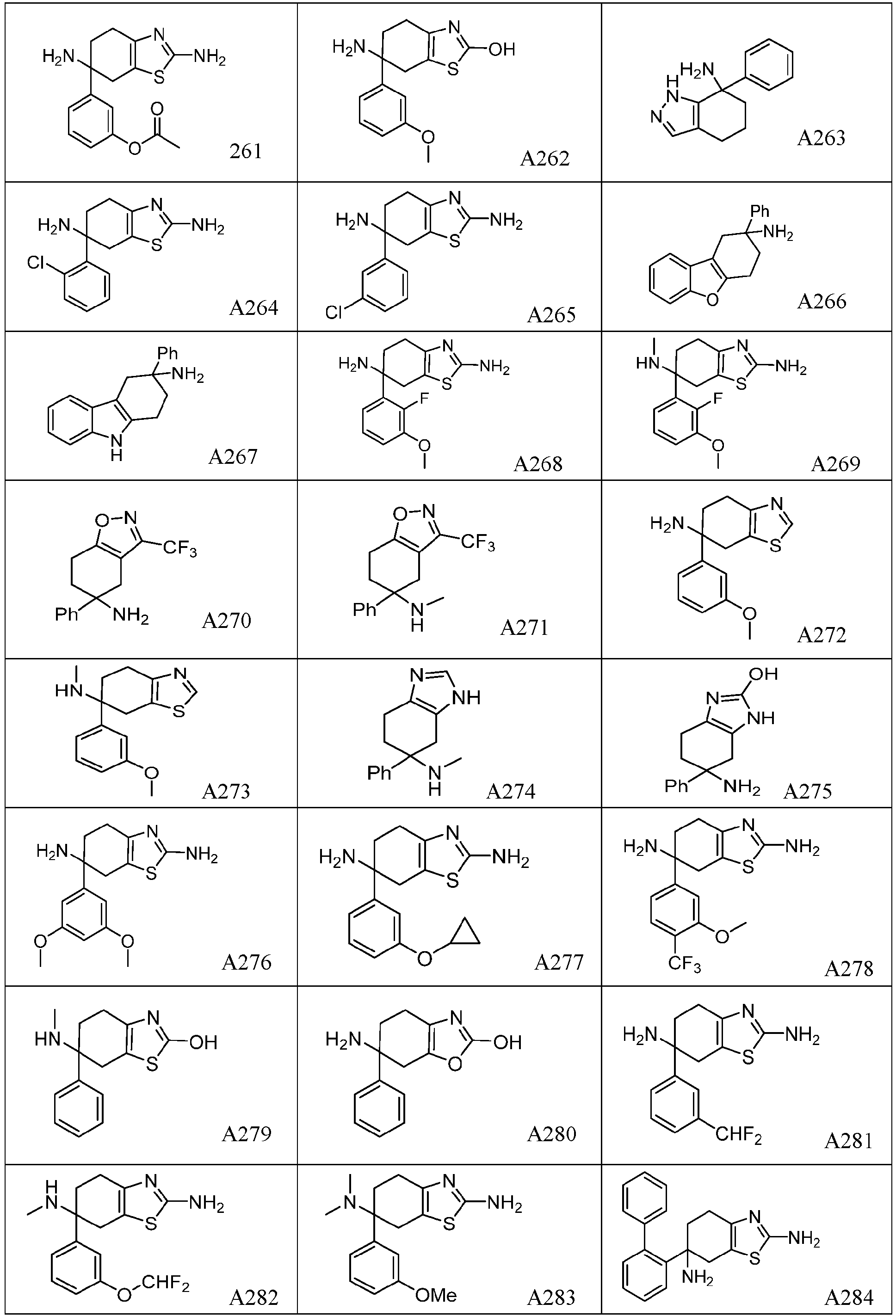

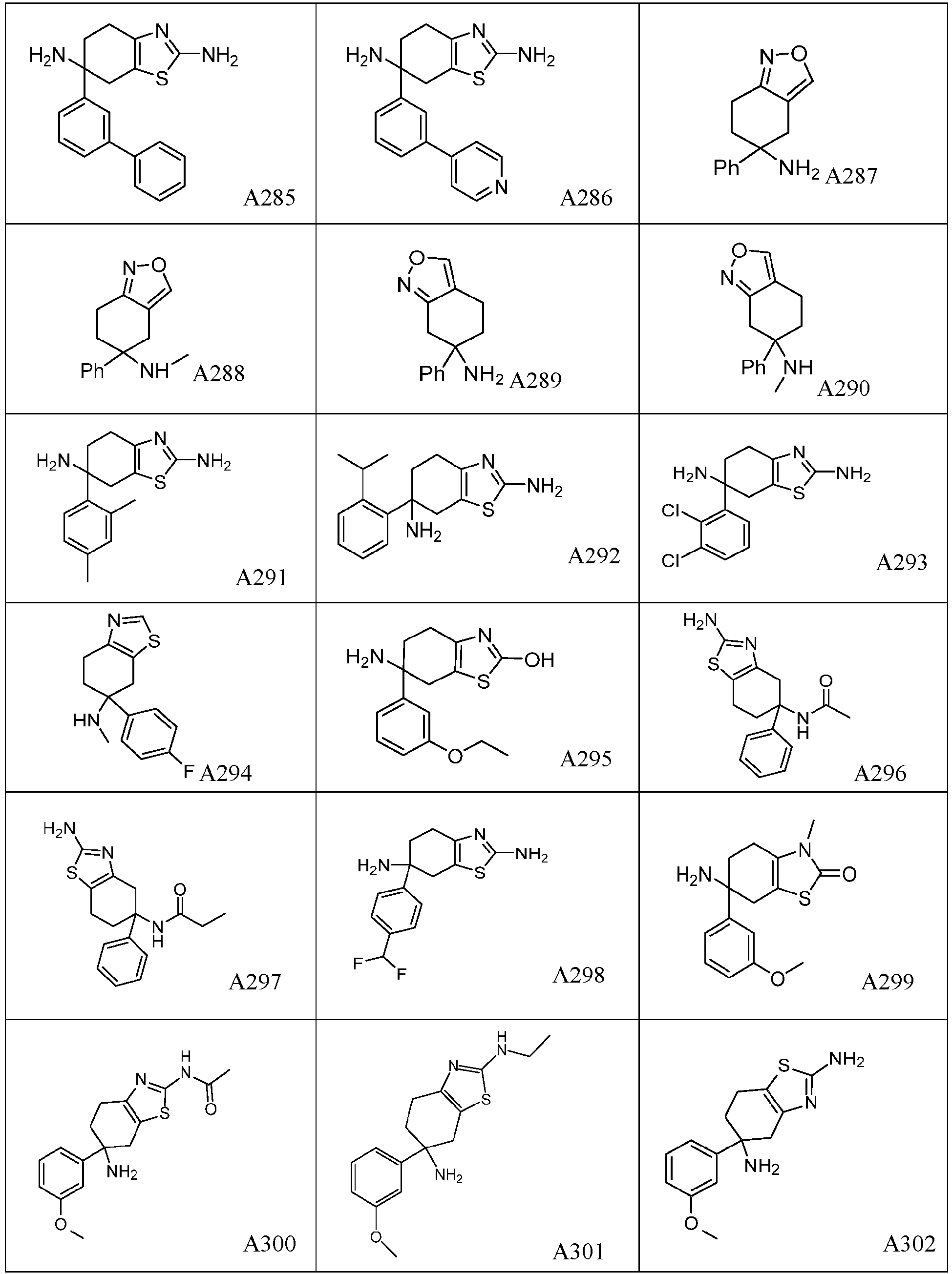

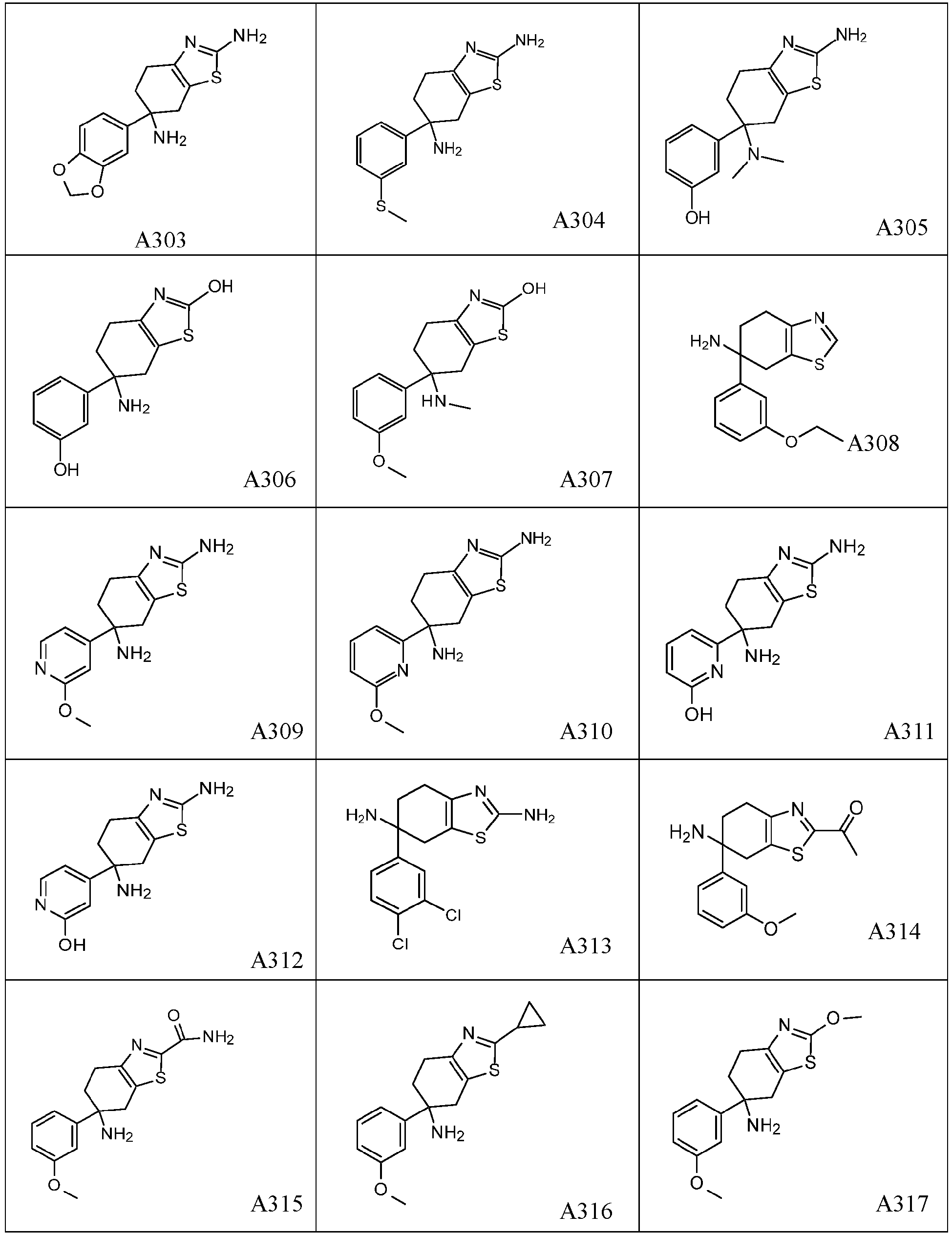

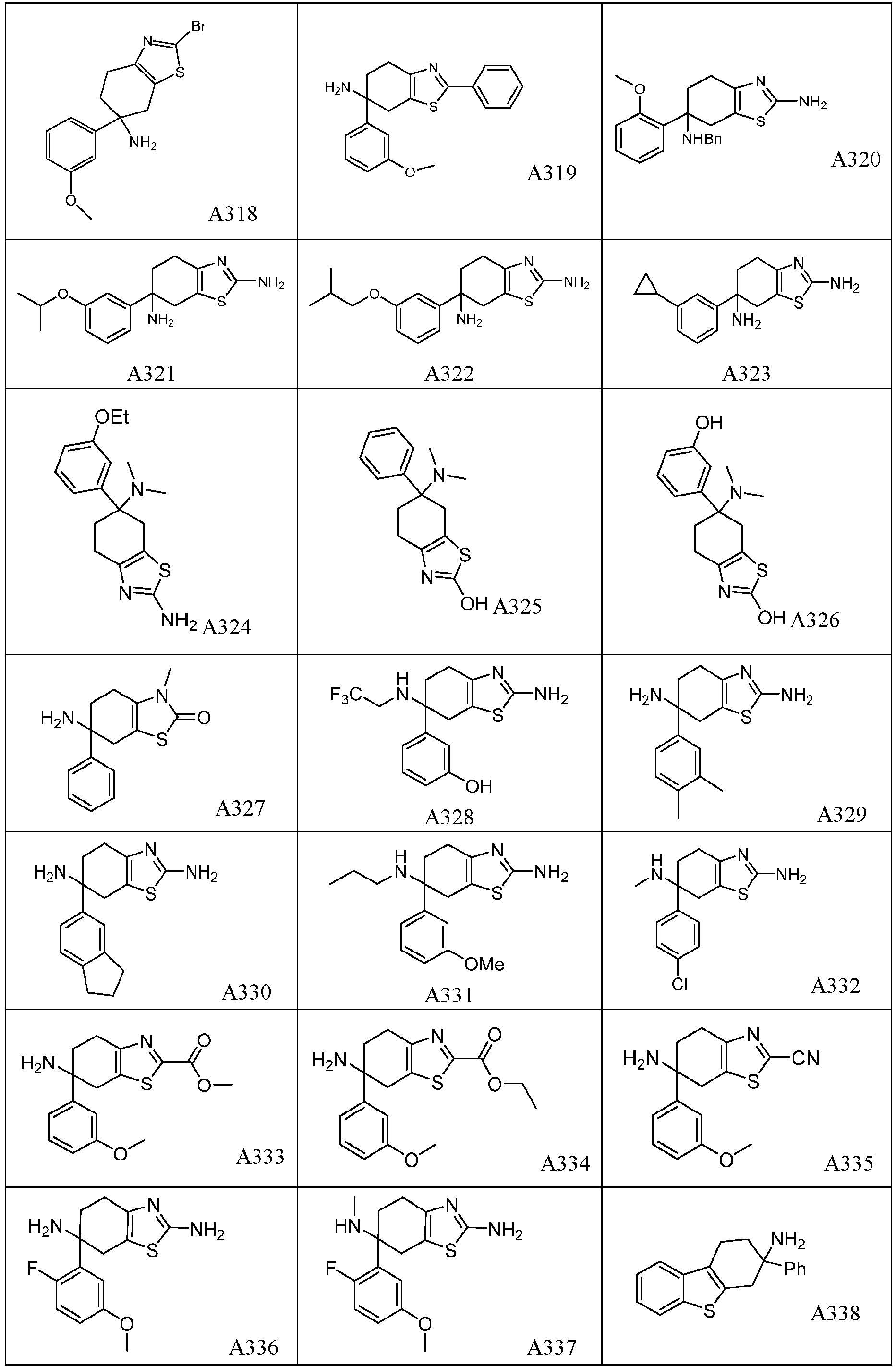

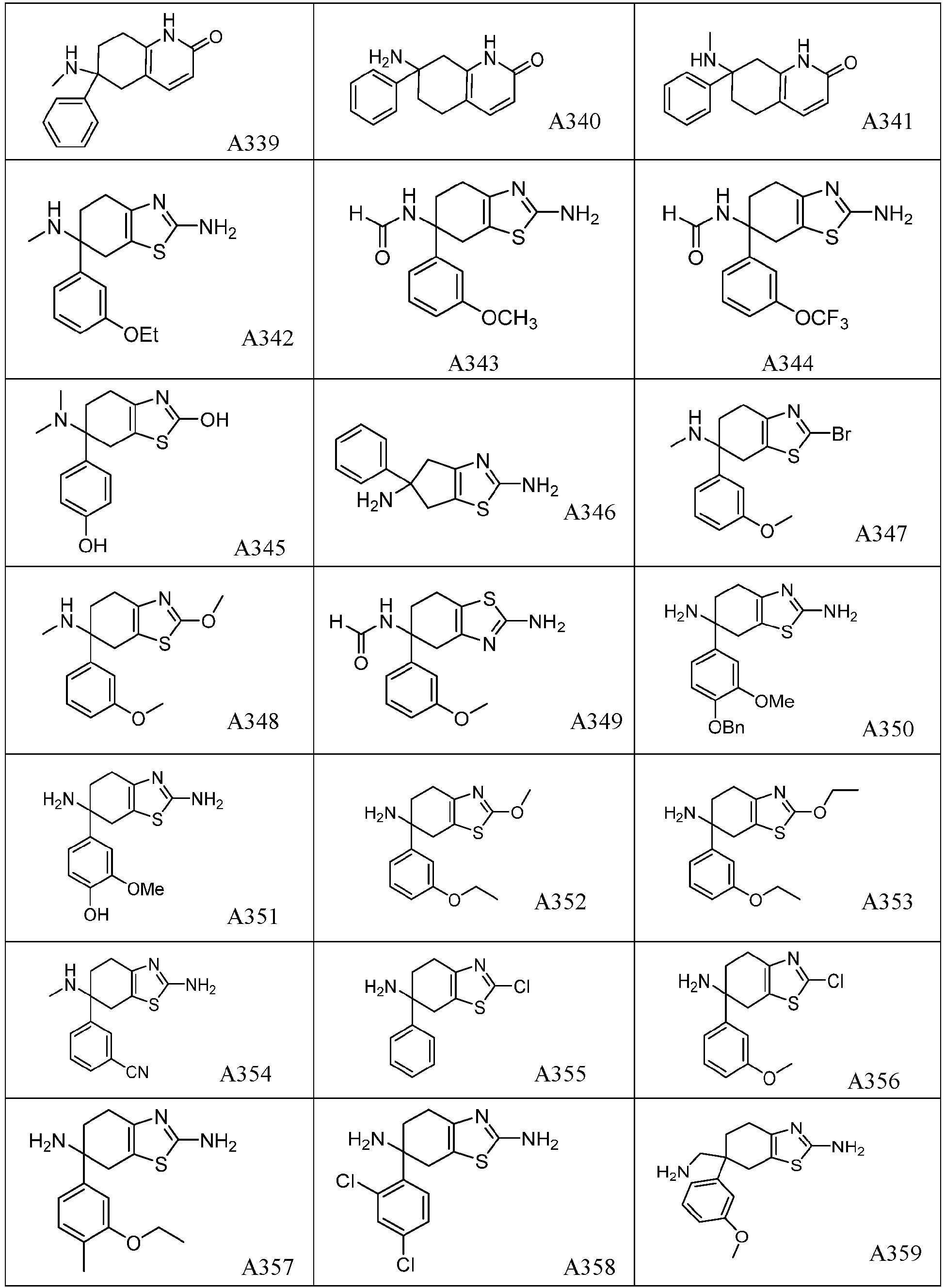

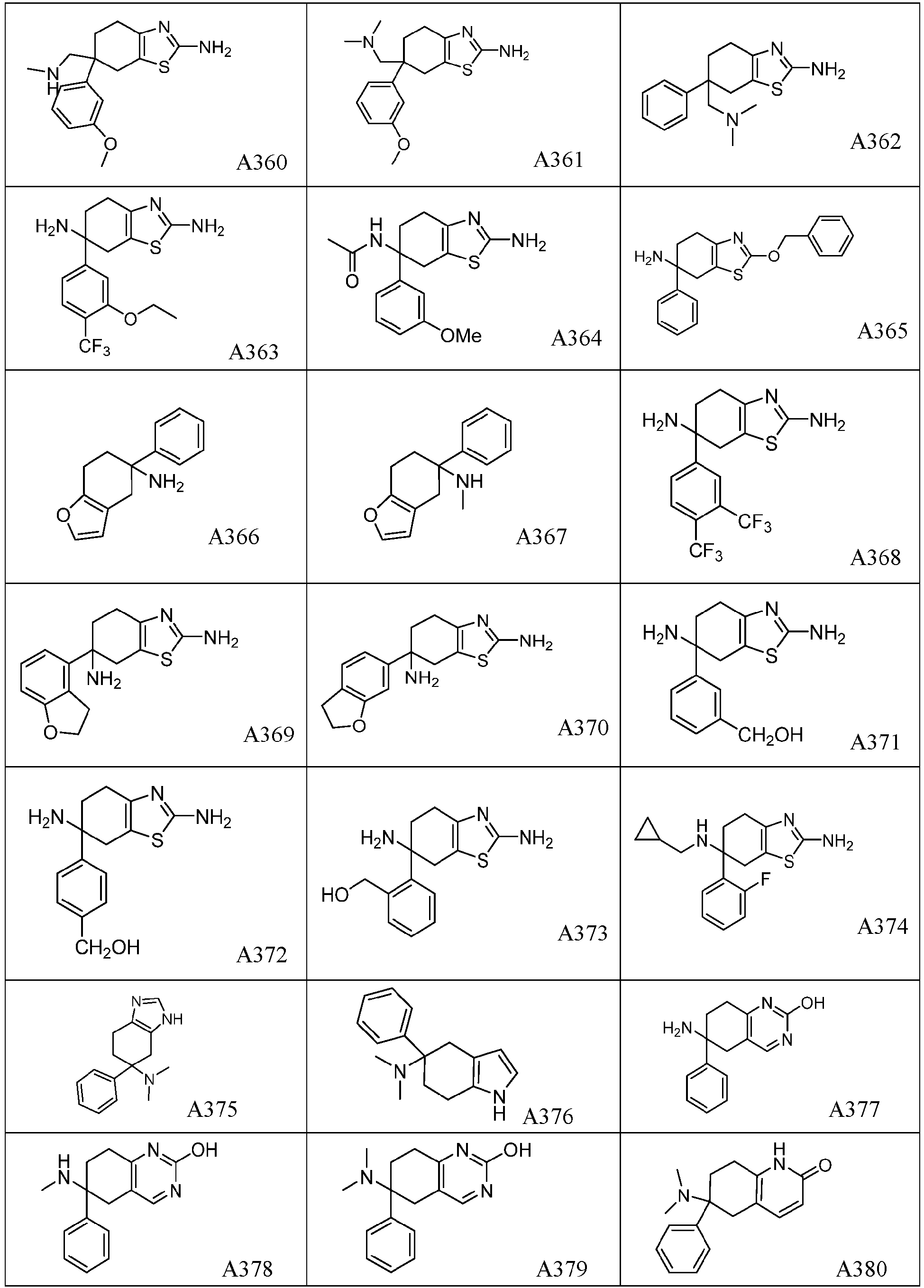

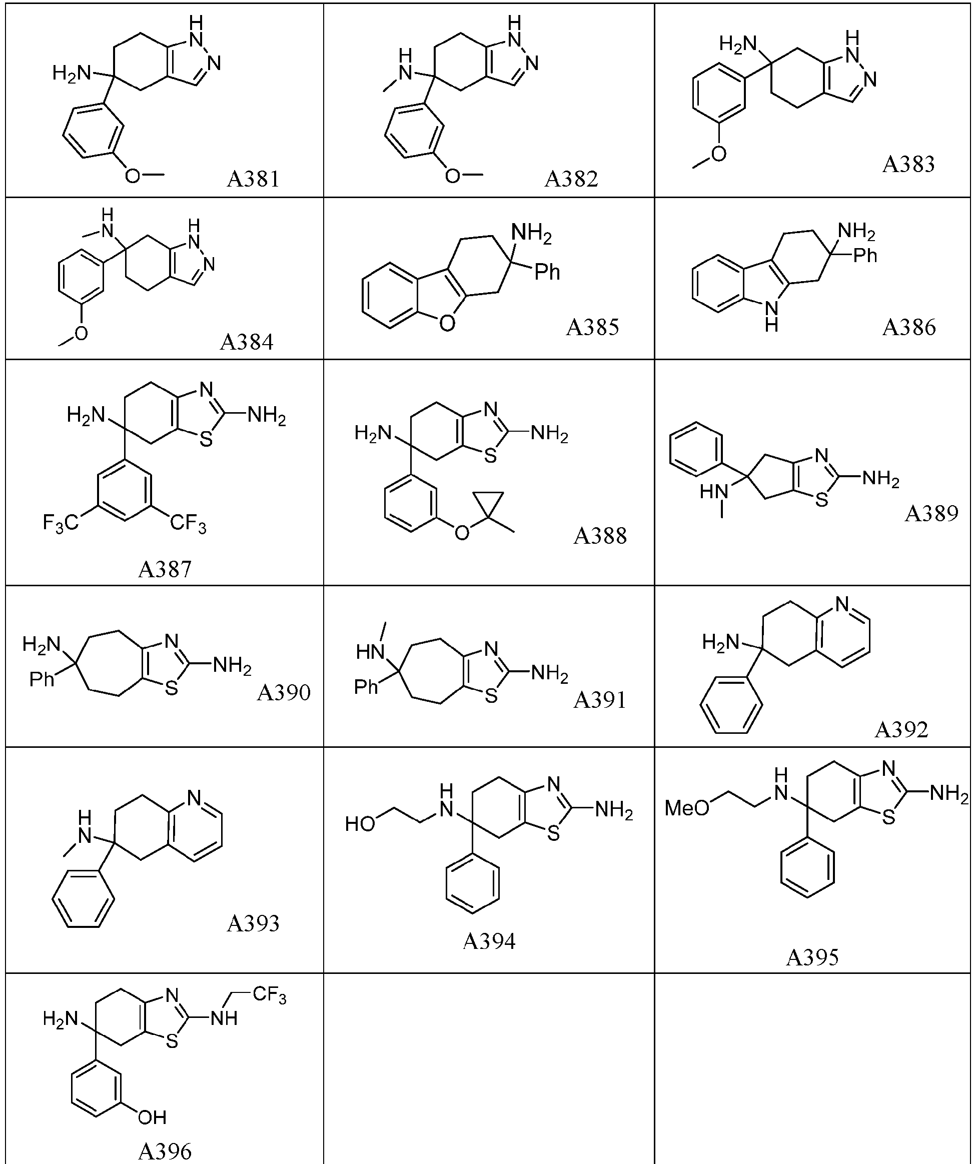

wherein, as conventionally understood in the art, "Bn" denotes benzyl, "Bz" denotes benzoyl, "Me" denotes methyl, "Et" denotes ethyl, and "Ph" denotes phenyl.

According to a second aspect of the present disclosure, provided is a method for preparing the aryl-containing amine compound of formula (I), wherein the method may be carried out through one or a combination of the following methods 1-3:
method 1:
   as shown in reaction formula 1, the method comprises the following steps:
      a) subjecting a compound of formula (II) and a compound of formula (III) to a condensation reaction to generate a compound of formula (IV);
      b) subjecting the compound of formula (IV) and a compound of formula (V) to a nucleophilic addition reaction to generate a compound of formula (I-a); 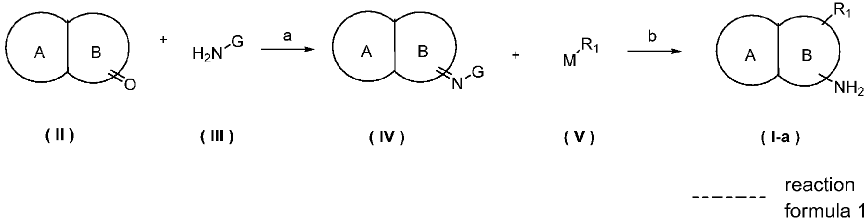
   wherein ring A, ring B, and R₁ are as defined and preferred above;
   G represents a leaving group, such as C1-C6 alkylsulfinyl, benzenesulfinyl, naphthalenesulfinyl, and benzyl, wherein the C1-C6 alkylsulfinyl, benzenesulfinyl, naphthalenesulfinyl, and benzyl described above are optionally further substituted with one or more groups selected from halogen, C1-C6 alkyl, nitro, hydroxy, amino, C1-C6 alkanoyl, C1-C6 alkoxy, and phenyl; preferably, G is C1-C4 alkylsulfinyl, benzenesulfinyl, naphthalenesulfinyl, or benzyl, wherein the C1-C4 alkylsulfinyl, benzenesulfinyl, naphthalenesulfinyl, and benzyl described above are optionally further substituted with one or more groups selected from halogen, C1-C4 alkyl, nitro, hydroxy, amino, C1-C4 alkanoyl, C1-C4 alkoxy, and phenyl; more preferably, G is *tert*-butylsulfinyl, *p*-toluenesulfinyl, trifluoromethylsulfinyl, *p*-bromosulfinyl, benzyl, *p*-methoxybenzyl, or triphenylmethyl;
   M represents a leaving group, such as a metal element, halogen, a metal complex, borane, silane, and a diazonium salt, preferably -MgBr, -MgCl, and -Li;
   step a) may be carried out in the presence or absence of an acid and in a solvent, and the solvent may be selected from: ethers such as dioxane, tetrahydrofuran, diethyl ether, methyl *tert-butyl* ether, diisopropyl ether, diethylene glycol dimethyl ether, and ethylene glycol dimethyl ether; aromatics such as benzene, toluene, nitrobenzene, and chlorobenzene; alcohols such as methanol, ethanol, isopropanol, butanol, *tert*-butanol, and ethylene glycol; halogenated hydrocarbons such as chloroform, dichloromethane, dichloroethane, and carbon tetrachloride; esters such as ethyl acetate, ethyl formate, methyl acetate, and isopropyl acetate; other solvents such as dimethyl sulfoxide and acetonitrile; or a mixture of the above solvents;
   the acid may be selected from organic acids, inorganic acids, or Lewis acids. The inorganic acids may include hydrochloric acid, hydrobromic acid, hydroiodic acid, sulfuric acid, nitric acid, phosphoric acid, and perchloric acid; the organic acids may include formic acid, acetic acid, trifluoroacetic acid, propionic acid, pyruvic acid, glycolic acid, oxalic acid, malonic acid, succinic acid, glutaric acid, maleic acid, fumaric acid, lactic acid, malic acid, citric acid, tartaric acid, picric acid, glutamic acid, benzoic acid, methanesulfonic acid, ethanesulfonic acid, benzenesulfonic acid, *p*-toluenesulfonic acid, salicylic acid, ascorbic acid, camphoric acid, or camphorsulfonic acid; the Lewis acids include aluminum chloride, ferric chloride, boron trifluoride, ethyl titanate, and the like; these acids may be used alone or in combination of two or more;
   step b) may be carried out in a solvent, and the solvent may be selected from: ethers such as dioxane, tetrahydrofuran, diethyl ether, methyl *tert*-butyl ether, diisopropyl ether, diethylene glycol dimethyl ether, and ethylene glycol dimethyl ether; aromatics such as benzene, toluene, nitrobenzene, and chlorobenzene; ketones such as acetone, methyl ethyl ketone, and 4-methyl-2-pentanone; amides such as *N,N*-dimethylformamide, *N,N*-dimethylacetamide, and 1-methyl-2-pyrrolidinone; halogenated hydrocarbons such as chloroform, dichloromethane, dichloroethane, and carbon tetrachloride; esters such as ethyl acetate, ethyl formate, methyl acetate, and isopropyl acetate; other solvents such as dimethyl sulfoxide and acetonitrile; or a mixture of the above solvents;
method 2 is as shown in reaction formula 2 and comprises the following steps:
   c) subjecting a compound of formula (II) and a compound of formula (V) to a nucleophilic addition reaction to generate a compound of formula (VI);
   d) subjecting the compound of formula (VI) and azide to a substitution reaction to generate a compound of formula (VII);
   e) subjecting the compound of formula (VII) to a reduction reaction to generate a compound of formula (I-a); 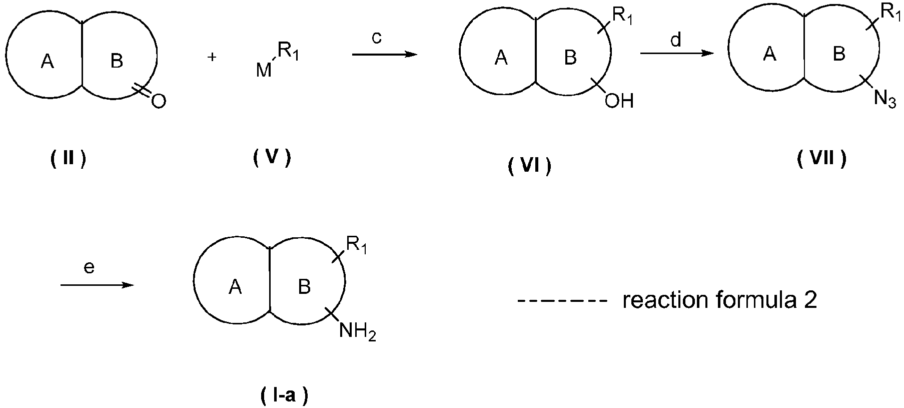
   wherein ring A, ring B, and R₁ are as defined and preferred above;
   M represents a leaving group, such as a metal element, halogen, a metal complex, borane, silane, and a diazonium salt, preferably -MgBr, -MgCl, and -Li;
   step c) may be carried out in a solvent, and the solvent may be selected from: ethers such as dioxane, tetrahydrofuran, diethyl ether, methyl *tert-butyl* ether, diisopropyl ether, diethylene glycol dimethyl ether, and ethylene glycol dimethyl ether; aromatics such as benzene, toluene, nitrobenzene, and chlorobenzene; amides such as *N,N*-dimethylformamide, *N,N*-dimethylacetamide, and 1-methyl-2-pyrrolidinone; halogenated hydrocarbons such as chloroform, dichloromethane, dichloroethane, and carbon tetrachloride; esters such as ethyl acetate, ethyl formate, methyl acetate, and isopropyl acetate; other solvents such as dimethyl sulfoxide and acetonitrile; or a mixture of the above solvents;
   step d) may be carried out under acid-catalyzed conditions in a solvent, wherein the acid may include hydrochloric acid, hydrobromic acid, hydroiodic acid, sulfuric acid, nitric acid, phosphoric acid, trifluoroacetic acid, acetic acid, and the like; the azide includes sodium azide, potassium azide, trimethylsilyl azide, and the like; the reaction solvent may be selected from water; ethers such as dioxane, tetrahydrofuran, diethyl ether, methyl *tert-butyl* ether, diisopropyl ether, diethylene glycol dimethyl ether, and ethylene glycol dimethyl ether; aromatics such as benzene, toluene, nitrobenzene, and chlorobenzene; ketones such as acetone, methyl ethyl ketone, and 4-methyl-2-pentanone; amides such as *N,N*-dimethylformamide, N,N-dimethylacetamide, and 1-methyl-2-pyrrolidinone; halogenated hydrocarbons such as chloroform, dichloromethane, dichloroethane, and carbon tetrachloride; esters such as ethyl acetate, ethyl formate, methyl acetate, and isopropyl acetate; other solvents such as dimethyl sulfoxide, acetonitrile, acetic acid, formic acid, and pivalic acid; or a mixture of the above solvents;
   step e) may be carried out in the presence of a reducing agent system in a solvent, wherein: the reducing agent system includes hydrogenation catalyzed by a Pd/C catalyst, hydrogenation catalyzed by a Pd/BaSO₄ catalyst, hydrogenation catalyzed by a PtO₂ catalyst, hydrogenation catalyzed by a Raney Ni catalyst, a PPh₃-THF-H₂O system, LAH, and the like; the solvent required is selected from water; ethers such as dioxane, tetrahydrofuran, diethyl ether, methyl *tert*-butyl ether, diisopropyl ether, diethylene glycol dimethyl ether, and ethylene glycol dimethyl ether; aromatic hydrocarbons such as benzene, toluene, nitrobenzene, and chlorobenzene; alcohols such as methanol, ethanol, isopropanol, butanol, *tert*-butanol, and ethylene glycol; or a mixture of the above solvents; and
method 3:
   subjecting the compound of formula (I-a) obtained by methods 1-2 to a functional group transformation of amino to give other aryl-containing amine compounds of formula (I), such as via alkylation or acylation reactions;
   the alkylation reaction may be carried out in the presence of an alkylating agent, including but not limited to methyl iodide, ethyl iodide, 2-bromopropane, bromocyclopropane, *tert*-butyl bromide, and the like;
   the acylation reaction may be carried out in the presence of an acylating agent, including but not limited to acetic anhydride, acetyl chloride, benzoyl chloride, acetic formic anhydride, propionic anhydride, cyclopropanecarboxylic anhydride, and the like.

The compounds of formulas (II), (III), (IV), (V), (VI), and (VII) are either commercially available, prepared by known technical methods, or prepared analogously to similar compounds.

The starting compounds used in the reaction formulas described above may be in the form of suitable salts, including: alkali metal salts and alkaline earth metal salts such as sodium, potassium, calcium, and magnesium salts; organic base salts such as pyridinium and triethylamine salts; inorganic acid salts such as hydrochloride, hydrobromide, hydroiodide, sulfate, nitrate, and phosphate; organic acid salts such as formate, acetate, propionate, glycolate, oxalate, malonate, succinate, fumarate, maleate, lactate, malate, citrate, tartrate, picrate, glutamate, methanesulfonate, and benzenesulfonate.

Furthermore, the starting compounds used in the reaction formulas described above may include solvate forms, such as hydrates and alcoholates.

The target compounds obtained according to the reaction formulas may be isolated and purified from the reaction mixture by the following method, such as after cooling, isolating a crude product from the reaction mixture by methods such as filtration, extraction, or concentration, and then subjecting the crude product to purification by conventional methods such as column chromatography, slurrying, or recrystallization.

According to a third aspect of the present disclosure, provided is a pharmaceutical composition comprising a therapeutically effective amount of one or more selected from the compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof, and optionally one or more pharmaceutically acceptable carriers, diluents, or excipients.

The compounds of the present disclosure exhibit multi-target effects on an NMDA receptor, a monoamine transporter, and/or a sigma receptor, and can be used for treating a central nervous system disease, particularly depressive disorder, manic-depressive psychosis, schizophrenia, anxiety disorder, phobia, autism, Alzheimer's disease, bipolar affective disorder, hysteria, obsessive-compulsive disorder, hyperactivity disorder, epilepsy, and the like.

Therefore, according to a fourth aspect of the present disclosure, provided is use of one or more selected from the compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof, or the pharmaceutical composition described above, in preparing a medicament for regulating activity of an NMDA receptor and/or a monoamine transporter and/or a sigma receptor, specifically, in preparing an NMDA receptor antagonist, in preparing a monoamine transporter inhibitor, and in preparing a sigma receptor agonist or antagonist.

According to a fifth aspect of the present disclosure, further provided is use of one or more selected from the aryl-containing amine compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof described above, or the pharmaceutical composition described above, in preparing a medicament for preventing and/or treating a disease associated with an NMDA receptor and/or a monoamine transporter and/or a sigma receptor, particularly a central nervous system disease.

The central nervous system disease is selected from: cerebral ischemia; stroke; cerebral infarction; traumatic brain injury; anti-NMDA receptor encephalitis; epilepsy; amyotrophic lateral sclerosis; schizophrenia; uncontrollable, intractable, or chronic schizophrenia; affective disorder; mental disorder; mood disorder; bipolar I disorder; bipolar II disorder; depressive disorder; endogenous depressive disorder; major depressive disorder; uncontrollable depressive disorder; dysthymic disorder; cyclothymic disorder; panic attack; panic disorder; social phobia; obsessive-compulsive disorder; impulse control disorder; post-traumatic stress disorder; anxiety disorder; acute stress disorder; hysteria; anorexia nervosa; sleep disorder; adjustment disorder; cognitive disorder; autism; neuropathic pain; mania; Parkinson's disease; Huntington's disease; Alzheimer's disease; various dementias; memory disorder; hyperactivity disorder; attention deficit/hyperactivity disorder; tic disorder; other neurological events or neurodegeneration caused by NMDA receptor activation.

In some embodiments, the neuropathic pain includes diabetic peripheral neuropathy, postherpetic neuralgia, complex regional pain syndrome, peripheral neuropathy, chemotherapy-induced neuropathic pain, cancer-related neuropathic pain, neuropathic low back pain, HIV-associated neuropathic pain, trigeminal neuralgia, and central post-stroke pain.

In some preferred embodiments, the central nervous system disease is selected from: bipolar I disorder; bipolar II disorder; depressive disorder; endogenous depressive disorder; major depressive disorder; uncontrollable depressive disorder; dysthymic disorder; cyclothymic disorder; panic attack; panic disorder; social phobia; obsessive-compulsive disorder; impulse control disorder; post-traumatic stress disorder; anxiety disorder; acute stress disorder; Parkinson's disease; diabetic peripheral neuropathy; postherpetic neuralgia; complex regional pain syndrome.

According to a sixth aspect of the present disclosure, further provided is a method for treating and/or preventing a disease associated with an NMDA receptor and/or a monoamine transporter and/or a sigma receptor, particularly a central nervous system disease, which comprises administering the aryl-containing amine compound of formula (I), the stereoisomer thereof, or the pharmaceutically acceptable salt thereof described above to a human or animal.

According to a sixth aspect of the present disclosure, further provided is a method for preparing the pharmaceutical composition described above, which comprises mixing the aryl-containing amine compound of formula (I), the stereoisomer thereof, or the pharmaceutically acceptable salt thereof described above with a pharmaceutically acceptable carrier.

The pharmaceutical composition of the present disclosure may be selected from various pharmaceutical formulation forms according to the therapeutic purpose, including but not limited to: tablets, pills, capsules, granules, suspensions, solutions, creams, ointments, powders, suppositories, aerosols, injections (e.g., fat-soluble or oil-soluble injections), and the like.

"Therapeutically effective amount" of the compound of the present disclosure refers to an amount of the compound of the present disclosure that can elicit a biological or medical response in an individual, or ameliorate symptoms, retard or delay disease progression, prevent a disease, or the like. The "therapeutically effective amount" may be determined by a physician or veterinarian and will vary depending on factors including the compound, the disease state being treated, the severity of the disease being treated, the age and health status of the individual, the route and form of administration, the judgment of the attending physician or veterinarian, and the like.

As used herein, "individual" refers to an animal. Preferably, the animal is a mammal. The individual also refers to, for example, a primate (e.g., human), cow, sheep, goat, horse, dog, cat, rabbit, rat, mouse, fish, bird, and the like. In one preferred embodiment, the individual is a human.

As used herein, "inhibit", "inhibiting", or "inhibition" refers to the alleviation or suppression of a specific illness, symptom, disorder, or disease, or a significant reduction in biological activity or baseline activity of a process.

As used herein, in one embodiment, the term "treat", "treating", or "treatment" of any disease or disorder refers to ameliorating the disease or disorder (i.e., arresting or slowing the progression of the disease or at least one of its clinical symptoms). In another embodiment, "treat", "treating", or "treatment" refers to ameliorating at least one physical parameter that may not be perceived by the patient. In another embodiment, "treat", "treating", or "treatment" refers to regulating a disease or disorder, either physically (e.g., stabilizing a perceptible symptom) or physiologically (e.g., stabilizing a physical parameter), or both.

As used herein, "prevent", "preventing", or "prevention" refers to administering one or more pharmaceutical substances, particularly the compounds of the present disclosure and/or pharmaceutically acceptable salts thereof, to an individual predisposed to developing the disease, to prevent the occurrence of the disease in the individual.

### Beneficial Effects

The present disclosure provides a brand new NMDAR antagonist, which belongs to channel pore blockers, and can inhibit channel opening caused by excessive activation of NMDA under pathological conditions thereby preventing excessive Ca²⁺ influx, without affecting the normal functions of NMDAR. The NMDAR antagonist described in the present disclosure is a reversible NMDAR antagonist that dissociates very rapidly after binding, without affecting the normal functions of NMDA receptors.

The compounds of the present disclosure have the following beneficial effects:
1) The compounds of the present disclosure have properties that modulate NMDA receptor activity, and thus can be used for treating and/or preventing a disease associated with an NMDA receptor.
2) *In vitro* study results show that the compounds of the present disclosure dissociate from NMDAR much faster than ketamine, indicating that the compounds of the present disclosure have lower psychotomimetic adverse effect risks and a larger safety window.
3) The compounds of the present disclosure have good inhibitory effects on a monoamine transporter (5-HT transporter and/or norepinephrine transporter and/or dopamine transporter), and can alleviate comorbidities such as anxiety or depression in anxiety disorder, depressive disorder, or other central nervous system diseases.
4) The compounds of the present disclosure have good binding activity to a sigma receptor and can exert neuroprotective effects, modulate cognitive functions, ameliorate drug addiction, and improve motor dysfunction through the regulation of a sigma receptor within the central nervous system.
5) The compounds of the present disclosure have the characteristics of high oral bioavailability, low drug effect dosage, and small toxic and side effects. They are effective for treating diseases related to the central nervous system, particularly for central nervous system events caused by NMDA receptor activation.

In summary, compared with the existing NMDAR antagonists, the compounds of the present disclosure have the advantages of the multi-target effect, lower drug effect dosage, less toxic and side effects, better safety and tolerability, and the like, have good general druggability, and have good clinical application prospects.

### DETAILED DESCRIPTION

The following examples and pharmacological examples further illustrate the present disclosure, but do not limit the scope of the present disclosure.

The starting materials, reagents, methods, and the like used in examples and pharmacological examples were those conventional in the art, unless otherwise specified.

### Abbreviation

| | | | |
|---|---|---|---|
| DMAP | 4-Dimethylaminopyridine | DIPEA | *N,N*-Diisopropylethylamine |
| THF | Tetrahydrofuran | DBU | 1,8-Diazabicycloundec-7-ene |
| DCM | Dichloromethane | DMA | Dimethylacetamide |
| TFA | Trifluoroacetic acid | TBDPSCl | *tert*-Butyldiphenylchlorosilane |
| BMS | Borane dimethyl sulfide complex | NMP | *N*-Methylpyrrolidone |
| EA | Ethyl acetate | DIAD | Diisopropyl azodicarboxylate |
| MTBE | Methyl *tert*-butyl ether | PTSA | *p*-Toluenesulfonic acid |
| TMSCl | Trimethylchlorosilane | Oxone | Potassium hydrogen monopersulfate |

The following examples are related to chiral separation operations. The chiral separation conditions are as follows:
chromatographic column: DAICEL CHIRALCEL OD-H 4.6 × 250 mm, 5 µm; mobile phase: n-hexane/0.1% diethylamine in isopropanol = 50/50; flow rate: 0.8 mL/min; column temperature: 35 °C; detection wavelength: 270 nm.

For chiral separation of different compounds, separation can be achieved by adjusting the mobile phase ratio or flow rate based on the conditions described above.

### Example

### Example 1 Preparation of 6-phenyl-4,5,6,7-tetrahydrobenzothiazole-2,6-diamine (Compound A1)

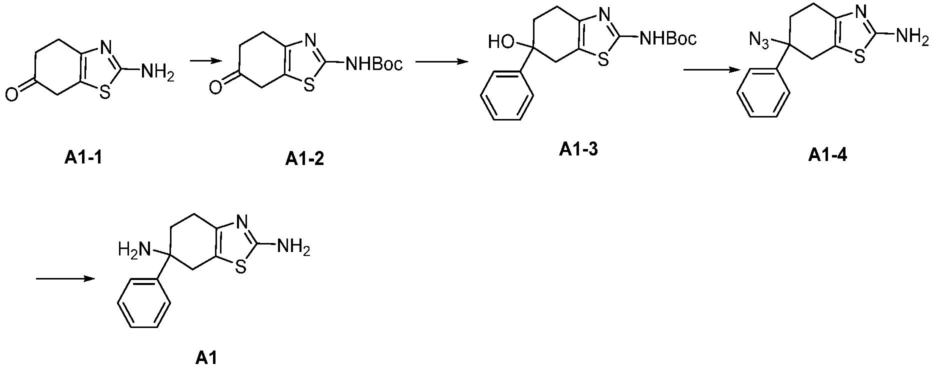

### Step 1:

Compound **A1-1** (765 mg) was dissolved in dichloromethane (20 mL), and DMAP (444 mg) and di-*tert*-butyl dicarbonate (1.5 g) were added. The mixture was reacted at room temperature overnight, and the reaction solution was concentrated and subjected to column chromatography to give compound **A1-2** (250 mg) as an off-white solid.

### Step 2:

Anhydrous THF (5 mL) and phenylmagnesium chloride (4.7 mL) were added to a 50 mL reaction flask, and under nitrogen atmosphere, the mixture was cooled to 0-5 °C. A solution of compound **A1-2** (500 mg) in THF (5 mL) was added dropwise, and the mixture was reacted at room temperature overnight. The reaction was quenched with a saturated aqueous ammonium chloride solution. The reaction solution was extracted with ethyl acetate, dried, concentrated, and purified by column chromatography to give compound **A1-3** (about 385 mg).

### Step 3:

Compound **A1-3** (500 mg) was dissolved in DCM (10 mL). Sodium azide (470 mg, 5 eq) was added, and TFA (1.32 g, 8 eq) was added dropwise under an ice bath. After the mixture was reacted for 30 min under the ice bath, the mixture was transferred to room temperature and left to stand overnight in the dark. Ice water was added to the reaction solution. The pH was adjusted to 9 with ammonia water. The mixture was transferred to a separating funnel and extracted with DCM. The organic phase was washed with a saturated aqueous sodium chloride solution, dried over anhydrous sodium sulfate, and concentrated to give compound **A1-4** (350 mg) as a brown solid.

### Step 4:

Compound **A1-4** (1.0 g) was dissolved in methanol (25 mL), and palladium on carbon (10%, 100 mg) was added. Hydrogen was introduced, and the mixture was reacted at room temperature overnight. The reaction solution was filtered and concentrated, and the residue was slurried with dichloromethane to give a solid (434 mg). The mother liquor was concentrated and purified by column chromatography to give a solid (172 mg). In total, about 606 mg of compound **A1** was obtained. LRMS-ESI (*m*/*z*): 246.16 [M + H]⁺.

¹H NMR (400 MHz, DMSO-d₆) δ 7.51 (d, *J =* 7.3 Hz, 2H), 7.28 (t, *J =* 7.6 Hz, 2H), 7.18 (t, *J* = 7.3 Hz, 1H), 6.60 (s, 2H), 2.98 (d, *J =* 16.0 Hz, 1H), 2.58 (d, *J =* 16.0 Hz, 1H), 2.49 (m, 1H), 2.10 (m, 2H), 1.91 (br, 2H), 1.79 (m, 1H).

Chiral column separation gave isomers **A1-P1** (R configuration) and **A1-P2** (S configuration):

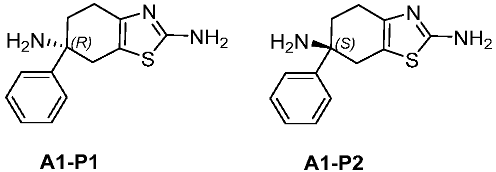

**A1-P1** (R configuration): HPLC purity: > 99%, retention time: 6.534 min. Chiral purity: 100%, retention time: 9.186 min.

**A1-P2** (S configuration): HPLC purity: > 99%, retention time: 6.553 min. Chiral purity: 100%, retention time: 14.432 min.

### Example 2 Preparation of N-(2-amino-6-phenyl-4,5,6,7-tetrahydrobenzothiazol-6-yl)formamide (Compound A2)

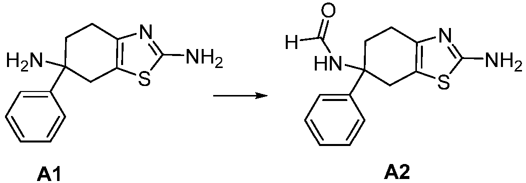

Formic acid (920 mg) and acetic anhydride (2.04 g) were added to a 50 mL reaction flask, and under nitrogen atmosphere, the mixture was reacted at reflux at 60 °C for 2 h and cooled to room temperature. Dichloromethane (40 mL) was added to prepare a 0.5 M acetic formic anhydride solution for later use. Compound A1 (3.8 g) was added to dichloromethane (50 mL), and the 0.5 M acetic formic anhydride solution (35 mL) described above was added dropwise. The mixture was reacted at room temperature for 1 h. The pH was adjusted to 9 with a saturated aqueous sodium bicarbonate solution. The mixture was extracted with dichloromethane, dried over anhydrous sodium sulfate, and filtered, and the filtrate was concentrated and subjected to column chromatography to give compound **A2** (3.8 g) as an off-white solid.

¹H NMR (400 MHz, DMSO-d₆) δ 8.28 (s, 1H), 7.97 (s, 1H), 7.38 (m, 2H), 7.30 (t, *J =* 7.6 Hz, 2H), 7.20 (t, *J =* 7.1 Hz, 1H), 6.65 (s, 2H), 2.99 (dd, *J =* 29.1, 16.3 Hz, 2H), 2.56 (m, 1H), 2.40 (m, 1H), 2.25 (m, 1H), 2.16 (m, 1H). LRMS-ESI (*m*/*z*): 274.16 [M + H]⁺.

### Example 3 Preparation of N⁶-methyl-6-phenyl-4,5,6,7-tetrahydrobenzothiazole-2,6-diamine (Compound A3) and Hydrochloride Thereof

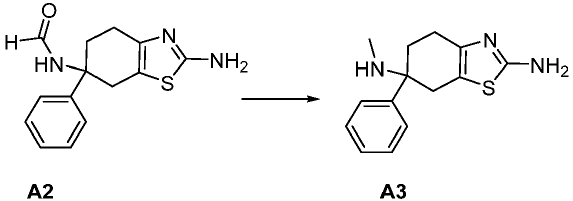

Compound **A2** (3.8 g) was added to anhydrous THF (60 mL), and BMS (136 mL) was added dropwise. The mixture was reacted at 65 °C overnight and cooled to room temperature, and the reaction was quenched by dropwise addition of methanol. The mixture was concentrated to dryness to remove the solvent. Methanol (50 mL) and water (50 mL) were added, and the mixture was reacted at reflux for 2 h, extracted with DCM, dried, concentrated, and purified by column chromatography to give basic **A3** (2.8 g) as an off-white solid. Methanol (30 mL) was added to give a clear solution, and 4 M HCl/EA (3.2 mL) was added dropwise. The mixture was stirred, concentrated to dryness to remove the solvent, slurried with MTBE, and filtered to give compound **A3** hydrochloride (2.9 g).

¹H NMR (400 MHz, DMSO-d₆) δ 10.36 (m, 1H), 9.82 (m, 1H), 9.26 (br, 2H), 7.61 (m, 2H), 7.46 (m, 3H), 3.64 (d, *J =* 16.5 Hz, 1H), 3.27 (d, *J =* 16.5 Hz, 1H), 2.66 (m, 2H), 2.52 (m, 1H), 2.14 (t, *J =* 4.8 Hz, 3H), 1.90 (m, 1H). LRMS-ESI (*m*/*z*): 246.16 [M + H]⁺.

Chiral column separation gave isomers **A3-P1** (R configuration) and **A3-P2** (S configuration):

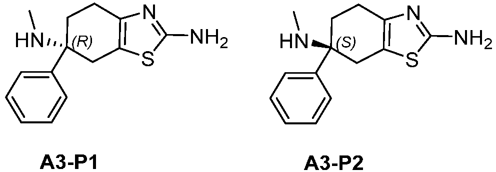

**A3-P1** (R configuration): HPLC purity: > 98%, retention time: 6.914 min. Chiral purity: > 95%, retention time: 6.847 min.

**A3-P2** (S configuration): HPLC purity: > 95%, retention time: 6.924 min. Chiral purity: > 95%, retention time: 6.782 min.

### Example 4 Preparation of N-(2-amino-6-phenyl-4,5,6,7-tetrahydrobenzothiazol-6-yl)acetamide (Compound A4)

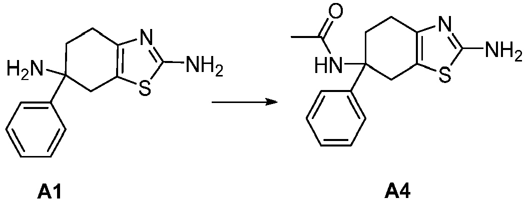

Compound **A1** (400 mg) was added to dichloromethane (20 mL), and acetic anhydride (167 mg, 1.1 eq) was added dropwise. The mixture was reacted at 25 °C overnight. The pH was adjusted to 9 with a saturated aqueous sodium bicarbonate solution. The mixture was extracted with dichloromethane, dried, concentrated, and subjected to column chromatography to give compound **A4** (359 mg, yield: 76.5%) as an off-white solid.

### Example 5 Preparation of N⁶-ethyl-6-phenyl-4,5,6,7-tetrahydrobenzothiazole-2,6-diamine (Compound A5) and Hydrochloride Thereof

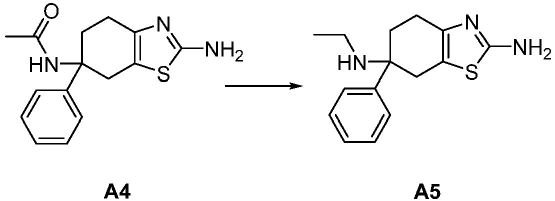

Compound **A4** (320 mg) was added to dichloromethane (20 mL), and TMSCl (500 mg, 5 eq) was added dropwise. The mixture was stirred for 30 min, and lithium aluminum hydride (480 mg, 12 eq) was added. The mixture was reacted at 25 °C for 5 h. The reaction was quenched with a 2 M aqueous sodium hydroxide solution (1 mL). Anhydrous magnesium sulfate and dichloromethane were added, and the mixture was stirred, filtered, concentrated, and subjected to column chromatography to give a white solid (247 mg). Ethyl acetate (3 mL) was added, and a 2 M solution of hydrogen chloride in ethyl acetate (0.5 mL) was added dropwise. The mixture was stirred for 30 min and filtered to give compound **A5** hydrochloride (250 mg).

¹H NMR (400 MHz, DMSO-d₆) δ 9.96 (s, 1H), 9.33 (s, 1H), 7.57 (m, *J =* 7.4 Hz, 2H), 7.44 (m, 3H), 6.81 (s, 2H), 3.61 (d, *J =* 15.8 Hz, 1H), 3.21 (d, *J =* 15.1 Hz, 1H), 2.78 (m, 1H), 2.60 (m, 1H), 2.43 (m, 2H), 2.27 (m, 1H), 1.71 (m, 1H), 1.14 (t, *J* = 6.8 Hz, 3H). LRMS-ESI (*m*/*z*): 274.17 [M + H]⁺.

### Example 6 Preparation of N-(2-amino-6-phenyl-4,5,6,7-tetrahydrobenzothiazol-6-yl)propionamide (Compound A6)

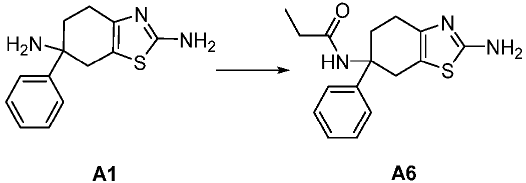

Compound **A1** (400 mg) was added to dichloromethane (20 mL), and propionic anhydride (234 mg, 1.1 eq) was added dropwise. The mixture was reacted at 25 °C overnight. The pH was adjusted to 9 with a saturated aqueous sodium bicarbonate solution. The mixture was extracted with dichloromethane, dried over anhydrous sodium sulfate, concentrated, and subjected to column chromatography to give compound **A6** (274 mg, yield: 55.8%) as an off-white solid.

### Example 7 Preparation of 6-phenyl-N⁶-propyl-4,5,6,7-tetrahydrobenzothiazole-2,6-diamine (Compound A7) and Hydrochloride Thereof

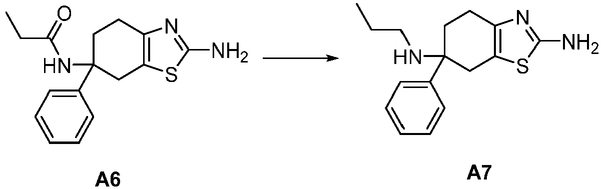

Compound **A6** (274 mg) was added to dichloromethane (20 mL), and TMSCl (990 mg, 10 eq) was added dropwise. The mixture was stirred for 30 min, and lithium aluminum hydride (968 mg, 28 eq) was added. The mixture was reacted at reflux for 24 h. The reaction was quenched with a 2 M aqueous sodium hydroxide solution (1 mL). Anhydrous magnesium sulfate and dichloromethane were added, and the mixture was stirred, filtered, concentrated, and subjected to column chromatography to give a white solid (229 mg). Ethyl acetate (3 mL) was added, and a 2 M solution of hydrogen chloride in ethyl acetate (0.5 mL) was added dropwise. The mixture was stirred for 30 min and filtered to give compound **A7** hydrochloride (240 mg).

LRMS-ESI (*m*/*z*): 288.19 [M + H]⁺. ¹H NMR (400 MHz, DMSO-*d*₆) δ 9.62 (s, 1H), 9.14 (s, 1H), 7.55 (m, 2H), 7.43 (m, 3H), 6.78 (s, 2H), 3.64 (d, *J =* 16.3 Hz, 1H), 3.15 (d, *J =* 16.2 Hz, 1H), 2.71 (m, 1H), 2.65-2.31 (m, 3H), 2.19 (m, 1H), 1.73 (m, 1H), 1.56 (m, 2H), 0.80 (t, *J =* 7.4 Hz, 3H).

### Example 8 Preparation of 6-(2-fluorophenyl)-4,5,6,7-tetrahydrobenzothiazole-2,6-diamine (Compound A9)

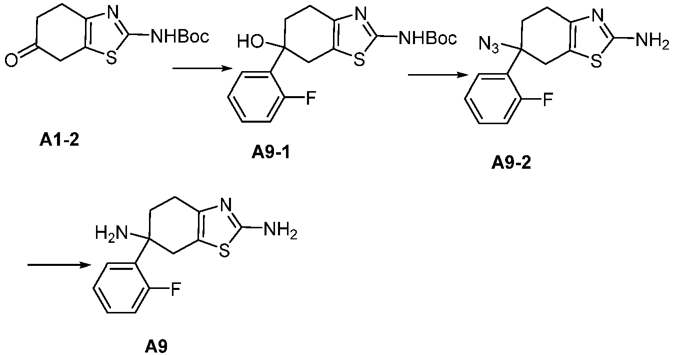

### Step 1:

*o*-Fluorobromobenzene (5 g, 1 eq) and lithium chloride (0.6 g, 0,5 eq) were added to THF (30 mL), and under nitrogen atmosphere, the mixture was cooled to -10 °C or lower. A 2.0 M solution of isopropylmagnesium chloride in THF (14.3 mL, 1 eq) was added, and after the addition was completed, the mixture was stirred for 3 h with the temperature controlled at -5 °C to -10 °C.

Another **A1-2** (1 g, 0.13 eq) was dispersed in THF (10 mL), and the mixture was added dropwise to the system described above. The resulting mixture was reacted for 2 h with the temperature controlled at -5 °C to -10°C. The reaction was quenched with methanol and water, and EA (100 mL) was added. The mixture was stirred and filtered, and the filtrate was washed with water (50 mL), dried over anhydrous sodium sulfate, concentrated, and purified by column chromatography to give **A9-1** (0.95 g, yield: 70.5%) as an off-white solid.

### Step 2:

**A9-1** (900 mg) was dissolved in DCM (10 mL). Sodium azide (0.83 g, 5 eq) was added, and TFA (9 mL) was added dropwise under an ice bath. After the mixture was reacted for 30 min under the ice bath, the mixture was transferred to room temperature and left to stand overnight in the dark. Ice water (50 mL) was added. The pH was adjusted to 9 with ammonia water. The mixture was extracted with DCM. The organic phase was washed with a saturated aqueous sodium chloride solution, dried over anhydrous sodium sulfate, concentrated, and subjected to column chromatography to give **A9-2** (70 mg).

### Step 3:

**A9-2** (70 mg) was dissolved in methanol (70 mL), and 10% palladium on carbon (50 mg) was added. Hydrogen was introduced, and the mixture was reacted at room temperature overnight. The mixture was filtered, and the filtrate was concentrated and subjected to column chromatography to give compound A9 (38 mg, yield: 59%) as a white solid.

¹H NMR (400 MHz, DMSO-*d*₆) δ7.47 (t, *J* = 16.3 Hz ,1H), 7.31-7.26 (m, 1H), 7.17-7.11 (m, 2H), 6.63 (s, 2H), 3.10 (d, *J =* 16 Hz, 1H), 2.62-2.50 (m, 2H), 2.35-2.31 (m, 1H), 2.16-2.12 (m, 1H), 1.82-1.78 (m, 1H). ESI (*m*/*z*): 264.13 [M + H]⁺. HPLC purity: > 97%, retention time: 6.229 min.

### Example 9 Preparation of 5-phenyl-5-(pyrrolidin-1-yl)-4,5,6,7-tetrahydro-1H-indazole (Compound A12) and Hydrochloride Thereof

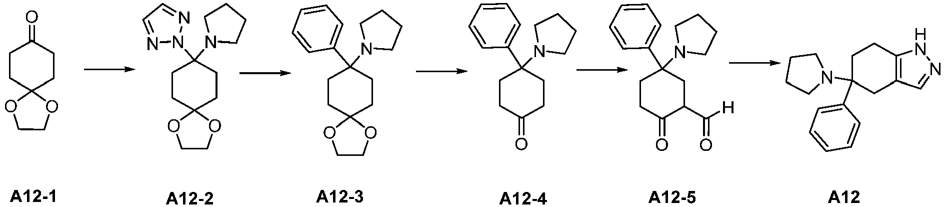

**Step 1:** Triazole (12.4 g, 1.1 eq), pyrrolidine (13.35 g, 1.2 eq), and 1,4-cyclohexanedione monoethylene acetal A12-1 (25.0 g, 160 mmol) were dissolved in toluene. The mixture was refluxed to remove water for 18 h and cooled to room temperature to give an **A12-2** solution for later use.

**Step 2:** Bromobenzene (75 g, 3 eq) was dissolved in anhydrous THF, and *n*-butyllithium (2.5 M, 150 mL, 3.3 eq) was slowly added dropwise at -78 °C. After the dropwise addition was completed, the temperature was maintained at -78 °C for 1 h, and the prepared **A12-2** solution described above was added dropwise. After the dropwise addition was completed, the mixture was stirred for 1 h with the temperature maintained, stirred at room temperature for 12 h, and subjected to column chromatography to give **A12-3** (10.5 g).

**Step 3:** Compound **A12-3** (10.5 g, 6.5 mmol) was dissolved in ethanol, and concentrated hydrochloric acid (12 M, 3 eq) was added. The mixture was stirred at room temperature overnight and concentrated to dryness to remove the solvent. The pH was adjusted to 11-12 with a saturated aqueous sodium bicarbonate solution. The mixture was extracted with DCM, dried, filtered, concentrated, and subjected to column chromatography to give **A12-4** (8.5 g).

### Step 4:

Compound **A12-4** (3.0 g, 11.1 mmol) was dissolved in toluene and dried over anhydrous magnesium sulfate for 2 days. The mixture was filtered for later use. Sodium *tert*-butoxide (2.46 g, 2 eq) was added to toluene, and ethyl formate (1.38 g, 1.5 eq) was added. The **A12-4** solution described above was added at 0-10 °C, and the mixture was stirred at room temperature overnight. The reaction solution was extracted twice with toluene. The aqueous phase was adjusted to pH = 6-7, and the mixture was extracted 3 times with dichloromethane, dried, filtered, concentrated, and subjected to column chromatography to give **A12-5** (2.5 g).

### Step 5:

Compound **A12-5** (200 mg, 7.3 mmol) was dissolved in ethanol, and hydrazine hydrate (0.5 mL) was added. The mixture was heated at reflux for 7 h. The mixture was concentrated to dryness to remove the solvent, and the residue was subjected to column chromatography to give **A12** (about 60 mg). The product obtained from the column chromatography described above was dissolved in methyl *tert-butyl* ether, and a commercially available 4 M hydrogen chloride/dioxane solution (1 eq, 0.056 mL) was added. A solid precipitated. The mixture was filtered and dried to give compound **A12** hydrochloride (25 mg).

LRMS-ESI (*m*/*z*): 268.49 [M + H]⁺. ¹H NMR (400 MHz, DMSO-*d*₆) δ 11.67 (s, 1H), 7.75 (m, 2H), 7.64 (s, 1H), 7.42 (m, 3H), 3.87 (d, *J =* 14.2 Hz, 1H), 3.48 (m, 1H), 3.32 (d, *J =* 15.3 Hz, 1H), 3.21 (m, 1H), 3.10 (m, 1H), 3.00 (m, 2H), 2.83 (dd, *J =* 16.8, 5.1 Hz, 1H), 2.55 (m, 1H), 2.13 (m, 1H), 1.83 (m, 2H), 1.66 (m, 2H).

Chiral column separation gave isomers **A12-P1** (S configuration) and **A12-P2** (R configuration):

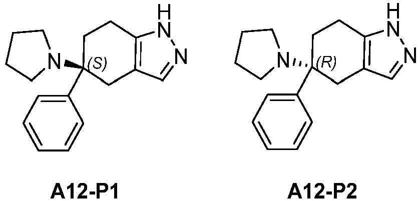

**A12-P1** (S configuration): HPLC purity: > 99%, retention time: 8.786 min. Chiral purity: > 99%, retention time: 7.703 min.

**A12-P2** (R configuration): HPLC purity: > 99%, retention time: 8.778 min. Chiral purity: > 95%, retention time: 10.236 min.

### Example 10 Preparation of N-methyl-6-phenyl-4,5,6,7-tetrahydrobenzothiazol-6-amine (Compound A13) and Hydrochloride Thereof

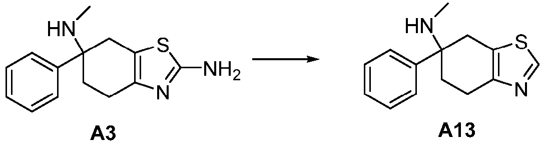

Compound **A3** (450 mg) was added to 85% phosphoric acid (25 mL), and the mixture was cooled to -10 °C. An aqueous sodium nitrite (6 eq) solution was added, and the mixture was stirred at -10 °C for 1 h and then added dropwise to a 50% hypophosphorous acid solution (0 °C, 7 mL). The resulting mixture was stirred at 0 °C for 1.5 h, neutralized with saturated sodium carbonate to pH 8-9, and extracted three times with EA. The organic phases were combined, concentrated, and subjected to column chromatography. The resulting **A13** sample was added to EA (50 mL), and a HCl/EA solution was added for salt formation to give compound **A13** hydrochloride (190 mg).

LRMS-ESI (*m*/*z*): 245.16 [M + H]⁺. ¹H NMR (400 MHz, DMSO-*d*₆) δ 10.32 (m, 1H), 9.75 (m, 1H), 8.95 (s, 1H), 7.57 (d, *J =* 6.9 Hz, 2H), 7.41 (m, 3H), 4.03 (d, *J =* 16.7 Hz, 1H), 3.48 (d, *J =* 16.7 Hz, 1H), 2.91 (d, *J =* 16.4 Hz, 1H), 2.72 (m, 1H), 2.55 (m, 1H), 2.19 (t, *J =* 5.2 Hz, 3H), 2.10 (m, 1H).

Chiral column separation gave isomers **A13-P1** (R configuration) and **A13-P2** (S configuration):

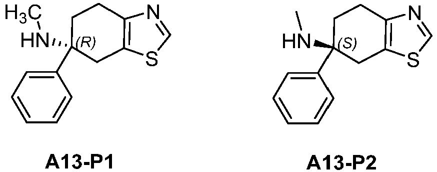

**A13-P1** (R configuration): HPLC purity: > 95%, retention time: 10.144 min. Chiral purity: > 99%, retention time: 4.501 min.

**A13-P2** (S configuration): HPLC purity: > 95%, retention time: 10.151 min. Chiral purity: > 99%, retention time: 6.418 min.

### Example 11 Preparation of 6-phenyl-4,5,6,7-tetrahydrobenzothiazol-6-amine (Compound A14) and Hydrochloride Thereof

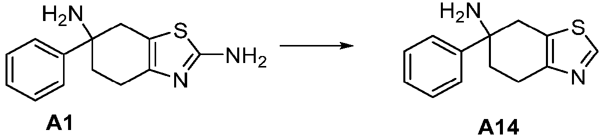

Compound **A1** (300 mg) was added to concentrated hydrochloric acid (12 M, 8 mL), and the mixture was stirred to give a clear solution. The solution was cooled to -30 °C, and a 1 M aqueous sodium nitrite solution (1.7 eq, 2 mL) was added dropwise. After the addition, the mixture was stirred for 1 h with the temperature maintained. Hypophosphorous acid (0.2 mL) was added, and the mixture was stirred under an ice-water bath for 1 h. Then the mixture was cooled to -30 °C and neutralized with a saturated aqueous sodium carbonate solution, extracted with dichloromethane-methanol, dried over anhydrous sodium sulfate, concentrated to dryness, and purified by column chromatography to give an **A14** sample (60 mg). EA (2 mL) was added, and a solution of hydrogen chloride in ethyl acetate was added dropwise. A solid precipitated. The mixture was filtered and dried to give compound **A14** hydrochloride (35 mg).

LRMS-ESI (*m*/*z*): 231.12 [M + H]⁺. ¹H NMR (400 MHz, DMSO-d₆) δ 8.97 (s, 1H), 8.93 (br, 3H), 7.58 (d, 2H), 7.40 (m, 3H), 3.78 (d, J = 16.6 Hz, 1H), 3.47 (d, J = 16.6 Hz, 1H), 2.90 (m, 1H), 2.59 (m, 1H), 2.44 (m, 1H), 2.27 (m, 1H).

Chiral column separation gave isomers **A14-P1** (R configuration) and **A14-P2** (S configuration):

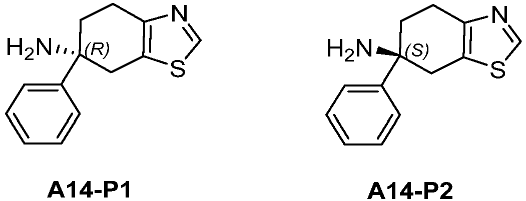

**A14-P1** (R configuration): HPLC purity: > 98%, retention time: 9.514 min. Chiral purity: 100%, retention time: 7.835 min.

**A14-P2** (S configuration): HPLC purity: > 95%, retention time: 9.607 min. Chiral purity: 100%, retention time: 21.014 min.

### Example 12 Preparation of 2-methyl-N-(4-phenyl-4,5,6,7-tetrahydrobenzothiophen-4-yl)propane-2-sulfinamide (Compound A15)

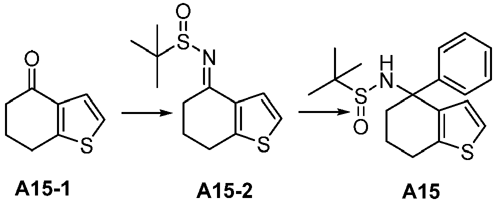

**Step 1: A15-1** (5 g, 1 eq), *tert*-butylsulfinamide (5.57 g, 1.4 eq), tetraethyl titanate (8.24 g, 1.1 eq), and THF were added to a reaction flask, and under nitrogen atmosphere, the mixture was reacted at reflux for 24 h (70 °C). The mixture was cooled to room temperature, and the reaction solution was poured into ice brine. EA was added, and the mixture was stirred and filtered. Liquid separation was performed, and the organic phase was retained. The aqueous phase was extracted with EA. The organic phases were combined, washed with saturated brine, dried, concentrated, and subjected to column chromatography to give compound **A15-2** (2.69 g, yield: 32.1%).

**Step 2:** Iodobenzene (1.0 g, 2.5 eq) was added to DCM (10 mL), and under nitrogen atmosphere, the mixture was cooled to -60 °C. *n*-Butyllithium (1.64 mL, 2.5 M, 2.1 eq) was added dropwise, and the mixture was reacted for 1.5 h with the temperature maintained. The mixture was designated as reaction solution 2 for later use. Compound **A15-2** (500 mg, 1 eq) was added to DCM (10 mL) to give a clear solution, and the mixture was designated as reaction solution 1 for later use. The reaction solution 1 was added dropwise to the reaction solution 2 with the temperature controlled at about -60 °C, and the mixture was reacted for 2 h with the temperature maintained. The reaction was quenched with ammonium chloride. The mixture was extracted with EA, washed with saturated brine, dried over anhydrous sodium sulfate, concentrated, and subjected to column chromatography to give compound **A15** (solid, 322 mg, yield: 49.3%).

### Example 13 Preparation of 4-phenyl-4,5,6,7-tetrahydrobenzothiophen-4-amine (Compound A16)

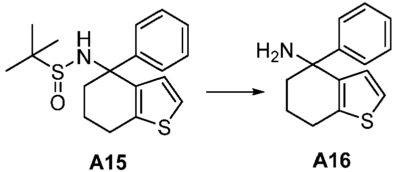

Compound **A15** (322 mg, 1 eq) was added to dioxane (9.7 mL), and a solution of hydrogen chloride in ethyl acetate (0.73 mL, 4 M, 3 eq) was added dropwise. The mixture was reacted at room temperature for 0.5 h. The mixture was neutralized with a saturated aqueous sodium bicarbonate solution, extracted with EA, washed with saturated brine, dried, concentrated, and subjected to column chromatography to give compound **A16** (192 mg, yield: 86.7%). DCM (2 mL) was added, and a 4 M solution of hydrogen chloride in ethyl acetate (0.3 mL) was added dropwise. The mixture was concentrated, slurried with isopropanol, and filtered to give **A16** in the hydrochloride form (HPLC purity: 96.7%).

¹H NMR (400 MHz, DMSO-*d*₆) δ 9.14 (s, 3H), 7.49 (d, *J =* 5.3 Hz, 1H), 7.44 - 7.33 (m, 3H), 7.30 - 7.22 (m, 2H), 7.03 (d, *J =* 5.3 Hz, 1H), 2.85 (m, 2H), 2.23 (t, *J =* 4.8 Hz, 1H), 1.95 (m, 1H), 1.49 (m, 1H).

### Example 14 Preparation of N-(4-phenyl-4,5,6,7-tetrahydrobenzothiophen-4-yl)formamide (Compound A17)

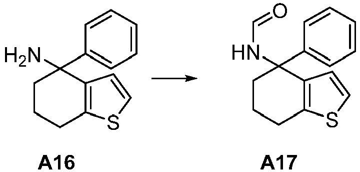

Anhydrous formic acid (347 mg, 9 eq) and acetic anhydride (770 mg, 9 eq) were added to a reaction flask, and under nitrogen atmosphere, the mixture was warmed to 60 °C and reacted for 2 h. Then the mixture was cooled to room temperature (about 25 °C). A solution of compound **A16** (192 mg, 1 eq) in DCM (4 mL) was added, and the mixture was stirred at room temperature for 0.5 h. The reaction was quenched with a saturated aqueous sodium bicarbonate solution, and the mixture was extracted with DCM and concentrated to give compound **A17** (226 mg) as an oily liquid.

### Example 15 Preparation of N-(4-phenyl-4,5,6,7-tetrahydrobenzothiophen-4-yl)formamide (Compound A18) and Hydrochloride Thereof

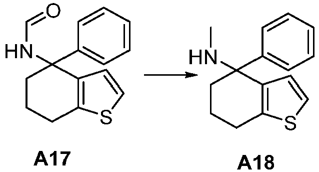

Compound **A17** (226 mg, over theoretical, 1 eq) was added to a THF (10 mL) solution. A 1.0 M solution of borane in tetrahydrofuran (8.4 mL, 10 eq, 1.0 M) was added dropwise under nitrogen atmosphere, and the mixture was warmed at reflux for 3 h. Methanol was added to quench the reaction, and the mixture was concentrated to dryness. Methanol and water in a ratio of 1:1 (5 mL) was added, and the mixture was warmed at reflux. After the reaction was completed as detected by TLC, the mixture was cooled to room temperature, extracted by DCM, and concentrated to give a crude product of **A18.** 2 mL of DCM was added, and 0.3 mL of a 4 M solution of hydrogen chloride in ethyl acetate was added dropwise. The mixture was concentrated to dryness, slurried with isopropanol, and filtered to give **A18** in the form of hydrochloride (150 mg, yield: 73.6%) as an off-white solid. HPLC: 98.3%.

¹H NMR (400 MHz, DMSO-*d*₆) δ 10.10 (s, 1H), 9.75 (s, 1H), 7.57 (d, *J* = 5.3 Hz, 1H), 7.49 - 7.37 (m, 3H), 7.36 - 7.31 (m, 2H), 7.10 (d, *J =* 5.3 Hz, 1H), 2.92 - 2.78 (m, 2H), 2.41 (t, *J =* 5.2 Hz, 3H), 2.35 (dd, *J =* 12.5, 2.4 Hz, 1H), 2.25 (m, 1H), 1.94 (m, 1H), 1.40 (m, 1H).

### Example 16 Preparation of 6-phenyl-4,5,6,7-tetrahydrobenzothiophen-6-amine (Compound A19)

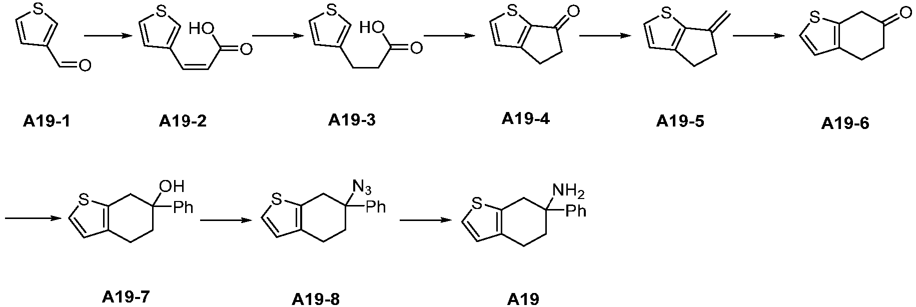

### Step 1:

Compound **A19-1** (10 g, 1 eq), malonic acid (13 g, 1.4 eq), and 12 mL of pyridine were sequentially added to a 100 mL reaction flask, and the mixture was warmed to 100 °C and reacted for 6 h. After the reaction solution was cooled, 3 M hydrochloric acid was added to adjust the pH to 3. A solid was precipitated, filtered, rinsed with water, and rinsed with *n*-heptane (50 mL × 2) to give compound **A19-2** (13.6 g) as an off-white solid.

### Step 2:

Compound **A19-2** (13.6 g), ammonium formate (22.47 g, 4 eq), 10% palladium on carbon (10 g), and isopropanol (150 mL) were added to a 500 mL reaction flask, and the mixture was warmed to 90 °C and reacted for 36 h under nitrogen atmosphere. The reaction solution was cooled to room temperature, filtered to remove the palladium on carbon through celite, concentrated to remove most of the isopropanol, diluted with 2 M hydrochloric acid (300 mL, pH = 2-3), extracted with EA (200 mL × 2), dried over sodium sulfate, and concentrated to give compound **A19-3** (10.5 g, two-step yield: 76.09%) as a solid.

### Step 3:

Polyphosphoric acid (PPA, 50 g) was added into a 250 mL reaction flask, which was warmed to 120 °C. When PPA can be stirred, compound **A19-3** (5 g, 1 eq) was added in batches, and the mixture was reacted for 1 h with the temperature maintained. The oil bath was removed, and ice blocks were slowly added into the reaction system while the reaction system was hot to quench the reaction (100 mL of ice water were added in total). After the feed liquid was cooled to room temperature, EA (100 mL) was added, and the mixture was stirred, filtered to remove black residues through celite, and left to stand for liquid separation. The aqueous phase was back-extracted once with 50 mL of EA, and the organic phases were combined, washed once with saturated sodium bicarbonate (100 mL), washed with water (100 mL), dried, filtered through silica gel, and concentrated to give compound **A19-4** (2.5 g, yield: 56.56%) as a brownish-yellow solid.

### Step 4:

Methyltriphenylphosphonium bromide (19.40 g, 1.5 eq) and anhydrous tetrahydrofuran (40 mL) were added to a 250 mL three-necked flask. The temperature was controlled at about 0 °C, and 2.5 M butyllithium (2.10 mL, 1.4 eq) was added dropwise. After the addition was completed, the mixture was reacted at room temperature for 1 h. The temperature was controlled at about 0 °C, and a solution of compound **A19-4** (5 g, 1 eq) in tetrahydrofuran (10 mL) was added dropwise to the reaction system described above. After the addition was completed, the mixture was reacted at room temperature overnight. Ice water (100 mL) and EA (100 mL) were added to the feed liquid, and the mixture was filtered through celite, and left to stand for liquid separation. The organic phase was concentrated to the minimum volume, and subjected to column chromatography to give compound **A19-5** (1.1 g, yield: 22.31%) as an oil.

### Step 5:

Compound **A19-5** (1.3 g, 1 eq) and methanol-water (volume ratio: 95/5, 39 mL in total) were added to a 100 mL reaction flask, and HTIB oxidant (3.56 g, 0.95 eq) was added in batches under an ice-water bath. After the addition was completed, the mixture was reacted at room temperature for 20 min. The reaction solution was concentrated to remove most of the methanol (35 °C). DCM and water (20 mL each) were added, and the mixture was stirred at room temperature for 20 min and subjected to phase separation. The DCM phase was concentrated and purified by column chromatography to give compound **A19-6** (630 mg, yield: 43.45%) as an oil.

### Step 6:

Compound **A19-6** (358 mg, 1 eq) and DCM (5 mL) were added to a 25 mL reaction flask, and 2 M phenylmagnesium chloride (1.18 mL, 1 eq) was added dropwise with the temperature maintained at 0-10 °C under nitrogen atmosphere. After the addition was completed, the mixture was reacted for 30 min with the temperature maintained. The reaction solution was poured into saturated ammonium chloride, and DCM was separated, dried, concentrated, and purified by column chromatography to give compound **A19-7** (263 mg, yield: 48.52%) as an oil.

### Step 7:

Compound **A19-7** (263 mg, 1 eq), sodium azide (1.11 g, 15 eq), and DCM (26.3 mL) were added to a 50 mL reaction flask, and TFA (13 mL) was added dropwise under nitrogen atmosphere under an ice-water bath. After the addition was completed, the mixture was reacted for 30 min with the temperature maintained. The reaction solution was poured into ice water (20 mL), and the pH was adjusted to 9 with ammonia water. The DCM phase was separated after standing, dried, and purified by column chromatography to give compound **A19-8.**

### Step 8:

Compound **A19-8** was dissolved in methanol (13 mL), and palladium on carbon (0.52 g) was added. The mixture was purged with hydrogen and reacted for 2 h under normal pressure. The reaction solution was filtered to remove the palladium on carbon and concentrated to give compound **A19** (90 mg, two-step yield: 34.35%) as a solid.

LRMS-ESI (*m*/*z*): 230.14 [M + H]⁺. ¹H NMR (400 MHz, CDCl₃) δ 7.54 (d, *J =* 7.3 Hz, 1H), 7.35 (t, *J =* 7.6 Hz, 1H), 7.25 (m, 1H), 7.11 (d, *J =* 5.1 Hz, 1H), 6.79 (d, *J =* 5.1 Hz, 1H), 3.34 (d, *J =* 16.6 Hz, 1H), 2.94 (d, *J =* 16.2 Hz, 1H), 2.87 - 2.74 (m, 1H), 2.61 (dt, *J =* 16.7, 5.5 Hz, 1H), 2.32 - 2.17 (m, 1H), 2.04 - 1.92 (m, 1H).

Chiral column separation gave isomers **A19-P1** (R configuration) and **A19-P2** (S configuration):

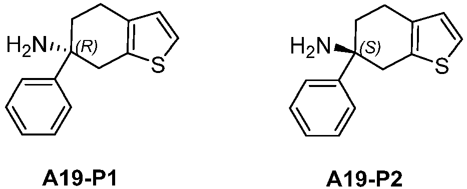

**A19-P1** (R configuration): HPLC purity: > 93%, retention time: 13.149 min. Chiral purity: 99%, retention time: 9.196 min.

**A19-P2** (S configuration): HPLC purity: > 97%, retention time: 13.279 min. Chiral purity: 98.8%, retention time: 10.021 min.

### Example 17 Preparation of N-(6-phenyl-4,5,6,7-tetrahydrobenzothiophen-6-yl)formamide (Compound A20)

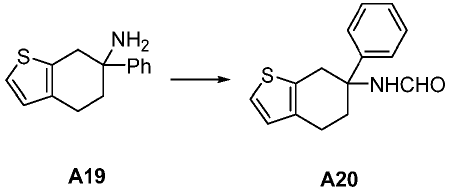

Preparation of acetic formic anhydride: Acetic anhydride (0.51 mL, 28 eq) and 98% formic acid (0.21 mL, 28 eq) were added to a 10 mL reaction flask, stirred at 65 °C for 1-2 h, and cooled to room temperature to give acetic formic anhydride for later use. A mixed solution of compound **A19** (45 mg, 1 eq, basic) in DCM/THF was added dropwise to the self-made acetic formic anhydride under an ice-water bath, and the mixture was stirred at room temperature for 1 h. A saturated aqueous sodium bicarbonate solution was slowly added to the reaction solution. The pH was adjusted to 8. The DCM phase was separated, concentrated, and purified by column chromatography to give compound **A20** (37 mg, yield: 73.27%) as a gum.

### Example 18 Preparation of N-methyl-6-phenyl-4,5,6,7-tetrahydrobenzothiophen-6-amine (Compound A21) and Hydrochloride Thereof

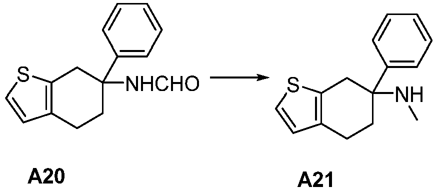

Compound **A20** (37 mg, 1 eq) was dissolved in THF (3.7 mL), lithium aluminium hydride (55 mg, 10 eq) was added in batches under an ice-water bath under nitrogen atmosphere, and the mixture was warmed at reflux for 3 h. The reaction solution was cooled to 0-10 °C, and 0.05 mL of water and 0.05 mL of a 20% sodium hydroxide solution were added dropwise. The mixture was filtered through celite and concentrated to the minimum volume, and 1 mL of a 4 M solution of hydrogen chloride in ethyl acetate was added. The mixture was stirred for 10 min and concentrated, and 0.5 mL of EA was added. The mixture was stirred for crystallization, and filtered to give compound **A21** in the form of hydrochloride (23 mg, yield: 57.5%) as an off-white solid.

¹H NMR (400 MHz, DMSO-*d*₆) δ 10.02 (s, 1H), 9.53 (s, 1H), 7.56 (d, *J =* 6.9 Hz, 2H), 7.46 - 7.36 (m, 3H), 7.31 (d, *J =* 5.1 Hz, 1H), 6.70 (d, *J =* 5.1 Hz, 1H), 3.90 (d, *J =* 16.5 Hz, 1H), 3.35 (d, *J =* 16.5 Hz, 1H), 2.73 (m, 1H), 2.62 (m, 1H), 2.41 (m, 1H), 1.96 (m, 1H). LRMS-ESI (*m*/*z*): 244.18 [M + H]⁺.

### Example 19 Preparation of 5-phenyl-4,5,6,7-tetrahydrobenzothiophen-5-amine (Compound A22)

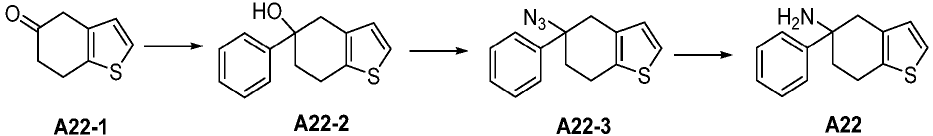

### Step 1:

**A22-1** (195 mg, 1.3 mmol) was placed in a 50 mL three-necked flask and purged with nitrogen. Dried THF (5 mL) was added, and the mixture was cooled to 0 °C. PhMgCl (1 mL, 1.97 mmol, 1.5 eq.) was added dropwise, and after the dropwise addition was completed, the mixture was reacted at 0 °C for 2 h. The reaction was quenched with a saturated aqueous ammonium chloride solution, and the mixture was extracted with ethyl acetate. The organic phase was washed with saturated brine, dried, filtered, concentrated to dryness, and subjected to column chromatography to give the product **A22-2** (115 mg) as a yellow oil.

### Step 2:

**A22-2** (115 mg, 0.5 mmol) was dissolved in DCM (3 mL), and NaN₃ (65 mg, 1 mmol, 2 eq.) was added. The mixture was purged with nitrogen and cooled to 0 °C. TFA (0.2 mL, 2.56 mmol, 5 eq.) was added dropwise, and after the dropwise addition was completed, the mixture was reacted at 0 °C for 2.5 h. The mixture was diluted with water under an ice-water bath, and ammonia water was added to quench the reaction. The mixture was extracted with ethyl acetate, and the organic phase was washed with saturated brine, dried, filtered, and concentrated to give **A22-3.**

### Step 3:

**A22-3** was dissolved in MeOH (10 mL), and Pd/C (150 mg) was added. The mixture was purged with hydrogen and reacted at room temperature for 2 h. The reaction solution was filtered to remove the Pd/C and concentrated, and the residue was dissolved in MTBE (10 mL). A 4 M solution of hydrogen chloride in ethyl acetate (0.1 mL) was added dropwise, a white solid precipitated, and the mixture was filtered to give compound **A22** (30 mg, three-step yield: 8.8%) as an off-white solid.

¹H NMR (400 MHz, CD₃OH) δ 7.52-7.50 (m, 2H), 7.46-7.38 (m, 3H), 7.28 (d, *J =* 4.0 Hz, 1H), 6.92 (d, *J =* 4.0 Hz, 1H), 3.62 (d, *J =* 16 Hz, 1H), 3.15 (d, *J =* 16 Hz, 1H), 2.97-2.92 (m,1H), 2.64-2.59 (m,1H), 2.55-2.47 (m,1H), 2.44-2.38 (m,1H). LRMS-ESI (*m*/*z*): 213.17 [M - NH₂]⁺. Chiral column separation gave isomers **A22-P1** (S configuration) and **A22-P2** (R configuration):

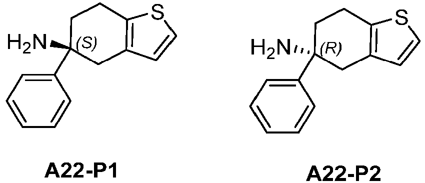

**A22-P1** (R configuration): HPLC purity: > 97%, retention time: 13.319 min. Chiral purity: 99%, retention time: 10.137 min.

**A22-P2** (S configuration): HPLC purity: > 97%, retention time: 13.321 min. Chiral purity: 98.8%, retention time: 10.871 min.

### Example 20 Preparation of 5-(N-methylbenzamido)-5-phenyl-4,5,6,7-tetrahydrobenzothiophen-2-carboxylic acid (Compound A23)

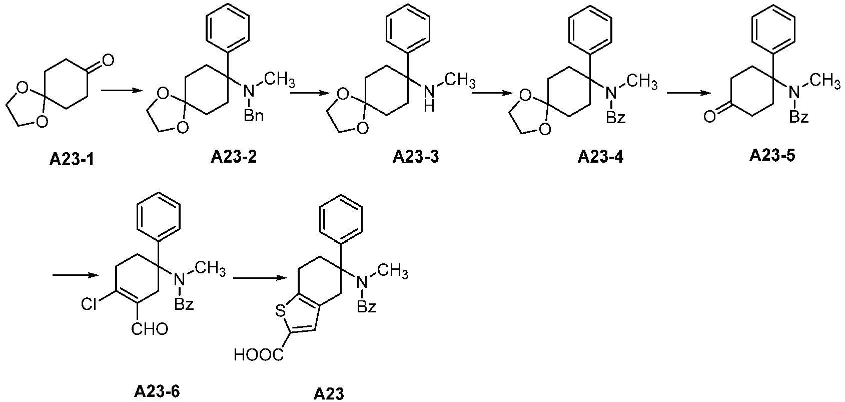

### Step 1:

In a reaction flask, 8 g of compound **A23-1,** *N*-methylbenzylamine (6.83 g, 1.1 eq), 1,2,3-triazole (4.25 g, 1.2 eq), and 50 mL of toluene were added, and the mixture was refluxed to remove water overnight and cooled to room temperature. Phenylmagnesium bromide (4 eq, in THF) was added to another reaction flask, and the reaction solution described above was added dropwise thereto under an ice bath and stirred for 90 min. The reaction solution was poured into an aqueous ammonium chloride solution, and EA was added. The organic phase was washed with water, dried, concentrated, and subjected to column chromatography to give compound **A23-2**(3.85 g, yield: 22%) as a light yellow oil.

### Step 2:

3.85 g of compound **A23-2,** 460 mg of Pd(OH)₂/C, 50 mL of ethanol, and 6.75 g of ammonium formate (10 eq) were added to a reaction flask, and the mixture was purged with nitrogen and refluxed for 50 min. The mixture was filtered to remove insoluble substances, and the residue was rinsed with ethanol. The filtrates were combined and concentrated, and the residue was diluted with DCM, separately washed once with water and a saturated sodium chloride solution, dried, and concentrated to give compound **A23-3** (2.49 g, yield: 92%) as a colorless oil.

### Step 3:

2.49 g of compound **A23-3** was added to a reaction flask, DCM and DIPEA 1.5 eq were added, and benzoyl chloride 1.1 eq was added under an ice bath. After the addition was completed, the mixture was reacted at room temperature overnight. The mixture was diluted with EA, separately washed once with an aqueous ammonium chloride solution and a saturated aqueous sodium chloride solution, dried, concentrated, and slurried with 15 mL of *n*-heptane to give compound **A23-4** (2.97 g, yield: 84%) as a light yellow solid.

### Step 4:

2.9 g of compound **A23-4,** 80 mL of acetone, 40 mL of water, and 0.2 eq of *p*-toluenesulfonic acid were added to a reaction flask, and the mixture was refluxed for 5 h. The mixture was diluted with EA, separately washed once with a saturated aqueous sodium bicarbonate solution and a saturated aqueous sodium chloride solution, dried, concentrated, and subjected to column chromatography to give compound **A23-5** (2.34 g, yield: 92%) as a colorless oil.

### Step 5:

4 eq of DMF was dissolved in 30 mL of dry DCM, and the mixture was stirred for 20 min under an ice bath. 3 eq of phosphorus oxychloride was added dropwise to the system, and after the addition was completed, the mixture was stirred at room temperature for 1 h and stirred again for 10 min under an ice bath. Compound **A23-5** (2 g, 1 eq) was dissolved in 15 mL of dry DCM, and the reaction solution described above was quickly added. The mixture was naturally warmed to room temperature and stirred overnight under nitrogen atmosphere. The reaction solution was poured into ice water. The pH was adjusted to weak basicity. The organic phase was separated, washed with saturated brine, dried, concentrated, and subjected to column chromatography to give compound **A23-6** (1.04 g, yield: 45%) as a light yellow oil.

### Step 6:

Sodium (3 eq) was added to anhydrous ethanol under an ice bath. After the reaction was completed, 2 eq of ethyl thioglycolate was added, and a solution of compound **A23-6** (1 g, 1 eq) in anhydrous ethanol was added. The mixture was stirred at room temperature for 3 h under nitrogen atmosphere. A 5 N aqueous sodium hydroxide solution (4 eq NaOH) was added directly, and the mixture was stirred at 60 °C for 4 h. After the reaction was completed, the mixture was diluted with water, and MTBE was added for phase separation. The organic layer was discarded, and the aqueous layer was separated, adjusted to about pH 2 with 1 M hydrochloric acid, and extracted with EA. The organic phase was washed with saturated brine, dried, and concentrated to give compound **A23** (1.03 g) as a light yellow solid.

### Example 21 Preparation of N-methyl-N-(5-phenyl-4,5,6,7-tetrahydrobenzothiophen-5-yl)benzamide (Compound A24)

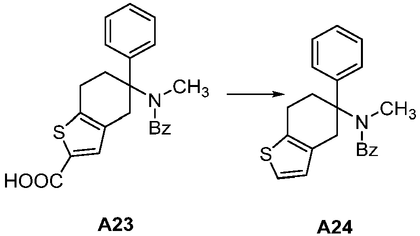

600 mg of compound **A23,** 0.5 eq of cuprous oxide, 0.4 eq of DBU, and a small amount of DMF were added to a reaction flask, and the mixture was purged with nitrogen many times and reacted at 160 °C for 5 h. The reaction solution was cooled, stirred with excess EA, and filtered to remove insoluble substances. The filter residue was rinsed with EA, and the filtrate was diluted with EA, separately washed once with an aqueous ammonium chloride solution and saturated brine, dried, concentrated, and subjected to column chromatography to give compound **A24** (405 mg) as a white solid.

### Example 22 Preparation of N-methyl-5-phenyl-4,5,6,7-tetrahydrobenzothiophen-5-amine (Compound A25) and Hydrochloride Thereof

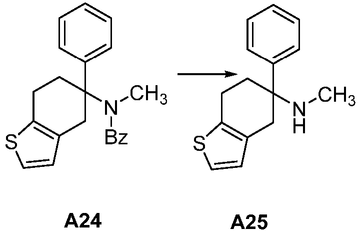

Dry THF and 360 mg of compound **A24** were added to a reaction flask, dissolved by stirring, and cooled under an ice bath, and 2 eq of LAH was added in batches for multiple times. After the addition was completed, the mixture was naturally warmed to ambient temperature, and stirred for 3 h. The reaction solution was poured into an ice water solution containing 2.7 g of potassium sodium tartrate tetrahydrate, and the mixture was extracted with EA. The organic phase was washed with saturated brine, dried, concentrated, and subjected to alkaline aluminum oxide column chromatography to give **A25** (about 220 mg) as a colorless oil, which was dissolved in methanol. A hydrogen chloride/methanol solution (about 1 eq of HCl) was added dropwise under an ice bath, and after the addition was completed, the mixture was stirred for 30 min and concentrated. An appropriate amount of acetonitrile was added to the residue, and the mixture was stirred. A white solid precipitated, and the mixture was filtered and dried to give compound **A25** hydrochloride (210 mg) as a white solid.

¹H NMR (400 MHz, DMSO-*d*₆) δ 10.21 (q, *J =* 6.2 Hz, 1H), 9.67 (m, 1H), 7.58 - 7.50 (m, 2H), 7.45 - 7.36 (m, 3H), 7.34 (d, *J =* 5.1 Hz, 1H), 6.94 (d, *J =* 5.1 Hz, 1H), 3.72 (dd, *J =* 16.1, 2.0 Hz, 1H), 3.25 (dd, *J =* 16.0, 2.3 Hz, 1H), 2.87 (dq, *J =* 16.9, 2.4 Hz, 1H), 2.67 (ddt, *J =* 12.8, 5.2, 2.4 Hz, 1H), 2.47 (m, 1H), 2.19 (t, *J =* 5.3 Hz, 3H), 2.14 - 2.00 (m, 1H). LRMS-ESI (*m*/*z*): 244.14 [M + H]⁺.

### Example 23 Preparation of methyl 7-(N-methylformamido)-7-phenyl-4,5,6,7-tetrahydrobenzothiophen-2-carboxylate (Compound A27)

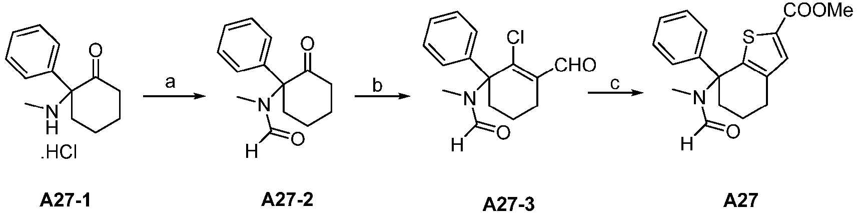

### Step 1:

Preparation of acetic formic anhydride: Acetic anhydride (127.8 mg, 3 eq) and anhydrous formic acid (57.6 mg, 3 eq) were added to a 10 mL reaction flask, stirred at 65 °C for 1-2 h, and cooled to room temperature to give acetic formic anhydride for later use.

Compound **A27-1** (100 mg) was suspended in DCM, 5 eq of DIPEA was added, and the mixture was subjected to an ice bath. 3 eq of the self-made acetic formic anhydride was added dropwise, and after the addition was completed, the mixture was reacted at ambient temperature for 40 min. The reaction was completed as detected by TLC. The mixture was diluted with DCM, washed with diluted hydrochloric acid, washed with brine, dried, and concentrated to give compound **A27-2** (90 mg) as an off-white solid. 1.1 g of compound **A27-2** was prepared again in this way.

### Step 2:

540 mg of compound **A27-2,** 2 eq of triethylamine, and 5 mL of DMF-DMA mixture were added to a reaction flask, and the mixture was reacted at 95 °C overnight. The mixture was concentrated to remove the solvent, and the residue was diluted with DCM, separately washed with an aqueous ammonium chloride solution and saturated brine, dried, and concentrated to give a yellow oil; 2.5 eq of DMF (calculated according to **A27-2**) and dry DCM were mixed and added to another reaction flask, and the mixture was stirred under an ice bath; 2.5 eq of phosphorus oxychloride was dropwise added. After the addition was completed, the mixture was stirred at room temperature for 40 min. The yellow oil obtained before was dissolved in a small amount of dry DCM and dropwise added to the system. After the addition was completed, the mixture was stirred at room temperature for 3 h under nitrogen atmosphere. The mixture was diluted with DCM, washed with an aqueous sodium hydroxide solution, washed with saturated brine, dried, concentrated, and subjected to column chromatography to give compound **A27-3** (613 mg, yield: 94%) as a light yellow oil.

### Step 3:

Sodium (2.5 eq) was added to anhydrous methanol under an ice bath. After the reaction was completed, 1.1 eq of ethyl thioglycolate was added, and a solution of compound **A27-3** (610 mg, 1 eq) in anhydrous methanol was added. The mixture was stirred at room temperature for 3 h. A small amount of the reaction solution was mixed with an ammonium chloride solution, and the mixture was extracted with EA. The organic phase was dried, concentrated, and subjected to column chromatography to give the title compound **A27.**

¹H NMR (400 MHz, Chloroform-*d*) δ 8.03 (s, 1H), 7.51 (s, 1H), 7.45 - 7.30 (m, 5H), 3.81 (s, 3H), 2.78 (s, 3H), 2.75 (m, 2H), 2.50 - 2.41 (m, 2H), 1.88 (m, 2H).

### Example 24 Preparation of 7-(N-methylformamido)-7-phenyl-4,5,6,7-tetrahydrobenzothiophen-2-carboxylic acid (Compound A28)

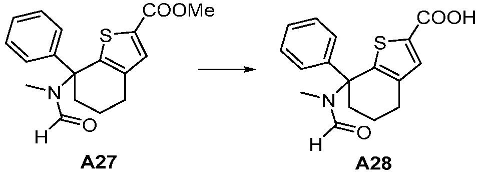

To the reaction solution of compound **A27** was added a 5 M aqueous sodium hydroxide solution (4 eq NaOH), and the mixture was stirred at room temperature overnight. The mixture was diluted with water, and MTBE was added for phase separation. The organic layer was discarded, and the aqueous layer was separated, adjusted to about pH 2 with 4 M hydrochloric acid, and extracted with DCM. The organic phase was washed with saturated brine, dried, and concentrated to give compound **A28** (730 mg, yield: 100%) as a light yellow oil.

LRMS-ESI (m/z): 314.39 [M - H]⁻.

### Example 25 Preparation of N-methyl-N-(7-phenyl-4,5,6,7-tetrahydrobenzothiophen-7-yl)formamide (Compound A29)

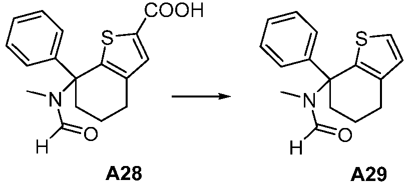

720 mg of compound **A28,** 0.5 eq of cuprous oxide, 0.4 eq of DBU, and a small amount of DMF were added, and the mixture was purged with nitrogen many times and reacted at 160 °C for 4 h. The reaction solution was cooled, stirred with excess EA, and filtered to remove insoluble substances. The filter residue was washed with EA, and the filtrate was diluted with EA, separately washed once with a saturated aqueous ammonium chloride solution and saturated brine, dried, concentrated, and subjected to column chromatography to give compound **A29** (350 mg, yield: 60%) as a white solid.

¹H NMR (400 MHz, Chloroform-d) δ 8.01 (s, 1H), 7.44 - 7.30 (m, 5H), 7.22 (d, *J =* 5.2 Hz, 1H), 6.79 (d, *J =* 5.2 Hz, 1H), 2.76 (s, 3H), 2.74 (m, 2H), 2.53 - 2.40 (m, 2H), 1.93 (m, 1H), 1.83 (m, 1H).

### Example 26 Preparation of N-methyl-7-phenyl-4,5,6,7-tetrahydrobenzothiophen-7-amine (Compound A30) and Hydrochloride Thereof

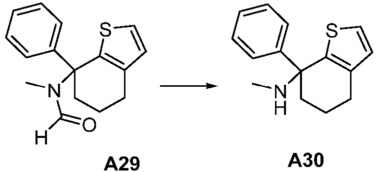

350 mg of compound **A29** was added, dissolved in dry THF, and cooled under an ice bath. 2 eq of lithium aluminum hydride was added in batches for multiple times, and after the addition was completed, the mixture was naturally warmed to room temperature and stirred for 40 min under nitrogen atmosphere. The reaction solution was poured into a solution of potassium sodium tartrate tetrahydrate in ice water, and the mixture was extracted with EA. When the phase separation was clear, the organic phase was washed with saturated brine, dried, concentrated, and separated by neutral aluminum oxide column chromatography to give a colorless oil (210 mg). The oil **A30** was dissolved in methanol and subjected to an ice bath for 10 min. A hydrogen chloride/methanol solution (about 1 eq of HCl) was added dropwise, and after the addition was completed, the mixture was stirred for 10 min and concentrated to dryness under reduced pressure. 2 mL of acetonitrile was added, and the mixture was stirred at ambient temperature overnight, filtered, and dried to give compound **A30** hydrochloride (170 mg) as a white solid.

¹H NMR (500 MHz, Methanol-*d*₄) δ 7.63 (d, *J* = 5.2 Hz, 1H), 7.51 - 7.41 (m, 3H), 7.36 - 7.33 (m, 2H), 7.03 (d, *J =* 5.2 Hz, 1H), 2.88 - 2.73 (m, 2H), 2.69 (s, 3H), 2.51 - 2.37 (m, 2H), 2.02 - 1.92 (m, 1H), 1.70 - 1.59 (m, 1H). LRMS-ESI (*m*/*z*): 213.07 [M - NHMe]⁺.

### Example 27 Preparation of 2-methyl-N-(4-phenyl-4,5,6,7-tetrahydrobenzothiazol-4-yl)propane-2-sulfinamide (Compound A31)

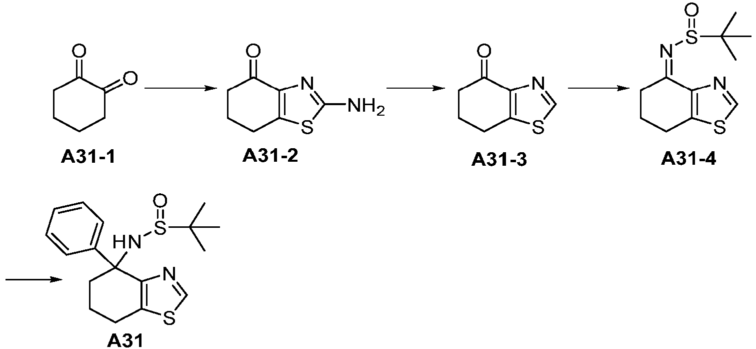

### Step 1:

Glacial acetic acid (200 mL) was added to compound **A31-1** (20 g, 1 eq), and a solution of liquid bromine (29 g, 1 eq) in acetic acid (20 mL) was slowly added dropwise under nitrogen atmosphere. The mixture was reacted at room temperature for 2 h. Thiourea (27.4 g, 2 eq) was added, and the mixture was heated at reflux for 3 h, concentrated to dryness to remove the solvent, slurried with ethanol, and filtered to give compound **A31-2** (15 g, 50%).

### Step 2:

THF was added to compound **A31-2** (20.6 g, 1 eq), and the mixture was cooled to 0-5 °C under nitrogen atmosphere. Isoamyl nitrite (17.3 g, 1.2 eq) was added dropwise, and after the dropwise addition was completed, the mixture was warmed to 45 °C, reacted for 2 h, quenched with ammonium chloride, extracted with DCM, washed with saturated brine, dried, concentrated, and subjected to column chromatography to give compound **A31-3** (5.7 g) as a dark brown solid.

### Step 3:

Compound **A31-3** (300 mg, 1 eq), *tert*-butylsulfinamide (330 mg, 1.4 eq), tetraethyl titanate (489 mg, 1.1 eq), and THF were added to a reaction flask, and under nitrogen atmosphere, the reaction solution was reacted at 40 °C overnight, cooled to room temperature, poured into ice brine, stirred with EA, filtered, subjected to liquid separation, extracted with EA, washed with saturated brine, dried, concentrated, and subjected to column chromatography to give compound A31-4 (about 288 mg). Gram-level compound **A31-4** was prepared in the same manner.

### Step 4:

Iodobenzene (35.4 g, 2.5 eq) was added to DCM (356 mL), and under nitrogen atmosphere, the mixture was cooled to -60 °C. *n*-Butyllithium (58.3 mL, 2.5 M, 2.1 eq) was added dropwise, and the mixture was reacted for 3 h with the temperature maintained. The mixture was designated as reaction solution 2 for later use. Compound **A31-4** (17.8 g, 1 eq) was added to DCM (338 mL) to give a clear solution, and the mixture was designated as reaction solution 1 for later use. The reaction solution 1 was added dropwise to the reaction solution 2 with the temperature controlled at about -60 °C, and the mixture was reacted for 2 h with the temperature maintained. The reaction was quenched with ammonium chloride. The mixture was extracted with EA, washed with saturated brine, dried over anhydrous sodium sulfate, concentrated, and subjected to column chromatography to give compound **A31** (5.5 g).

### Example 28 Preparation of 4-phenyl-4,5,6,7-tetrahydrobenzothiazol-4-amine (Compound A32)

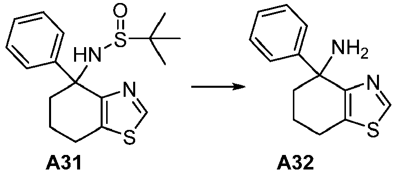

Compound **A31** (5.5 g, 1 eq) was added to dioxane (165 mL), and a solution of hydrogen chloride in ethyl acetate (12.4 mL, 4 M, 3 eq) was added dropwise. The mixture was reacted at room temperature for 0.5 h. The mixture was neutralized with a saturated aqueous sodium bicarbonate solution, extracted with EA, washed with saturated brine, dried, concentrated, and subjected to column chromatography to give compound **A32** (2.81 g).

¹H NMR (400 MHz, DMSO-*d*₆) δ 8.85 (s, 1H), 7.68 - 6.74 (m, 5H), 2.85 (m, 2H), 2.27 (s, 2H), 1.92 (m, 2H), 1.85 (m, 1H), 1.56 (m, 1H). LRMS-ESI (*m*/*z*): 214.09 [M - NH₂]⁺.

### Example 29 Preparation of N-(4-phenyl-4,5,6,7-tetrahydrobenzothiazol-4-yl)formamide (Compound A33)

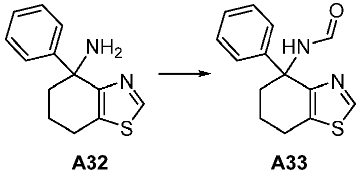

Anhydrous formic acid (1.8 g, 9 eq) was added to acetic anhydride (3.99 g, 9 eq), and under nitrogen atmosphere, the mixture was warmed to 60 °C and reacted for 2 h. Then the mixture was cooled to room temperature (about 25 °C). A solution of compound **A32** (1.0 g, 1 eq) in DCM (20 mL) was added, and the mixture was stirred at room temperature for 1.5 h. The reaction was quenched with a saturated aqueous sodium bicarbonate solution, and the mixture was extracted with DCM. The organic phase was concentrated and subjected to column chromatography to give compound **A33** (952 mg) as a light yellow solid.

### Example 30 Preparation of N-methyl-4-phenyl-4,5,6,7-tetrahydrobenzothiazol-4-amine (Compound A34) and Hydrochloride Thereof

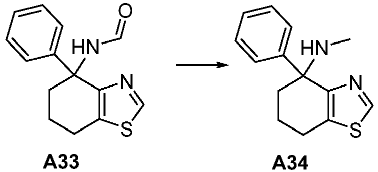

Compound A33 (950 mg, 1 eq) was added to a THF (40 mL) solution, and under nitrogen atmosphere, borane tetrahydrofuran (36.8 mL, 10 eq, 1.0 M) was added dropwise. The mixture was warmed at reflux and reacted for 2 h. The reaction was completed as shown by TLC. The mixture was cooled to 0-5 °C, and the reaction was quenched with methanol under an ice bath. The mixture was concentrated to dryness and stirred with methanol and water 1:1 (17 mL) at room temperature. The reaction was completed as detected by TLC. The mixture was extracted with DCM, and the organic phase was concentrated and subjected to column chromatography to give a crude product (364 mg). 10 mL of DCM was added, and a solution of hydrogen chloride in ethyl acetate (0.37 mL, 1.0 eq, 4.0 M) was added dropwise. The mixture was concentrated to dryness under reduced pressure, slurried with isopropyl ether, and filtered to give compound A34 (330 mg).

¹H NMR (400 MHz, DMSO-*d*₆) δ 9.94 (m, 2H), 9.21 (s, 1H), 7.46-7.36 (m, 3H), 7.29 (m, 2H), 2.90 (m, 2H), 2.47 (m, 1H), 2.43 (t, *J* = 5.2 Hz, 3H), 2.36 (td, *J* = 13.1, 2.4 Hz, 1H), 2.00 (m, 1H), 1.43 (m, 1H). LRMS-ESI (*m*/*z*): 214.08 [M - NHCH₃]⁺.

### Example 31 Preparation of 4-phenyl-4,5,6,7-tetrahydrobenzothiazole-2,4-diamine (Compound A35)

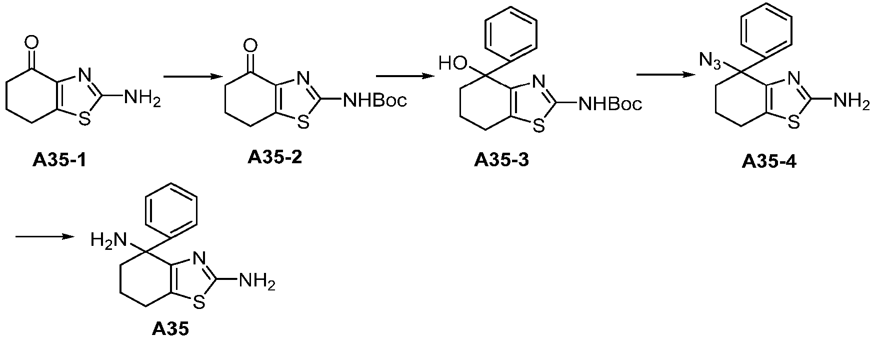

### Step 1:

Compound **A35-1** (800 mg) was added to dichloromethane (20 mL), and DMAP (464 mg) and di-*tert*-butyl dicarbonate (1.57 g) were added. The mixture was reacted at room temperature overnight. The mixture was concentrated and subjected to column chromatography to give compound **A35-2** (250 mg) as an off-white solid.

### Step 2:

2.7 mL (26.087 mmol, 7 eq) of bromobenzene and 6 mL of anhydrous tetrahydrofuran were added to a 100 mL three-necked flask, and under nitrogen atmosphere, the mixture was stirred in a low-temperature reaction kettle at -78 °C. When the internal temperature decreased to -60 °C or below, 10.4 mL (26.087 mmol, 7 eq) of 2.5 M *n*-butyllithium was added dropwise, while controlling the internal temperature to not exceed -60 °C. 1 h after the dropwise addition was completed, a solution of 1 g (3.726 mmol, 1 eq) of compound **A35-2** in THF (10 mL) was added slowly, while controlling the internal temperature to not exceed -60 °C. After the dropwise addition was completed, the mixture was stirred at -78 °C for 5 h. An aqueous saturated ammonium chloride solution was added to the reaction system, and the mixture was extracted with EA. The EA phase was washed with water, washed with saturated brine, dried over anhydrous sodium sulfate, concentrated, and subjected to column chromatography to give compound **A35-3** (1.13 g) as a light yellow solid.

LRMS-ESI (m/z): 347.18 [M + H]⁺.

### Step 3:

1.13 g (3.261 mmol, 1 eq) of compound **A35-3** was added to a 50 mL three-necked flask, and 8 mL of DCM was added. The mixture was stirred for 10 min under an ice bath under nitrogen atmosphere, and 2.1 g (32.61 mmol, 10 eq) of NaN₃ was added. After the addition was completed, 4 mL of CF₃COOH was slowly added dropwise, and the mixture was stirred at room temperature overnight. The system was poured into a proper amount of ice water, and the pH was adjusted to about 10 by slowly adding ammonia water dropwise. The mixture was extracted with a proper amount of DCM, and the DCM phase was washed with water, washed with saturated brine, dried over anhydrous sodium sulfate, and concentrated to give compound **A35-4** as an oil.

LRMS-ESI (m/z): 272.06 [M + H]⁺.

### Step 4:

2 mL of THF and 122 mg (0.2 eq) of cobalt chloride hexahydrate were added to the oil **A35-4** obtained in the above step, and the mixture was stirred under nitrogen atmosphere. A solution of 390 mg (4 eq) NaBH₄ in water (10 mL) was added slowly dropwise, and the mixture was stirred at room temperature for 2 h. The pH was adjusted to about 3 with 2 M hydrochloric acid, and the mixture was filtered to remove insoluble substances. The pH was then adjusted to about 10 with a saturated sodium carbonate solution, and the mixture was extracted with DCM/MeOH = 20:1, dried over anhydrous sodium sulfate, concentrated, and subjected to column chromatography to give compound A35 (320 mg) as a white solid.

¹H NMR (400 MHz, DMSO-*d*₆) δ 7.30-7.22 (m, 4H), 7.16-7.12 (m, 1H), 6.67 (s, 2H), 2.57-2.54 (m, 2H),2.22 (s, 2H), 1.84-1.81(m, 2H), 1.78-1.72 (m, 1H), 1.54-1.43 (m, 1H). LRMS-ESI (m/z): 274.17 [M-NH₂]⁺. HPLC purity: > 97%, retention time: 10.848 min.

### Examples 32-35 Preparation of N-(2-amino-4-phenyl-4,5,6,7-tetrahydrobenzothiazol-4-yl)formamide (Compound A36), N,N'-(4-phenyl-4,5,6,7-tetrahydrobenzothiazole-2,4-diyl)diformamide (Compound A37), N⁴-methyl-4-phenyl-4,5,6,7-tetrahydrobenzothiazole-2,4-diamine (Compound A38), and N⁴-methyl-4-phenyl-4,5,6,7-tetrahydrobenzothiazole-2,4-diamine (Compound A39)

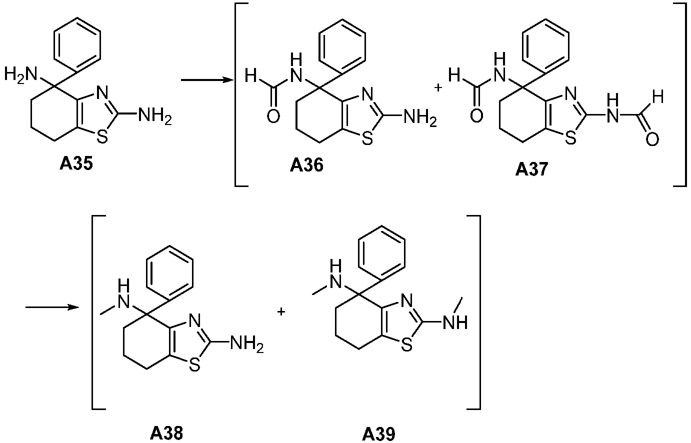

### Step 1:

240 mg of compound **A35** was added to a 25 mL single-necked flask, and 8 mL of ethyl formate was added. The mixture was refluxed overnight under nitrogen atmosphere. The reaction system was concentrated to dryness. An appropriate amount of DCM:MeOH = 20:1 was added. The organic phase was washed once with saturated brine, dried over sodium sulfate, and concentrated to give a brown solid (200 mg). A small amount thereof was separated by column chromatography to give compound **A36** and compound **A37.**

**A36:** LRMS-ESI (m/z): 274.17 [M + H]⁺.

**A37:** LRMS-ESI (m/z): 320.20 [M + H]⁺.

### Step 2:

5 mL of a solution of borane in tetrahydrofuran was added to the mixture of **A36** and **A37.** The mixture was refluxed overnight under nitrogen atmosphere. The system was cooled to room temperature. Methanol was slowly added dropwise to quench excess borane, and the mixture was concentrated, 6 mL of methanol and 2 drops of concentrated hydrochloric acid were added. The mixture was refluxed overnight under nitrogen atmosphere. The system was cooled to room temperature. A small amount of ammonia water was added to adjust the pH to 8-9. The mixture was concentrated and subjected to column chromatography to give compound **A39** (50 mg), which was subjected to salification with HCl/MeOH to give a light yellow powder (56 mg). The fragment peak was obtained by mass spectrometry. LRMS-ESI (*m*/*z*): 243.32 [M -NHCH₃]⁺.

¹H NMR (400 MHz, DMSO-*d*₆) δ 9.03 (br, 1H), 7.57 (m, 1H), 7.39 (m, 4H), 7.35 (m, 1H), 2.80 (d, *J* = 4.7 Hz, 3H), 2.60 (m, 2H), 2.43 (s, 3H), 2.22 (m, 2H), 1.85 (m, 1H), 1.33 (m, 1H).

Compound **A38** (18 mg) was further obtained by column chromatography.

¹H NMR (400 MHz, DMSO-*d*₆) δ 7.25 (d, *J =* 4.0 Hz, 4H), 7.19-7.14 (m, 1H), 6.69 (s, 2H), 2.56-2.54 (m, 2H), 2.17 (s, 3H), 2.05-1.98 (m, 1H), 1.78-1.66 (m, 2H), 1.49-1.39 (m, 1H). LRMS-ESI (m/z): 260.21 [M + H]⁺.

### Example 36 Preparation of N-(2-amino-5-phenyl-4,5,6,7-tetrahydrobenzothiazol-5-yl)formamide (Compound A40)

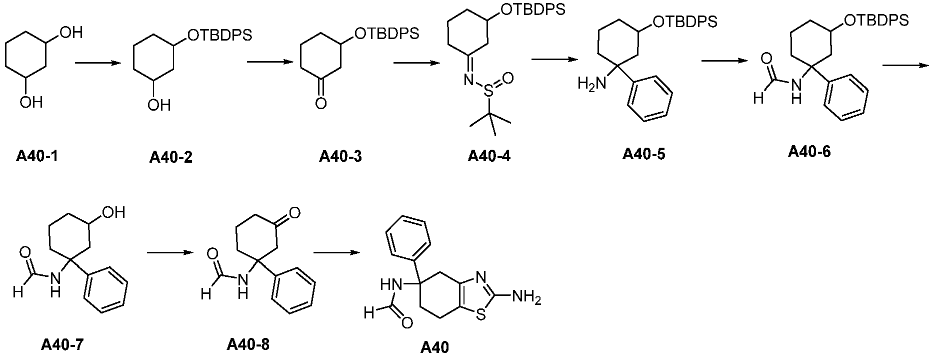

### Step 1:

Compound **A40-1** (5.0 g, 43.1 mmol) was dissolved in DMF. Imidazole (6.5 g, 2 eq) was added, and TBDPSCl (12 g, 1.1 eq) was added at 0-10 °C. The mixture was stirred at room temperature overnight, quenched with water, and extracted with EA/water, and the organic phase was dried, concentrated, and subjected to column chromatography to give compound **A40-2** (6.0 g) as a light yellow oil.

### Step 2:

Compound **A40-2** (6.0 g, 16.9 mmol) was dissolved in DCM. Dess-Martin reagent (24 g, 1.5 eq) was added. The mixture was stirred at room temperature overnight. The reaction solution was poured into water, extracted with EA, dried, concentrated, and subjected to column chromatography to give compound **A40-3** (5.0 g).

### Step 3:

Compound **A40-3** (5.0 g, 14.2 mmol) was dissolved in anhydrous THF, and *tert*-butylsulfinamide (2.6 g, 1.5 eq) was added. Ethyl titanate (11 mL, 3 eq) was added dropwise at 0-10 °C, and the mixture was at room temperature overnight. The reaction solution was poured into water and filtered. The filtrate was extracted with EA, dried, concentrated, and subjected to column chromatography to give compound **A40-4** (6.6 g).

### Step 4:

Preparation of Grignard reagent: Mg (3.4 g, 10 eq) was suspended in anhydrous THF, and the mixture was purged with nitrogen 3 times. Bromobenzene (29 g, 10 eq) was slowly added dropwise, and the mixture was heated at 50 °C for 1 h and cooled to -10-0 °C for later use. Compound **A40-4** (6.6 g, 14.2 mol) was dissolved in anhydrous THF, and the Grignard reagent described above was slowly added dropwise. The mixture was stirred at 0-10 °C for 2 h and stirred at room temperature for 1 h. 2 M hydrochloric acid was added, and the mixture was stirred at room temperature overnight. The reaction solution was poured into water, adjusted to pH = 8-9, extracted with dichloromethane, dried, concentrated, and subjected to column chromatography to give compound **A40-5** (3.5 g).

### Step 5:

Compound **A40-5** (3.5 g) was dissolved in ethyl formate, and the mixture was heated at reflux for 24 h. The mixture was concentrated to dryness to remove the solvent and subjected to column chromatography to give compound **A40-6** (3.5 g).

### Step 6:

Compound **A40-6** (3.5 g) was dissolved in THF, and TABF (1 M, 16 mL, 2 eq) was added. The mixture was stirred at room temperature overnight. The mixture was concentrated to dryness to remove the solvent and subjected to column chromatography to give compound **A40-7** (110 mg).

### Step 7:

Compound **A40-7** (1.1 g, 5.1 mmol) was dissolved in DCM. Dess-Martin reagent (3.8 g, 2 eq) was added. The mixture was stirred at room temperature overnight. The reaction was quenched with sodium thiosulfate, and the mixture was extracted with dichloromethane, dried, filtered, concentrated, and subjected to column chromatography to give compound **A40-8** (700 mg).

### Step 8:

Compound **A40-8** (350 mg, 1.6 mmol) was dissolved in DCM, and pyridinium tribromide (700 mg, 1.1 eq) was added at 0-10 °C. The mixture was stirred at room temperature for 3 h. The reaction was quenched with sodium thiosulfate, and the mixture was extracted with dichloromethane, dried, filtered, concentrated, and subjected to column chromatography to give a bromo intermediate (380 mg) as a light yellow solid. The light yellow solid described above was dissolved in dioxane, and thiourea (350 mg, 3 eq) and diisopropylethylamine (400 mg, 2 eq) were added. The mixture was stirred at 80-90 °C for 5 h. The mixture was extracted with DCM/water, and the organic phase was dried, filtered, concentrated, and subjected to column chromatography to give the title compound (200 mg).

¹H NMR (400 MHz, DMSO-*d*₆) δ 8.31-8.19 (m, 1H), 8.02-7.97 (m, 1H), 7.42-7.19 (m, 5H), 6.72-6.70 (m, 2H), 2.97-2.90 (m, 2H), 2.67-2.62 (m, 1H), 2.52-2.47 (m, 1H), 2.41-2.32 (m, 1H), 2.28-2.14 (m, 1H).

### Example 37 Preparation of N-(2-amino-7-phenyl-4,5,6,7-tetrahydrobenzothiazol-7-yl)formamide (Compound A41)

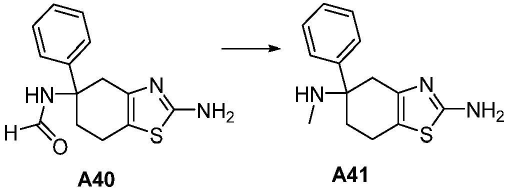

Compound **A40** (200 mg, 0.73 mmol) was suspended in THF, and a solution of borane in tetrahydrofuran (10 eq) was added. The mixture was heated at reflux for 3 h. Methanol was added to quench the reaction, and the mixture was concentrated to dryness to remove the solvent. Methanol/water was added, and the mixture was refluxed overnight, extracted with chloroform, dried, concentrated, and subjected to column chromatography to give compound **A41** (100 mg).

¹H NMR (400 MHz, DMSO-*d*₆) δ 7.40 (m, 2H), 7.35 (t, *J =* 7.5 Hz, 2H), 7.26 (t, *J =* 6.9 Hz, 1H), 6.69 (s, 2H), 3.09 (d, *J =* 16.7 Hz, 1H), 2.69 (d, *J =* 16.2 Hz, 1H), 2.16 (m, 2H), 2.00 (m, 5H). LRMS-ESI (*m*/*z*): 260.19 [M + H]⁺.

### Example 38 Preparation of N-(2-amino-7-phenyl-4,5,6,7-tetrahydrobenzothiazol-7-yl)formamide (Compound A42)

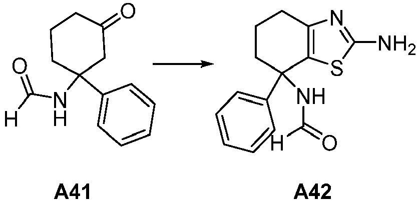

Compound **A41** (350 mg, 1.6 mmol) was dissolved in DCM. The mixture was heated at reflux. Pyridinium tribromide (700 mg, 1.1 eq) was added. The mixture was stirred for 1 h. The reaction was quenched with sodium thiosulfate, and the mixture was extracted with dichloromethane, dried, filtered, concentrated, and subjected to column chromatography to give a bromo intermediate (380 mg) as a light yellow solid. The light yellow solid described above was dissolved in dioxane, and thiourea (350 mg, 3 eq) and diisopropylethylamine (400 mg, 2 eq) were added. The mixture was heated at 80-90 °C for 5 h. The mixture was extracted with DCM/water, and the organic phase was dried, filtered, concentrated, and subjected to column chromatography to give compound **A42** (150 mg).

### Example 39 Preparation of N⁷-methyl-7-phenyl-4,5,6,7-tetrahydrobenzothiazole-2,7-diamine (Compound A43)

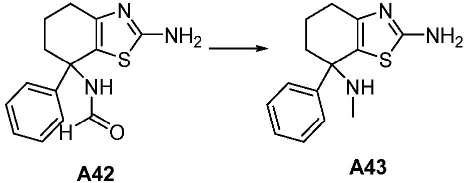

Compound **A42** (150 mg, 0.56 mmol) was suspended in THF, and a solution of borane in tetrahydrofuran (10 eq) was added. The mixture was heated at reflux for 3 h. Methanol was added to quench the reaction, and the mixture was concentrated to dryness to remove the solvent. A mixed solution of methanol/water with equal volume was added, and the mixture was refluxed overnight, extracted with chloroform, dried, filtered, and subjected to column chromatography to give compound **A43** (80 mg).

¹H NMR (400 MHz, DMSO-*d*₆) δ 7.36 (m, 2H), 7.29 (t, *J* = 7.5 Hz, 2H), 7.19 (t, *J* = 7.1 Hz, 1H), 6.63 (s, 2H), 2.92 (d, *J* = 16.3 Hz, 1H), 2.55 (d, *J* = 16.6 Hz, 1H), 2.03 (m, 3H), 1.92 (s, 3H), 1.88 (m, 1H). LRMS-ESI (*m*/*z*): 260.18 [M + H]⁺.

### Example 40 Preparation of ethyl 5-morpholino-5-phenyl-4,5,6,7-tetrahydrobenzothiophen-2-carboxylate (Compound A44) and Hydrochloride Thereof

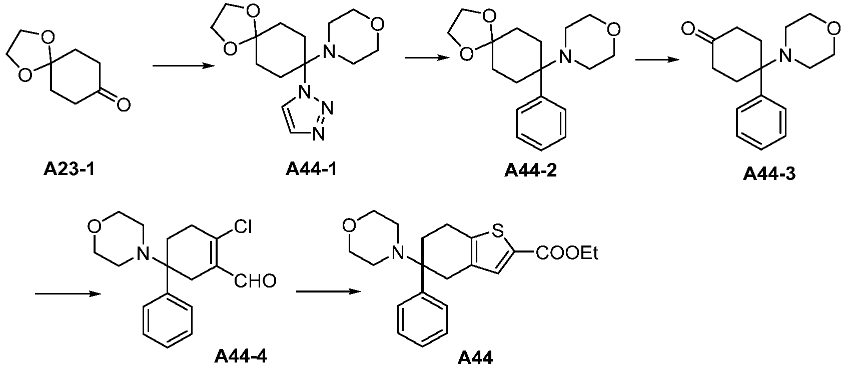

### Step 1:

5 g of compound **A23-1,** 1.1 eq of morpholine, and 1.2 eq of triazole were mixed in 40 mL of toluene, and the mixture was refluxed to remove water overnight to give a solution containing compound **A44-1.**

### Step 2:

Under nitrogen atmosphere, 4 eq of a solution of phenylmagnesium bromide in tetrahydrofuran was stirred for 10 min under an ice bath, and the solution of compound **A44-1** described above was added dropwise to the reaction system. After the addition was completed, the mixture was stirred at room temperature for 2 h. The reaction solution was poured into an aqueous ammonium chloride solution and extracted with EA. The organic phase was washed twice with water, and directly concentrated to give a crude product containing compound **A44-2.**

### Step 3:

Compound **A44-2** was dissolved in 20 mL of tetrahydrofuran, and 20 mL of 1 M hydrochloric acid was added. The mixture was reacted at 40 °C overnight. The mixture was adjusted to weak basicity and extracted with EA. The organic phase was washed with water, washed with saturated brine, dried, concentrated, and subjected to column chromatography to give compound **A44-3** (4.4 g, three-step yield: 53%) as an oil. The compound was slurried in PE and filtered to give a light yellow solid (3.4 g).

### Step 4:

Under an ice bath and nitrogen atmosphere, 2 eq of phosphorus oxychloride was dropwise added to a dichloromethane solution containing 1.6 eq of DMF. After the addition was completed, the mixture was stirred at room temperature for 20 min and cooled under an ice bath again. 200 mg of compound **A44-3** (1 eq) was added to the reaction system in batches for multiple times, and after the addition was completed, the mixture was reacted at room temperature overnight. An aqueous sodium bicarbonate solution was added for liquid separation, The organic phase was washed with saturated brine, concentrated, and subjected to column chromatography to give 115 mg of compound **A44-4.**

### Step 5:

Metal sodium (2 eq) was added to anhydrous ethanol under an ice bath. After the sodium completely disappeared in the solution under nitrogen atmosphere, 1.1 eq of ethyl thioglycolate was added, and a solution of compound **A44-4** (110 mg, 1 eq) in ethanol was added dropwise. After the addition was completed, the mixture was reacted at room temperature overnight. Ammonium chloride was added to quench the reaction, and the mixture was extracted with DCM. The organic phase was dried, concentrated, and subjected to column chromatography to give a colorless oil (105 mg). Hydrogen chloride/methanol solution was added to methanol to form hydrochloride, which was slurried in EA, and filtered to give compound **A44** hydrochloride (85 mg) as a white solid.

¹H NMR (400 MHz, DMSO-*d*₆) δ 11.65 (s, 1H), 7.69 (m, 2H), 7.57 (s, 1H), 7.52 - 7.42 (m, 3H), 4.23 (q, *J =* 7.1 Hz, 2H), 4.20 - 3.82 (m, 5H), 3.60 - 3.50 (m, 3H), 3.24 (d, *J =* 12.5 Hz, 1H), 3.12 - 3.03 (m, 1H), 2.70 - 2.55 (m, 3H), 2.35 (m, 1H), 1.25 (t, *J* = 7.1 Hz, 3H). LRMS-ESI (*m*/*z*): 372.43 [M + H]⁺.

### Example 41 Preparation of ethyl 3-amino-5-morpholino-5-phenyl-4,5,6,7-tetrahydrobenzothiophen-2-carboxylate (Compound A45) and Hydrochloride Thereof

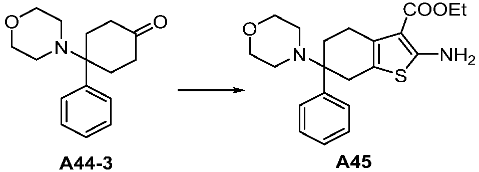

200 mg of compound **A44-3** was mixed with 1.5 eq of ethyl cyanoacetate, 1.5 eq of morpholine, 1.5 eq of elemental sulfur in ethanol, and the mixture was stirred at 50 °C overnight. The reaction solution was cooled and a solid precipitated. The mixture was filtered, and the filter cake was purified by column chromatography to give **A45** (80 mg) as a solid. Hydrogen chloride/methanol solution was added to methanol to form hydrochloride, which was slurried in acetonitrile, and filtered to give the title compound **A45** hydrochloride (65 mg) as a light yellow solid.

¹H NMR (400 MHz, DMSO-*d*₆) δ 11.30 (s, 1H), 7.67 (s, 2H), 7.48 (m, 3H), 7.25 (m, 2H), 4.16 - 3.82 (m, 7H), 3.65 - 3.40 (m, 3H), 3.09 (d, *J* = 12.9 Hz, 1H), 2.99 - 2.89 (m, 1H), 2.55 (m, 2H), 2.39 (m, 1H), 2.11 (m, 1H), 1.17 (t, *J =* 7.1 Hz, 3H). LRMS-ESI (*m*/*z*): 387.39 [M + H]⁺. **Example 42 Preparation** of

### 5-morpholino-5-phenyl-4,5,6,7-tetrahydrobenzothiophen-2-carboxylic acid (Compound A46)

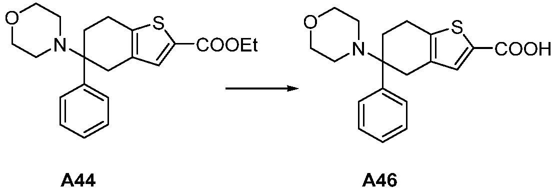

Compound **A44** (590 mg) was added to a reaction flask, and ethanol and 1.27 mL of 5 N aqueous sodium hydroxide solution (4 eq) were added. The mixture was reacted at 60 °C for 8 h and diluted with water. MTBE was added. The aqueous phase was separated, adjusted to be neutral, and extracted with DCM (5 mL × 5). The organic phases were combined, washed with a small amount of saturated brine, dried, and concentrated to give a crude product (455 mg), which was slurried with MTBE and filtered. The filter cake was dried to give compound **A46** (320 mg) as a light pink solid.

¹H NMR (400 MHz, DMSO-*d*₆) δ 12.78 (s, 1H), 7.53 (s, 1H), 7.35 - 7.17 (m, 5H), 3.50 (t, *J* = 4.5 Hz, 4H), 3.23 (d, *J* = 16.5 Hz, 1H), 3.00 (d, *J* = 16.4 Hz, 1H), 2.80 (dt, *J* = 17.0, 4.4 Hz, 1H), 2.45 (m, 2H), 2.27 (m, 3H), 2.19 - 2.01 (m, 2H). LRMS-ESI (*m*/*z*): 344.47 [M + H]⁺.

### Example 43 Preparation of 4-(5-phenyl-4,5,6,7-tetrahydrobenzothiophen-5-yl)morpholine (Compound A47) and Hydrochloride Thereof

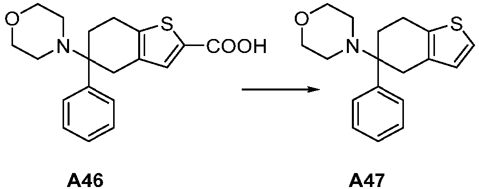

235 mg of compound **A46,** 0.5 eq of cuprous oxide, and 0.4 eq of DBU were mixed in a small amount of DMF, and the mixture was purged with nitrogen for multiple times, reacted at 160 °C for 4 h, cooled, diluted with EA, washed with an aqueous ammonium chloride solution, washed with saturated brine, dried, concentrated, and subjected to column chromatography to give A47 (100 mg) as a white solid. Hydrogen chloride/methanol solution was added to methanol to form hydrochloride, which was slurried in acetonitrile, and filtered to give a white solid (100 mg), namely compound **A47** hydrochloride.

¹H NMR (400 MHz, DMSO-*d*₆) δ 11.67 (s, 1H), 7.77 - 7.60 (m, 2H), 7.45 (m, 3H), 7.28 (d, *J* = 5.1 Hz, 1H), 6.87 (d, *J =* 5.1 Hz, 1H), 4.17 - 3.81 (m, 5H), 3.63 (d, *J* = 12.4 Hz, 1H), 3.49 (d, *J* = 14.4 Hz, 2H), 3.21 (q, *J =* 4.5 Hz, 1H), 2.98 (dd, *J =* 17.2, 5.4 Hz, 1H), 2.60 (m, 3H), 2.29 (m, 1H). LRMS-ESI (*m*/*z*): 300.45 [M + H]⁺.

### Example 44 Preparation of 6-phenyl-6-(piperidin-1-yl)-4,5,6,7-tetrahydrobenzothiazol-2-amine (Compound A48) and Hydrochloride Thereof

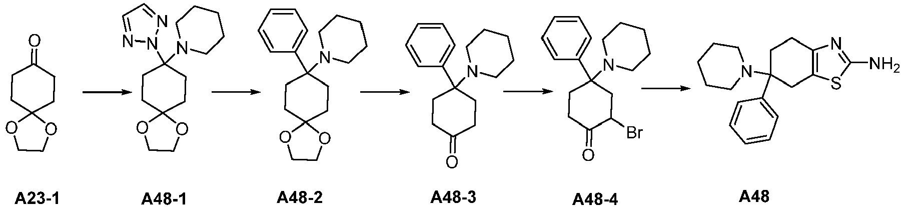

### Step 1:

Preparation of Grignard reagent: magnesium (345.6 mg, 4.8 eq) was suspended in dry tetrahydrofuran, bromobenzene (200 mg, 0.48 eq) was added dropwise, and the mixture was warmed to 50-60 °C for initiation. Bromobenzene (1.8 g, 3.6 eq) was then added dropwise with slight boiling. After 30 min, the dropwise addition was completed, and the mixture was warmed to 60 °C and stirred for 1 h. Most of magnesium disappeared. The mixture was cooled to 0-10 °C for later use.

Triazole (1.0 g, 1.2 eq), piperidine (1.4 mL, 1.2 eq), and 1,4-cyclohexanedione monoethylene acetal **A23-1** (2.0 g, 12.8 mmol) were dissolved in toluene in another reaction flask. The mixture was refluxed to remove water for 18 h to give a solution of **A48-1** in toluene. The mixture was cooled to room temperature and added dropwise to the above Grignard reagent (0-10 °C). After the dropwise addition was completed, the mixture was stirred at room temperature for 12 h, and subjected to column chromatography to give compound **A48-2** (1.0 g).

### Step 2:

Compound **A48-2** (900 mg) was dissolved in ethanol, and hydrochloric acid was added. The mixture was heated to 70 °C and stirred for 12 h. The reaction solution was concentrated to dryness and extracted with methyl *tert*-butyl ether/water. The aqueous phase was adjusted to pH = 11-12 with a sodium hydroxide solution, and the mixture was extracted with dichloromethane, dried, filtered, and concentrated to give compound **A48-3** (500 mg).

### Step 3:

Compound **A48-3** (500 mg, 2 mmol) was dissolved in acetic acid. Tribromopyridine (800 mg, 1.2 eq) was added, and the mixture was heated at reflux for 3 h. Thiourea (300 mg, 2 eq) was added, and the mixture was heated at reflux for 18 h and concentrated to dryness to remove the solvent. A saturated sodium carbonate solution was added to adjust the pH to be 9-10, and the mixture was extracted with dichloromethane. The organic phase was dried, concentrated, and subjected to column chromatography to give **A48** (200 mg) as a white solid. Methanol (5 mL) was added to give a clear solution, and HCl/EA solution was dropwise added. The mixture was stirred for reaction and concentrated to dryness to remove the solvent to give compound **A48** hydrochloride as a white solid.

LRMS-ESI (*m*/*z*): 314.28 [M + H]⁺. ¹H NMR (400 MHz, DMSO-*d*₆) δ 10.75 (s, 1H), 7.69 (br, 2H), 7.45 (m, 3H), 7.15 (br, 2H), 3.99 (d, *J =* 15.1 Hz, 1H), 3.68 (d, *J =* 10.9 Hz, 1H), 3.61 (d, *J* = 11.2 Hz, 1H), 3.47 (d, *J =* 15.0 Hz, 1H), 3.11 (m, 1H), 2.55 (m, 2H), 2.32 (m, 2H), 2.10 (m, 1H), 1.71 (d, *J =* 13.2 Hz, 2H), 1.60 (d, *J* = 11.7 Hz, 1H), 1.18 (m, 1H).

### Example 45 Preparation of 6-(3-chlorophenyl)-6-(piperidin-1-yl)-4,5,6,7-tetrahydrobenzothiazol-2-amine (Compound A49)

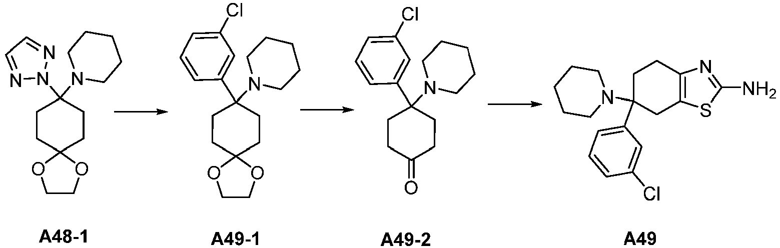

### Step 1:

*m*-Chloroiodobenzene (12 g, 2 eq) was dissolved in anhydrous THF, and *n*-butyllithium (2.5 M, 23 mL, 1.1 eq) was slowly added dropwise at -78 °C. After the dropwise addition was completed, the temperature was maintained at -78 °C for 1 h, and the newly prepared solution of **A48-1** in toluene was added dropwise. After the dropwise addition was completed, the mixture was stirred for 1 h with the temperature maintained at -78 °C, stirred at room temperature for 12 h, and subjected to column chromatography to give compound **A49-1** (3.0 g).

### Step 2:

Compound **A49-1** (3.0 g, 1.35 mmol) was dissolved in ethanol, and concentrated hydrochloric acid (3 eq, 12 M) was added. The mixture was stirred at room temperature overnight and concentrated to dryness to remove the solvent. The pH was adjusted to 11-12 with saturated sodium bicarbonate. The mixture was extracted with DCM, dried, filtered, concentrated, and subjected to column chromatography to give **A49-2** (1.8 g).

### Step 3:

Compound **A49-2** (200 mg, 0.7 mmol) was dissolved in acetic acid. Pyridinium tribromide (1.1 g, 4 eq) was added. The mixture was heated at reflux for 4 h. Thiourea (420 mg, 8 mmol) was added. The mixture was heated at reflux for 18 h, concentrated to dryness to remove the solvent, adjusted to pH = 9-10 with a saturated sodium carbonate solution, extracted with dichloromethane, dried, filtered, concentrated, and subjected to column chromatography to give the title compound **A49** (25 mg).

LRMS-ESI (*m*/*z*): 348.38 [M + H]⁺.

### Example 46 Preparation of 6-phenyl-6-(pyrrolidin-1-yl)-4,5,6,7-tetrahydrobenzothiazol-2-amine (Compound A50)

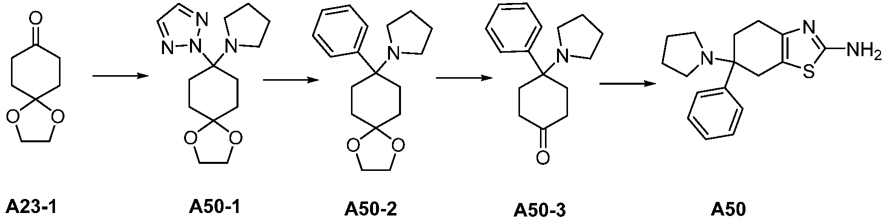

### Step 1:

Triazole (12.4 g, 1.1 eq), pyrrolidine (13.35 g, 1.2 eq), and 1,4-cyclohexanedione monoethylene acetal (25.0 g, 160 mmol) were dissolved in toluene. The mixture was refluxed to remove water for 18 h and cooled to room temperature to give a solution of compound **A50-1** in toluene for later use.

### Step 2:

Bromobenzene (75 g, 3 eq) was dissolved in anhydrous THF, and n-butyllithium (2.5 M, 150 mL, 3.3 eq) was slowly added dropwise at -78 °C. After the dropwise addition was completed, the temperature was maintained at -78 °C for 1 h, and the prepared solution of A50-1 in toluene described above was added dropwise. After the dropwise addition was completed, the mixture was stirred for 1 h with the temperature maintained at -78 °C, stirred at room temperature for 12 h, and subjected to column chromatography to give compound **A50-2** (10.5 g).

### Step 3:

Compound **A50-2** (10.5 g, 6.5 mmol) was dissolved in ethanol, and concentrated hydrochloric acid (12 M, 1.6 mL) was added. The mixture was stirred at room temperature overnight and concentrated to dryness to remove the solvent. The pH was adjusted to 11-12 with saturated sodium bicarbonate. The mixture was extracted with DCM, dried, filtered, concentrated, and subjected to column chromatography to give compound **A50-3** (8.5 g).

### Step 4:

Compound **A50-3** (200 mg, 0.7 mmol) was dissolved in acetic acid. Pyridinium tribromide (1.1 g, 4 eq) was added. The mixture was heated at reflux for 4 h. Thiourea (420 mg, 8 mmol) was added. The mixture was heated at reflux for 18 h, concentrated to dryness to remove the solvent, adjusted to pH = 9-10 with a saturated sodium carbonate solution, extracted with dichloromethane, dried, filtered, concentrated, and subjected to column chromatography to give the title compound (200 mg).

LRMS-ESI (*m*/*z*): 300.33 [M + H]⁺. ¹H NMR (400 MHz, DMSO-*d*₆) δ 7.37 (m, 2H), 7.28 (t, *J* = 7.4 Hz, 2H), 7.20 (t, *J =* 6.9 Hz, 1H), 6.59 (s, 2H), 3.15 (d, *J =* 14.6 Hz, 1H), 2.89 (d, *J =* 15.7 Hz, 1H), 2.55 (br, 2H), 2.39 (m, 3H), 2.16 (m, 2H), 1.73 (m, 1H), 1.56 (br, 4H).

Chiral column separation gave isomers **A50-P1** (S configuration) and **A50-P2 (R** configuration):

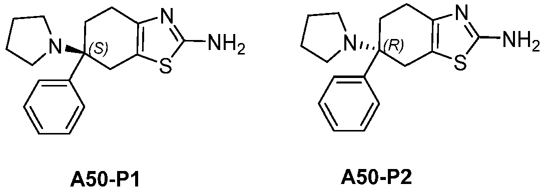

**A50-P1** (S configuration): HPLC purity: > 99%, retention time: 5.761 min. Chiral purity: > 95%, retention time: 7.617 min.

**A50-P2** (R configuration): HPLC purity: > 99%, retention time: 7.452 min. Chiral purity: > 95%, retention time: 7.623 min.

### Example 47 Preparation of 6-(3-chlorophenyl)-6-(pyrrolidin-1-yl)-4,5,6,7-tetrahydrobenzothiazol-2-amine (Compound A51)

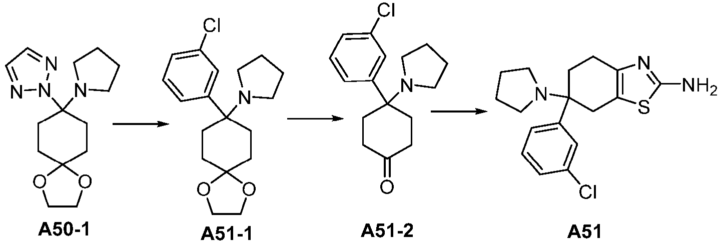

### Step 1:

m-Chloroiodobenzene (45 g, 1.2 eq) was dissolved in anhydrous THF, and n-butyllithium (2.5 M, 88 mL, 1.5 eq) was slowly added dropwise at -78 °C. After the dropwise addition was completed, the temperature was maintained at -78 °C for 1 h, and the newly prepared solution of A50-1 in toluene was added dropwise. After the dropwise addition was completed, the mixture was stirred for 1 h with the temperature maintained at -78 °C, stirred at room temperature for 12 h, and subjected to column chromatography to give compound **A51-1** (8.5 g).

### Step 2:

Compound **A51-1** (8.5 g, 6.5 mmol) was dissolved in ethanol, and concentrated hydrochloric acid (12 M, 1.6 mL) was added. The mixture was stirred at room temperature overnight and concentrated to dryness to remove the solvent. The pH was adjusted to 11-12 with a saturated sodium bicarbonate solution. The mixture was extracted with DCM, dried, filtered, concentrated, and subjected to column chromatography to give compound **A51-2** (5.2 g).

### Step 3:

Compound **A51-2** (300 mg, 9.4 mmol) was dissolved in acetic acid. Pyridinium tribromide (1.02 g, 4 eq) was added. The mixture was heated at reflux for 4 h to give a bromo intermediate. Dimethyl thiourea (560 mg, 8 eq) was added. The mixture was heated at reflux for 18 h, concentrated to dryness to remove the solvent, adjusted to pH = 9-10 with a saturated sodium carbonate solution, extracted with dichloromethane, dried, filtered, concentrated, and subjected to column chromatography to give compound **A51** (90 mg) as a white solid.

LRMS-ESI (*m*/*z*): 334.39 [M + H]⁺. ¹H NMR (400 MHz, CDCl₃) δ 7.38 (s, 1H), 7.29 (m, 3H), 6.62 (s, 2H), 3.13 (d, *J =* 16.2 Hz, 1H), 2.88 (d, *J =* 16.0 Hz, 1H), 2.55 (br, 2H), 2.37 (m, 3H), 2.15 (m, 2H), 1.70 (m, 1H), 1.58 (br, 4H).

### Example 48 Preparation of 5-phenyl-5-(pyrrolidin-1-yl)-4,5,6,7-tetrahydrobenzothiophen-2-carboxylic acid (Compound A52)

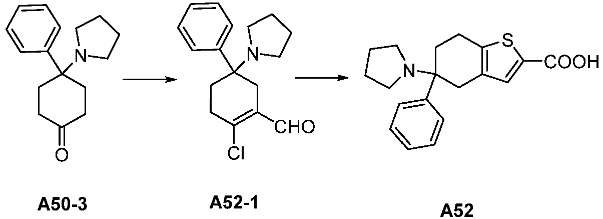

Step 1: phosphorus oxychloride (126 mg, 2 eq) was added dropwise to a solution of DMF (50 mg, 1.5 eq) in dichloromethane at 0-10 °C, and the mixture was stirred for 2 h under an ice bath. Compound **A50-3** (100 mg, 0.5 mmol) was dissolved in dichloromethane and added to the above reaction solution. The mixture was stirred at room temperature overnight. The mixture was slowly dropwise added to an aqueous sodium acetate solution. The mixture was subjected to liquid separation. The organic phase was dried, filtered, and concentrated to give compound **A52-1** as an oil.

Step 2: the oil **A52-1** described above was dissolved in ethanol, and ethyl thioglycolate (74 mg, 1.5 eq) was added. The mixture was stirred for 10 min. Sodium ethoxide (55 mg, 4 eq) was added under an ice bath. The mixture was reacted at room temperature overnight. Water was added, and the mixture was heated at reflux for 2 h. The mixture was concentrated to remove ethanol, adjusted to pH = 5-6 with 1 M hydrochloric acid solution, and extracted with dichloromethane. The organic phase was dried, concentrated, and subjected to column chromatography to give compound **A52** (55 mg) as a light yellow oil.

### Example 49 Preparation of 1-(5-phenyl-4,5,6,7-tetrahydrobenzothiophen-5-yl)pyrrolidine (Compound A53)

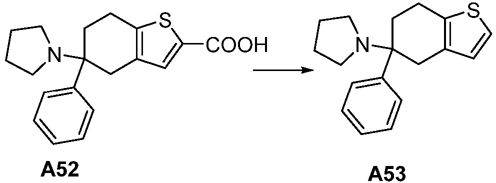

Compound **A52** (55 mg, 0.17 mmol) was dissolved in NMP, followed by the addition of cuprous oxide (24 mg, 1 eq) and quinoline (26 mg, 1.1 eq). The mixture was warmed to 140 °C for 12 h. After the starting materials were substantially depleted, the reaction solution was poured into water, extracted with dichloromethane, dried, filtered, and concentrated to give compound **A53** (20 mg).

### Example 50 Preparation of 6-phenyl-6-(pyrrolidin-1-yl)-4,5,6,7-tetrahydrobenzothiadiazole (Compound A54)

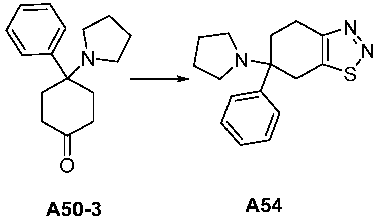

Compound **A50-3** (200 mg, 0.9 mmol) was dissolved in methanol, followed by the addition of semicarbazide (200 mg, 2 eq) and potassium acetate (200 mg, 2.1 eq). The mixture was heated at reflux for 6 h. The mixture was concentrated to dryness to remove the solvent, extracted with dichloromethane/water, dried, filtered, and concentrated to give a solid (300 mg). The above solid was dissolved in dichloromethane, and slowly added to thionyl chloride at -20 °C. The mixture was stirred at room temperature for 1 h. The reaction solution was poured into water, adjusted to pH = 12 with sodium hydroxide, extracted with dichloromethane, dried, filtered, concentrated, and subjected to column chromatography to give compound **A54** (100 mg).

¹H NMR (400 MHz, CDCl₃) δ 7.29 (m, 5H) 3.65 (d, *J* = 17.3 Hz, 1H), 3.41 (d, *J* = 17.1 Hz, 1H), 3.28 (dt, *J* = 16.4, 4.4 Hz, 1H), 2.64 (m, 3H), 2.55 (m, 2H), 2.43 (m, 2H), 1.68 (m, 4H). LRMS-ESI (*m*/*z*): 286.35 [M + H]⁺.

### Example 51 Preparation of 5-phenyl-5-(pyrrolidin-1-yl)-4,5,6,7-tetrahydrobenzo[d]isoxazole (Compound A55)

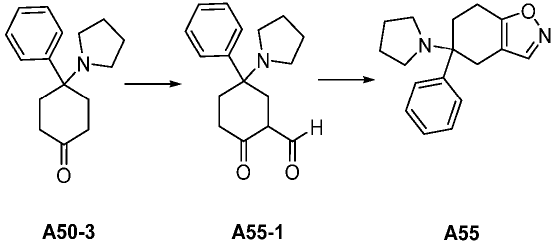

### Step 1:

Compound **A50-3** (300 mg, 0.7 mmol) was dissolved in toluene and dried over anhydrous magnesium sulfate for 2 days. The mixture was filtered for later use. Sodium *tert*-butoxide (246 mg, 2 eq) was added to toluene, and ethyl formate (138 mg, 1.5 eq) was added. The solution of **A50-3** in toluene was added at 0-10 °C, and the mixture was stirred at room temperature overnight. The reaction solution was extracted twice with toluene. The aqueous phase was retained and adjusted to pH = 6-7 with 1 M hydrochloric acid solution, and the mixture was extracted 3 times with dichloromethane, dried, filtered, concentrated, and subjected to column chromatography to give compound **A55-1** (800 mg).

### Step 2:

Compound **A55-1** (110 mg) was dissolved in acetic acid, followed by the addition of hydroxylamine hydrochloride (70 mg, 2 eq). The mixture was warmed to 80 °C and heated for 5 h. The mixture was concentrated to dryness to remove the solvent, adjusted to pH = 9-10 with saturated sodium carbonate, extracted with dichloromethane, dried, filtered, concentrated, and subjected to column chromatography to give compound **A55** (66 mg).

ESI-MS m/z 269.36 [M+H]⁺.

### Example 52 Preparation of N,N-dimethyl-6-phenyl-6-(pyrrolidin-1-yl)-4,5,6,7-tetrahydrobenzothiazol-2-amine (Compound A56)

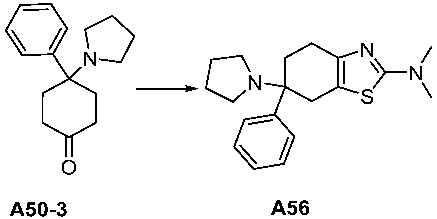

Compound **A50-3** (50 mg, 0.16 mmol) was dissolved in acetic acid. Pyridinium tribromide (300 g, 4 eq) was added. The mixture was heated at reflux for 4 h to give a bromo intermediate. Dimethyl thiourea (100 mg, 8 mmol) was added. The mixture was heated at reflux for 18 h. The mixture was concentrated to dryness to remove the solvent, adjusted to pH= 9-10 with an aqueous saturated sodium carbonate solution, extracted with dichloromethane, dried, filtered, concentrated, and subjected to column chromatography to give compound **A56** (10 mg).

LRMS-ESI (*m*/*z*): 328.45 [M + H]⁺.

¹H NMR (400 MHz, DMSO-*d*₆) δ 7.36 (m, 2H) 7.31 (t, *J* = 7.6 Hz, 2H), 7.21 (t, *J* = 7.1 Hz, 1H), 2.97 (d, *J* = 16.8 Hz, 1H), 2.94 (s, 3H), 2.83 (s, 3H), 2.78 (d, *J* = 16.8 Hz, 1H), 2.50 (m, 2H), 2.46-2.33 (m, 3H), 2.26 (m, 1H), 2.15 (m, 1H), 1.68 (m, 1H), 1.55 (m, 4H).

### Example 53 Preparation of 6-(3-chlorophenyl)-N,N-dimethyl-6-(pyrrolidin-1-yl)-4,5,6,7-tetrahydrobenzothiazol-2-ami ne (Compound A57) and Hydrochloride Thereof

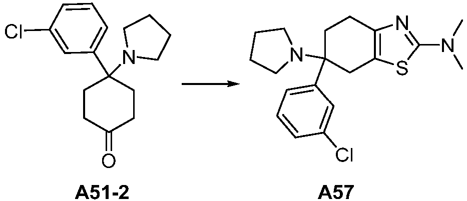

Compound **A51-2** (300 mg, 9.4 mmol) was dissolved in acetic acid. Pyridinium tribromide (1.1 g, 4 eq) was added. The mixture was heated at reflux for 4 h to give a bromo intermediate. Dimethyl thiourea (600 mg, 8 eq) was added. The mixture was heated at reflux for 18 h, concentrated to dryness to remove the solvent, adjusted to pH = 9-10 with an aqueous saturated sodium carbonate solution, extracted with dichloromethane, dried, filtered, concentrated, and subjected to column chromatography to give compound **A57** (150 mg) as an oil. The oil described above was dissolved in methyl *tert-*butyl ether and adjusted to pH = 2-3 with 12 M concentrated hydrochloric acid, and a large amount of solid precipitated. The mixture was filtered and dried to give compound **A57** hydrochloride (95 mg).

¹H NMR (400 MHz, DMSO-*d*₆) δ 12.28 (s, 1H), 10.32 (s, 1H), 7.93 (s, 1H), 7.77 (d, *J* = 7.6 Hz, 1H), 7.53 (m, 2H), 4.15 (d, *J =* 16.3 Hz, 1H), 3.57 (d, *J =* 17.9 Hz, 1H), 3.40 (s, 3H), 3.35 (m, 1H), 3.17 (m, 1H), 2.96 (s, 3H), 2.88 (m, 4H), 2.63 (td, *J* = 13.1, 5.9 Hz, 1H), 2.11 (m, 1H), 1.84 (m, 2H), 1.67 (m, 2H). LRMS-ESI (*m*/*z*): 362.41 [M + H]⁺.

### Example 54 Preparation of 6-(3-chlorophenyl)-6-(piperidin-1-yl)-4,5,6,7-tetrahydrobenzothiadiazole (Compound A58) and Maleate Salt Thereof

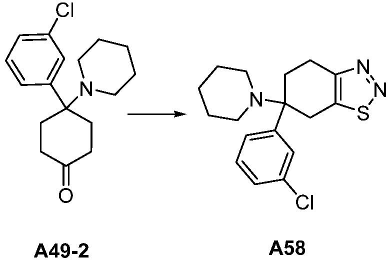

Compound **A49-2** (300 mg, 1.3 mmol) was dissolved in methanol, followed by the addition of semicarbazide (300 mg, 2 eq) and potassium acetate (300 mg, 2.1 eq). The mixture was heated at reflux for 6 h. The mixture was concentrated to dryness to remove the solvent, and extracted with dichloromethane/water. The organic phase was dried, filtered, and concentrated to give a solid (300 mg). The above solid was dissolved in dichloromethane, and slowly added to thionyl chloride at -20 °C. The mixture was stirred at room temperature for 1 h. The reaction solution was poured into water, adjusted to pH = 12 with a sodium hydroxide solution, extracted with dichloromethane, dried, filtered, and subjected to column chromatography to give basic compound **A58** (100 mg) as a solid. The above solid was dissolved in acetone, followed by the addition of maleic acid under stirring. A large amount of solid precipitated. The mixture was filtered and dried to give compound **A58** maleate (100 mg) as a white solid.

¹H NMR (400 MHz, DMSO-*d*₆) δ 7.58 (br, 1H), 7.41 (m, 3H), 6.16 (s, 2H), 3.54 (d, *J =* 17.3 Hz, 1H), 3.30 (d, *J =* 14.6 Hz, 1H), 2.99-2.37 (m, 8H), 1.58 (br, 4H), 1.39 (br, 2H). ESI-MS m/z 334.38 [M+H]⁺.

### Example 55 Preparation of 6-(3-chlorophenyl)-N⁶-methyl-4,5,6,7-tetrahydrobenzothiazole-2,6-diamine (Compound A59)

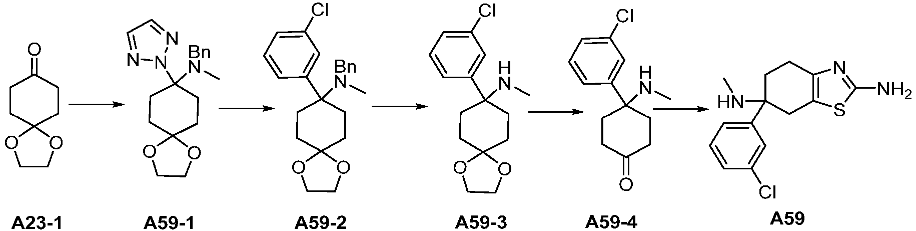

### Step 1:

Triazole (1.0 g, 1.2 eq), N-methylbenzylamine (1.8 mL, 1.2 eq), and 1,4-cyclohexanedione monoethylene acetal (2.0 g, 1.28 mmol) were dissolved in toluene. The mixture was refluxed to remove water for 18 h and cooled to room temperature to give a solution containing **A59-1** for later use.

### Step 2:

*m*-Chloroiodobenzene (6 g, 2 eq) was dissolved in anhydrous THF, and *n*-butyllithium (2.5 M, 6 mL, 1.1 eq) was slowly added dropwise at -78 °C. After the dropwise addition was completed, the temperature was maintained at -78 °C for 1 h, and the prepared **A59-1** described above was added dropwise. After the dropwise addition was completed, the mixture was stirred for 1 h with the temperature maintained, stirred at room temperature for 12 h, and subjected to column chromatography to give compound **A59-2** (600 mg).

### Step 3:

Compound **A59-2** (500 mg, 1.35 mmol) was dissolved in toluene, and DIAD (275 mg, 1.1 eq) was added. The mixture was heated at reflux for 18 h, and concentrated to remove toluene. A saturated aqueous ammonium chloride solution and ethanol were added, and the mixture was refluxed for 18 h, concentrated to dryness to remove ethanol, adjusted to pH 7-8 with ammonia water, extracted with EA, dried, concentrated, and subjected to column chromatography to give compound **A59-3** (120 mg).

### Step 4:

Compound **A59-3** (50 mg) was dissolved in ethanol, and 12 M hydrochloric acid (3 eq) was added. The mixture was stirred at room temperature for 12 h. The mixture was concentrated to dryness to remove the solvent, adjusted to pH = 9-10 with saturated sodium carbonate, extracted with dichloromethane, dried, filtered, concentrated, and subjected to column chromatography to give compound **A59-4** (20 mg).

### Step 5:

Compound **A59-4** (20 mg, 0.09 mmol) was dissolved in acetic acid. Pyridinium tribromide (135 mg, 0.36 mmol) was added. The mixture was heated at reflux for 4 h to give a bromo intermediate. Thiourea (54 mg, 0.72 mmol) was added. The mixture was heated at reflux for 18 h, concentrated to dryness to remove the solvent, adjusted to pH = 9-10 with an aqueous saturated sodium carbonate solution, extracted with dichloromethane, dried, filtered, concentrated, and subjected to column chromatography to give compound **A59** (5 mg).

¹H NMR (400 MHz, DMSO-*d*₆) δ 7.64 (s, 1H), 7.49 (d, *J* = 7.7 Hz, 1H), 7.36 (t, *J* = 7.8 Hz, 1H), 7.30 (d, *J* = 7.9 Hz, 1H), 6.72 (s, 2H), 3.10 (m, 3H), 3.00 (d, *J* = 15.8 Hz, 1H), 2.67 (d, *J* = 16.0 Hz, 1H), 2.50 (m, 2H), 2.32 (d, *J =* 10.7 Hz, 1H), 1.90 (dd, *J =* 10.6, 3.0 Hz, 1H). ESI-MS m/z 294.33 [M+H]⁺.

### Example 56 Preparation of 4-(2-chlorophenyl)-4,5,6,7-tetrahydrobenzothiazol-2,4-diamine (Compound A60) and Maleate Salt Thereof

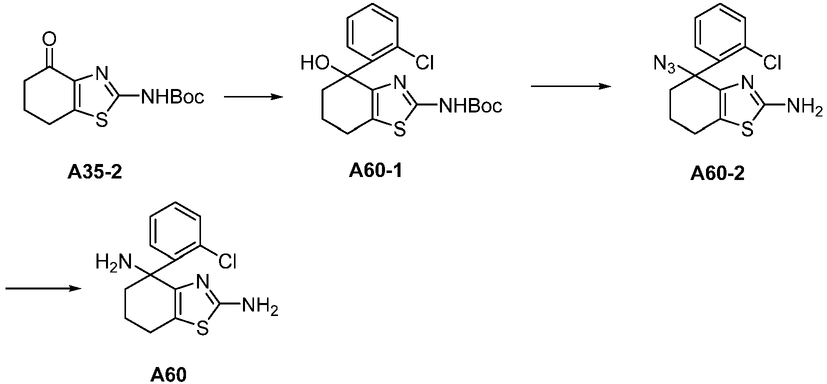

### Step 1:

o-Chlorobromobenzene (24.83 g, 130 mmol) was dissolved in dry THF (100 mL) and cooled to -78 °C, and n-BuLi (48 mL, 2.5 M) was added dropwise over about 1 h. After 1 h, a solution of A35-2 (5.340 g, 20 mmol) in THF (80 mL)was added dropwise. The mixture was stirred at -78 °C for 5 h. The reaction was quenched with a saturated aqueous ammonium chloride solution, and the mixture was extracted with EA. The organic phase was washed with a saturated aqueous sodium chloride solution, dried, concentrated, and subjected to column chromatography to give compound **A60-1** (2.8 g) as a yellow foamy solid.

### Step 2:

Compound **A60-1** (2.3 g, 6.0 mmol) was dissolved in DCM (100 mL), and sodium azide (7.8 g, 120 mmol) was added. TFA (80 mL) was added dropwise under an ice bath within 1 h. After the addition was completed, the mixture was reacted under an ice bath for another 1 h and at room temperature overnight. Ice water (100 mL) was added, and the pH was adjusted to 9 with 5 M ammonia water. The mixture was extracted with DCM, and the organic phase was washed with saturated brine, dried over anhydrous sodium sulfate, and subjected to column chromatography to give compound **A60-2** (1.050 g) as a white foamy solid.

### Step 3:

Compound **A60-2** (1.050 g) was dissolved in THF (20 mL), and cobalt chloride hexahydrate (167 mg) was added, followed by dropwise addition of an aqueous solution (20 mL) of sodium borohydride (260 mg). The mixture was reacted at room temperature for 30 min. The reaction was quenched with 1 M hydrochloric acid. The mixture was adjusted to pH 12 and extracted with DCM, and the organic phases were combined, dried over anhydrous sodium sulfate, concentrated, and subjected to column chromatography to give basic compound **A60** (light brown solid), which was converted to the maleate salt according to the method described in Example 54 to give compound **A60** maleate (682 mg).

¹H NMR (400 MHz, DMSO-*d*₆) δ 8.89 (br, 2H), 7.44 (m, 2H), 7.38 (m, 1H), 7.30 (d, *J =* 7.5 Hz, 1H), 6.92 (s, 2H), 6.02 (s, 2H), 2.71 (m, 1H), 2.61 (m, 1H), 2.43 (m, 1H), 2.04 (m, 2H), 1.80 (m, 1H). ESI-MS m/z 308.11 [M+H]⁺.

### Example 57 Preparation of N-(2-amino-4-(2-chlorophenyl)-4,5,6,7-tetrahydrobenzothiazol-4-yl)formamide (Compound A61)

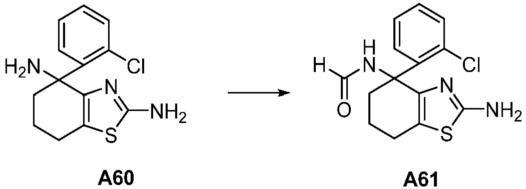

Compound **A60** (260 mg) was dissolved in ethyl formate (12 mL) and heated at 90 °C for 18 h. The reaction solution was concentrated to dryness and subjected to column chromatography to give compound **A61.**

### Example 58 Preparation of 4-(2-chlorophenyl)-N⁴-methyl-4,5,6,7-tetrahydrobenzothiazol-2,4-diamine (Compound A62) and Maleate Salt Thereof

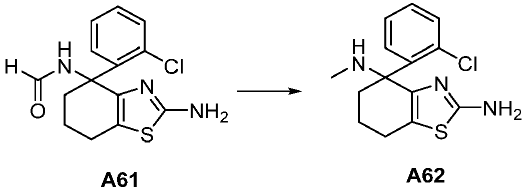

Compound **A61** was dissolved in dry THF (20 mL), and borane-dimethyl sulfide solution (3.8 mL, 2 M) was added dropwise. The mixture was stirred at room temperature for 20 min and then refluxed for 5 h. After cooling to room temperature, methanol (15 mL) was cautiously added to quench the reaction. The mixture was concentrated to remove the solvent. Methanol (30 mL) was added, and the mixture was refluxed overnight, concentrated, and subjected to column chromatography to give basic compound **A62** (light brown solid), which was converted to the maleate salt according to the method described in Example 54 to give compound **A62** maleate.

¹H NMR (400 MHz, MeOD) δ 7.58 (d, *J =* 7.2 Hz, 1H), 7.46 (t, *J* = 7.0 Hz, 1H), 7.36 (t, *J =* 7.7 Hz, 1H), 6.92 (d, *J =* 7.8 Hz, 1H), 6.31 (s, 3H), 2.94 (m, 1H), 2.85 (s, 3H), 2.71 (m, 2H), 2.20 (t, *J=* 11.9 Hz, 1H), 1.97 (m, 1H), 1.46 (m, 1H). ESI-MS m/z 294.05 [M+H]⁺.

### Example 59 Preparation of 4-(2-chlorophenyl)-N⁴-ethyl-4,5,6,7-tetrahydrobenzothiazole-2,4-diamine (Compound A63)

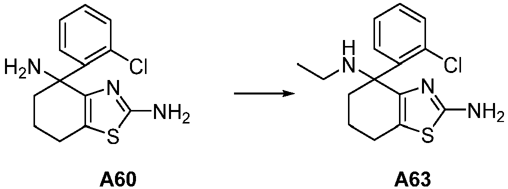

Compound **A60** (195 mg, 0.7 mmol) was dissolved in MeOH (5 mL), followed by addition of acetic acid (84 mg, 1.4 mmol) and acetaldehyde (168 µL, 5 M in THF). The mixture was stirred at room temperature for 1 h, followed by addition of sodium cyanoborohydride (91 mg). After the mixture was reacted at room temperature for 24 h, acetaldehyde (42 µL) and sodium cyanoborohydride (23 mg) were further added, and the mixture was reacted for another 20 h. The pH was adjusted to 12 with a 2 M sodium hydroxide solution. The mixture was extracted with DCM, dried, concentrated, and subjected to column chromatography to give compound **A63** (72 mg) as a light yellow oil.

¹H NMR (400 MHz, DMSO-*d*₆) δ 7.63 (s, 1H), 7.37-7.12 (m, 4H), 6.61 (br, 2H), 2.58 (m, 3H), 2.21 (m, 1H), 2.05 (m, 2H), 1.67 (m, 2H), 1.01 (m, 3H). ESI-MS m/z 308.11 [M+H]⁺.

### Example 60 Preparation of 4-(2-chlorophenyl)-N⁴-propyl-4,5,6,7-tetrahydrobenzothiazole-2,4-diamine (Compound A64)

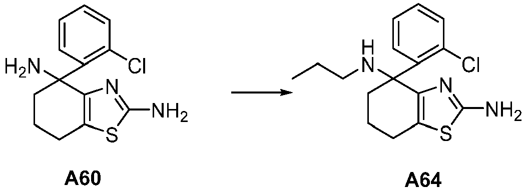

Compound A60 (20 mg, 0.07 mmol) was dissolved in methanol (1 mL), followed by the addition of acetic acid (9 mg, 0.14 mmol) and propionaldehyde (5 mg, 1.2 eq). After 5 min, sodium cyanoborohydride (9 mg, 2 eq) was added. The mixture was stirred for 5 h. Water (10 mL) was added, and the pH was adjusted to 10 with a 1 M sodium hydroxide solution. The mixture was extracted with DCM, dried, concentrated, and subjected to column chromatography to give compound **A64** (13 mg) as a white solid.

¹H NMR (400 MHz, MeOD) δ 7.49 (d, *J* = 7.8 Hz, 1H), 7.36 (t, *J* = 7.2 Hz, 1H), 7.30 (t, *J* = 7.4 Hz, 1H), 7.06 (s, 1H), 3.37 (m, 1H), 2.79 (m, 1H), 2.74-2.56 (m, 3H), 2.09 (m, 1H), 1.97 (m, 1H), 1.68 (m, 2H), 1.49 (m, 1H), 0.97 (t, *J* = 7.4 Hz, 3H).

### Example 61 Preparation of 5-phenyl-4,5,6,7-tetrahydro-1H-indazol-5-amine (Compound A65)

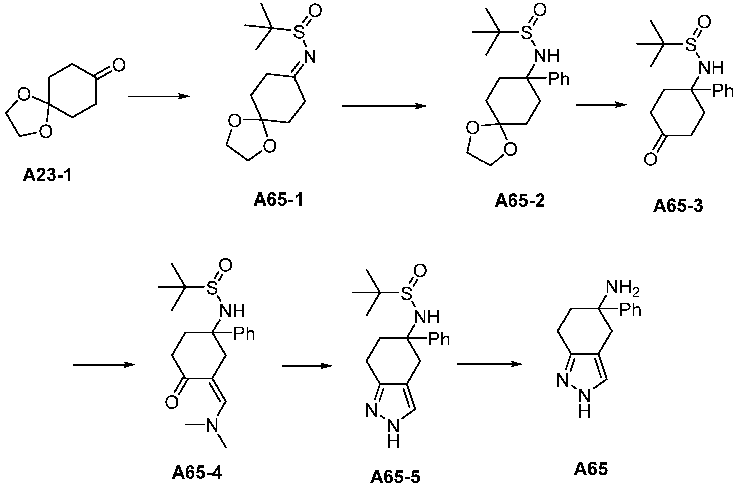

### Step 1:

Compound A23-1 (1 eq, 5 g), tert-butylsulfinamide (1.5 eq, 5.82 g), and tetraethyl titanate (3 eq, 21 g) were dissolved in extra dry tetrahydrofuran (100 mL), and the mixture was stirred at room temperature overnight under nitrogen atmosphere. The reaction was quenched with a saturated aqueous sodium bicarbonate solution. After filtration, the filtrate was extracted 3 times with ethyl acetate, dried, concentrated, and subjected to column chromatography to give A65-1 (7.06 g, yield: 85%).

### Step 2:

Phenylmagnesium chloride (3 eq, 20 mL) was added to a 250 mL three-necked flask under nitrogen atmosphere. A solution of **A65-1** in tetrahydrofuran was added slowly under an ice bath. After the addition was completed, the mixture was stirred for 20 min, removed from the ice bath, and stirred at room temperature. The reaction was completed as monitored by TLC. The reaction was quenched by addition of a saturated aqueous ammonium chloride solution. After filtration, the mixture was extracted three times with EA, dried, concentrated, and subjected to column chromatography to give **A65-2** (3.01 g) as a white solid.

### Step 3:

**A65-2** (1 eq, 2.5 g) was dissolved in 40 mL of a mixed solvent of acetone and water (acetone:water = 3:1). The mixture was stirred with PTSA (0.5 eq, 0.705 g). The reaction was completed as monitored by TLC. The reaction was quenched by addition of a saturated aqueous sodium bicarbonate solution. The mixture was extracted three times with EA, dried, and concentrated to give **A65-3** (1.675 g, yield: 77.15%) as a brownish red liquid.

### Step 4:

**A65-3** (1 eq, 1.675 g) and DMF-DMA (5 eq, 3.79 mL) were heated at 110 °C at reflux overnight. After the reaction was completed, the **A65-4** solution was obtained, which was directly used in the next step without treatment.

### Step 5:

The **A65-4** solution from the previous step and hydrazine hydrate (5 eq, 1.68 g) were dissolved in methanol and heated at 80 °C overnight. The mixture was concentrated to dryness to give **A65-5** (2 g) as a brownish red liquid.

### Step 6:

**A65-5** (1 eq, 500 mg) was dissolved in 2 mL of anhydrous ethanol. The mixture was stirred for ten min, and stirred with 2 M hydrochloric acid solution for 2 h. The reaction was quenched with a saturated aqueous sodium bicarbonate solution. The mixture was adjusted to pH 8-9, extracted three times with EA, dried, concentrated, and subjected to column chromatography on an amino column to give compound **A65** (340 mg) as a dark red compound.

¹H NMR (500 MHz, Chloroform-d) δ 7.58-7.52 (m, 2H), 7.40 (s, 1H), 7.37 (t, *J =* 7.6 Hz, 2H), 7.26 (s, 1H), 3.67 (d, *J =* 2.0 Hz, 2H), 3.17 (d, *J =* 15.6 Hz, 1H), 2.88 (dt, *J =* 15.7, 7.9 Hz, 1H), 2.76 (d, *J =* 15.6 Hz, 1H), 2.69-2.58 (m, 1H), 2.31 (ddd, *J =* 14.2, 8.8, 6.0 Hz, 1H), 2.06 (dq, *J* = 13.6, 6.8, 5.8 Hz, 1H). ESI-MS m/z 197.34 [M+H]⁺.

### Example 62 Preparation of N-(2-amino-6-phenyl-4,5,6,7-tetrahydrobenzothiazol-6-yl)cyclopropaneformamide (Compound A66)

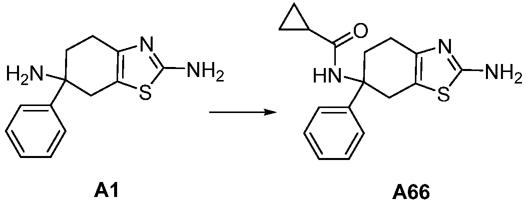

**A1** (15 mg, 1 eq) was dissolved in 2 mL of THF, and triethylamine (9 mg, 1.1 eq) was added. The mixture was cooled to 0 °C under an ice-water bath, followed by slow addition of cyclopropanecarboxylic anhydride (14 mg, 1.1 eq). The mixture was reacted at room temperature for 14 h. The reaction was quenched with a saturated aqueous sodium bicarbonate solution. The mixture was extracted 3 times with DCM, and the organic phases were combined, dried, concentrated, and purified by column chromatography to give compound **A66** (18 mg).

¹H NMR (400 MHz, DMSO-*d*₆) δ 8.21 (s, 1H), 7.34 (d, *J =* 7.6 Hz, 2H), 7.28 (t, *J =* 7.6 Hz, 2H), 7.18 (t, *J =* 7.1 Hz, 1H), 6.63 (s, 2H), 3.04 (d, *J =* 16.5 Hz, 1H), 2.93 (d, *J =* 16.1 Hz, 1H), 2.62 (m, 1H), 2.35 (m, 1H), 2.15 (m, 2H), 1.69 (m, 1H), 0.57 (m, 4H). ESI-MS m/z 314.22 [M+H]⁺.

### Example 63 Preparation of 6-phenyl-N⁶-(cyclopropylmethyl)-4,5,6,7-tetrahydrobenzothiazole-2,6-diamine (Compound A67) Hydrochloride

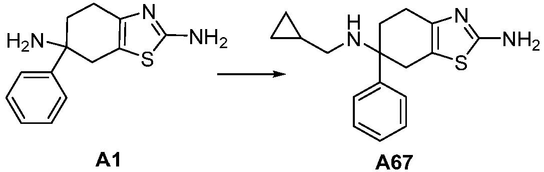

**A1** (150 mg, 1 eq) was dissolved in 2 mL of DCM, and cyclopropanecarboxaldehyde (43 mg, 1 eq) was added. The mixture was reacted at room temperature for 14 h, followed by slow addition of sodium triacetoxyborohydride (130 mg, 1 eq). The mixture was reacted at room temperature for 1 h. The reaction was quenched with water. The mixture was extracted 3 times with DCM, and the organic phases were combined, dried, concentrated, and purified by column chromatography. The residue **A67** was dissolved in methanol, and a hydrogen chloride/methanol solution was added to form the hydrochloride, which was concentrated to give compound **A67** in the form of hydrochloride (105 mg).

¹H NMR (400 MHz, DMSO-*d*₆) δ 10.42 (s, 1H), 9.59 (s, 1H), 9.16 (br, 2H), 7.59 (m, 2H), 7.44 (m, 3H), 3.60 (m, 2H), 2.78-2.53 (m, 4H), 2.13 (m, 1H), 1.76 (m, 1H), 0.98 (m, 1H), 0.50 (d, *J* = 8.0 Hz, 2H), 0.24 (m, 1H), 0.064 (m, 1H).

ESI-MS m/z 300.18 [M+H]⁺.

### Example 64 Preparation of N-benzyl-N-methyl-5-phenyl-4,5,6,7-tetrahydro-1H-indazol-5-amine (Compound A68)

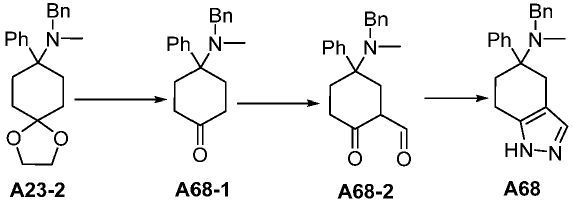

### Step 1:

Compound **A23-2** (1 eq) was dissolved in ethanol, and concentrated hydrochloric acid (12 M, 3 eq) was added. The mixture was stirred at room temperature overnight, dried over sodium sulfate, and concentrated to remove the solvent. The pH was adjusted to 11-12 with a saturated aqueous sodium bicarbonate solution. The mixture was extracted with DCM, dried, filtered, concentrated, and subjected to column chromatography to give **A68-1** (yield: 83%).

### Step 2:

Compound **A68-1** was dissolved in toluene and dried over anhydrous magnesium sulfate. The mixture was filtered for later use. Toluene and sodium *tert*-butoxide (2 eq) were added to another reaction flask, and ethyl formate (1.5 eq) was added. The solution of compound **A68-1** described above was added at 0-10 °C, and the mixture was stirred at room temperature overnight. The reaction solution was extracted twice with toluene. The aqueous phase was retained and adjusted to pH = 6-7 with 1 M hydrochloric acid solution, and the mixture was extracted 3 times with dichloromethane, dried, concentrated, and subjected to column chromatography to give compound **A68-2** (yield: 93%).

### Step 3:

Compound **A68-2** (1 eq, 90 mg) was dissolved in ethanol, and 85% hydrazine hydrate (3 eq, 97 mg) was added. The mixture was warmed to 85 °C and heated for 5 h. The mixture was dried over anhydrous sodium sulfate and concentrated to dryness to remove the solvent, and the residue was subjected to column chromatography to give compound **A68** (yield: 98%) as a white solid.

¹H NMR (500 MHz, Chloroform-d) δ 7.63 - 7.51 (m, 2H), 7.47 (s, 1H), 7.31 (d, *J* = 8.1 Hz, 3H), 7.27 (d, *J =* 2.5 Hz, 3H), 7.23 (qd, *J =* 6.9, 3.7 Hz, 2H), 3.57 (d, *J =* 13.3 Hz, 1H), 3.43 (d, *J = 13.3* Hz, 1H), 3.16 (s, 2H), 2.70 (ddd, *J =* 16.0, 4.9, 3.0 Hz, 1H), 2.43 (td, *J =* 12.2, 5.0 Hz, 1H), 2.24 (s, 3H), 2.21 (s, 1H), 1.97 (ddd, *J* = 16.4, 11.9, 4.8 Hz, 1H). ESI-MS m/z 318.18 [M+H]⁺.

### Example 65 Preparation of N-methyl-5-phenyl-4,5,6,7-tetrahydro-1H-indazol-5-amine (Compound A69)

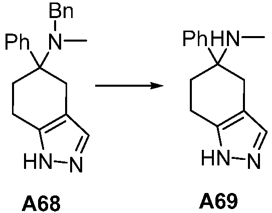

Compound **A68** (1 eq, 25 mg) was dissolved in nitromethane, and Oxone oxidant (3 eq, 81 mg) was added at room temperature. The mixture was stirred at 30 °C for 24 h. The reaction was quenched with sodium thiosulfate. The mixture was extracted with EA three times, washed twice with saturated brine, dried, concentrated, and subjected to column chromatography to give compound **A69.**

¹H NMR (500 MHz, Chloroform-d) δ 7.50 - 7.40 (m, 2H), 7.35 (s, 1H), 7.32 (dd, *J =* 8.4, 7.0 Hz, 2H), 7.25 - 7.21 (m, 1H), 3.13 (d, *J =* 15.5 Hz, 1H), 2.82 (d, *J =* 15.5 Hz, 1H), 2.73 (ddd, *J* = 16.3, 7.4, 5.6 Hz, 1H), 2.46 (dt, *J =* 16.9, 6.6 Hz, 1H), 2.30 (q, *J =* 6.7 Hz, 2H), 2.13 (s, 3H). ESI-MS m/z 228.11 [M+H]⁺.

### Example 66 Preparation of 5-phenyl-5-(piperidin-1-yl)-4,5,6,7-tetrahydro-1H-indazole (Compound A70)

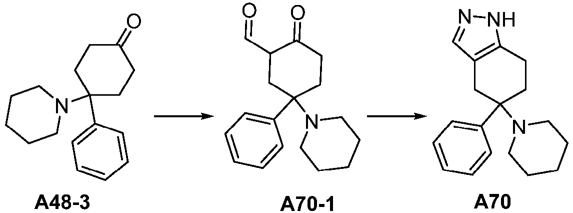

**Step 1:** compound **A48-3** (1 eq, 0.7 g) was dissolved in toluene and dried over anhydrous magnesium sulfate. The mixture was filtered for later use. Toluene and sodium *tert*-butoxide (2 eq, 523 mg) were added to another reaction flask, and ethyl formate (1.5 eq, 303 mg) was added. The solution of compound **A48-3** in toluene described above was added at 0-10 °C, and the mixture was stirred at room temperature overnight. The reaction solution was extracted twice with toluene. The aqueous phase was retained and adjusted to pH = 6-7 with 1 M hydrochloric acid solution, and the mixture was extracted 3 times with dichloromethane, dried, concentrated, and subjected to column chromatography to give compound **A70-1** (0.72 g, yield: 93%).

**Step 2:** compound **A70-1** (1 eq, 90 mg) was dissolved in ethanol, and 85% hydrazine hydrate (3 eq, 97 mg) was added. The mixture was warmed to 85 °C and heated for 5 h. The mixture was dried over anhydrous sodium sulfate and concentrated to dryness to remove the solvent, and the residue was subjected to column chromatography to give compound A70 (87 mg, yield: 98%) as a white solid.

¹H NMR (500 MHz, Chloroform-d) δ 7.46 - 7.32 (m, 3H), 7.19 (dt, *J =* 14.0, 7.0 Hz, 3H), 3.37 - 2.83 (m, 2H), 2.61 (d, *J* = 15.9 Hz, 3H), 2.34 (s, 3H), 2.10 - 1.75 (m, 2H), 1.68 - 1.21 (m, 6H). ESI-MS m/z 282.31 [M+H]⁺.

Chiral column separation gave isomers **A70-P1** (S configuration) and **A70-P2** (R configuration):

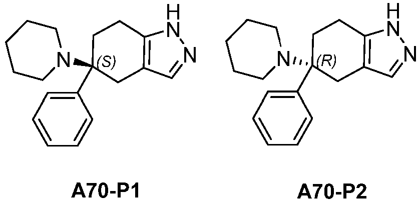

**A70-P1** (S configuration): HPLC purity: > 95%, retention time: 10.024 min. Chiral purity: > 99%, retention time: 6.319 min.

**A70-P2** (R configuration): HPLC purity: > 95%, retention time: 10.009 min. Chiral purity: > 95%, retention time: 7.971 min.

### Example 67 Preparation of 6-phenyl-6-(piperidin-1-yl)-4,5,6,7-tetrahydrobenzothiazole (Compound A71)

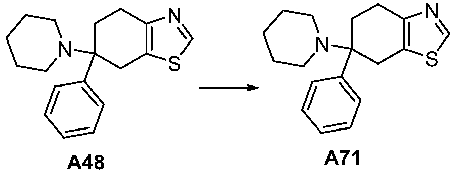

Basic A48 (160 mg, 1.0 eq) was dissolved in THF (2 mL), and *tert-*butyl nitrite (65 mg, 1.1 eq) was added. The mixture was reacted at 60 °C for 5 h. The reaction was quenched with a saturated sodium bicarbonate solution, and the mixture was extracted with ethyl acetate, concentrated, and purified by column chromatography to give compound A71 (25 mg) as a colorless oil. ¹H NMR (500 MHz, Chloroform-d) δ 8.57 (s, 1H), 7.33 (m, 2H), 7.22 (m, 3H), 3.27 (m, 2H), 2.84 (d, *J =* 15.4 Hz, 1H), 2.60 (m, 2H), 2.36 (m, 3H), 2.05 (m, 2H), 1.80 - 1.33 (m, 6H). ESI-MS m/z 299.20 [M+H]⁺.

### Example 68 Preparation of 6-((methylamino)methyl)-6-phenyl-4,5,6,7-tetrahydrobenzo [d]thiazol-2-amine (Compound A252)

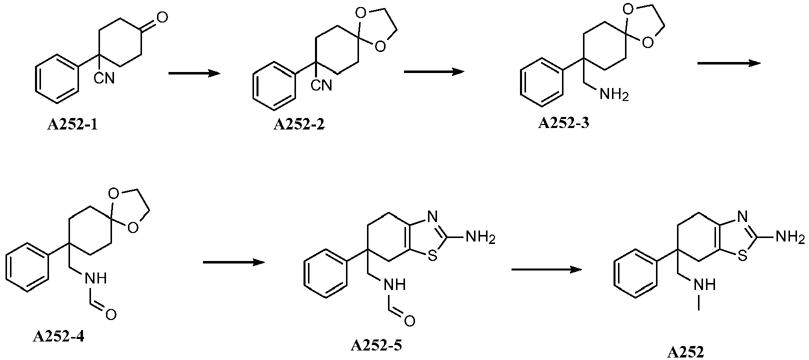

**Step** 1: A252-1 (5.5 g, 1.0 eq), ethylene glycol (1.7 g, 1.05 eq), and toluene (100 mL) were added to a 250 mL three-necked flask, and under nitrogen atmosphere, the mixture was warmed to 120 °C and reacted for 2-3 h. The reaction solution was cooled to room temperature and washed with 100 mL of saturated sodium bicarbonate. The organic phase was collected, washed with 100 mL of a saturated sodium chloride solution, dried over anhydrous sodium sulfate, and concentrated under reduced pressure to give A252-2 (5.2 g) as an oil.

**Step 2:** the above oil A252-2 was added to a 250 mL three-necked flask and stirred with 50 mL of tetrahydrofuran to give a clear solution. The mixture was purged with nitrogen and cooled to about 0 °C under an ice-water bath, and lithium aluminum hydride (1.5 g, 1.5 eq) was added in batches. After the addition was completed, the mixture was warmed to about 70 °C and stirred for 2 h. The reaction solution was cooled to 0 °C under an ice-water bath and quenched with a saturated ammonium chloride solution. The mixture was extracted with 100 mL of ethyl acetate, and the organic phase was washed with 50 mL of a saturated sodium chloride solution, dried over anhydrous sodium sulfate, and concentrated under reduced pressure to give A252-3 (5.5 g) as an off-white solid.

**Step 3:** A252-3 (2.2 g) was added to a 100 mL three-necked flask and stirred with 20 mL of ethyl formate to give a clear solution. The mixture was warmed to 70 °C and reacted for about 2 h under nitrogen atmosphere. The mixture was concentrated under reduced pressure to give A252-4 (2 g) as an off-white solid.

**Step 4:** A252-4 (650 mg) was added to a 50 mL three-necked flask and stirred with 10 mL of dichloromethane to give a clear solution. Pyridinium tribromide (950 mg, 1.1 eq) was added, and the mixture was stirred at room temperature for 4-5 h. The reaction was quenched with 5 mL of a saturated sodium thiosulfate solution. The mixture was extracted with 20 mL of dichloromethane, and the organic phases were combined, dried, and concentrated to give an off-white solid (450 mg). The mixture was stirred with 10 mL of dioxane to give a clear solution. Thiourea (615 mg, 3.0 eq) and DIPEA (700 mg, 2.0 eq) were added, and the mixture was warmed to 80-90 °C and stirred overnight. The reaction solution was cooled to room temperature, and separately extracted with 10 mL of water and dichloromethane. The organic phase was dried, concentrated, and subjected to column chromatography to give A252-5 (350 mg) as an off-white solid.

**Step 5:** A252-5 (200 mg) was added to a 50 mL three-necked flask and stirred with 10 mL of tetrahydrofuran. A solution (7 mL, 10 eq) of borane in tetrahydrofuran was added under nitrogen atmosphere, and the mixture was warmed to 60 °C and reacted for 2 h with the temperature maintained. The mixture was cooled to about 0 °C, and 5 mL of methanol was slowly added dropwise to quench the reaction. The mixture was concentrated to remove the solvent, and water (10 mL) and methanol (10 mL) were added. The mixture was warmed to 70 °C, stirred for 1-2 h, cooled to room temperature, and extracted with dichloromethane (20 mL). The organic phase was dried over anhydrous sodium sulfate, concentrated, and subjected to column chromatography to give an off-white solid (100 mg). ¹H NMR (400 MHz, DMSO-*d*₆) δ 7.43 (s, 1H), 7.37-7.26 (m, 3H), 7.24-7.17 (m, 1H), 6.63 (s, 2H), 5.48 (s, 1H), 3.44 (d, *J =* 16.2 Hz, 1H), 3.05-2.94 (m, 2H), 2.88 (d, *J =* 15.7 Hz, 1H), 2.83-2.78 (m, 1H), 2.20-2.06 (m, 2H), 1.87-1.79 (m, 1H), 1.70 (d, *J =* 5.2 Hz, 3H). ESI-MS m/z 274.18 [M+H]⁺

The compounds shown in the following table, **A8**, **A10**, **A11**, **A26**, and **A72-A396**, were prepared by the same methods as in the above examples, except that starting materials and intermediates corresponding to the final products were used.

| Compound | Structure | ESI-MS/Proton nuclear magnetic resonance |
|---|---|---|
| **A8** | 6-amino-6-phenyl-4,5,6,7-tetrahydrobenz othiazol-2-ol 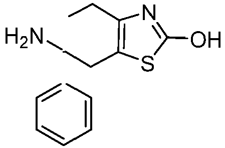 | ESI-MS m/z 230.1 [M-NH₂]⁺. |
| | | ¹H NMR (400 MHz, DMSO-*d*₆) δ 7.52 (d, *J* = 7.6 Hz, 2H), 7.32 (t, *J* = 7.6 Hz, 2H), 7.22 (t, *J* = 7.3 Hz, 1H), 2.83 (d, *J* = 16.1 Hz, 1H), 2.48 - 2.36 (m, 2H), 2.15-2.09 (m, 1H), 2.05-1.98 (m, 1H), 1.84-1.78 (m, 1H). |
| **A8-P1** | (*R*)-6-amino-6-phenyl-4,5,6,7-tetrahydrob enzothiazol-2-ol 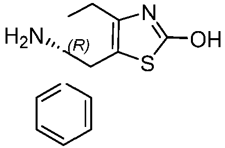 | **A8-P1** (R configuration): HPLC purity: > 98%, retention time: 9.035 min. Chiral purity: > 99%, retention time: 5.719 min. |
| **A8-P2** | (*S*)-6-amino-6-phenyl-4,5,6,7-tetrahydrob enzothiazol-2-ol 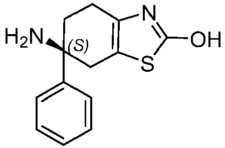 | **A8-P1** (S configuration): HPLC purity: > 98%, retention time: 9.027 min. Chiral purity: > 99%, retention time: 6.954 min. |
| **A10** | 6-(3-fluorophenyl)-4,5,6,7-tetrahydrobenz othiazole-2,6-diamine 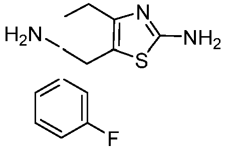 | ESI-MS m/z 264.50 [M+H]⁺. |
| | | ¹H NMR (500 MHz, Methanol-*d*₄) δ 7.45 - 7.37 (m, 1H), 7.36 - 7.33 (m, 1H), 7.31 - 7.26 (m, 1H), 7.09 - 7.01 (m, 1H), 3.26 (d, *J* = 16.2 Hz, 1H), 2.87 (d, *J* = 16.2 Hz, 1H), 2.68 - 2.56 (m, 1H), 2.40 - 2.22 (m, 2H), 2.18 - 2.08 (m, 1H). |
| **A11** | 6-(4-fluorophenyl)-4,5,6,7-tetrahydrobenz othiazole-2,6-diamine 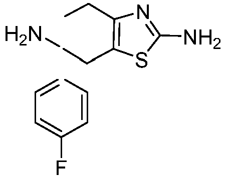 | ESI-MS m/z 264.57 [M+H]⁺. |
| | | ¹H NMR (400 MHz, Methanol-*d*₄) δ 7.64-7.51 (m, 2H), 7.33-7.09 (m, 2H), 3.51 - 3.41 (m, 1H), 3.12 - 3.03 (m, 1H), 2.78 - 2.66 (m, 1H), 2.56 - 2.43 (m, 1H), 2.41 - 2.27 (m, 2H). |
| **A26** | 7-phenyl-4,5,6,7-tetrahydrobenzothiophen 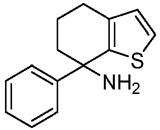 -7-amine | ESI-MS m/z 230.14 [M+H]⁺. |
| **A72** | 6-phenyl-6-(pyrrol-1-yl)-4,5,6,7-tetrahydr obenzothiazole 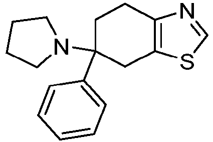 | ESI-MS m/z 285.42 [M+H]⁺. |
| **A73** | 5-phenyl-5-(pyrrolidin-1-yl)-4,5,6,7-tetra hydrobenzothiazol-2-amine 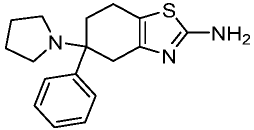 | ESI-MS m/z 285.42 [M+H]⁺. |
| **A74** | 5-phenyl-5-(piperidin-1-yl)-4,5,6,7-tetrah ydrobenzothiazol-2-amine 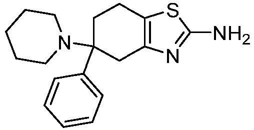 | ESI-MS m/z 314.46 [M+H]⁺. |
| **A75** | 6-phenyl-5-(pyrrol-1-yl)-4,5,6,7-tetrahydr obenzothiazole 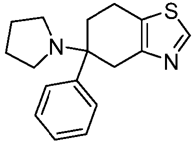 | ESI-MS m/z 285.42 [M+H]⁺. |
| **A76** | 6-phenyl-5-(piperidin-1-yl)-4,5,6,7-tetrah ydrobenzothiazole 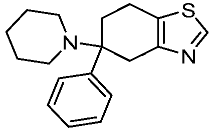 | ESI-MS m/z 299.45 [M+H]⁺. |
| **A77** | 6-phenyl-6-(piperidin-1-yl)-4,5,6,7-tetrah ydrobenzothiadiazole 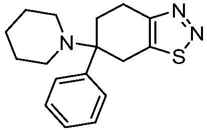 | ESI-MS m/z 300.44 [M+H]⁺. |
| **A78** | 6-phenyl-6-(piperidin-1-yl)-4,5,6,7-tetrah ydrobenzo[d]isoxazole 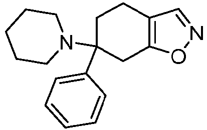 | ESI-MS m/z 283.39 [M+H]⁺. |
| **A79** | 5-phenyl-5-(piperidin-1-yl)-4,5,6,7-tetrah ydrobenzothiophen-2-carboxylic acid 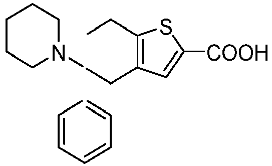 | ESI-MS m/z 342.47 [M+H]⁺. |
| **A80** | 1-(5-phenyl-4,5,6,7-tetrahydrobenzothiop hen-5-yl)piperidine 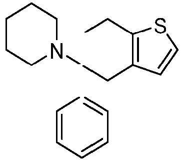 | ESI-MS m/z 298.46 [M+H]⁺. |
| **A81** | 6-(2-fluorophenyl)-*N*⁶-methyl-4,5,6,7-tetr ahydrobenzothiazole-2,6-diamine 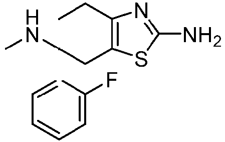 | ESI-MS m/z 278.16 [M+H]⁺. |
| | | ¹H NMR (400 MHz, DMSO-*d*₆) δ 7.32 (q, *J =* 6.6 Hz, 1H), 7.28 - 7.08 (m, 3H), 6.65 (s, 2H), 3.25 (d, *J* = 16.7 Hz, 1H), 2.74 (d, *J =* 16.1 Hz, 1H), 2.48-2.43 (m, 1H), 2.39 - 2.28 (m, 1H), 2.12-2.06 (m, 4H), 2.01 - 1.92 (m, 1H). |
| **A82** | 6-(3-fluorophenyl)-*N*⁶-methyl-4,5,6,7-tetr ahydrobenzothiazole-2,6-diamine 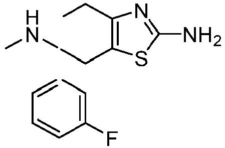 | ESI-MS m/z 278.36 [M+H]⁺. |
| | | ¹H NMR (400 MHz, Methanol-*d*₄) δ 7.48 - 7.39 (m, 1H), 7.31 - 7.21 (m, 2H), 7.13 - 7.05 (m, 1H), 3.42 (d, *J =* 16.1 Hz, 1H), 2.91 (d, *J =* 16.1 Hz, 1H), 2.62 - 2.51 (m, 1H), 2.40 - 2.24 (m, 2H), 2.21 (s, 3H), 2.12 - 2.03 (m, 1H). |
| **A83** | 6-(4-fluorophenyl)-*N*⁶-methyl-4,5,6,7-tetr ahydrobenzothiazole-2,6-diamine 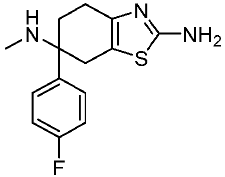 | ESI-MS m/z 278.36 [M+H]⁺. |
| | | ¹H NMR (400 MHz, Methanol-*d*₄) δ 7.54 - 7.44 (m, 2H), 7.17 - 7.02 (m, 2H), 3.44 (d, *J* = 16.0 Hz, 1H), 2.91 (d, *J* = 16.0 Hz, 1H), 2.58 - 2.48 (m, 1H), 2.37 - 2.28 (m, 2H), 2.21 (s, 3H), 2.10 - 1.99 (m, 1H). |
| **A84** | methyl 5-morpholino-5-phenyl-4,5,6,7-tetrahydro benzothiophen-2-carboxylate 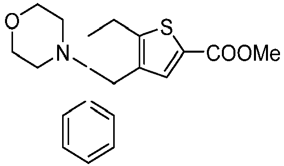 | ESI-MS m/z 358.47 [M+H]⁺. |
| **A85** | 6-(2-methoxyphenyl)-4,5,6,7-tetrahydrob enzothiazole-2,6-diamine 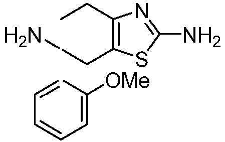 | ESI-MS m/z 276.37 [M+H]⁺. |
| | | ¹H NMR (500 MHz, DMSO-*d*₆) δ 7.34 - 7.28 (m, 1H), 7.16 (dd, *J* = 7.8, 1.7 Hz, 1H), 7.10 (d, *J =* 8.2 Hz, 1H), 6.91 (t, *J =* 7.6 Hz, 1H), 6.71 (s, 2H), 3.88 (s, 3H), 3.21 (d, *J* = 15.4 Hz, 1H), 2.92 (d, *J =* 13.9 Hz, 1H), 2.60 (dd, *J* = 11.8, 5.0 Hz, 1H), 2.49 (q, *J =* 5.2 Hz, 1H), 2.00 - 1.88 (m, 2H). |
| **A86** | 6-(2-methoxyphenyl)-*N*⁶-methyl-4,5,6,7-t etrahydrobenzothiazole-2,6-diamine 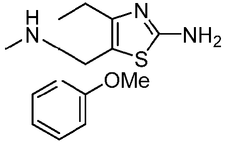 | ESI-MS m/z 290.40 [M+H]⁺. |
| **A87** | 6-(3-methoxyphenyl)-*N*⁶-methyl-4,5,6,7-t etrahydrobenzothiazole-2,6-diamine 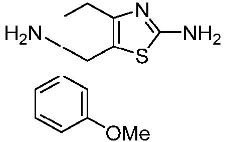 | ESI-MS m/z 259.37 [M+H]⁺. ¹H NMR (400 MHz, DMSO-*d*₆) δ 7.21 (t, *J = 7.9* Hz, 1H), 7.13-7.00 (m, 2H), 6.77 (dd, *J* = 8.1, 2.4 Hz, 1H), 6.61 (s, 2H), 3.73 (s, 3H), 2.97 (d, *J* = 15.9 Hz, 1H), 2.58 (d, *J =* 16.1 Hz, 1H), 2.52-2.48 (m, 1H), 2.17-2.06 (m, 2H), 1.82-1.75 (m, 1H). |
| **A87-P1** | (*R*)-6-(3-methoxyphenyl)-*N*⁶-methyl-4,5,6 ,7-tetrahydrobenzothiazole-2,6-diamine 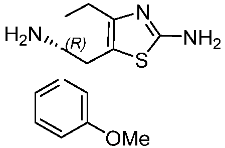 | **A87-P1** (R configuration): HPLC purity: > 97%, retention time: 7.558 min. Chiral purity: > 99%, retention time: 6.700 min. |
| **A87-P2** | (*S*)-6-(3-methoxyphenyl)-*N*⁶-methyl-4,5,6 ,7-tetrahydrobenzothiazole-2,6-diamine 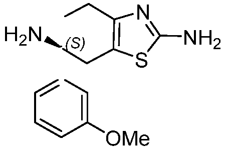 | **A87-P2** (S configuration): HPLC purity: > 99%, retention time: 7.594 min. Chiral purity: > 99%, retention time: 10.307 min. |
| **A88** | 6-(3-methoxyphenyl)-*N*⁶-methyl-4,5,6,7-t etrahydrobenzothiazole-2,6-diamine 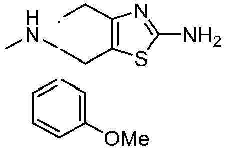 | ESI-MS m/z 290.20 [M+H]⁺. |
| | | ¹H NMR (400 MHz, DMSO-*d*₆) δ 7.30 (t, *J =* 8.0 Hz, 1H), 7.10 (s, 1H), 7.04 (d, *J =* 7.8 Hz, 1H), 6.90 (dd, *J* = 8.2, 2.4 Hz, 1H), 6.72 (s, 2H), 3.75 (s, 3H), 3.39 (d, *J* = 16.0 Hz, 1H), 2.99 (d, *J* = 16.0 Hz, 1H), 2.44 (m, 1H), 2.40 - 2.20 (m, 2H), 2.10 (s, 3H), 1.87 (m, 1H). |
| **A88-P1** | (*R*)-6-(3-methoxyphenyl)-*N*⁶-methyl-4,5,6 ,7-tetrahydrobenzothiazole-2,6-diamine 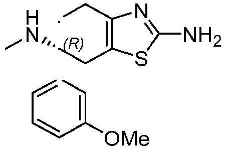 | **A88-P1** (R configuration): HPLC purity: > 97%, retention time: 7.829 min. Chiral purity: > 99%, retention time: 6.316 min. |
| **A88-P2** | (*S*)-6-(3-methoxyphenyl)-*N*⁶-methyl-4,5,6 ,7-tetrahydrobenzothiazole-2,6-diamine 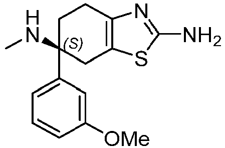 | **A88-P2** (S configuration): HPLC purity: > 98%, retention time: 7.999 min. Chiral purity: > 99%, retention time: 7.463 min. |
| **A89** | 6-(4-methoxyphenyl)-4,5,6,7-tetrahydrob enzothiazole-2,6-diamine 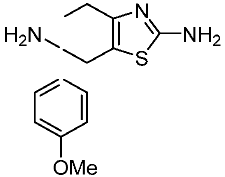 | ESI-MS m/z 276.14 [M+H]⁺. |
| | | ¹H NMR (400 MHz, DMSO-*d*₆) δ 7.47 (d, *J* = 8.8 Hz, 2H), 6.90 (d, *J* = 8.8 Hz, 2H), 6.68 (s, 2H), 3.78 (s, 3H), 3.02 (d, *J* = 15.9 Hz, 1H), 2.63 (d, *J* = 15.9 Hz, 1H), 2.56-2.50 (m, 1H), 2.26-2.04 (m, 4H), 1.84 (m, 1H). |
| **A90** | 6-(4-methoxyphenyl)-*N*⁶-methyl-4,5,6,7-t etrahydrobenzothiazole-2,6-diamine 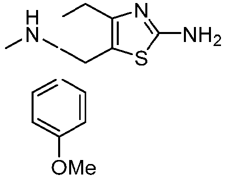 | ESI-MS m/z 290.40 [M+H]⁺. |
| **A91** | 6-(2-trifluoromethylphenyl)-4,5,6,7-tetrah ydrobenzothiazole-2,6-diamine 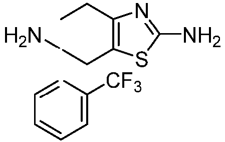 | ESI-MS m/z 314.34 [M+H]⁺. |
| **A92** | 6-(2-trifluoromethylphenyl)-*N*⁶-methyl-4, 5,6,7-tetrahydrobenzothiazole-2,6-diamin e 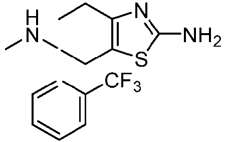 | ESI-MS m/z 328.37 [M+H]⁺. |
| **A93** | 6-(3-trifluoromethylphenyl)-4,5,6,7-tetrah ydrobenzothiazole-2,6-diamine hydrobromide 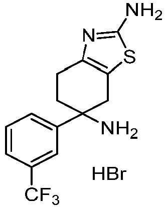 | ESI-MS m/z 314.22 [M+H]⁺. |
| hydrobrom ide | | ¹H NMR (400 MHz, DMSO-*d*₆) δ 9.20 (s, 2H), 8.81 (s, 3H), 7.98 (d, *J =* 2.0 Hz, 1H), 7.93 - 7.88 (m, 1H), 7.83 - 7.81 (m, 1H), 7.75-7.71 (m, 1H), 3.55 (d, *J* = 16.9 Hz, 1H), 3.17 (d, *J =* 16.8 Hz, 1H), 2.73-2.65 (m, 1H), 2.58-2.53 (m, 1H), 2.42-2.36 (m, 1H), 2.24-2.16 (m, 1H). |
| **A94** | 6-(3-trifluoromethylphenyl)-*N*⁶-methyl-4, 5,6,7-tetrahydrobenzothiazole-2,6-diamin e hydrobromide 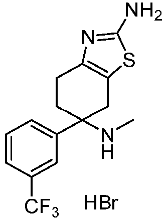 | ESI-MS m/z 328.09 [M+H]⁺. |
| hydrobrom ide | | ¹H NMR (400 MHz, DMSO-*d*₆) δ 9.68 (s, 1H), 9.49 (s, 1H), 9.12 (s, 2H), 7.97 (s, 1H), 7.90 (d, *J =* 8.0 Hz, 1H), 7.85 (d, *J =* 7.8 Hz, 1H), 7.75 (t, *J =* 7.9 Hz, 1H), 3.76 (d, *J* = 16.6 Hz, 1H), 3.24 (d, *J =* 16.9 Hz, 1H), 2.74-2.66 (m, 2H), 2.58 - 2.53 (m, 1H), 2.23 (d, *J* = 4.8 Hz, 3H), 2.03 - 1.98 (m, 1H). |
| **A95** | 6-(4-trifluoromethylphenyl)-4,5,6,7-tetrah ydrobenzothiazole-2,6-diamine 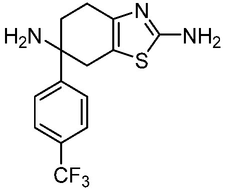 | ESI-MS m/z 314.34 [M+H]⁺. |
| | | ¹H NMR (500 MHz, Methanol-*d*₄) δ 7.76 - 7.70 (m, 4H), 3.38 (d, *J* = 16.4 Hz, 1H), 2.97 (d, *J* = 16.2 Hz, 1H), 2.68 - 2.60 (m, 1H), 2.52 - 2.40 (m, 1H), 2.32 - 2.17 (m, 2H). |
| **A96** | 6-(4-trifluoromethylphenyl)-*N*⁶-methyl-4, 5,6,7-tetrahydrobenzothiazole-2,6-diamin e hydrobromide 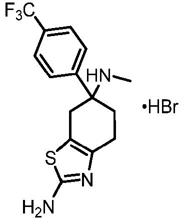 | ESI-MS m/z 328.01 [M+H]⁺. |
| hydrobrom ide | | ¹H NMR (400 MHz, DMSO-*d*₆) δ 9.79 (s, 1H), 9.58 (s, 1H), 9.22 (s, 2H), 7.87 (q, *J =* 8.6 Hz, 4H), 3.76 (d, *J =* 16.6 Hz, 1H), 3.26 (d, *J =* 16.6 Hz, 1H), 2.76-2.66 (m, 2H), 2.58 - 2.54 (m, 1H), 2.23 (d, *J =* 4.6 Hz, 3H), 2.01-1.95 (m, 1H). |
| **A97** | *N*⁶-isopropyl-6-phenyl-4,5,6,7-tetrahydro benzothiazole-2,6-diamine 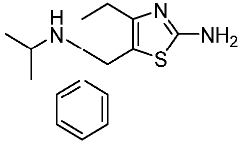 | ESI-MS m/z 288.43 [M+H]⁺. |
| **A98** | 6-(pyridin-3-yl)-4,5,6,7-tetrahydrobenzot hiazole-2,6-diamine 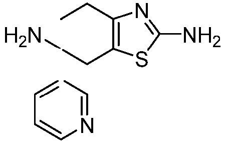 | ESI-MS m/z 247.2 [M+H]⁺. |
| | | ¹H NMR (400 MHz, DMSO-*d*₆) δ 8.71 (d, *J =* 2.1 Hz, 1H), 8.41 (dd, *J =* 4.7, 1.5 Hz, 1H), 7.90 - 7.87 (m, 1H), 7.32 (dd, *J* = 7.9, 4.7 Hz, 1H), 6.65 (s, 2H), 3.05 (d, *J* = 15.9 Hz, 1H), 2.65 (d, *J* = 16.1 Hz, 1H), 2.55-2.50 (m, 1H), 2.17 - 2.04 (m, 2H), 1.89-1.84 (m, 1H). |
| **A99** | *N*⁶-methyl-6-(pyridin-3-yl)-4,5,6,7-tetrahy drobenzothiazole-2,6-diamine 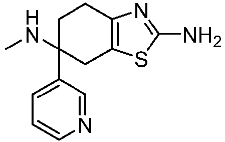 | ESI-MS m/z 261.2 [M+H]⁺. |
| | | ¹H NMR (400 MHz, DMSO-*d*₆) δ 8.56 (d, *J =* 2.1 Hz, 1H), 8.41 (dd, *J* = 4.7, 1.4 Hz, 1H), 7.79 - 7.76 (m, 1H), 7.33 (dd, *J* = 8.0, 4.7 Hz, 1H), 6.64 (s, 2H), 3.14 (d, *J* = 16.0 Hz, 1H), 2.66 (d, *J* = 16.0 Hz, 1H), 2.45 - 2.38 (m, 1H), 2.10 - 1.98 (m, 2H), 1.94 (s, 3H), 1.91-1.83 (m, 1H). |
| **A100** | 6-(2-methylphenyl)-4,5,6,7-tetrahydroben zothiazole-2,6-diamine 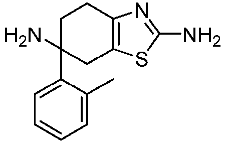 | ESI-MS m/z 260.14 [M+H]⁺. |
| | | ¹H NMR (400 MHz, DMSO-*d*₆) δ 7.33 - 7.24 (m, 1H), 7.18 - 6.99 (m, 3H), 6.61 (s, 2H), 3.07 (d, *J* = 16.2 Hz, 1H), 2.72 (d, *J =* 16.2 Hz, 1H), 2.65 (s, 3H), 2.51-2.46 (m, 1H), 2.38-2.31 (m, 1H), 2.06-2.01 (m, 3H), 1.86-1.80 (m, 1H). |
| **A101** | 6-(2-methylphenyl)-*N*⁶-methyl-4,5,6,7-tet rahydrobenzothiazole-2,6-diamine 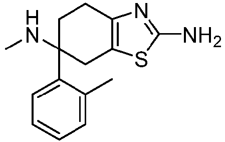 | ESI-MS m/z 274.40 [M+H]⁺. |
| **A102** bis(trifluor oacetic acid) salt | 6-(3-methylphenyl)-4,5,6,7-tetrahydroben zothiazole-2,6-diamine bis(trifluoroacetic acid) salt 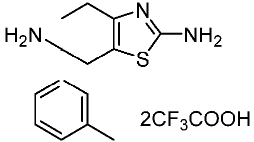 | ESI-MS m/z 260.37 [M+H]⁺. |
| | | ¹H NMR (400 MHz, CD₃OD) δ 7.44 - 7.25 (m, 4H), 3.55 (d, *J* = 18.1 Hz, 1H), 3.09 (d, *J =* 16.7 Hz, 1H), 2.77 - 2.53 (m, 2H), 2.48 - 2.30 (m, 5H). |
| **A102** | 6-(3-methylphenyl)-4,5,6,7-tetrahydroben zothiazole-2,6-diamine hydrochloride 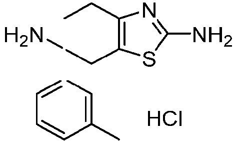 | ESI-MS m/z 260.37 [M+H]⁺. |
| hydrochlor ide | | ¹H NMR (400 MHz, DMSO-*d*₆) δ 9.43 (s, 2H), 9.00 (s, 3H), 7.48 (s, 1H), 7.38 (d, *J* = 8.1 Hz, 1H), 7.32 (t, *J* = 7.7 Hz, 1H), 7.21 (d, *J =* 7.4 Hz, 1H), 3.42 (d, *J* = 16.8 Hz, 1H), 3.21 (s, 1H), 2.68 (d, *J =* 16.8 Hz, 1H), 2.46 (d, *J* = 6.3 Hz, 1H), 2.40 (q, *J* = 6.9 Hz, 1H), 2.33 (s, 3H), 2.14 (dd, *J =* 17.0, 7.7 Hz, 1H). |
| **A103** | 6-(3-methylphenyl)-*N*⁶-methyl-4,5,6,7-tet rahydrobenzothiazole-2,6-diamine 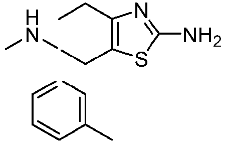 | ESI-MS m/z 274.40 [M+H]⁺. |
| **A104** | 6-(4-methylphenyl)-4,5,6,7-tetrahydroben zothiazole-2,6-diamine 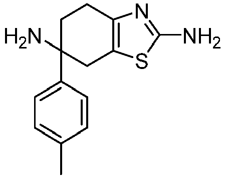 | ESI-MS m/z 260.27 [M+H]⁺. |
| | | ¹H NMR (400 MHz, DMSO-*d*₆) δ 7.42 (d, *J* = 8.0 Hz, 2H), 7.16 (d, *J* = 8.0 Hz, 2H), 6.72 (s, 2H), 3.18 (d, *J* = 15.9 Hz, 1H), 2.90 (d, *J =* 16.0 Hz, 1H), 2.50-2.45 (m, 1H), 2.30-2.22 (m, 4H), 2.11 - 1.99 (m, 2H). |
| **A105** | 6-(4-methylphenyl)-*N*⁶-methyl-4,5,6,7-tet rahydrobenzothiazole-2,6-diamine 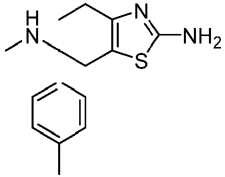 | ESI-MS m/z 274.40 [M+H]⁺. |
| **A106** | 6-phenyl-6-(piperidin-1-yl)-4,5,6,7-tetrah ydrobenzo[d]isothiazole 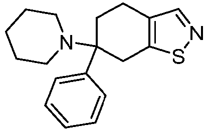 | ESI-MS m/z 299.45 [M+H]⁺. |
| **A107** | 6-phenyl-4,5,6,7-tetrahydrobenzo[*d*]isothi azol-6-amine 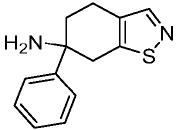 | ESI-MS m/z 231.33 [M+H]⁺. |
| **A108** | *N*-methyl-6-phenyl-4,5,6,7-tetrahydroben zo[d]isothiazol-6-amine 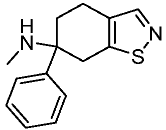 | ESI-MS m/z 245.36 [M+H]⁺. |
| **A109** | 2-methyl-6-phenyl-4,5,6,7-tetrahydrobenz othiazol-6-amine 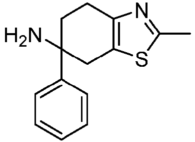 | ESI-MS m/z 245.36 [M+H]⁺. |
| **A110** | N,2-dimethyl-6-phenyl-4,5,6,7-tetrahydro benzothiazol-6-amine 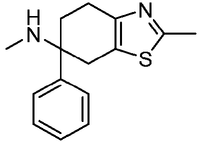 | ESI-MS m/z 259.38 [M+H]⁺. |
| **A111** | 6-(azepan-1-yl)-6-phenyl-4,5,6,7-tetrahyd robenzothiazol-2-amine 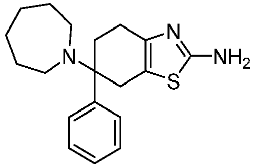 | ESI-MS m/z 328.49 [M+H]⁺. |
| **A112** | *N*⁶-cyclopropyl-6-phenyl-4,5,6,7-tetrahyd robenzothiazole-2,6-diamine 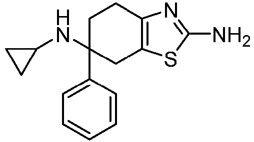 | ESI-MS m/z 289.41 [M+H]⁺. |
| **A113** | 2-cyclopropyl-6-phenyl-4,5,6,7-tetrahydr obenzothiazol-6-amine 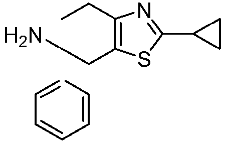 | ESI-MS m/z 271.39 [M+H]⁺. |
| **A114** | *N*⁶,*N*⁶-dimethyl-6-phenyl-4,5,6,7-tetrahyd robenzothiazole-2,6-diamine 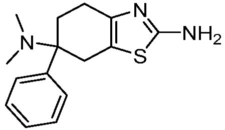 | ESI-MS m/z 274.20 [M+H]⁺. |
| | | ¹H NMR (400 MHz, DMSO-*d*₆) δ 7.53 - 7.15 (m, 5H), 6.64 (m, 2H), 3.17 - 2.93 (m, 2H), 2.39 (d, *J* = 16.7 Hz, 1H), 2.30 - 2.06 (m, 8H), 1.75-1.55 (m, 1H). |
| **A115** | 4-(2-amino-6-(methylamino)-4,5,6,7-tetra hydrobenzothiazol-6-yl)phenol 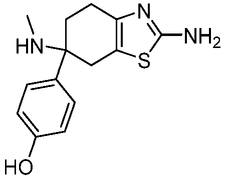 | ESI-MS m/z 276.37 [M+H]⁺. |
| **A116** | 6-(azetidin-1-yl)-6-phenyl-4,5,6,7-tetrahy drobenzothiazol-2-amine 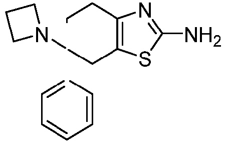 | ESI-MS m/z 286.41 [M+H]⁺. |
| **A117** | 6-(4-(trifluoromethoxy)phenyl)-4, 5,6,7-tet rahydrobenzothiazole-2,6-diamine 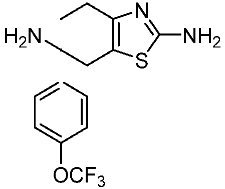 | ESI-MS m/z 330.34 [M+H]⁺. |
| **A118** | 6-(naphthalen-2-yl)-4,5,6,7-tetrahydroben zothiazole-2,6-diamine 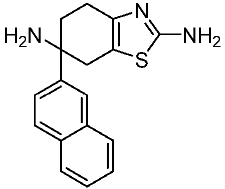 | ESI-MS m/z 296.16 [M+H]⁺. |
| | | ¹H NMR (400 MHz, DMSO-*d*₆) δ 7.98 (d, *J* = 1.8 Hz, 1H), 7.88-7.84 (m, 3H), 7.76 (dd, *J* = 8.7, 1.9 Hz, 1H), 7.49 (m, 2H), 6.68 (s, 2H), 3.18 (d, *J* = 16.0 Hz, 1H), 2.75 (d, *J* = 15.9 Hz, 1H), 2.63 - 2.51 (m, 1H), 2.32-2.25 (m, 1H), 2.21-2.13 (m, 1H), 2.01-1.92 (m, 1H). |
| **A119** | 6-phenyl-4,5,6,7-tetrahydro-1*H*-indazol-6 -amine 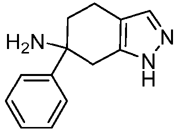 | ESI-MS m/z 214.28 [M+H]⁺. |
| **A120** | *N*-methyl-6-phenyl-4,5,6,7-tetrahydro-1*H* -indazol-6-amine 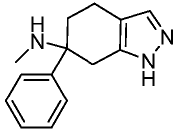 | ESI-MS m/z 228.31 [M+H]⁺. |
| **A121** | 2-methyl-6-phenyl-5,6,7,8-tetrahydroquin azolin-6-amine 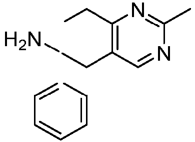 | ESI-MS m/z 240.32 [M+H]⁺. |
| **A122** | 6-(4-cyanophenyl)-*N*⁶-methyl-4,5,6,7-tetr ahydrobenzothiazole-2,6-diamine 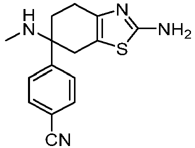 | ESI-MS m/z 285.38 [M+H]⁺. |
| **A123** | 5-phenyl-5-(piperidin-1-yl)-4,5,6,7-tetrah ydrobenzothiophen-2-formamide 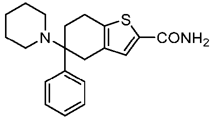 | ESI-MS m/z 341.49 [M+H]⁺. |
| **A124** | *N*-methyl-5-phenyl-4,5,6,7-tetrahydroben zo[*c*][1,2,5]thiadiazol-5-amine 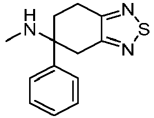 | ESI-MS m/z 246.34 [M+H]⁺. |
| **A125** | *N*⁶-(cyclobutylmethyl)-6-phenyl-4,5,6,7-t etrahydrobenzothiazole-2,6-diamine 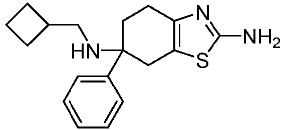 | ESI-MS m/z 314.46 [M+H]⁺. |
| **A126** | 6-amino-6-phenyl-5,6,7,8-tetrahydroquin olin-2(1H)-one 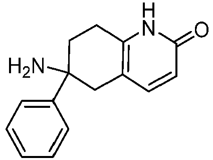 | ESI-MS m/z 241.31 [M+H]⁺. |
| **A127** | 5-(2-fluorophenyl)-5-(pyrrolidin-1-yl)-4,5 ,6,7-tetrahydro-1*H*-indazole 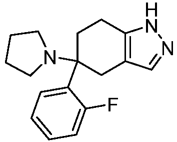 | ESI-MS m/z 286.37 [M+H]⁺. |
| **A128** | 5-(3-fluorophenyl)-5-(pyrrolidin-1-yl)-4,5 ,6,7-tetrahydro-1*H*-indazole 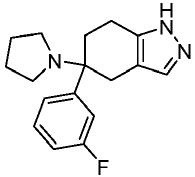 | ESI-MS m/z 286.47 [M+H]⁺. |
| **A129** | 5-(4-fluorophenyl)-5-(pyrrolidin-1-yl)-4,5 ,6,7-tetrahydro-1*H*-indazole 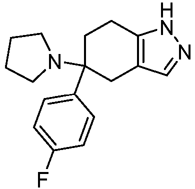 | ESI-MS m/z 286.50 [M+H]⁺. |
| **A130** | N-(2-amino-6-phenyl-4,5,6,7-tetrahydrob enzo[d]thiazol-6-yl)benzamide 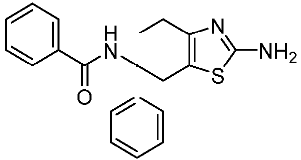 | ESI-MS m/z 350.45 [M+H]⁺. |
| **A131** | 6-phenyl-2-(trifluoromethyl)-4,5,6,7-tetra hydrobenzo[d]thiazol-6-amine hydrochloride 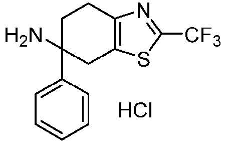 | ESI-MS m/z 299.33 [M+H]⁺. |
| hydrochlor ide | | ¹H NMR (400 MHz, DMSO-*d*₆) δ 8.90 (s, 3H), 7.66-7.56 (m, 2H), 7.51-7.35 (m, 3H), 3.93 (d, *J =* 17.3 Hz, 1H), 3.53 (d, *J =* 17.3 Hz, 1H), 2.96 (dd, *J =* 10.5, 5.9 Hz, 1H), 2.62 (d, *J* = 6.5 Hz, 1H), 2.45 (d, *J* = 9.2 Hz, 2H). |
| **A132** | 5-phenyl-4,5,6,7-tetrahydrobenzo[d]thiaz ole-2,5-diamine 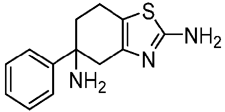 | ESI-MS m/z 246.17 [M+H]⁺. |
| | | ¹H NMR (400 MHz, DMSO-*d*₆) δ 7.49 (d, *J =* 7.7 Hz, 2H), 7.31 (t, *J =* 7.6 Hz, 2H), 7.21 (t, *J* = 7.3 Hz, 1H), 6.67 (s, 2H), 2.93 (d, *J* = 16.5 Hz, 1H), 2.66 - 2.53 (m, 2H), 2.21-2.11 (m, 2H), 1.95 - 1.79 (m, 1H). |
| **A132-P1** | (*S*)-5-phenyl-4,5,6,7-tetrahydrobenzo[*d*]th iazole-2,5-diamine 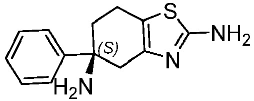 | **A132-P1** (S configuration): HPLC purity: > 96%, retention time: 7.036 min. Chiral purity: > 99%, retention time: 4.789 min. |
| **A132-P2** | (*R*)-5-phenyl-4,5,6,7-tetrahydrobenzo[*d*]t hiazole-2,5-diamine 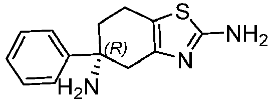 | **A132-P2** (R configuration): HPLC purity: > 97%, retention time: 7.0214 min. Chiral purity: > 99%, retention time: 5.617 min. |
| **A133** | 7-phenyl-4,5,6,7-tetrahydrobenzo[d]thiaz ole-2,7-diamine 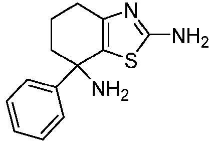 | ESI-MS m/z 246.60 [M+H]⁺. |
| **A134** | 5-phenyl-4,5,6,7-tetrahydrobenzo[d]thiaz ol-5-amine 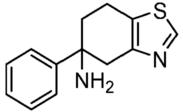 | ESI-MS m/z 231.13 [M+H]⁺. |
| | | ¹H NMR (400 MHz, Chloroform-d) δ 8.67 (s, 1H), 7.50 (d, *J* = 7.7 Hz, 2H), 7.35 (t, *J =* 7.5 Hz, 2H), 7.30 - 7.21 (m, 1H), 3.47 (d, *J* = 16.6 Hz, 1H), 3.07 (d, *J* = 16.5 Hz, 1H), 3.02 - 2.87 (m, 1H), 2.71-2.67 (m, 1H), 2.40 - 2.22 (m, 1H), 2.17 - 2.05 (m, 1H). |
| **A135** | *N*-methyl-5-phenyl-4,5,6,7-tetrahydroben zo[*d*]thiazol-5-amine 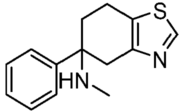 | ESI-MS m/z 245.14 [M+H]⁺. |
| | | ¹H NMR (400 MHz, Chloroform-d) δ 8.66 (s, 1H), 7.46 (d, *J* = 7.5 Hz, 2H), 7.35 (t, *J* = 7.5 Hz, 2H), 7.29 (d, *J* = 7.2 Hz, 1H), 3.61 (d, *J* = 16.4 Hz, 1H), 3.30 (d, *J =* 16.4 Hz, 1H), 2.91-2.85 (m, 2H), 2.53-2.50 (m, 2H), 2.45-2.37 (m, 1H), 2.25 (s, 3H). |
| **A136** | 7-phenyl-4,5,6,7-tetrahydrobenzo[*d*]thiaz ol-7-amine 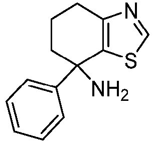 | ESI-MS m/z 231.50 [M+H]⁺. |
| **A137** | *N*-methyl-7-phenyl-4,5,6,7-tetrahydroben zo[*d*]thiazol-7-amine 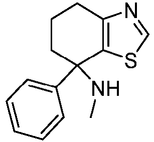 | ESI-MS m/z 245.80 [M+H]⁺. |
| **A138** | 5-(2-fluorophenyl)-4,5,6,7-tetrahydrobenz o[*d*]thiazole-2,5-diamine 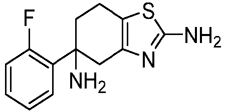 | ESI-MS m/z 264.33 [M+H]⁺. |
| **A139** | 5-(3-fluorophenyl)-4,5,6,7-tetrahydrobenz o[*d*]thiazole-2,5-diamine 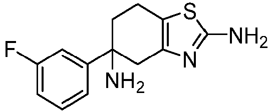 | ESI-MS m/z 264.50 [M+H]⁺. |
| **A140** | 5-(4-fluorophenyl)-4,5,6,7-tetrahydrobenz o[*d*]thiazole-2,5-diamine 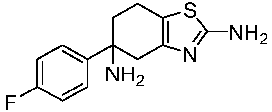 | ESI-MS m/z 264.33 [M+H]⁺. |
| **A141** | *N*⁵-ethyl-5-phenyl-4,5,6,7-tetrahydrobenz o[*d*]thiazole-2,5-diamine 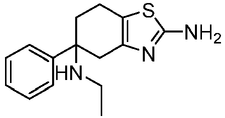 | ESI-MS m/z 274.20 [M+H]⁺. |
| | | ¹H NMR (400 MHz, DMSO-*d*₆) δ 7.63 - 7.19 (m, 5H), 6.75 (s, 2H), 3.51 (m, 1H), 3.17 (m, 1H), 2.86 (m, 1H), 2.55 (m, 1H), 2.33-2.20 (m, 3H), 1.87 (m, 1H), 1.14 - 0.91 (m, 3H). |
| **A142** | 5-phenyl-*N*⁵-propyl-4,5,6,7-tetrahydroben zo[*d*]thiazole-2,5-diamine 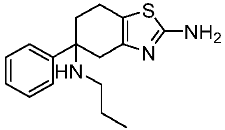 | ESI-MS m/z 288.20 [M+H]⁺. |
| | | ¹H NMR (400 MHz, DMSO-*d*₆) δ 7.43 (m, 5H), 6.79 (s, 2H), 3.38 (m, 2H), 3.17 (d, *J* = 4.9 Hz, 1H), 3.00 (m, 1H), 2.84 (m, 1H), 2.59 (m, 1H), 2.34 (m, 1H), 2.12 (m, 1H), 1.81 (m, 1H), 1.65 - 1.39 (m, 2H), 0.79 (t, *J =* 7.4 Hz, 3H). |
| **A143** | 6-phenyl-4,5,6,7-tetrahydrobenzo[*d*]isoxa zol-6-amine 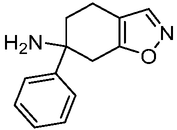 | ESI-MS m/z 215.27 [M+H]⁺. |
| **A144** | *N*-methyl-6-phenyl-4,5,6,7-tetrahydroben zo[*d*]isoxazol-6-amine 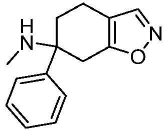 | ESI-MS m/z 229.30 [M+H]⁺. |
| **A145** | phenyl-4,5,6,7-tetrahydrobenzo[d]isoxazo l-5-amine 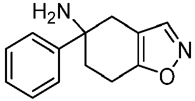 | ESI-MS m/z 215.12 [M+H]⁺. |
| | | ¹H NMR (400 MHz, DMSO-*d*₆) δ 8.57 (s, 1H), 7.55 (dd, *J* = 7.8, 1.5 Hz, 2H), 7.32 (t, *J* = 7.6 Hz, 2H), 7.21 (t, *J* = 7.3 Hz, 1H), 2.94 (d, *J =* 16.0 Hz, 1H), 2.92-2.84 (m, 1H), 2.62 (d, *J* = 15.9 Hz, 1H), 2.55-2.48(m, 1H), 2.23-2.16 (m, 1H), 1.99 (s, 2H), 1.94-1.88 (m, 1H). |
| **A146** | *N*-methyl-5-phenyl-4,5,6,7-tetrahydroben zo[*d*]isoxazol-5-amine 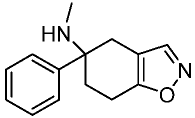 | ESI-MS m/z 229.50 [M+H]⁺. |
| | | ¹H NMR (400 MHz, Chloroform-d) δ 8.16 (s, 1H), 7.43-7.32 (m, 4H), 7.29-7.23 (m, 1H), 3.05 (d, *J =* 16.0 Hz, 1H), 2.91-2.83 (m, 1H), 2.79 (d, *J* = 16.0 Hz, 1H), 2.70-2.63 (m, 1H), 2.35 - 2.25 (m, 1H), 2.23-2.17 (m, 1H), 2.11 (s, 3H). |
| **A147** | 7-phenyl-4,5,6,7-tetrahydrobenzo[*d*]isoxa zol-7-amine 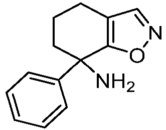 | ESI-MS m/z 215.27 [M+H]⁺. |
| **A148** | 4-phenyl-4,5,6,7-tetrahydrobenzo[*d*]isoxa zol-4-amine 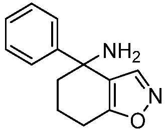 | ESI-MS m/z 215.30 [M+H]⁺. |
| **A149** | 5-phenyl-4,5,6,7-tetrahydrobenzo[*d*]isothi azol-5-amine 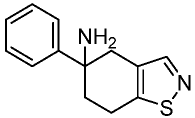 | ESI-MS m/z 231.33 [M+H]⁺. |
| | | ¹H NMR (400 MHz, DMSO-*d*₆) δ 8.34 (s, 1H), 7.74 - 7.41 (m, 2H), 7.32 (dd, *J* = 8.4, 6.9 Hz, 2H), 7.25 - 7.21 (m, 1H), 3.18 (d, *J =* 16.2 Hz, 1H), 3.07 - 2.99 (m, 1H), 2.83 (d, *J =* 16.0 Hz, 1H), 2.72-2.65 (m, 1H), 2.26-2.20 (m, 1H), 1.99- 1.92 (m, 1H). |
| **A150** | *N*-methyl-5-phenyl-4,5,6,7-tetrahydroben zo[*d*]isothiazol-5-amine hydrochloride | ESI-MS m/z 245.36 [M+H]⁺. |
| hydrochlor ide | | ¹H NMR (400 MHz, DMSO-*d*₆) δ 10.16 (d, *J* = 11.3 Hz, 1H), 9.67 (s, 1H), 8.45 (s, 1H), 7.63 - 7.53 (m, 2H), 7.50 - 7.31 (m, 3H), 4.08 - 3.73 (m, 1H), 3.43 - 3.18 (m, 1H), 3.20 - 3.06 (m, 1H), 2.75-2,72 (m, 1H), 2.54-2.47 (m, 1H), 2.26-2.17 (m, 4H). |
| | 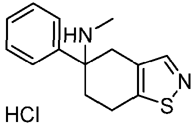 | |
| **A151** | 7-phenyl-4,5,6,7-tetrahydrobenzo[*d*]isothi azol-7-amine 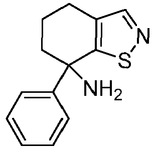 | ESI-MS m/z 231.60 [M+H]⁺. |
| **A152** | *N*-methyl-7-phenyl-4,5,6,7-tetrahydroben zo[*d*]isothiazol-7-amine 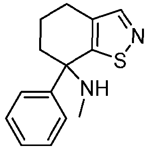 | ESI-MS m/z 245.40 [M+H]⁺. |
| **A153** | 4-phenyl-4,5,6,7-tetrahydrobenzo[*d*]isothi azol-4-amine 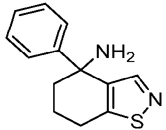 | ESI-MS m/z 231.62 [M+H]⁺. |
| **A154** | *N*-methyl-4-phenyl-4,5,6,7-tetrahydroben zo[*d*]isothiazol-4-amine 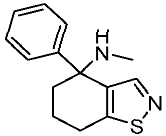 | ESI-MS m/z 245.46 [M+H]⁺. |
| **A155** | 4-phenyl-4,5,6,7-tetrahydro-1*H*-indazol-4 -amine 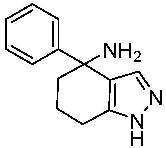 | ESI-MS m/z 214.28 [M+H]⁺. |
| **A156** | N-methyl-4-phenyl-4,5,6,7-tetrahydro-1H -indazol-4-amine 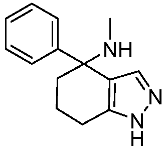 | ESI-MS m/z 228.31 [M+H]⁺. |
| **A157** | 7-phenyl-4,5,6,7-tetrahydro-1*H*-indazol-7 -amine 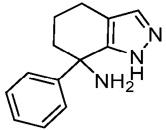 | ESI-MS m/z 214.30 [M+H]⁺. |
| **A158** | *N*-methyl-7-phenyl-4,5,6,7-tetrahydro-1*H* -indazol-7-amine 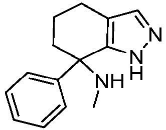 | ESI-MS m/z 228.35 [M+H]⁺. |
| **A159** | 7-phenyl-4,5,6,7-tetrahydro-1*H*-indol-7-a mine 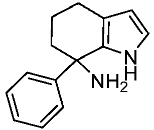 | ESI-MS m/z 213.30 [M+H]⁺. |
| **A160** | N-methyl-7-phenyl-4,5,6,7-tetrahydro-1H -indol-7-amine 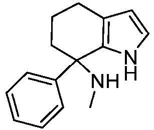 | ESI-MS m/z 227.32 [M+H]⁺. |
| **A161** | 4-phenyl-4,5,6,7-tetrahydro-1*H*-indol-4-a mine 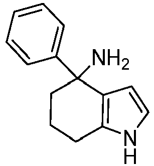 | ESI-MS m/z 213.32 [M+H]⁺. |
| **A162** | *N*-methyl-4-phenyl-4,5,6,7-tetrahydro-1*H* -indol-4-amine 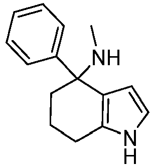 | ESI-MS m/z 227.70 [M+H]⁺. |
| **A163** | 5-phenyl-4,5,6,7-tetrahydro-1H-indol-5-a mine 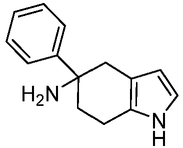 | ESI-MS m/z 213.50 [M+H]⁺. |
| **A164** | N-methyl-5-phenyl-4,5,6,7-tetrahydro-1H -indol-5-amine 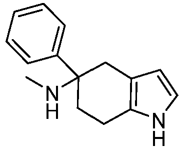 | ESI-MS m/z 227.50 [M+H]⁺. |
| **A165** | 6-phenyl-4,5,6,7-tetrahydro-1H-indol-5-a mine 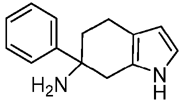 | ESI-MS m/z 213.54 [M+H]⁺. |
| **A166** | *N*-methyl-6-phenyl-4,5,6,7-tetrahydro-1*H* -indol-5-amine 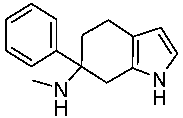 | ESI-MS m/z 227.56 [M+H]⁺. |
| **A167** | 5-(2-aminoethyl)-5-phenyl-4,5,6,7-tetrahy drobenzo[d]thiazol-2-amine 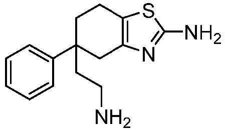 | ESI-MS m/z 274.40 [M+H]⁺. |
| **A168** | 5-(2-(methylamino) ethyl)-5-phenyl-4,5,6,7-tetrahydrobenzo[*d* ]thiazol-2-amine 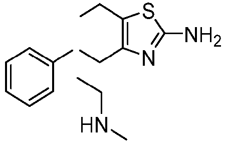 | ESI-MS m/z 288.43 [M+H]⁺. |
| **A169** | 6-(2-aminoethyl)-6-phenyl-4,5,6,7-tetrahy drobenzo[*d*]thiazol-2-amine 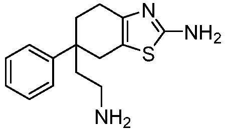 | ESI-MS m/z 274.46 [M+H]⁺. |
| **A170** | 6-(2-(methylamino) ethyl)-5-phenyl-4,5,6,7-tetrahydrobenzo[d ]thiazol-2-amine 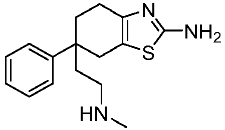 | ESI-MS m/z 288.45 [M+H]⁺. |
| **A171** | 5-phenyl-4,5,6,7-tetrahydrobenzo[*d*]oxaz ole-2,5-diamine 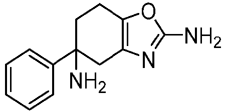 | ESI-MS m/z 230.28 [M+H]⁺. |
| **A172** | 6-phenyl-4,5,6,7-tetrahydrobenzo[*d*]oxaz ole-2,6-diamine 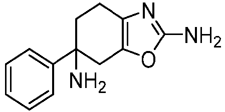 | ESI-MS m/z 230.30 [M+H]⁺. |
| **A173** | 2-methyl-5-phenyl-4,5,6,7-tetrahydrobenz o[*b*]thiophen-5-amine 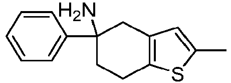 | ESI-MS m/z 244.37 [M+H]⁺. |
| **A174** | 3-methyl-5-phenyl-4,5,6,7-tetrahydrobenz o[*b*]thiophen-5-amine 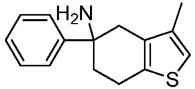 | ESI-MS m/z 244.50 [M+H]⁺. |
| **A175** | *N*-methyl-5-phenyl-4,5,6,7-tetrahydroben zo[*c*]thiophen-5-amine 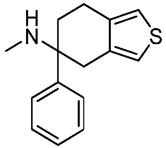 | ESI-MS m/z 244.53 [M+H]⁺. |
| **A176** | 2-fluoro-5-phenyl-4,5,6,7-tetrahydrobenz o[b]thiophen-5-amine 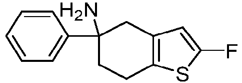 | ESI-MS m/z 248.33 [M+H]⁺. |
| **A177** | 2-chloro-5-phenyl-4,5,6,7-tetrahydrobenz o[b]thiophen-5-amine 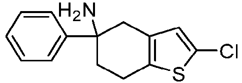 | ESI-MS m/z 264.78 [M+H]⁺. |
| **A178** | 5-phenyl-2-(trifluoromethyl)-4,5,6,7-tetra hydrobenzo[*b*]thiophen-5-amine 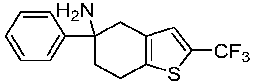 | ESI-MS m/z 298.34 [M+H]⁺. |
| **A179** | 2-ethyl-5-phenyl-4,5,6,7-tetrahydrobenzo[ b]thiophen-5-amine 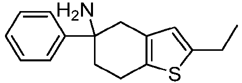 | ESI-MS m/z 258.40 [M+H]⁺. |
| **A180** | 2,5-diphenyl-4,5,6,7-tetrahydrobenzo[*b*]th iophen-5-amine 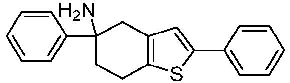 | ESI-MS m/z 306.44 [M+H]⁺. |
| **A181** | 5-phenyl-2-(pyridin-4-yl)-4,5,6,7-tetrahyd robenzo[*b*]thiophen-5-amine 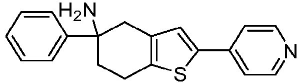 | ESI-MS m/z 307.43 [M+H]⁺. |
| **A182** | 5-phenyl-2-(pyridin-3-yl)-4,5,6,7-tetrahyd robenzo[*b*]thiophen-5-amine 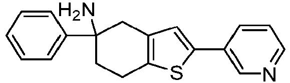 | ESI-MS m/z 307.40 [M+H]⁺. |
| **A183** | 5-phenyl-2-(pyridin-2-yl)-4,5,6,7-tetrahyd robenzo[b]thiophen-5-amine 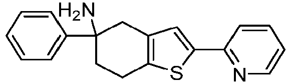 | ESI-MS m/z 307.64 [M+H]⁺. |
| **A184** | 2-morpholino-5-phenyl-4,5,6,7-tetrahydro benzo[b]thiophen-5-amine 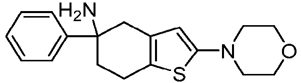 | ESI-MS m/z 315.45 [M+H]⁺. |
| **A185** | 2-methoxy-5-phenyl-4,5,6,7-tetrahydrobe nzo[*b*]thiophen-5-amine 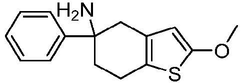 | ESI-MS m/z 260.37 [M+H]⁺. |
| **A186** | 5-phenyl-2-(pyrrolidin-1-yl)-4,5,6,7-tetra hydrobenzo[*b*]thiophen-5-amine 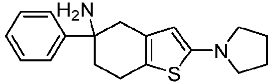 | ESI-MS m/z 299.45 [M+H]⁺. |
| **A187** | 5-phenyl-2-(piperidin-1-yl)-4,5,6,7-tetrah ydrobenzo[*b*]thiophen-5-amine 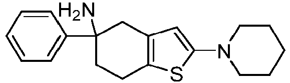 | ESI-MS m/z 313.48 [M+H]⁺. |
| **A188** | 5-phenyl-5,6,7,8-tetrahydro-4*H*-cyclohept a[*d*]thiazole-2,5-diamine 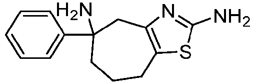 | ESI-MS m/z 260.37 [M+H]⁺. |
| **A189** | 7-phenyl-5,6,7,8-tetrahydro-4*H*-cyclohept a[*d*]thiazole-2,7-diamine 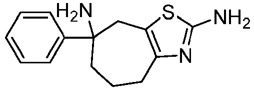 | ESI-MS m/z 260.50 [M+H]⁺. |
| **A190** | 1-(5-phenyl-5-(pyrrolidin-1-yl)-4,5,6,7-tet rahydro-1*H*-indazol-1-yl]ethan-1-one 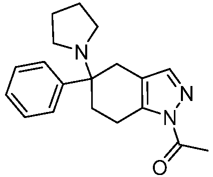 | ESI-MS m/z 310.41 [M+H]⁺. |
| | | ¹H NMR (400 MHz, Chloroform-d) δ 7.88 (s, 1H), 7.27-7.22 (m, 2H), 7.21 - 7.14 (m, 2H), 7.13 - 7.05 (m, 1H), 3.11 (d, *J* = 16.1 Hz, 1H), 2.94 (dd, *J* = 16.3, 1.5 Hz, 1H), 2.75 - 2.58 (m, 1H), 2.56 - 2.49 (m, 2H), 2.47 (s, 3H), 2.41 (td, *J* = 7.1, 2.6 Hz, 2H), 2.33 - 2.21 (m, 2H), 2.19 - 2.07 (m, 1H), 1.62 - 1.42 (m, 4H). |
| **A191** | 5-phenyl-5-(piperidin-1-yl)-4,5,6,7-tetrah ydrobenzo[*d*]isoxazole 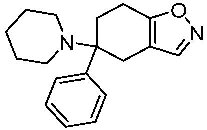 | ESI-MS m/z 283.41 [M+H]⁺. |
| **A192** | 5-(aminomethyl)-5-phenyl-4,5,6,7-tetrahy drobenzo[*d*]thiazol-2-amine 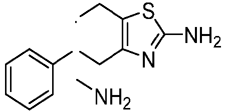 | ESI-MS m/z 260.37 [M+H]⁺. |
| **A193** | 6-(aminomethyl)-6-phenyl-4,5,6,7-tetrahy drobenzo[*d*]thiazol-2-amine 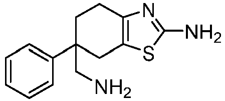 | ESI-MS m/z 260.57 [M+H]⁺. |
| **A194** | 5-((methylamino)methyl)-5-phenyl-4,5,6, 7-tetrahydrobenzo[*d*]thiazol-2-amine 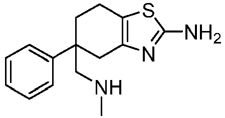 | ESI-MS m/z 274.40 [M+H]⁺. |
| **A195** | 5-((dimethylamino)methyl)-5-phenyl-4,5, 6,7-tetrahydrobenzo[d]thiazol-2-amine 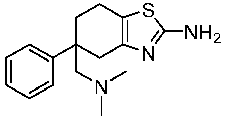 | ESI-MS m/z 288.43 [M+H]⁺. |
| **A196** | *N*⁶-(tert-butyl)-6-phenyl-4,5,6,7-tetrahydr obenzo[*d*]thiazole-2,6-diamine 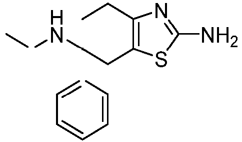 | ESI-MS m/z 302.45 [M+H]⁺. |
| **A197** | 6-(quinolin-2-yl)-4,5,6,7-tetrahydrobenzo [*d*]thiazole-2,6-diamine 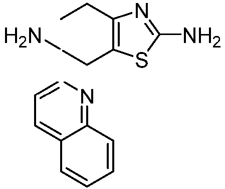 | ESI-MS m/z 297.39 [M+H]⁺. |
| **A198** | 5-phenyl-4,5,6,7-tetrahydrobenzo[*c*][1,2,5 ]thiadiazol-5-amine 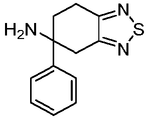 | ESI-MS m/z 232.34 [M+H]⁺. |
| **A199** | 3-methyl-5-phenyl-4,5,6,7-tetrahydro-1*H-*indazol-5-amine 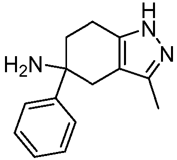 | ESI-MS m/z 228.30 [M+H]⁺. |
| **A200** | 5-phenyl-3-(trifluoromethyl)-4,5,6,7-tetra hydro-1*H*-indazol-5-amine 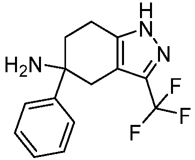 | ESI-MS m/z 282.30 [M+H]⁺. |
| **A201** | 1-methyl-5-phenyl-4,5,6,7-tetrahydro-1*H-*indazol-5-amine 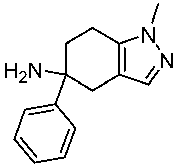 | ESI-MS m/z 228.40 [M+H]⁺. |
| **A202** | 1-isopropyl-5-phenyl-4,5,6,7-tetrahydro-1 *H*-indazol-5-amine 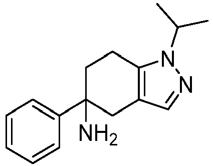 | ESI-MS m/z 256.40 [M+H]⁺. |
| **A203** | 1-cyclopropyl-5-phenyl-4,5,6,7-tetrahydr o-1*H*-indazol-5-amine 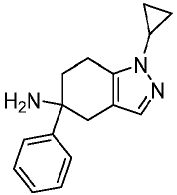 | ESI-MS m/z 254.35 [M+H]⁺. |
| **A204** | 4-(5-phenyl-4,5,6,7-tetrahydro-1*H*-indazo l-5-yl)morpholine 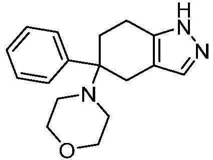 | ESI-MS m/z 284.38 [M+H]⁺. |
| **A205** | 6-(pyridin-2-yl)-4,5,6,7-tetrahydrobenzo[ *d*]thiazole-2,6-diamine 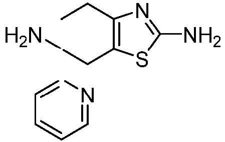 | ESI-MS m/z 247.13 [M+H]⁺. |
| **A206** | 5-(pyridin-4-yl)-4,5,6,7-tetrahydrobenzo[ *d*]thiazole-2,6-diamine 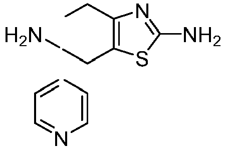 | ESI-MS m/z 247.35 [M+H]⁺. |
| **A207** | 6-(5-fluoropyridin-2-yl)-4,5,6,7-tetrahydr obenzo[*d*]thiazole-2,6-diamine 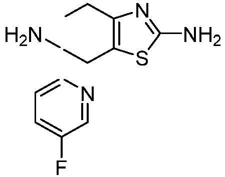 | ESI-MS m/z 265.34 [M+H]⁺. |
| **A208** | 6-(5-chloropyridin-2-yl)-4,5,6,7-tetrahydr obenzo[*d*]thiazole-2,6-diamine 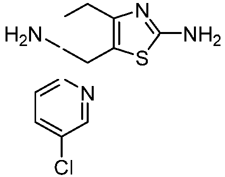 | ESI-MS m/z 281.79 [M+H]⁺. |
| **A209** | 6-(6-methylpyridin-2-yl)-4,5,6,7-tetrahydr obenzo[*d*]thiazole-2,6-diamine 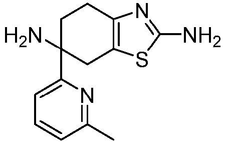 | ESI-MS m/z 261.38 [M+H]⁺. |
| **A210** | 6-(pyrimidin-5-yl)-4,5,6,7-tetrahydrobenz o[*d*]thiazole-2,6-diamine 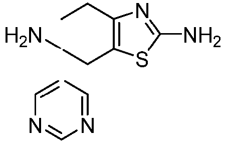 | ESI-MS m/z 248.42 [M+H]⁺. |
| **A211** | 6-(pyrazin-2-yl)-4,5,6,7-tetrahydrobenzo[ *d*]thiazole-2,6-diamine 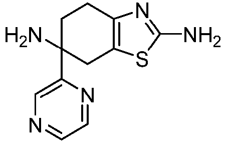 | ESI-MS m/z 248.36 [M+H]⁺. |
| **A212** | 6-(pyridazin-4-yl)-4,5,6,7-tetrahydrobenz o[*d*]thiazole-2,6-diamine 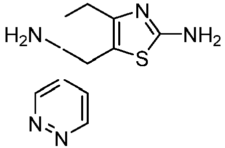 | ESI-MS m/z 248.40 [M+H]⁺. |
| **A213** | 6-(6-(trifluoromethyl)pyridin-3-yl)-4,5,6, 7-tetrahydrobenzo[*d*]thiazole-2,6-diamine 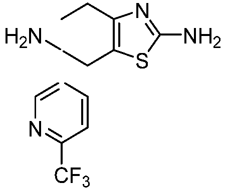 | ESI-MS m/z 315.30 [M+H]⁺. |
| **A214** | 5-(2-fluorophenyl)-4,5,6,7-tetrahydrobenz o[*d*]oxazole-2,5-diamine 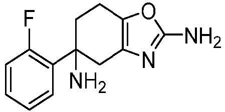 | ESI-MS m/z 248.30 [M+H]⁺. |
| **A215** | 5-(3-fluorophenyl)-4,5,6,7-tetrahydrobenz o[*d*]oxazole-2,5-diamine 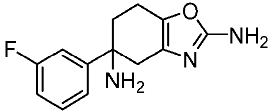 | ESI-MS m/z 248.20 [M+H]⁺. |
| **A216** | 5-(4-fluorophenyl)-4,5,6,7-tetrahydrobenz o[*d*]oxazole-2,5-diamine 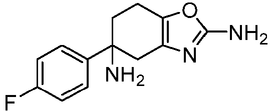 | ESI-MS m/z 248.51 [M+H]⁺. |
| **A217** | 5-phenyl-4,5,6,7-tetrahydrobenzo[*d*]oxaz ol-5-amine 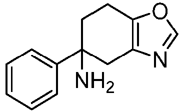 | ESI-MS m/z 215.27 [M+H]⁺. |
| **A218** | 6-phenyl-4,5,6,7-tetrahydrobenzo[*d*]oxaz ol-6-amine 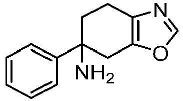 | ESI-MS m/z 215.57 [M+H]⁺. |
| **A219** | 1(2,6-diamino-4,5,6,7-tetrahydrobenzo[*d*] thiazol-6-yl)benzonitrile 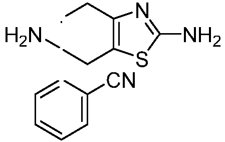 | ESI-MS m/z 271.35 [M+H]⁺. |
| **A220** | 2(2,6-diamino-4,5,6,7-tetrahydrobenzo[*d*] thiazol-6-yl)benzonitrile 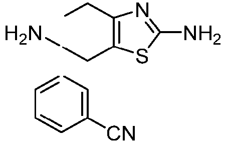 | ESI-MS m/z 271.2 [M+H]⁺. |
| | | ¹H NMR (400 MHz, DMSO-*d*₆) δ 7.97 (t, *J =* 1.8 Hz, 1H), 7.84 (dt, *J* = 8.1, 1.5 Hz, 1H), 7.67 (dt, *J* = 7.7, 1.3 Hz, 1H), 7.51 (t, *J =* 7.8 Hz, 1H), 6.64 (s, 2H), 3.00 (d, *J* = 16.0 Hz, 1H), 2.60 (d, *J* = 16.2 Hz, 1H), 2.26 - 2.08 (m, 4H), 1.91 - 1.75 (m, 2H). |
| **A221** | 3(2,6-diamino-4,5,6,7-tetrahydrobenzo[*d*] thiazol-6-yl)benzonitrile 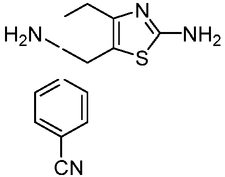 | ESI-MS m/z 271.2 [M+H]⁺. |
| | | ¹H NMR (400 MHz, DMSO-*d*₆) δ 7.74 (q, *J =* 8.6 Hz, 4H), 6.64 (s, 2H), 2.99 (d, *J* = 16.0 Hz, 1H), 2.61 (d, *J* = 16.0 Hz, 1H), 2.51 - 2.50 (m, 1H), 2.15-2.16 (m, 2H), 1.83-1.78 (m, 1H). |
| **A222** | 7-(4-fluorophenyl)-4,5,6,7-tetrahydro-1*H-*indazol-7-amine 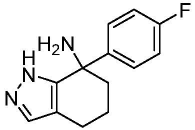 | ESI-MS m/z 215.15 [M-NH₂]⁺. |
| | | ¹H NMR (400 MHz, CDCl₃) δ 7.20 (s, 1H), 7.20 - 7.15 (m, 2H), 6.96 - 6.88 (m, 2H), 4.74 (s, 2H), 2.62 - 2.47 (m, 2H), 2.09 (ddd, *J* = 13.3, 7.6, 2.6 Hz, 1H), 1.87 (ddd, *J* = 13.1, 10.4, 2.6 Hz, 1H), 1.81 - 1.71 (m, 1H), 1.60 - 1.47 (m, 1H). |
| **A223** | 3-(2-amino-6-(methylamino)-4,5,6,7-tetra hydrobenzo[*d*]thiazol-6-yl)phenol 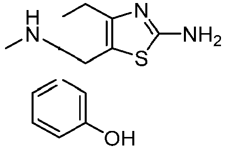 | ESI-MS m/z 276.30 [M+H]⁺. |
| | | ¹H NMR (400 MHz, DMSO-*d*₆) δ 9.24 (s, 1H), 7.08 (d, *J =* 7.8 Hz, 1H), 6.90 - 6.78 (m, 2H), 6.62 (d, *J* = 5.8 Hz, 3H), 3.47-3.41 (m, 1H), 3.04 (d, *J =* 16.0 Hz, 1H), 2.66 (d, *J* = 15.8 Hz, 1H), 2.41-2.37 (m, 1H), 2.05-1.94 (m, 6H). |
| **A224** | 3-(2,6-diamino-4,5,6,7-tetrahydrobenzo[*d* ]thiazol-6-yl)phenol 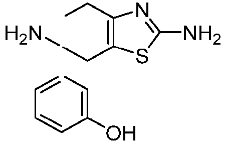 | ESI-MS m/z 262.10 [M+H]⁺ |
| | | ¹H NMR (400 MHz, DMSO-*d*₆) δ 9.20 (s, 1H), 7.07 (t, *J* = 7.8 Hz, 1H), 7.01 - 6.79 (m, 2H), 6.73 - 6.43 (m, 3H), 2.93 (d, *J* = 15.9 Hz, 1H), 2.56 (d, *J* = 16.3 Hz, 1H), 2.50-2.46 (m, 1H), 2.20 - 1.99 (m, 4H), 1.77 (dt, *J* = 12.3, 5.8 Hz, 1H). |
| **A225** | 2-methoxy-6-phenyl-4,5,6,7-tetrahydrobe nzo[*d*]thiazol-6-amine 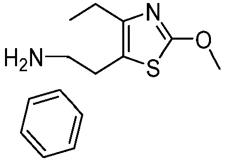 | ESI-MS m/z 261.35 [M+H]⁺ |
| | | ¹H NMR (400 MHz, DMSO-*d*₆) δ 7.59 - 7.46 (m, 2H), 7.30 (t, *J* = 7.6 Hz, 2H), 7.25 - 7.14 (m, 1H), 3.94 (s, 3H), 3.09 (d, *J* = 16.3 Hz, 1H), 2.78 - 2.57 (m, 2H), 2.40 - 2.21 (m, 1H), 2.16 (m, 1H), 1.98 - 1.81 (m, 1H). |
| **A226** | *N*⁶-ethyl-6-(3-methoxyphenyl)-4,5,6,7-tetr ahydrobenzo[*d*]thiazole-2,6-diamine 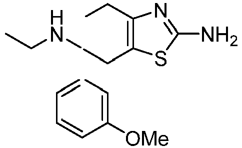 | ESI-MS m/z 304.42 [M+H]⁺ |
| | | ¹H NMR (400 MHz, DMSO-*d*₆) δ 7.27 (m, 1H), 7.04 (m, 2H), 6.85 (m, 1H), 6.67 (s, 2H), 3.73 (s, 3H), 2.44 - 1.75 (m, 7H), 1.00 (m, 4H). |
| **A227** | 6-(4-fluoro-3-methoxyphenyl)-4,5,6,7-tetr ahydrobenzo[*d*]thiazole-2,6-diamine 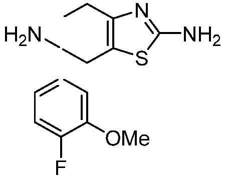 | ESI-MS m/z 294.10 [M+H]⁺ |
| | | ¹H NMR (400 MHz, DMSO-*d*₆) δ 7.35 (dd, *J* = 8.6, 2.2 Hz, 1H), 7.17 - 6.97 (m, 2H), 6.61 (s, 2H), 3.82 (s, 3H), 2.97 (d, *J* = 15.9 Hz, 1H), 2.57 (d, *J* = 16.2 Hz, 1H), 2.52-2.50 (m, 1H), 2.24 - 2.04 (m, 2H), 1.99 (s, 2H), 1.77 (dt, *J =* 12.0, 5.2 Hz, 1H). |
| **A228** | 6-(3-ethoxyphenyl)-4,5,6,7-tetrahydroben zo[*d*]thiazole-2,6-diamine 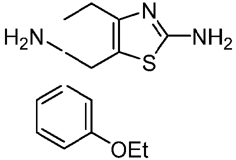 | ESI-MS m/z 290.20 [M+H]⁺ |
| | | ¹H NMR (400 MHz, DMSO-*d*₆) δ 7.18 (t, *J* = 7.9 Hz, 1H), 7.12 - 7.02 (m, 2H), 6.79 - 6.66 (m, 1H), 6.60 (s, 2H), 3.99 (q, *J =* 6.9 Hz, 2H), 2.96 (d, *J =* 15.9 Hz, 1H), 2.57 (d, *J* = 16.2 Hz, 1H), 2.52 - 2.44 (m, 1H), 2.10 (m, 2H), 1.91 (s, 2H), 1.78 (dt, *J =* 12.2, 6.5 Hz, 1H), 1.31 (t, *J* = 7.0 Hz, 3H). |
| **A228-P1** | (*R*)-6-(3-ethoxyphenyl)-4,5,6,7-tetrahydro benzo[*d*]thiazole-2,6-diamine 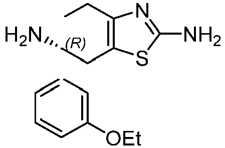 | **A228-P1** (R configuration): HPLC purity: > 97%, retention time: 9.062 min. Chiral purity: > 99%, retention time: 7.522 min. |
| **A228-P2** | (*S*)-6-(3 -ethoxyphenyl)-4, 5, 6,7-tetrahydro benzo[*d*]thiazole-2,6-diamine 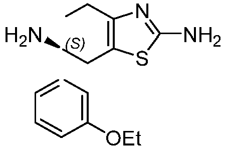 | **A228-P1** (S configuration): HPLC purity: > 95%, retention time: 9.066 min. Chiral purity: > 98%, retention time: 8.512 min. |
| **A229** | 6-(3-(trifluoromethoxy)phenyl)-4, 5,6,7-tet rahydrobenzo[*d*]thiazole-2,6-diamine 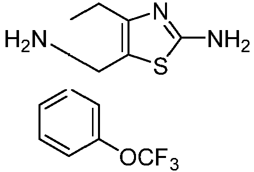 | ESI-MS m/z 330.1 [M+H]⁺ |
| | | ¹H NMR (400 MHz, DMSO-*d*₆) δ 7.54-7.52 (m, 2H), 7.42 (t, *J* = 7.9 Hz, 1H), 7.18 (d, *J* = 8.2 Hz, 1H), 6.62 (s, 2H), 2.97 (d, *J* = 16.0 Hz, 1H), 2.60 (d, *J =* 16.0 Hz, 1H), 2.55 -2.50(m, 1H), 2.14-2.06 (m, 4H), 1.80 (dt, *J =* 12.1, 6.3 Hz, 1H). |
| **A230** | *N*⁶-methyl-6-(3-(tiifluoromethoxy)phenyl )-4,5,6,7-tetrahydrobenzo[*d*]thiazole-2,6-diamine 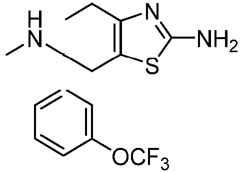 | ESI-MS m/z 344.1 [M+H]⁺ |
| | | ¹H NMR (400 MHz, DMSO-*d*₆) δ 7.48 - 7.35 (m, 3H), 7.19 (d, *J* = 7.5 Hz, 1H), 6.61 (s, 2H), 3.04 (d, *J* = 16.0 Hz, 1H), 2.65 (d, *J* = 15.9 Hz, 1H), 2.43-2.39 (m, 1H), 2.04-2.01 (t, *J* = 6.2 Hz, 2H), 1.96-1.90 (m, 4H). |
| **A231** | 6-(4-fluorophenyl)-4,5,6,7-tetrahydrobenz o[*d*]thiazol-6-amine 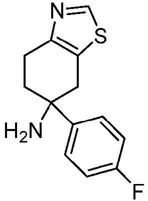 | ESI-MS m/z 249.1 [M+H]⁺ |
| | | ¹H NMR (400 MHz, Chloroform-d) δ 8.65 (s, 1H), 7.79 - 7.38 (m, 2H), 7.03 (t, *J* = 8.7 Hz, 2H), 3.32 (d, *J* = 16.5 Hz, 1H), 3.13 - 2.91 (m, 2H), 2.92 - 2.72 (m, 1H), 2.36-2.29 (m, 1H), 2.16 - 1.98 (m, 1H). |
| **A232** hydrochlor ide | 6-(4-chlorophenyl)-4,5,6,7-tetrahydroben zo[*d*]thiazole-2,6-diamine hydrochloride 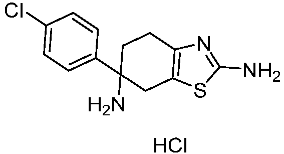 | ESI-MS m/z 280.1 [M+H]⁺ |
| | | ¹H NMR (400 MHz, Methanol-*d*₄) δ 7.62 (d, *J* = 8.5 Hz, 2H), 7.54 (d, *J* = 8.5 Hz, 2H), 3.60 (d, *J =* 16.8 Hz, 1H), 3.20 (d, *J* = 16.8 Hz, 1H), 2.86 - 2.72 (m, 1H), 2.71 - 2.61 (m, 1H), 2.57 - 2.46 (m, 1H), 2.45 - 2.33 (m, 1H). |
| **A233** | *N*,*N*-dimethyl-6-phenyl-4,5,6,7-tetrahydro benzo[*d*]thiazol-6-amine 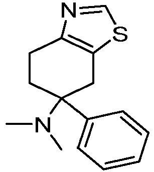 | ESI-MS m/z 259.1 [M+H]⁺ |
| | | ¹H NMR (400 MHz, DMSO-*d*₆) δ 8.80 (s, 1H), 7.32 (d, *J =* 7.4 Hz, 2H), 7.27 (t, *J =* 7.5 Hz, 2H), 7.20 (t, *J =* 7.1 Hz, 1H), 3.38 (d, *J* = 17.7 Hz, 1H), 3.17 (d, *J =* 16.6 Hz, 1H), 2.74 (d, *J* = 15.9 Hz, 1H), 2.33-2.26 (m, 1H), 2.20-2.16 (m, 1H), 2.09 (s, 6H), 2.02-1.94 (m, 1H). |
| **A234** | 6-(2-fluorophenyl)-*N',N*' -dimethyl-4,5,6, 7-tetrahydrobenzo[*d*]thiazole-2,6-diamine 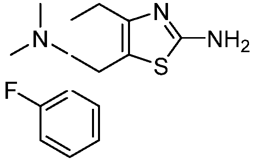 | ESI-MS m/z 292.1 [M+H]⁺ |
| | | ¹H NMR (400 MHz, DMSO-*d*₆) δ 7.32-7.22 (m, 2H), 7.14-7.07 (m, 2H), 6.62 (s, 2H), 3.34-3.19(m, 1H), 2.88 (d, *J =* 15.9 Hz, 1H), 2.63-2.54 (m, 1H), 2.46 (d, *J* = 16.0 Hz, 1H), 2.17-2.10 (m,7H), 1.86-1.78 (m, 1H). |
| **A235** | 6-(2,4-difluorophenyl)-4,5,6,7-tetrahydro benzo[*d*]thiazole-2,6-diamine 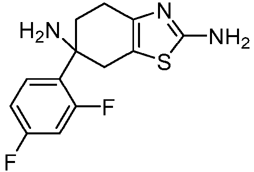 | ESI-MS m/z 282.1 [M+H]⁺ |
| | | ¹H NMR (400 MHz, DMSO-*d*₆) δ 7.54-7.47 (m, 1H), 7.21-7.15 (m, 1H), 7.05-7.00 (m, 1H), 6.65 (s, 2H), 3.08 (d, *J =* 16.1 Hz, 1H), 2.60 (d, *J* = 16.4 Hz, 1H), 2.54-2.50 (m, 1H), 2.33-2.27 (m, 1H), 2.19 - 2.09 (m, 1H), 1.81-1.75 (m, 1H). |
| **A236** | 6-(2,4-difluorophenyl)-*N*⁶-methyl-4,5,6, 7-tetrahydrobenzo[*d*]thiazole-2,6-diamine 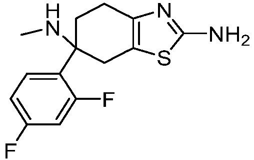 | ESI-MS m/z 296.1 [M+H]⁺ |
| | | ¹H NMR (400 MHz, DMSO-*d*₆) δ 7.29-7.18 (m, 2H), 7.05-7.01 (m, 1H), 6.65 (s, 2H), 3.24 (d, *J* = 16.0 Hz, 1H), 2.72 (d, *J* = 16.0 Hz, 1H), 2.46 -2.43 (m, 1H), 2.33-2.25 (m, 1H), 2.13-1.95 (m, 5H). |
| **A237** | 2-phenyl-1,2,3,4-tetrahydrodibenzo[*b*,*d*]th iophen-2-amine 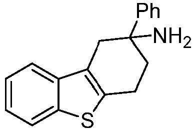 | ESI-MS m/z 280.4 [M+H]⁺ |
| | | ¹H NMR (400 MHz, DMSO-*d*₆) δ 7.88 (d, *J* = 7.8 Hz, 1H), 7.66 (d, *J* = 7.5 Hz, 1H), 7.56 (d, *J* = 7.4 Hz, 2H), 7.39 - 7.28 (m, 4H), 7.21 (t, *J =* 7.3 Hz, 1H), 3.19 (d, *J* = 16.5 Hz, 1H), 3.06 - 2.96 (m, 1H), 2.86 (d, *J =* 16.4 Hz, 1H), 2.68 - 2.55 (m, 1H), 2.44 - 2.23 (m, 3H), 2.01 -1.94 (m, 1H). |
| **A238** | 5-(2-fluorophenyl)*-N*-methyl-4,5,6,7-tetra hydrobenzo[*b*]thiophen-5-amine hydrochloride 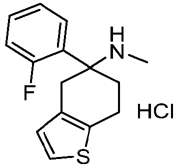 | ESI-MS m/z 262.1 [M+H]⁺ |
| hydrochlor ide | | ¹H NMR (400 MHz, DMSO-*d*₆) δ 10.24 (s, 1H), 8.90 (s, 1H), 7.56 - 7.43 (m, 1H), 7.41-7.29 (m, 2H), 7.26-7.15 (m, 2H), 6.95 (d, *J* = 5.2 Hz, 1H), 3.81 (d, *J* = 16.0 Hz, 1H), 3.32 (d, *J* = 16.0 Hz, 1H), 2.95 (d, *J* = 17.2 Hz, 1H), 2.76 (d, *J* = 13.1 Hz, 1H), 2.47-2.39 (m, 1H), 2.34 (s, 3H), 2.17-2.10 (m, 1H). |
| **A239** | 5-(2-fluorophenyl)-4,5,6,7-tetrahydrobenz o[*b*]thiophen-5-amine hydrochloride; 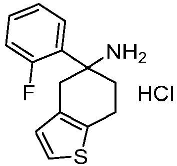 | ESI-MS m/z 248.1 [M+H]⁺ |
| hydrochlor ide | | ¹H NMR (400 MHz, DMSO-*d*₆) δ 8.87 (s, 3H), 7.48-7.43 (m, 1H), 7.38 (d, *J* = 5.1 Hz, 1H), 7.32 (dd, *J* = 12.9, 8.2 Hz, 1H), 7.27-7.22 (m, 1H), 7.19-7.15 (m, 1H), 6.97 (d, *J =* 5.1 Hz, 1H), 3.57 (d, *J =* 16.0 Hz, 1H), 3.32 (d, *J* = 16.6 Hz, 1H), 2.96-2.90 (m, 1H), 2.69-2.65 (m, 1H), 2.39-2.25 (m, 2H). |
| **A240** | 6-(4-bromophenyl)-4,5,6,7-tetrahydroben zo[*d*]thiazole-2,6-diamine hydrochloride 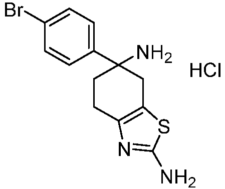 | ESI (*m*/*z*): 324.0 [M + H]⁺. |
| hydrochlor ide | | ¹H NMR (400 MHz, Methanol-*d*₄) δ 7.71 - 7.64 (m, 2H), 7.55 - 7.49 (m, 2H), 3.58 (d, *J* = 15.8 Hz, 1H), 3.16 (d, *J* = 16.9 Hz, 1H), 2.81 - 2.70 (m, 1H), 2.69 - 2.59 (m, 1H), 2.53 - 2.42 (m, 1H), 2.42 - 2.30 (m, 1H). |
| **A241** | 6-phenyl-4,5,6,7-tetrahydro-1*H*-benzo[*d*]i midazol-6-amine trifluoroacetic acid salt 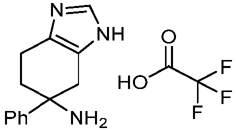 | ESI-MS m/z 214.1 [M+H]⁺ |
| trifluoroac etic acid salt | | ¹H NMR (400 MHz, DMSO-*d*₆) δ 14.31(s,1H), 8.89 (s, 1H), 8.69 (s, 3H), 7.54 (d, *J* = 7.6 Hz, 2H), 7.45 (dt, *J =* 12.5, 6.9 Hz, 3H), 3.59 (d, *J* = 16.5 Hz, 1H), 3.17 (d, *J* = 16.5 Hz, 1H), 2.779-2.72 (m, 1H), 2.55-2.51 (m, 1H), 2.38-2.30 (m, 2H). |
| **A242** | 6-(naphthalen-1-yl)-4,5,6,7-tetrahydroben zo[*b*]thiophen-6-amine hydrochloride 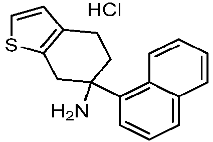 | ESI-MS m/z 263.13 [M-NH₂]⁺ |
| hydrochlor ide | | ¹H NMR (400 MHz, Methanol-*d*₄) δ 8.25 (d, *J =* 8.6 Hz, 1H), 8.02 (d, *J =* 7.4 Hz, 1H), 7.97 (d, *J =* 7.7 Hz, 1H), 7.71-7.55 (m, 2H), 7.54-7.44 (m, 2H), 7.33 (d, *J* = 5.2 Hz, 1H), 6.84 (d, *J* = 5.2 Hz, 1H), 4.15 (d, *J* = 17.5 Hz, 1H), 3.41 (d, *J =* 17.6 Hz, 1H), 3.28-3.22 (m, 1H), 2.93-2.81 (m, 1H), 2.63-2.53 (m, 1H), 2.46-2.36 (m, 1H). |
| **A243** | 6-(naphthalen-2-yl)-4,5,6,7-tetrahydroben zo[*b*]thiophen-6-amine hydrochloride 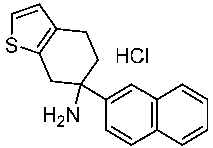 | ESI-MS m/z 263.08 [M-NH₂]⁺ |
| hydrochlor ide | | ¹H NMR (400 MHz, Methanol-*d*₄) δ 8.03-7.94 (m, 2H), 7.92-7.83 (m, 2H), 7.67 (dd, *J* = 8.8, 2.1 Hz, 1H), 7.58-7.49 (m, 2H), 7.26 (d, *J* = 5.1 Hz, 1H), 6.76 (d, *J* = 5.1 Hz, 1H), 3.88 (d, *J* = 16.4 Hz, 1H), 3.41 (d, *J =* 16.5 Hz, 1H), 2.92-2.77 (m, 1H), 2.74-2,64 (m, 1H), 2.51-2.43 (m, 2H). |
| **A244** | 6-phenyl-4,5,6,7-tetrahydrobenzo[*d*][1,2,3 ]thiadiazol-6-amine hydrochloride 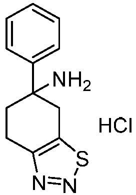 | ESI-MS m/z 215.07 [M-NH₂]⁺ |
| hydrochlor ide | | ¹H NMR (400 MHz, DMSO-*d*₆) δ 8.99 (s, 3H), 7.56 (d, *J* = 7.3 Hz, 2H), 7.52-7.15 (m, 3H), 4.13 (d, *J =* 17.7 Hz, 1H), 3.62 (d, *J =* 17.7 Hz, 1H), 3.36-3.25 (m, 1H), 2.76-2.56 (m, 2H), 2.50 -2.47(m, 1H). |
| **A245** | *N*-methyl-6-phenyl-4,5,6,7-tetrahydroben zo[*d*][1,2,3]thiadiazol-6-amine hydrochloride 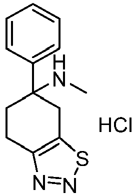 | ESI-MS m/z 246.14 [M+H]⁺ |
| hydrochlor ide | | ¹H NMR (400 MHz, DMSO-*d*₆) δ 10.45-10.30 (m, 1H), 9.90-9.85 (m, 1H), 7.63-7.51 (m, 2H), 7.51 - 7.34 (m, 3H), 4.41 (dd, *J* = 17.3, 2.1 Hz, 1H), 3.65 (d, *J =* 17.4 Hz, 1H), 3.39-3.32 (m, 1H), 2.89-2.79 (m, 1H), 2.68-2.56 (m, 1H), 2.48-2.39 (m, 1H), 2.23 (t, *J =* 5.1 Hz, 3H). |
| **A246** | 5-(naphthalen-1-yl)-4,5,6,7-tetrahydroben zo[*b*]thiophen-5-amine 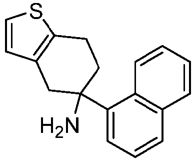 | ESI-MS m/z 263.12 [M-NH₂]⁺ |
| | | ¹H NMR (400 MHz, Methanol-*d*₄) δ 8.24 (d, *J* = 8.6 Hz, 1H), 8.06 - 8.00 (m, 1H), 7.99-7.91 (m, 1H), 7.70-7.53 (m, 2H), 7.51-7.43 (m, 2H), 7.34 (d, *J* = 5.2 Hz, 1H), 6.95 (d, *J* = 5.2 Hz, 1H), 4.02 (d, *J* = 17.3 Hz, 1H), 3.26 (d, *J* = 17.4 Hz, 2H), 3.09-2.95 (m, 1H), 2.73-2.65 (m 1H), 2.50-2.37 (m, 1H). |
| **A247** | 5-(naphthalen-2-yl)-4,5,6,7-tetrahydroben zo[*b*]thiophen-5-amine 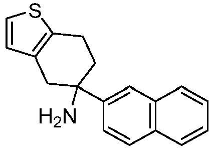 | ESI-MS m/z 263.10 [M-NH₂]⁺ |
| | | ¹H NMR (400 MHz, Methanol-*d*₄) δ 7.978-7.96(m, 2H), 7.92-7.82 (m, 2H), 7.65 (dd, *J =* 8.8, 2.0 Hz, 1H), 7.59-7.48 (m, 2H), 7.30 (d, *J* = 5.2 Hz, 1H), 6.98 (d, *J =* 5.2 Hz, 1H), 3.77 (d, *J =* 16.3 Hz, 1H), 3.25 (d, *J =* 16.4 Hz, 1H), 3.05-2.94 (m, 1H), 2.76-2.71 (m, 1H), 2.63-2.43 (m, 2H). |
| **A248** | 5-amino-5-phenyl-4,5,6,7-tetrahydrobenz o[*d*]thiazol-2-ol 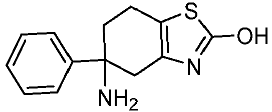 | ESI-MS m/z 247.33 [M+H]⁺ |
| **A249** | *N*⁵,*N*⁵-dimethyl-5-phenyl-4, 5, 6,7-tetrahyd robenzo[*d*]thiazole-2,5-diamine 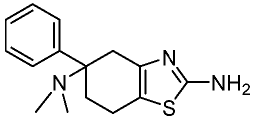 | ESI-MS m/z 274.16 [M+H]⁺ |
| | | ¹H NMR (400 MHz, DMSO-*d*₆) δ 7.34-7.27 (m, 5H), 6.74 (s, 2H), 3.04-2.73 (m, 2H), 2.49 (d, *J =* 15.5 Hz, 1H), 2.24-2.08 (m, 8H), 1.76-1.65 (m, 1H). |
| **A250** | *N*⁴-ethyl-4-phenyl-4,5,6,7-tetrahydrobenz o[*d*]thiazole-2,4-diamine 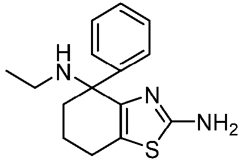 | ESI-MS m/z 229.10 [M-CH₃CH₂NH₂]⁺ |
| | | ¹H NMR (400 MHz, DMSO-*d*₆) δ 9.33 (s, 1H), 7.32 (s, 5H), 6.94 (s, 2H), 2.60-2.52 (m, 2H), 2.27-2.16 (m, 2H), 2.00-1.72 (m, 2H), 1.32 (s, 2H), 1.10 (s, 3H). |
| **A251** | 4-phenyl-*N*⁴-propyl-4,5,6,7-tetrahydroben zo[*d*]thiazole-2,4-diamine 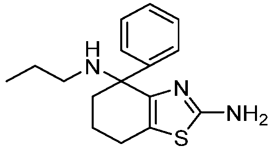 | ESI-MS m/z 229.21 [M-CH₃CH₂CH₂NH₂]⁺ |
| | | ¹H NMR (400 MHz, DMSO-*d*₆) δ 9.30 (s, 1H), 7.32 (s, 5H), 6.98 (s, 2H), 2.57 (s, 2H), 2.43-2.12 (m, 2H), 2.02-1.78 (m, 2H), 1.65-1.30 (m, 4H), 0.84 (t, *J =* 7.4 Hz, 3H). |
| **A252** | 6-((methylamino)methyl)-6-phenyl-4,5,6, 7-tetrahydrobenzo[*d*]thiazol-2-amine 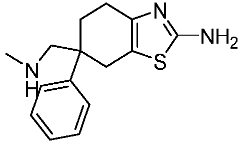 | ESI-MS m/z 274.18 [M+H]⁺ |
| | | ¹H NMR (400 MHz, DMSO-*d*₆) δ 7.43 (s, 1H), 7.37-7.26 (m, 3H), 7.24-7.17 (m, 1H), 6.63 (s, 2H), 5.48 (s, 1H), 3.44 (d, *J =* 16.2 Hz, 1H), 3.05-2.94 (m, 2H), 2.88 (d, *J =* 15.7 Hz, 1H), 2.83-2.78 (m, 1H), 2.20-2.06 (m, 2H), 1.87-1.79 (m, 1H), 1.70 (d, *J* = 5.2 Hz, 3H). |
| **A253** | 6-(3,4-dimethoxyphenyl)-4,5,6,7-tetrahyd robenzo[*d*]thiazole-2,6-diamine 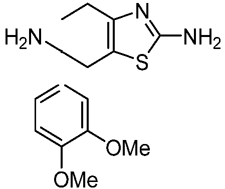 | ESI-MS m/z 306.40 [M+H]⁺ |
| | | ¹H NMR (400 MHz, DMSO-*d*₆) δ 7.16 (d, *J* = 2.2 Hz, 1H), 6.96 (dd, *J =* 8.4, 2.2 Hz, 1H), 6.83 (d, *J =* 8.5 Hz, 1H), 6.60 (s, 2H), 3.73(s, 3H), 3.71(s,3H), 2.96 (d, *J* = 15.9 Hz, 1H), 2.57 (d, *J* = 16.2 Hz, 1H), 2.53-2.47 (m, 1H), 2.27-1.92 (m, 4H), 1.77 (dt, *J =* 12.2, 5.6 Hz, 1H). |
| **A254** | 6-(3,4-dimethoxyphenyl)-*N*⁶-methyl-4,5,6 ,7-tetrahydrobenzo[*d*]thiazole-2,6-diamin e 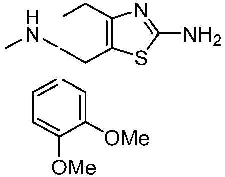 | ESI-MS m/z 320.42 [M+H]⁺ |
| | | ¹H NMR (400 MHz, DMSO-*d*₆) δ 7.23 (s, 1H), 6.95-6.84 (m, 2H), 6.72 (s, 2H), 3.75 (s, 3H), 3.74 (s, 3H),3.37 (m, 1H), 2.94 (d, *J =* 15.9 Hz, 1H), 2.48-2.21 (m, 3H), 2.10 (s, 3H), 1.91 (m, 1H). |
| **A255** | 6-(3-(2,2,2-trifluoroethoxy)phenyl)-4,5,6, 7-tetrahydrobenzo[*d*]thiazole-2,6-diamine 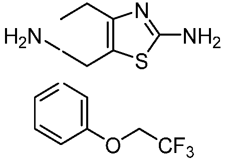 | ESI-MS m/z 344.37 [M+H]⁺ |
| **A256** | 6-(3-(difluoromethoxy)phenyl)-4,5,6,7-tet rahydrobenzo[*d*]thiazole-2,6-diamine 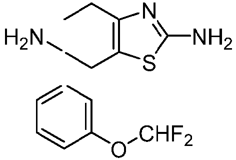 | ESI-MS m/z 312.35 [M+H]⁺ |
| **A257** | 2-ethoxy-6-phenyl-4,5,6,7-tetrahydrobenz o[*d*]thiazol-6-amine 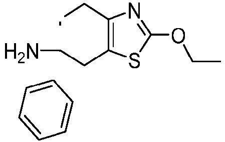 | ESI-MS m/z 275.2 [M+H]⁺ |
| | | ¹H NMR (400 MHz, DMSO-*d*₆) δ 7.58 - 7.46 (m, 2H), 7.30 (t, *J* = 7.7 Hz, 2H), 7.21 (d, *J* = 7.2 Hz, 1H), 4.33 (q, *J* = 7.0 Hz, 2H), 3.07 (d, *J* = 16.5 Hz, 1H), 2.71 - 2.59 (m, 2H), 2.31 - 2.03 (m, 4H), 1.88 - 1.78 (m, 1H), 1.32 (t, *J =* 7.0 Hz, 3H). |
| **A258** | 6-(3,4-dimethoxyphenyl)-*N*⁶-ethyl-4,5,6,7 -tetrahydrobenzo[*d*]thiazole-2,6-diamine 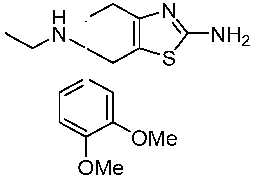 | ESI-MS m/z 334.45 [M+H]⁺ |
| **A259** | 6-(3-(cyclopropylmethoxy)phenyl)-4,5,6, 7-tetrahydrobenzo[*d*]thiazole-2,6-diamine 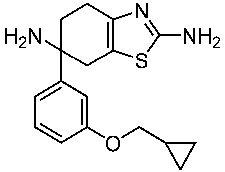 | ESI-MS m/z 316.44 [M+H]⁺ |
| **A260** | 6-(3-methoxy-4-methylphenyl)-4,5,6,7-tet rahydrobenzo[*d*]thiazole-2,6-diamine 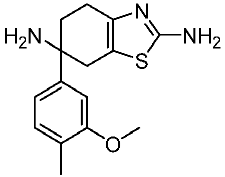 | ESI-MS m/z 290.16 [M+H]⁺ |
| | | ¹H NMR (400 MHz, DMSO-*d*₆) δ 7.12 (d, *J* = 1.7 Hz, 1H), 7.04 (d, *J* = 7.8 Hz, 1H), 6.94 (d, *J* = 1.8 Hz, 1H), 6.62 (s, 2H), 3.77 (s, 3H), 3.03 (d, *J* = 16.0 Hz, 1H), 2.63 (d, *J* = 16.2 Hz, 1H),2.55-2.51(m, 1H), 2.24-2.12 (m, 2H), 2.10 (s, 3H), 1.85 (d, *J =* 7.3 Hz, 1H). |
| **A261** | phenyl 3-(2,6-diamino-4,5,6,7-tetrahydrobenzo[*d* ]thiazol-6-yl)acetate 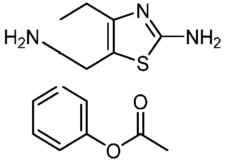 | ESI-MS m/z 304.38 [M+H]⁺ |
| **A262** | 6-amino-6-(3-methoxyphenyl)-4,5,6,7-tetr ahydrobenzo[*d*]thiazol-2-ol 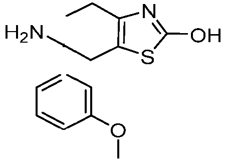 | ESI-MS m/z 260.2 [M-NH₂]⁺ |
| | | ¹H NMR (400 MHz, DMSO-*d*₆) δ 7.27 (t, *J* = 8.0 Hz, 1H), 7.15 - 7.02 (m, 2H), 6.85 (dd, *J* = 8.0, 2.4 Hz, 1H), 3.75 (s, 3H), 2.92 (d, *J =* 16.3 Hz, 1H), 2.56 (d, *J* = 16.3 Hz, 1H), 2.43 (d, *J* = 17.4 Hz, 1H), 2.19 (dd, *J* = 12.9, 6.8 Hz, 1H), 2.03-1.91 (m, 2H). |
| **A263** | 7-phenyl-4,5,6,7-tetrahydro-1*H*-indazol-7 -amine 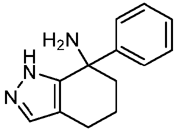 | ESI-MS m/z 214.28 [M+H]⁺ |
| **A264** | 4-(2-chlorophenyl)-4,5,6,7-tetrahydroben zo[*d*]thiazole-2,6-diamine 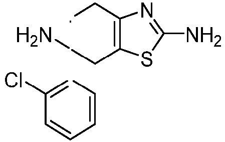 | ESI-MS m/z 280.79 [M+H]⁺ |
| **A265** | 6-(3-chlorophenyl)-4,5,6,7-tetrahydroben zo[*d*]thiazole-2,6-diamine 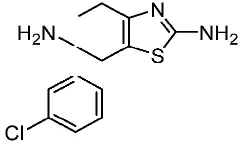 | ESI-MS m/z 280.10 [M+H]⁺ |
| | | ¹H NMR (400 MHz, DMSO-*d*₆) δ 7.59 (t, *J* = 2.0 Hz, 1H), 7.47 - 7.42 (m, 1H), 7.32 (t, *J* = 7.8 Hz, 1H), 7.28 - 7.22 (m, 1H), 6.62 (s, 2H), 2.96 (d, *J* = 16.0 Hz, 1H), 2.58 (d, *J* = 16.4 Hz, 2H), 2.10 (m , 4H), 1.82-1.75 (m, 1H). |
| **A266** | 2-phenyl-1,2,3,4-tetrahydrodibenzo[b,d]f uran-2-amine 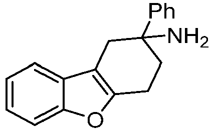 | ESI-MS m/z 264.34 [M+H]⁺ |
| **A267** | 3-phenyl-2,3,4,9-tetrahydro-1*H*-carbazol-3-amine 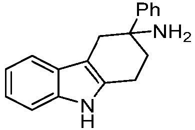 | ESI-MS m/z 263.36 [M+H]⁺ |
| **A268** | 6-(2-fluoro-3-methoxyphenyl)-4,5,6,7-tetr ahydrobenzo[*d*]thiazole-2,6-diamine 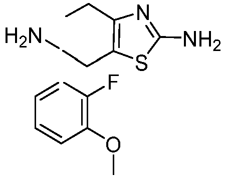 | ESI-MS m/z 294.14 [M+H]⁺ |
| | | ¹H NMR (400 MHz, DMSO-*d*₆) δ 7.03 (s, 3H), 6.63 (s, 2H), 3.81 (s, 3H), 3.07 (d, *J* = 16.1 Hz, 1H), 2.58 (d, *J* = 15.8 Hz, 1H), 2.32-2.29 (m, 1H), 2.16-2.12 (m, 2H), 1.84-1.70 (m, 1H). |
| **A269** | 6-(2-fluoro-3-methoxyphenyl)-*N*⁶-methyl-4,5,6,7-tetrahydrobenzo[*d*]thiazole-2,6-di amine 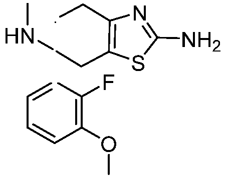 | ESI-MS m/z 308.39 [M+H]⁺ |
| **A270** | 5-phenyl-3-(trifluoromethyl)-4,5,6,7-tetra hydrobenzo[*d*]isoxazol-5-amine 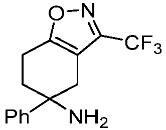 | ESI-MS m/z 283.27 [M+H]⁺ |
| **A271** | *N*-methyl-5-phenyl-3-(trifluoromethyl)-4, 5,6,7-tetrahydrobenzo[*d*]isoxazol-5-amine 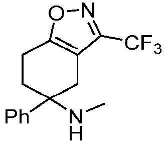 | ESI-MS m/z 297.29 [M+H]⁺ |
| **A272** | 6-(3-methoxyphenyl)-4,5,6,7-tetrahydrob enzo[*d*]thiazol-6-amine hydrobromide 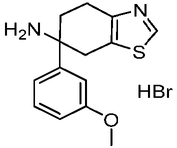 | ESI-MS m/z 261.2 [M+H]⁺ |
| hydrobrom ide | | ¹H NMR (400 MHz, DMSO-*d*₆) δ 8.95 (s, 1H), 8.52 (s, 3H), 7.35 (t, *J* = 8.0 Hz, 1H), 7.12 (t, *J =* 2.2 Hz, 1H), 7.07 (d, *J* = 8.3 Hz, 1H), 6.97 (dd, *J* = 8.2, 2.4 Hz, 1H), 3.76-3.72 (m, 4H), 3.30 (d, *J =* 16.0 Hz,1H), 2.94-2.88 (m, 1H), 2.56 - 2.53 (m, 1H), 2.45 - 2.30 (m, 2H). |
| **A273** | 6-(3-methoxyphenyl)-N-methyl-4,5,6,7-te trahydrobenzo[d]thiazol-6-amine 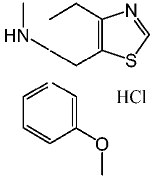 hydrochloride | ESI-MS m/z 275.2 [M+H]⁺ |
| hydrochlor ide | | ¹H NMR (400 MHz, DMSO-*d*₆) δ 10.09 (s, 1H), 9.62 (s, 1H), 8.92 (s, 1H), 7.33 (t, *J* = 8.0 Hz, 1H), 7.16 (t, *J =* 2.1 Hz, 1H), 7.06 (dd, *J* = 7.8, 1.8 Hz, 1H), 6.97 (dd, *J* = 8.1, 2.4 Hz, 1H), 3.74 (s, 3H), 3.44-3.36 (m, 1H), 2.91 (d, *J* = 16.6 Hz, 1H), 2.70-2.67 (m, 1H), 2.55-2.49 (m, 2H), 2.24 - 2.16 (m, 4H). |
| **A274** | *N*-methyl-6-phenyl-4,5,6,7-tetrahydro-1*H* -benzo[*d*]imidazol-6-amine 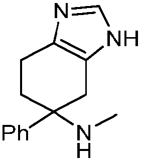 | ESI-MS m/z 228.31 [M+H]⁺ |
| **A275** | 6-amino-6-phenyl-4,5,6,7-tetrahydro-1*H-*benzo[*d*]imidazol-2-ol 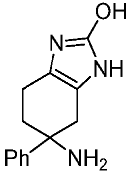 | ESI-MS m/z 230.28 [M+H]⁺ |
| **A276** | 6-(3,5-dimethoxyphenyl)-4,5,6,7-tetrahyd robenzo[*d*]thiazole-2,6-diamine 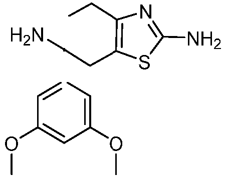 | ESI-MS m/z 306.40 [M+H]⁺ |
| **A277** | 6-(3-cyclopropoxyphenyl)-4,5,6,7-tetrahy drobenzo[*d*]thiazole-2,6-diamine 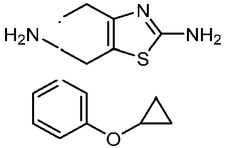 | ESI-MS m/z 302.41 [M+H]⁺ |
| | | ¹H NMR (500 MHz, DMSO-*d*₆) δ 7.26 (t, *J =* 7.9 Hz, 1H), 7.17 (s, 1H), 7.12 (d, *J* = 8.0 Hz, 1H), 6.97 (d, *J* = 8.1 Hz, 1H), 6.69 (s, 2H), 3.79 (tt, *J* = 6.1, 3.1 Hz, 1H), 3.07 (d, *J =* 15.9 Hz, 1H), 2.75 (d, *J* = 16.0 Hz, 1H), 2.51 (s, 1H), 2.20 - 2.15 (m, 1H), 2.11 - 2.05 (m, 1H), 1.96 - 1.91 (m, 1H), 0.82 - 0.73 (m, 2H), 0.69 - 0.59 (m, 2H). |
| **A278** | 6-(3-methoxy-4-(trifluoromethyl)phenyl)-4,5,6,7-tetrahydrobenzo[*d*]thiazole-2,6-di amine 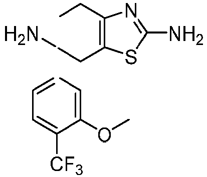 | ESI-MS m/z 344.37 [M+H]⁺ |
| **A279** | 6-(methylamino)-6-phenyl-4,5,6,7-tetrahy drobenzo[*d*]thiazol-2-ol 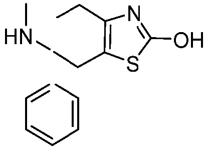 | ESI-MS m/z 261.2 [M+H]⁺ |
| | | ¹H NMR (400 MHz, DMSO-*d*₆) δ 10.80 (s, 1H), 7.40 (d, *J =* 7.7 Hz, 2H), 7.32 (t, *J =* 7.6 Hz, 2H), 7.21 (t, *J =* 7.1 Hz, 1H), 2.84 (d, *J* = 16.2 Hz, 1H), 2.48 (d, *J* = 9.7 Hz, 1H), 2.34 - 2.26 (m, 1H), 2.04 (t, *J* = 6.2 Hz, 2H), 1.91 (s, 3H), 1.88 - 1.79 (m, 1H). |
| **A280** | 6-amino-6-phenyl-4,5,6,7-tetrahydrobenz o[*d*]oxazol-2-ol 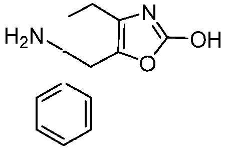 | ESI-MS m/z 231.27 [M+H]⁺ |
| **A281** | 6-(3-(difluoromethyl)phenyl)-4,5,6,7-tetra hydrobenzo[*d*]thiazole-2,6-diamine 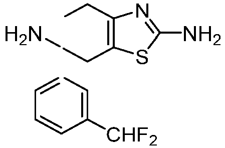 | ESI-MS m/z 296.35 [M+H]⁺ |
| **A282** | 6-(3-(difluoromethoxy)phenyl)-*N*⁶-methyl -4,5,6,7-tetrahydrobenzo[*d*]thiazole-2,6-di amine 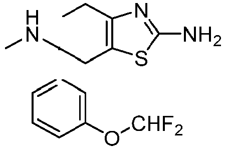 | ESI-MS m/z 326.38 [M+H]⁺ |
| **A283** | 6-(3-methoxyphenyl)-*N*⁶,*N*⁶ -dimethyl-4,5, 6,7-tetrahydrobenzo[*d*]thiazole-2,6-diami ne 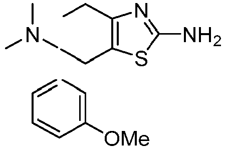 | ESI-MS m/z 304.11[M+H]⁺ |
| | | ¹H NMR (500 MHz, Chloroform-d) δ 7.17 (t, *J =* 8.1 Hz, 1H), 6.97 - 6.91 (m, 2H), 6.76 - 6.73 (m, 1H), 4.87 (s, 2H), 3.76 (s, 3H), 2.99 (d, *J* = 2.6 Hz, 2H), 2.52-2.47 (m, 1H), 2.28-2.19 (m, 7H), 2.11-2.08 (m, 1H), 1.898-1.82(m, 1H). |
| **A284** | 6-([1,1'-biphenyl]-2-yl)-4,5,6,7-tetrahydro benzo[*d*]thiazole-2,6-diamine 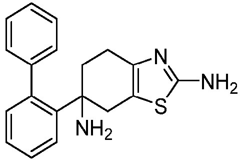 | ESI-MS m/z 322.44 [M+H]⁺ |
| **A285** | 6-([1,1'-biphenyl]-3-yl)-4,5,6,7-tetrahydro benzo[*d*]thiazole-2,6-diamine 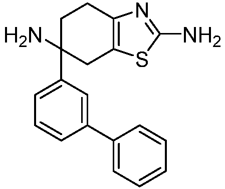 | ESI-MS m/z 322.44 [M+H]⁺ |
| **A286** | 6-(3-(pyridin-4-yl)phenyl)-4,5,6,7-tetrahy drobenzo[*d*]thiazole-2,6-diamine 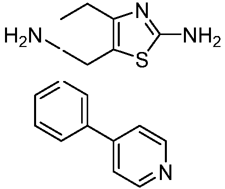 | ESI-MS m/z 323.43 [M+H]⁺ |
| **A287** | 5-phenyl-4,5,6,7-tetrahydrobenzo[*c*]isoxa zol-5-amine 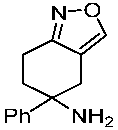 | ESI-MS m/z 198.10 [M-NH₂]⁺ |
| | | ¹H NMR (400 MHz, DMSO-*d*₆) δ 8.40 (s, 1H), 7.57-7.55 (m, 2H), 7.37-7.30 (m, 2H), 7.24-7.20 (m, 1H), 2.90 (d, *J* = 16.1 Hz, 1H), 2.87-2.78 (m, 1H), 2.55 (dd, *J* = 9.7, 3.3 Hz, 2H), 2.25-2.18 (m, 1H), 1.96-1.84 (m, 1H). |
| **A288** | *N*-methyl-5-phenyl-4,5,6,7-tetrahydroben zo[c]isoxazol-5-amine 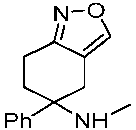 | ESI-MS m/z 229.10 [M+H]⁺ |
| | | ¹H NMR (400 MHz, DMSO-*d*₆) δ 8.12 (s, 1H), 7.42-7.40 (m, 2H), 7.37-7.33 (m, 2H), 7.29-7.26 (m, 1H), 3.07 (d, *J* = 16.0 Hz, 1H), 2.81-2.77 (m, 2H), 2.49-2.36 (m, 3H), 2.16 (s, 3H). |
| **A289** | 6-phenyl-4,5,6,7-tetrahydrobenzo[*c*]isoxa zol-6-amine 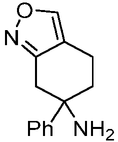 | ESI-MS m/z 215.27 [M+H]⁺ |
| **A290** | *N*-methyl-6-phenyl-4,5,6,7-tetrahydroben zo[c]isoxazol-6-amine 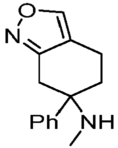 | ESI-MS m/z 229.30 [M+H]⁺ |
| **A291** | 6-(2,4-dimethylphenyl)-4,5,6,7-tetrahydro benzo[*d*]thiazole-2,6-diamine 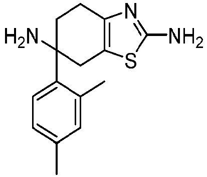 | ESI-MS m/z 274.40 [M+H]⁺ |
| **A292** | 6-(2-isopropylphenyl)-4,5,6,7-tetrahydrob enzo[*d*]thiazole-2,6-diamine 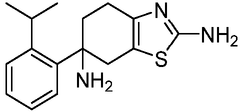 | ESI-MS m/z 288.43 [M+H]⁺ |
| **A293** | 6-(2,3-dichlorophenyl)-4,5,6,7-tetrahydro benzo[*d*]thiazole-2,6-diamine 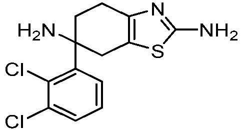 | ESI-MS m/z 315.23 [M+H]⁺ |
| **A294** | 6-(4-fluorophenyl)-*N*-methyl-4,5,6,7-tetra hydrobenzo[*d*]thiazol-6-amine 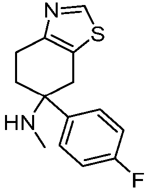 | ESI-MS m/z 263.10 [M+H]⁺ |
| | | ¹H NMR (400 MHz, DMSO-*d*₆) δ 8.84 (s, 1H), 7.50 - 7.38 (m, 2H), 7.17 - 7.09 (m, 2H), 3.39 - 3.36 (m, 1H), 2.98 (d, *J* = 16.4 Hz, 1H), 2.84 - 2.72 (m, 1H), 2.42 - 2.30 (m, 1H), 2.22 - 2.15 (m, 2H), 1.97 (s, 3H). |
| **A295** | 6-amino-6-(3-ethoxyphenyl)-4,5,6,7-tetra hydrobenzo[*d*]thiazol-2-ol hydrochloride 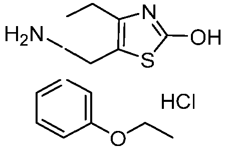 | ESI-MS m/z 289.2 [M-H]⁻ |
| hydrochlor ide | | ¹H NMR (400 MHz, DMSO-*d*₆) δ 11.03 (s, 1H), 8.57 (s, 3H), 7.37 (t, *J =* 8.0 Hz, 1H), 7.11 (d, *J* = 2.2 Hz, 1H), 7.07 (d, *J* = 7.8 Hz, 1H), 6.97 (dd, *J* = 8.2, 2.3 Hz, 1H), 4.08 - 4.02 (m, 2H), 3.19 (d, *J* = 16.6 Hz, 1H), 2.88 (d, *J =* 16.6 Hz, 1H), 2.42 (d, *J =* 8.3 Hz, 2H), 2.28 - 2.21 (m, 1H), 1.99 (d, *J =* 13.0 Hz, 1H), 1.33 (t, *J* = 7.0 Hz, 3H). |
| **A296** | *N*-(2-amino-5-phenyl-4,5,6,7-tetrahydrob enzo[*d*]thiazol-5-yl)acetamide 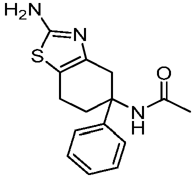 | ESI-MS m/z 288.15 [M+H]⁺ |
| | | ¹H NMR (400 MHz, DMSO-*d*₆) δ 8.05 (s, 1H), 7.38-7.24 (m, 4H), 7.24 - 7.12 (m, 1H), 6.69 (s, 2H), 2.90-2.75 (m, 2H), 2.67 (dt, *J =* 11.3, 4.8 Hz, 1H), 2.45 (d, *J* = 9.0 Hz, 1H), 2.30 (d, *J* = 16.3 Hz, 1H), 2.11 (ddd, *J* = 12.4, 9.1, 5.3 Hz, 1H), 1.80 (s, 3H). |
| **A297** | *N*-(2-amino-5-phenyl-4,5,6,7-tetrahydrob enzo[*d*]thiazol-5-yl)propionamide 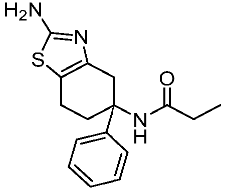 | ESI-MS m/z 302.19 [M+H]⁺ |
| | | ¹H NMR (400 MHz, DMSO-*d*₆) δ 7.96 (s, 1H), 7.35-7.23 (m, 4H), 7.23-7.14 (m, 1H), 6.69 (s, 2H), 2.87-2.77 (m, 2H), 2.69 (dd, *J* = 12.5, 4.8 Hz, 1H), 2.43 (d, *J* = 9.0 Hz, 1H), 2.31 (d, *J* = 16.4 Hz, 1H), 2.16 - 2.04 (m, 3H), 0.92 (t, *J* = 7.6 Hz, 3H). |
| **A298** | 5(4-(difluoromethyl)phenyl)-4,5,6,7-tetra hydrobenzo[*d*]thiazole-2,6-diamine 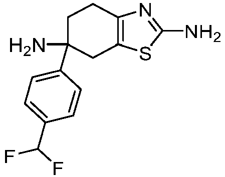 | ESI-MS m/z 296.35 [M+H]⁺ |
| **A299** | 6-amino-6-(3-methoxyphenyl)-3-methyl-4,5,6,7-tetrahydrobenzo[*d*]thiazol-2-one 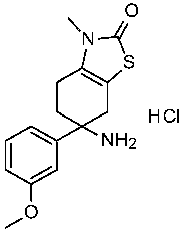 hydrochloride | ESI-MS m/z 291.2 [M+H]⁺ |
| hydrochlor ide | | ¹H NMR (400 MHz, DMSO-*d*₆) δ 8.81 (s, 3H), 7.36 (t, *J =* 8.0 Hz, 1H), 7.22 (t, *J* = 2.2 Hz, 1H), 7.11 (dd, *J =* 7.8, 1.8 Hz, 1H), 6.97 (dd, *J =* 8.3, 2.4 Hz, 1H), 3.78 (s, 3H), 3.22 (d, *J* = 16.5 Hz, 1H), 3.07 (s, 3H), 3.02 (d, *J =* 16.5 Hz, 1H), 2.62-2.58 m, 1H), 2.50-2.45(m, 1H), 2.39-2.32 (m, 1H), 2.18 - 2.14 (m, 1H). |
| **A300** | *N*-(6-amino-6-(3-methoxyphenyl)-4,5,6,7-tetrahydrobenzo[*d*]thiazol-2-yl)acetamide 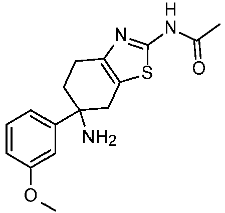 | ESI-MS m/z 316.18 [M-H]⁻ |
| | | ¹H NMR (400 MHz, DMSO-*d*₆) δ 11.82 (s, 1H), 7.20 (t, *J* = 7.9 Hz, 1H), 7.12 - 7.05 (m, 2H), 6.77 (dd, *J* = 8.0, 2.5 Hz, 1H), 3.73 (s, 3H), 3.11 (d, *J* = 16.3 Hz, 1H), 2.71 (dd, *J* = 16.8, 10.5 Hz, 2H), 2.33 (dt, *J* = 10.8, 5.5 Hz, 1H), 2.20 - 2.13 (m, 1H), 2.10 (s, 3H), 1.86 (dd, *J* = 12.7, 6.0 Hz, 1H). |
| **A301** | *N*²-ethyl-6-(3-methoxyphenyl)-4,5,6,7-tetr ahydrobenzo[*d*]thiazole-2,6-diamine hydrochloride 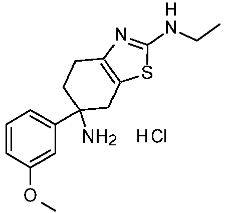 | ESI-MS m/z 304.22 [M+H]⁺ |
| hydrochlor ide | | ¹H NMR (400 MHz, DMSO-*d*₆) δ 9.99 (s, 1H), 8.92 (s, 3H), 7.36 (t, *J =* 8.0 Hz, 1H), 7.21 (d, *J* = 2.2 Hz, 1H), 7.10 (d, *J* = 7.9 Hz, 1H), 6.98 (dd, *J* = 8.3, 2.3 Hz, 1H), 3.78 (s, 3H), 3.51-3.38 (m, 3H), 3.15 (d, *J* = 16.7 Hz, 1H), 2.70 (d, *J =* 18.1 Hz, 1H), 2.50-2.45 (m, 1H), 2.40 - 2.31 (m, 1H), 2.19 - 2.09 (m, 1H), 1.19 (t, *J =* 7.2 Hz, 3H). |
| **A302** | 5-(3-methoxyphenyl)-4,5,6,7-tetrahydrob enzo[*d*]thiazole-2,5-diamine 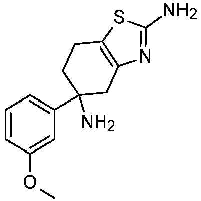 | ESI-MS m/z 276.1 [M+H]⁺ |
| | | ¹H NMR (400 MHz, DMSO-*d*₆) δ 7.21 (t, *J* = 8.0 Hz, 1H), 7.07 - 7.02 (m, 2H), 6.77 (dd, *J* = 8.0, 2.3 Hz, 1H), 6.65 (s, 2H), 3.73 (s, 3H), 2.88 (d, *J* = 16.4 Hz, 1H), 2.64 - 2.56 (m, 1H), 2.52 (d, *J* = 16.0 Hz, 1H), 2.25 - 2.18 (m, 1H), 2.14-2.07 (m, 1H), 1.84-1.78 (m, 1H). |
| **A303** | 6-(benzo[*d*][1,3]dioxol-5-yl)-4,5,6,7-tetra hydrobenzo[*d*]thiazole-2,6-diamine 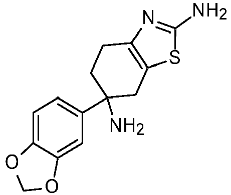 | ESI-MS m/z 290.2 [M+H]⁺ |
| | | ¹H NMR (400 MHz, DMSO-*d*₆) δ 7.11 (d, *J* = 1.8 Hz, 1H), 6.95 (dd, *J* = 8.2, 1.9 Hz, 1H), 6.80 (d, *J =* 8.2 Hz, 1H), 6.62 (s, 2H), 5.96 (d, *J =* 1.9 Hz, 2H), 2.94 (d, *J* = 15.9 Hz, 1H), 2.57 (d, *J* = 16.0 Hz, 1H), 2.53-2.47 (m, 1H), 2.16 - 2.02 (m, 2H), 1.80-1.74 (m, 1H). |
| **A304** | 6-(3-methylthio)phenyl-4,5,6,7-tetrahydro benzo[*d*]thiazole-2,6-diamine 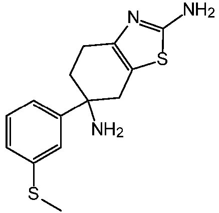 | ESI-MS m/z 292.2 [M+H]⁺ |
| | | ¹H NMR (400 MHz, DMSO-*d*₆) δ 7.42 (s, 1H), 7.35 - 7.19 (m, 2H), 7.10 (d, *J* = 7.3 Hz, 1H), 6.63 (s, 2H), 2.99 (d, *J* = 16.0 Hz, 1H), 2.60 (d, *J =* 16.1 Hz, 1H), 2.50-2.45 (m, 4H), 2.14-2.08 (m, 2H), 1.82 - 1.78 (m, 1H). |
| **A305** | 3-(2-amino-6-dimethylamino-4,5,6,7-tetra hydrobenzo[*d*]thiazol-6-yl)phenol 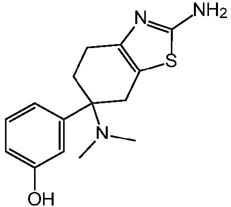 | ESI-MS m/z 290.12[M+H]⁺ |
| | | ¹H NMR (500 MHz, Chloroform-d) δ 7.10 (t, *J* = 7.9 Hz, 1H), 6.93 - 6.86 (m, 1H), 6.83 (t, *J =* 2.1 Hz, 1H), 6.71 - 6.63 (m, 1H), 5.09 - 4.65 (m, 2H), 2.99 - 2.91 (m, 2H), 2.51 - 2.46 (m, 1H), 2.23-2.16 (m, 7H), 2.08 - 2.04 (m, 1H), 1.93-1.84 (m, 1H). |
| **A306** | 6-amino-6-(3-hydroxyphenyl)-4,5,6,7-tetr ahydrobenzo[*d*]thiazol-2-ol 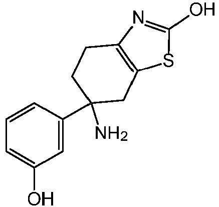 | ESI-MS m/z 263.1 [M+H]⁺ |
| | | ¹H NMR (400 MHz, DMSO-*d*₆) δ 9.29 (s, 1H), 7.11 (t, *J =* 8.1 Hz, 1H), 6.93 - 6.91 (m, 2H), 6.63 (dd, *J =* 8.0, 2.2 Hz, 1H), 2.80 (d, *J* = 16.2 Hz, 1H), 2.47 - 2.38 (m, 2H), 2.12 - 2.00 (m, 2H), 1.85-1.81 (m, 1H). |
| **A307** | 6-(3-methoxyphenyl)-6-methylamino-4,5, 6,7-tetrahydrobenzo[*d*]thiazol-2-ol 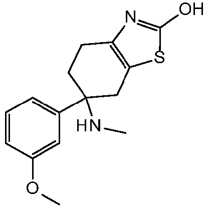 | ESI-MS m/z 291.1 [M+H]⁺ |
| | | ¹H NMR (400 MHz, Methanol-*d*₄) δ 7.45 (t, *J* = 7.9 Hz, 1H), 7.09 (t, *J* = 6.3 Hz, 3H), 3.83 (s, 3H), 3.48 (d, *J* = 16.1 Hz, 1H), 2.97 (d, *J* = 16.1 Hz, 1H), 2.64 - 2.46 (m, 3H), 2.41 (s, 3H), 2.20 (t, *J =* 7.6 Hz, 1H). |
| **A308** | 6-(3-ethoxyphenyl)-4,5,6,7-tetrahydroben zo[*d*]thiazol-6-amine 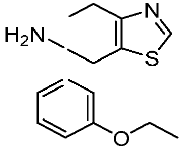 | ESI-MS m/z 275.4 [M+H]⁺ |
| **A309** | 6-(2-methoxypyridin-4-yl)-4,5,6,7-tetrahy drobenzo[*d*]thiazole-2,6-diamine 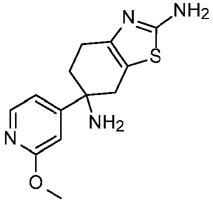 | ESI-MS m/z 277.2 [M+H]⁺ |
| | | ¹H NMR (400 MHz, DMSO-*d*₆) δ 8.08 (d, *J* = 5.5 Hz, 1H), 7.12 (d, *J =* 5.4 Hz, 1H), 6.87 (s, 1H), 6.65 (s, 2H), 3.82 (s, 3H), 2.97 (d, *J =* 16.0 Hz, 1H), 2.60 (d, *J* = 16.0 Hz, 1H), 2.55-2.47 (m, 1H), 2.14-2.07 m, 2H), 1.84-1.78 (m, 1H). |
| **A310** | 6-(6-methoxypyridin-2-yl)-4,5,6,7-tetrahy drobenzo[*d*]thiazole-2,6-diamine 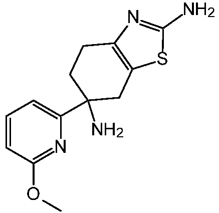 | ESI-MS m/z 277.2 [M+H]⁺ |
| | | ¹H NMR (400 MHz, DMSO-*d*₆) δ 7.67 (t, *J* = 7.8 Hz, 1H), 7.13 (d, *J* = 7.4 Hz, 1H), 6.69 - 6.64 (m, 3H), 3.85 (s, 3H), 3.22 (d, *J* = 16.2 Hz, 1H), 2.62 (d, *J* = 15.8 Hz, 1H), 2.56-2.50 (m, 1H), 2.30-2.19 (m, 2H), 1.88-1.83 (m, 1H). |
| **A311** | 6-(2,6-diamino-4,5,6,7-tetrahydrobenzo[*d* ]thiazol-6-yl)pyridin-2-ol 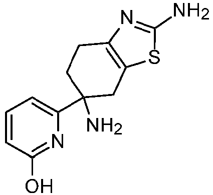 | ESI-MS m/z 263.2 [M+H]⁺ |
| | | ¹H NMR (400 MHz, DMSO-*d*₆) δ 7.40 - 7.36 (m, 1H), 6.66 (s, 2H), 6.22-6.17 (m, 2H), 2.93 (d, *J =* 16.1 Hz, 1H), 2.60 (d, *J* = 16.1 Hz, 1H), 2.57-2.53 (m, 1H), 2.23 - 2.09 (m, 2H), 1.82 - 1.76 (m, 1H). |
| **A312** | 4-(2,6-diamino-4,5,6,7-tetrahydrobenzo[*d* ]thiazol-6-yl)pyridin-2-ol 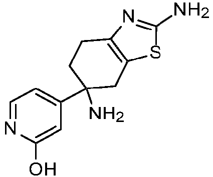 | ESI-MS m/z 263.2 [M+H]⁺ |
| | | ¹H NMR (400 MHz, DMSO-*d*₆) δ 11.33 (s, 1H), 7.27 (d, *J =* 6.7 Hz, 1H), 6.64 (s, 2H), 6.38-6.30 (m, 2H), 2.89 (d, *J =* 15.9 Hz, 1H), 2.56-2.50 (m, 2H), 2.19-2.13 (m, 1H), 2.03-2.00 (m, 1H), 1.77-1.73 (m, 1H). |
| **A313** | 6-(3,4-dichlorophenyl)-4,5,6,7-tetrahydro benzo[*d*]thiazole-2,6-diamine 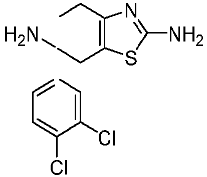 | ESI-MS m/z 314.1 [M+H]⁺ |
| | | ¹H NMR (400 MHz, DMSO-*d*₆) δ 7.77 (d, *J* = 2.2 Hz, 1H), 7.54 (d, *J =* 8.5 Hz, 1H), 7.47-7.45 (m, 1H), 6.64 (s, 2H), 2.97 (d, *J* = 16.0 Hz, 1H), 2.60 (d, *J =* 16.0 Hz, 1H), 2.14-2.07 (m, 2H), 2.03-1.97 (m, 1H), 1.81-1.77 (m, 1H). |
| **A314** | 1-(6-amino-6-(3-methoxyphenyl)-4,5,6,7-tetrahydrobenzo[*d*]thiazol-2-yl)ethan-1-o ne hydrochloride 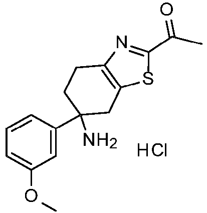 | ESI-MS m/z 303.16[M+H]⁺ |
| hydrochlor ide | | ¹H NMR (400 MHz, DMSO-*d*₆) δ 8.89 (s, 3H), 7.33 (t, *J =* 8.0 Hz, 1H), 7.19 (t, *J* = 2.1 Hz, 1H), 7.07 (dd, *J =* 7.7, 1.8 Hz, 1H), 6.96 (dd, *J =* 8.1, 2.4 Hz, 1H), 3.89 (d, *J* = 17.4 Hz, 1H), 3.76 (s, 3H), 3.51 (m, 1H), 2.97 (m, 1H), 2.62 (m, 1H), 2.56 (s, 3H), 2.48 - 2.34 (m, 2H). |
| **A315** | 6-amino-6-(3-methoxyphenyl)-4,5,6,7-tetr ahydrobenzo[*d*]thiazole-2-formamide 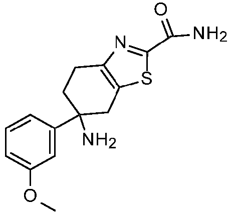 | ESI-MS m/z 304.19[M+H]⁺ |
| | | ¹H NMR (400 MHz, DMSO-*d*₆) δ 8.03 (s, 1H), 7.68 (s, 1H), 7.23 (t, *J =* 7.9 Hz, 1H), 7.15 - 7.06 (m, 2H), 6.79 (dd, *J* = 8.1, 2.6 Hz, 1H), 3.74 (s, 3H), 3.33-3.29 (m, 1H), 2.96-2.88 (m, 2H), 2.61 - 2.54 (m, 1H), 2.21 (m, 2H), 2.03 - 1.88 (m, 2H). |
| **A316** | 2-cyclopropyl-6-(3-methoxyphenyl)-4,5,6 ,7-tetrahydrobenzo[*d*]thiazol-6-amine hydrochloride 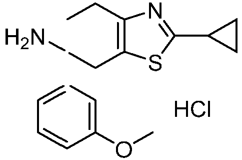 | ESI-MS m/z 301.21[M+H]⁺ |
| hydrochlor ide | | ¹H NMR (400 MHz, DMSO-*d*₆) δ 8.93 (s, 3H), 7.32 (t, *J* = 8.0 Hz, 1H), 7.21 (t, *J* = 2.2 Hz, 1H), 7.07 (dd, *J =* 7.8, 1.8 Hz, 1H), 6.94 (dd, *J =* 8.2, 2.4 Hz, 1H), 3.61 (d, *J* = 16.7 Hz, 1H), 3.36 (d, *J* = 16.7 Hz, 1H), 2.81-2.75 (m, 1H), 2.55 - 2.50 (m, 1H), 2.42-2.33 (m, 2H), 2.24-2.18 (m, 1H), 1.13-1.09 (m, 2H), 0.95-0.92 (m, 2H). |
| **A317** | 2-methoxy-6-(3-methoxyphenyl)-4,5,6,7-t etrahydrobenzo[*d*]thiazol-6-amine 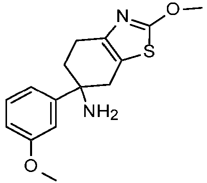 | ESI-MS m/z 291.16[M+H]⁺ |
| | | ¹H NMR (400 MHz, DMSO-*d*₆) δ 7.21 (t, *J* = 7.9 Hz, 1H), 7.14 - 7.05 (m, 2H), 6.82 - 6.75 (m, 1H), 3.94 (s, 3H), 3.73 (s, 3H), 3.05 (d, *J* = 15.8 Hz, 1H), 2.68 - 2.61 (m, 2H), 2.32 - 2.26 (m, 1H), 2.17-1.99 (m, 3H), 1.86 - 1.79 (m, 1H). |
| **A318** | 2-bromo-6-(3-methoxyphenyl)-4,5,6,7-tet rahydrobenzo[*d*]thiazol-6-amine hydrochloride 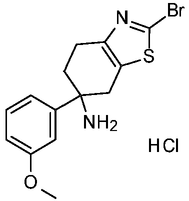 | ESI-MS m/z 340.11 [M+H]⁺ |
| hydrochlor ide | | ¹H NMR (400 MHz, DMSO-*d*₆) δ 8.88 (s, 3H), 7.34 (t, *J =* 8.0 Hz, 1H), 7.20 (t, *J* = 2.2 Hz, 1H), 7.11 - 7.04 (m, 1H), 6.96 (dd, *J* = 8.3, 2.3 Hz, 1H), 3.77 (s, 3H), 3.69 (d, *J* = 17.0 Hz, 1H), 3.34 (d, *J =* 16.7 Hz, 1H), 2.85 (m, 1H), 2.54 (m, 1H), 2.43 - 2.27 (m, 2H). |
| **A319** | 6-(3-methoxyphenyl)-2-phenyl-4,5,6,7-tet rahydrobenzo[*d*]thiazol-6-amine hydrochloride 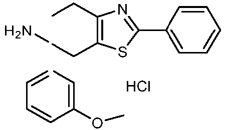 | ESI-MS m/z 337.1 [M+H]⁺ |
| hydrochlor ide | | ¹H NMR (400 MHz, DMSO-*d*₆) δ 8.85 (s, 3H), 7.88 - 7.86 (m, 2H), 7.50 - 7.46 (m, 3H), 7.34 (t, *J =* 8.1 Hz, 1H), 7.22 (t, *J* = 2.1 Hz, 1H), 7.13 - 7.11 (m, 1H), 6.96 (dd, *J =* 8.3, 2.3 Hz, 1H), 3.78-3.74 (m, 4H), 3.44 (d, *J =* 17.0 Hz, 1H), 2.95 - 2.91 (m, 1H), 2.67-2.57 (m, 1H), 2.45 - 2.33 (m, 2H). |
| **A320** | *N*⁶-benzyl-6-(2-methoxyphenyl)-4,5,6,7-t etrahydrobenzo[*d*]thiazole-2,6-diamine 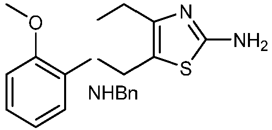 | ESI-MS m/z 366.49[M+H]⁺ |
| | | ¹H NMR (400 MHz, DMSO-*d*₆) δ 7.31 - 7.13 (m, 6H), 7.10 - 7.04 (m, 2H), 6.89 (t, *J =* 7.5 Hz, 1H), 6.61 (s, 2H), 3.84 (s, 3H), 3.27 - 2.68 (m, 4H), 2.49 - 2.37 (m, 2H), 2.13 - 1.99 (m, 1H), 1.89 (d, *J =* 11.7 Hz, 1H). |
| **A321** | 6-(3-isopropoxyphenyl)-4,5,6,7-tetrahydr obenzo[*d*]thiazole-2,6-diamine 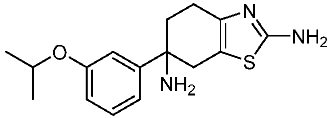 | ESI-MS m/z 304.42[M+H]⁺ |
| | | ¹H NMR (400 MHz, DMSO-*d*₆) δ 7.17 (t, *J* = 7.9 Hz, 1H), 7.08 - 6.93 (m, 2H), 6.73 (dd, *J* = 8.3, 2.5 Hz, 1H), 6.60 (s, 2H), 4.62-4.52 (m, 1H), 2.96 (d, *J* = 16.0 Hz, 1H), 2.57 (d, *J* = 15.9 Hz, 1H), 2.16 - 2.03 (m, 3H), 2.03- 1.8 (m, 1H), 1.24 (dd, *J=* 8.1, 6.0 Hz, 6H). |
| **A322** | 6-(3-isobutoxyphenyl)-4,5,6,7-tetrahydro benzo[*d*]thiazole-2,6-diamine 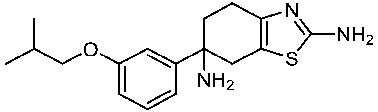 | ESI-MS m/z 318.45[M+H]⁺ |
| | | ¹H NMR (500 MHz, DMSO-*d*₆) δ 7.30 - 7.00 (m, 3H), 6.81 (s, 1H), 6.70 (s, 2H), 3.72 (s, 2H), 3.10 (d, *J* = 15.6 Hz, 1H), 2.77 (d, *J* = 16.5 Hz, 1H), 2.42 - 1.57 (m, 5H), 0.97 (s, 6H). |
| **A323** | 6-(3-cyclopropylphenyl)-4,5,6,7-tetrahydr obenzo[*d*]thiazole-2,6-diamine 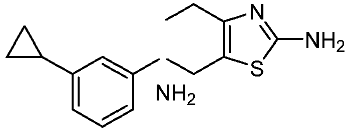 | ESI-MS m/z 286.4 [M+H]⁺ |
| **A324** | 3-(2-amino-6-dimethylamino-4,5,6,7-tetra hydrobenzo[*d*]thiazol-6-yl)phenetole 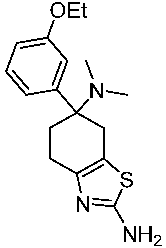 | ESI-MS m/z 304.1 [M+H]⁺ |
| **A325** | 6-(dimethylamino)-6-phenyl-4,5,6,7-tetra hydrobenzo[*d*]thiazol-2-ol oxalate salt 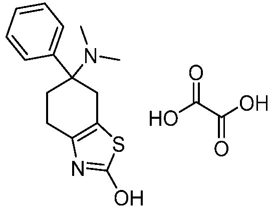 | ESI-MS m/z 275.19 [M+H]⁺ |
| oxalate salt | | 1H NMR (400 MHz, DMSO-*d*₆) δ 10.84 (s, 1H), 7.53 (s, 2H), 7.45-7.38 (m, 3H), 3.29 (d, *J* = 17.0 Hz, 1H), 2.94 (d, *J =* 14.1 Hz, 1H), 2.67-2.56 (m, 1H), 2.38-2.29 (m, 8H), 1.67-1.60 (m, 1H). |
| **A326** | 6-(dimethylamino)-6-(3-hydroxyphenyl)-4,5,6,7-tetrahydrobenzo[*d*]thiazol-2-ol 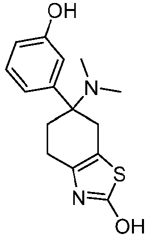 | ESI-MS m/z 291.1 [M+H]⁺ |
| **A327** | 6-amino-3-methyl-6-phenyl-4,5,6,7-tetrah ydrobenzo[*d*]thiazol-2(3H)-one 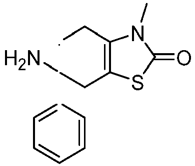 | ESI-MS m/z 261.2 [M+H]⁺ |
| **A328** | 3-(2-amino-6-((2,2,2-trifluoroethyl)amino )-4,5,6,7-tetrahydrobenzo[*d*]thiazol-6-yl)p henol 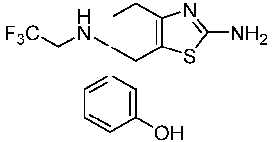 | ESI-MS m/z 344.37 [M+H]⁺ |
| **A329** | 6-(3,4-dimethylphenyl)-4,5,6,7-tetrahydro benzo[*d*]thiazole-2,6-diamine 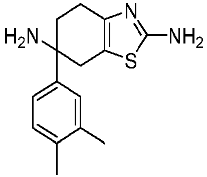 | ESI-MS m/z 274.40 [M+H]⁺ |
| **A330** | 6-(2,3-dihydro-1*H*-inden-5-yl)-4,5,6,7-tet rahydrobenzo[*d*]thiazole-2,6-diamine 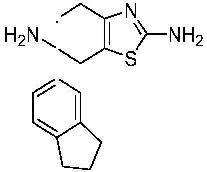 | ESI-MS m/z 286.4 [M+H]⁺ |
| **A331** | *N*⁶-propyl-6-(3-methoxyphenyl)-4,5,6,7-te trahydrobenzo[*d*]thiazole-2,6-diamine 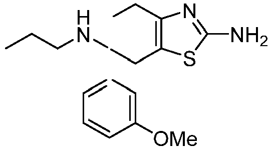 | ESI-MS m/z 318.42 [M+H]⁺ |
| **A332** | 6-(4-chlorophenyl)-*N*⁶-methyl-4,5,6,7-tetr ahydrobenzo[*d*]thiazole-2,6-diamine 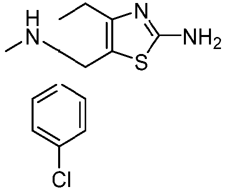 | ESI-MS m/z 294.2 [M+H]⁺ |
| | | ¹H NMR (400 MHz, DMSO-*d*₆) δ 7.48 - 7.37 (m, 2H), 7.42-7.37 (m, 2H), 6.68 (s, 2H), 3.20 (d, *J =* 16.0 Hz, 1H), 2.78 (d, *J* = 16.0 Hz, 1H), 2.52-2.38 (m, 1H), 2.20-2.07 (m, 2H), 1.92-1.84 (m, 1H). |
| **A333** | methyl 6-amino-6-(3-methoxyphenyl)-4,5,6,7-tetr ahydrobenzo[*d*]thiazole-2-carboxylate 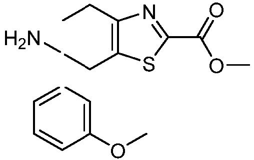 | ESI-MS m/z 319.1 [M+H]⁺ |
| **A334** | ethyl 6-amino-6-(3-methoxyphenyl)-4,5,6,7-tetr ahydrobenzo[*d*]thiazole-2-carboxylate 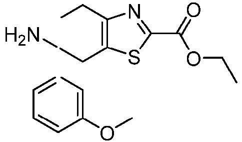 | ESI-MS m/z 333.1 [M+H]⁺ |
| **A335** | 6-amino-6-(3-methoxyphenyl)-4,5,6,7-tetr ahydrobenzo[*d*]thiazole-2-carbonitrile 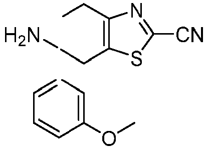 | ESI-MS m/z 286.1 [M+H]⁺ |
| **A336** | 6-(2-fluoro-5-methoxyphenyl)-4,5,6,7-tetr ahydrobenzo[*d*]thiazole-2,6-diamine 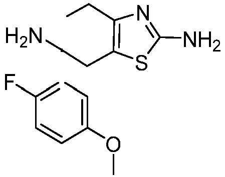 | ESI-MS m/z 294.36 [M+H]⁺ |
| **A337** | 6-(2-fluoro-5-methoxyphenyl)-*N*⁶-methyl-4,5,6,7-tetrahydrobenzo[*d*]thiazole-2,6-di amine 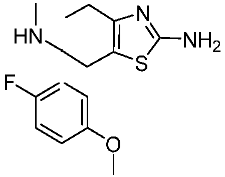 | ESI-MS m/z 308.39 [M+H]⁺ |
| **A338** | 3-phenyl-1,2,3,4-tetrahydrodibenzo[*b*,*d*]th iophen-3-amine 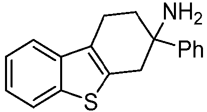 | ESI-MS m/z 280.4 [M+H]⁺ |
| **A339** | 6-(methylamino)-6-phenyl-5,6,7,8-tetrahy droquinolin-2(1*H*)-one 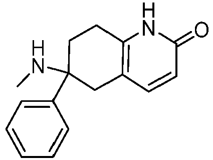 | ESI-MS m/z 255.3 [M+H]⁺. |
| **A340** | 7-amino-7-phenyl-5,6,7,8-tetrahydroquin olin-2(1*H*)-one 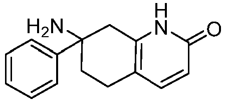 | ESI-MS m/z 241.3 [M+H]⁺. |
| **A341** | 7-(methylamino)-7-phenyl-5,6,7,8-tetrahy droquinolin-2(1*H*)-one 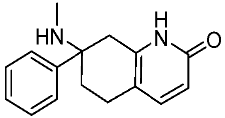 | ESI-MS m/z 255.3 [M+H]⁺. |
| **A342** | 6-(3-ethoxyphenyl)-*N*⁶-methyl-4,5,6,7-tetr ahydrobenzo[*d*]thiazole-2,6-diamine 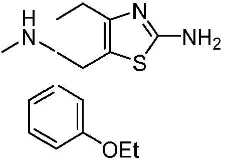 | ESI-MS m/z 304.4 [M+H]⁺ |
| | | ¹H NMR (400 MHz, DMSO-*d*₆) δ 7.22 (t, *J* = 8.2 Hz, 1H), 7.00-6.92 (m, 2H), 6.79 (dd, *J* = 8.0, 2.3 Hz, 1H), 6.64 (s, 2H), 3.99 (m, *J* = 7.0, 2.1 Hz, 2H), 3.44 (dd, *J* = 7.1, 4.6 Hz, 1H), 3.14 (d, *J* = 16.2 Hz, 1H), 2.72 (d, *J* = 16.0 Hz, 1H), 2.44-2.37 (m, 1H), 2.16-2.04 (m, 2H), 1.99 (s, 3H), 1.95 - 1.88 (m, 1H), 1.31 (t, *J* = 7.0 Hz, 3H). |
| **A343** | *N*-(2-amino-6-(3-methoxyphenyl)-4,5,6,7-tetrahydrobenzo[*d*]thiazol-6-yl)formamid e 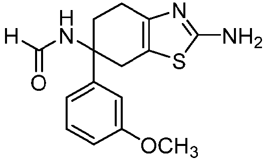 | ESI-MS m/z 304.4 [M+H]⁺ |
| **A344** | *N*-(2-amino-6-(3-(trifluoromethoxy)pheny l)-4,5,6,7-tetrahydrobenzo[*d*]thiazol-6-yl) formamide 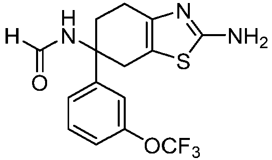 | ESI-MS m/z 358.3 [M+H]⁺ |
| **A345** | 6-(dimethylamino)-6-(4-hydroxyphenyl)-4,5,6,7-tetrahydrobenzo[*d*]thiazol-2-ol 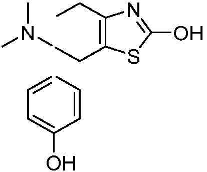 | ESI-MS m/z 291.4 [M+H]⁺ |
| **A346** | 5-phenyl-5,6-dihydro-4*H*-cyclopenta[*d*]th iazole-2,5-diamine 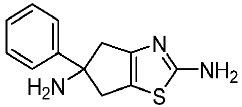 | ESI-MS m/z 232.1 [M+H]⁺ |
| **A347** | 2-bromo-6-(3-methoxyphenyl)-N-methyl-4,5,6,7-tetrahydrobenzo[*d*]thiazol-6-amin e 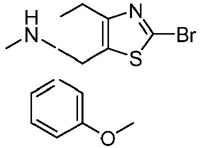 | ESI-MS m/z 353.0 [M+H]⁺ |
| **A348** | 2-methoxy-6-(3-methoxyphenyl)-*N*-meth yl-4,5,6,7-tetrahydrobenzo[*d*]thiazol-6-am ine 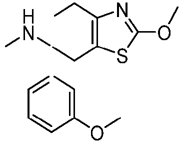 | ESI-MS m/z 305.4 [M+H]⁺ |
| **A349** | *N*-(2-amino-5-(3-methoxyphenyl)-4,5,6,7-tetrahydrobenzo[*d*]thiazol-5-yl)formamid e 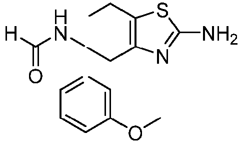 | ESI-MS m/z 304.4 [M+H]⁺ |
| **A350** | 6-(4-(benzyloxy)-3-methoxyphenyl)-4,5,6 ,7-tetrahydrobenzo[*d*]thiazole-2,6-diamin e 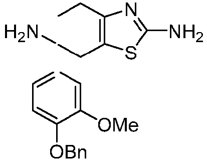 | ESI-MS m/z 382.5 [M+H]⁺ |
| **A351** | 4-(2,6-diamino-4,5,6,7-tetrahydrobenzo[*d* ]thiazol-6-yl)-2-methoxyphenol 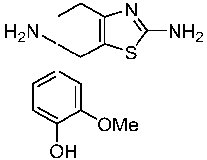 | ESI-MS m/z 292.4 [M+H]⁺ |
| **A352** | 6-(3-ethoxyphenyl)-2-methoxy-4,5,6,7-tet rahydrobenzo[*d*]thiazol-6-amine 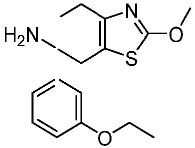 | ESI-MS m/z 305.4 [M+H]⁺ |
| **A353** | 2-ethoxy-6-(3-ethoxyphenyl)-4,5,6,7-tetra hydrobenzo[*d*]thiazol-6-amine 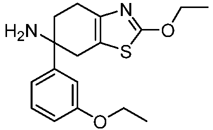 | ESI-MS m/z 319.4 [M+H]⁺ |
| **A354** | 3-(2-amino-6-(methylamino)-4,5,6,7-tetra hydrobenzo[*d*]thiazol-6-yl)benzonitrile 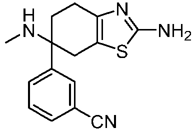 | ESI-MS m/z 285.4 [M+H]⁺. |
| **A355** | 2-chloro-6-phenyl-4,5,6,7-tetrahydrobenz o[*d*]thiazol-6-amine 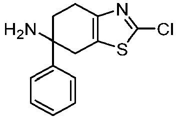 | ESI-MS m/z 265.8 [M+H]⁺. |
| **A356** | 2-chloro-6-(3-methoxyphenyl)-4,5,6,7-tet rahydrobenzo[*d*]thiazol-6-amine 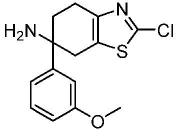 | ESI-MS m/z 295.8 [M+H]⁺. |
| **A357** | 6-(3-ethoxy-4-methylphenyl)-4,5,6,7-tetra hydrobenzo[*d*]thiazole-2,6-diamine 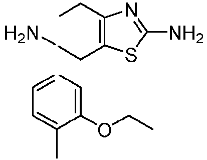 | ESI-MS m/z 304.4 [M+H]⁺ |
| **A358** | 6-(2,4-dichlorophenyl)-4,5,6,7-tetrahydro benzo[*d*]thiazole-2,6-diamine 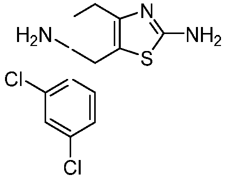 | ESI-MS m/z 315.2 [M+H]⁺ |
| **A359** | 6-(aminomethyl)-6-(3-methoxyphenyl)-4, 5,6,7-tetrahydrobenzo[*d*]thiazol-2-amine 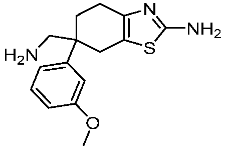 | ESI-MS m/z 390.4 [M+H]⁺ |
| **A360** | 6-(3-methoxyphenyl)-6-((methylamino)m ethyl)-4,5,6,7-tetrahydrobenzo[*d*]thiazol-2 -amine 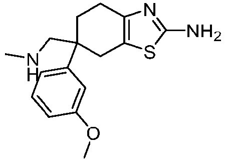 | ESI-MS m/z 304.4 [M+H]⁺ |
| **A361** | 6-((dimethylamino)methyl)-6-(3-methoxy phenyl)-4,5,6,7-tetrahydrobenzo[*d*]thiazol -2-amine 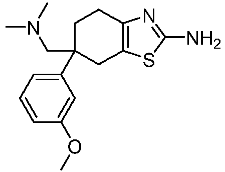 | ESI-MS m/z 318.5 [M+H]⁺ |
| **A362** | 6-((dimethylamino)methyl)-6-phenyl-4,5, 6,7-tetrahydrobenzo[*d*]thiazol-2-amine 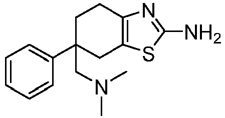 | ESI-MS m/z 288.4 [M+H]⁺. |
| **A363** | 6-(3-ethoxy-4-(trifluoromethyl)phenyl)-4, 5,6,7-tetrahydrobenzo[*d*]thiazole-2,6-dia mine 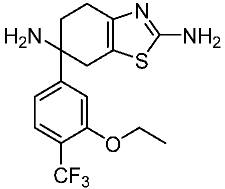 | ESI-MS m/z 358.4 [M+H]⁺ |
| **A364** | *N*-(2-amino-6-(3-methoxyphenyl)-4,5,6,7-tetrahydrobenzo[*d*]thiazol-6-yl)acetamide 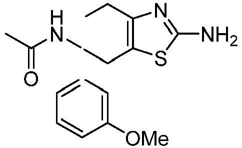 | ESI-MS m/z 318.4 [M+H]⁺ |
| **A365** | 2-(benzyloxy)-6-phenyl-4,5,6,7-tetrahydr obenzo[*d*]thiazol-6-amine 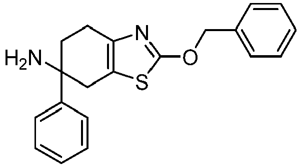 | ESI-MS m/z 337.5 [M+H]⁺ |
| **A366** | 5-phenyl-4,5,6,7-tetrahydrobenzofuran-5-amine 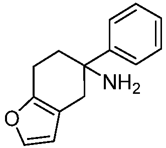 | ESI-MS m/z 214.3 [M+H]⁺ |
| **A367** | *N*-methyl-5-phenyl-4,5,6,7-tetrahydroben zofuran-5-amine 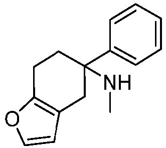 | ESI-MS m/z 228.3 [M+H]⁺ |
| **A368** | 6-(3,4-bis(trifluoromethyl)phenyl)-4,5,6,7 -tetrahydrobenzo[*d*]thiazole-2,6-diamine 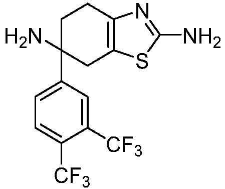 | ESI-MS m/z 382.3 [M+H]⁺ |
| **A369** | 6-(2,3-dihydrobenzofuran-4-yl)-4,5,6,7-te trahydrobenzo[*d*]thiazole-2,6-diamine 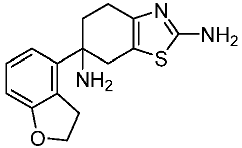 | ESI-MS m/z 288.4 [M+H]⁺ |
| **A370** | 6-(2,3-dihydrobenzofuran-6-yl)-4,5,6,7-te trahydrobenzo[*d*]thiazole-2,6-diamine 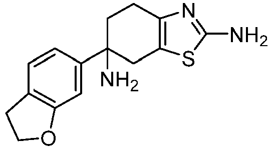 | ESI-MS m/z 288.4 [M+H]⁺ |
| **A371** | (3-(2,6-diamino-4,5,6,7-tetrahydrobenzo[ *d*]thiazol-6-yl)phenyl)methanol 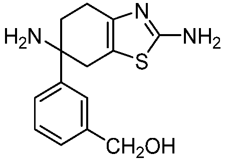 | ESI-MS m/z 276.4 [M+H]⁺ |
| **A372** | (4-(2,6-diamino-4,5,6,7-tetrahydrobenzo[ *d*]thiazol-6-yl)phenyl)methanol 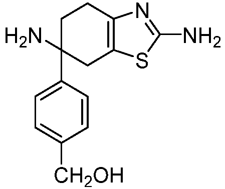 | ESI-MS m/z 276.4 [M+H]⁺ |
| **A373** | (2-(2,6-diamino-4,5,6,7-tetrahydrobenzo[ *d*]thiazol-6-yl)phenyl)methanol 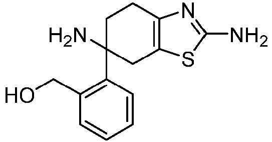 | ESI-MS m/z 276.4 [M+H]⁺ |
| **A374** | *N*⁶-(cyclopropylmethyl)-6-(2-fluoropheny l)-4,5,6,7-tetrahydrobenzo[*d*]thiazole-2,6-diamine 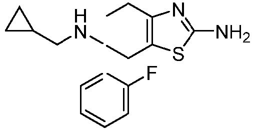 | ESI-MS m/z 318.24 [M+H]⁺ |
| | | ¹H NMR (400 MHz, DMSO-*d*₆) δ 7.27 (q, *J* = 6.9 Hz, 1H), 7.19-7.06 (m, 3H), 6.63 (s, 2H), 3.15 (d, *J* = 16.1 Hz, 1H), 2.73 (d, *J* = 16.1 Hz, 1H), 2.44 (d, *J =* 16.5 Hz, 1H), 2.27 (dd, *J =* 11.9, 6.0 Hz, 2H), 2.10-2.04 (m, 1H), 1.97-1.85 (m, 2H), 0.81-0.74 (m, 1H), 0.33 (d, *J =* 8.1 Hz, 2H), 0.04-0.15 (m, 2H). |
| **A375** | *N,N*-dimethyl-6-phenyl-4,5,6,7-tetrahydro -1*H*-benzo[*d*]imidazol-6-amine 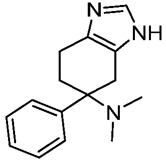 | ESI-MS m/z 242.3 [M+H]⁺ |
| **A376** | *N,N*-dimethyl-5-phenyl-4,5,6,7-tetrahydro -1*H*-indol-5-amine 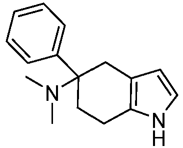 | ESI-MS m/z 241.4 [M+H]⁺ |
| **A377** | 6-amino-6-phenyl-5,6,7,8-tetrahydroquina zolin-2-ol 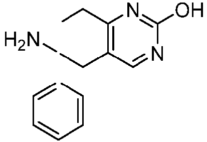 | ESI-MS m/z 242.3 [M+H]⁺ |
| **A378** | 6-(methylamino)-6-phenyl-5,6,7,8-tetrahy droquinazolin-2-ol 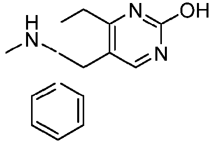 | ESI-MS m/z 256.3 [M+H]⁺ |
| **A379** | 6-(dimethylamino)-6-phenyl-5,6,7,8-tetra hydroquinazolin-2-ol 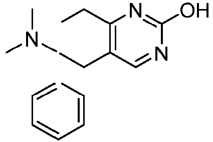 | ESI-MS m/z 270.3 [M+H]⁺ |
| **A380** | 6-(dimethylamino)-6-phenyl-5,6,7,8-tetra hydroquinolin-2(1*H*)-one 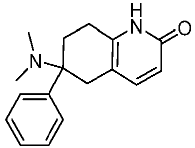 | ESI-MS m/z 269.4 [M+H]⁺. |
| **A381** | 5-(3-methoxyphenyl)-4,5,6,7-tetrahydro-1 *H*-indazol-5-amine 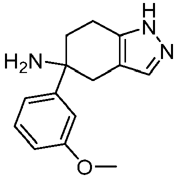 | ESI-MS m/z 244.3 [M+H]⁺. |
| **A382** | 5-(3-methoxyphenyl)-*N*-methyl-4,5,6,7-te trahydro-1*H*-indazol-5-amine 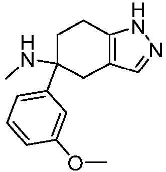 | ESI-MS m/z 258.2 [M+H]⁺. |
| **A383** | 6-(3-methoxyphenyl)-4,5,6,7-tetrahydro-1 *H*-indazol-6-amine 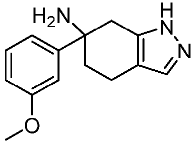 | ESI-MS m/z 244.1 [M+H]⁺. |
| **A384** | 6-(3-methoxyphenyl)-N-methyl-4,5,6,7-te trahydro-1*H*-indazol-6-amine 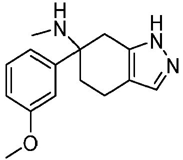 | ESI-MS m/z 258.3 [M+H]⁺. |
| **A385** | 3-phenyl-1,2,3,4-tetrahydrodibenzo[*b,d*]f uran-3-amine 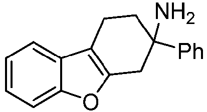 | ESI-MS m/z 264.3 [M+H]⁺ |
| **A386** | 2-phenyl-2,3,4,9-tetrahydro-1*H*-carbazol-2-amine 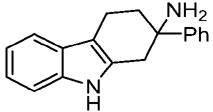 | ESI-MS m/z 263.4 [M+H]⁺ |
| **A387** | 6-(3,5-bis(trifluoromethyl)phenyl)-4,5,6,7 -tetrahydrobenzo[*d*]thiazol-2,6-diamine 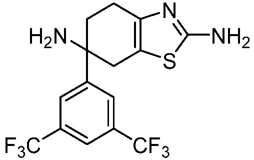 | ESI-MS m/z 382.3 [M+H]⁺ |
| **A388** | 6-(3-(1-methylcyclopropoxy)phenyl)-4,5, 6,7-tetrahydrobenzo[*d*]thiazole-2,6-diami ne 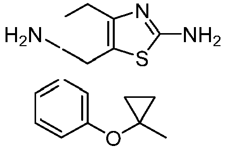 | ESI-MS m/z 316.4 [M+H]⁺ |
| **A389** | *N*⁵-methyl-5-phenyl-5,6-dihydro-4*H*-cycl openta[d]thiazole-2,5-diamine 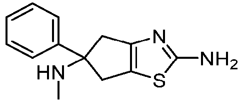 | ESI-MS m/z 246.3 [M+H]⁺ |
| **A390** | 6-phenyl-5,6,7,8-tetrahydro-4*H*-cyclohept a[*d*]thiazole-2,6-diamine 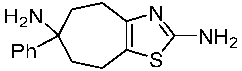 | ESI-MS m/z 260.4 [M+H]⁺. |
| **A391** | *N*⁶-methyl-6-phenyl-5,6,7,8-tetrahydro-4 *H*-cyclohepta[*d*]thiazole-2,6-diamine 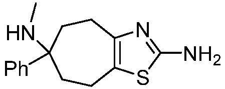 | ESI-MS m/z 274.4 [M+H]⁺. |
| **A392** | 6-phenyl-5,6,7,8-tetrahydroquinolin-6-am ine 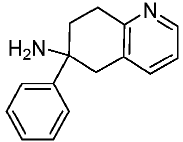 | ESI-MS m/z 225.3 [M+H]⁺. |
| **A393** | *N*-methyl-6-phenyl-56,7,8-tetrahydroquin olin-6-amine 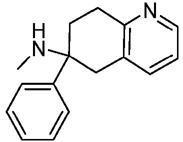 | ESI-MS m/z 239.3 [M+H]⁺. |
| **A394** | 2-((2-amino-6-phenyl-4,5,6,7-tetrahydrob enzo[*d*]thiazol-6-yl)amino)ethan-1-ol 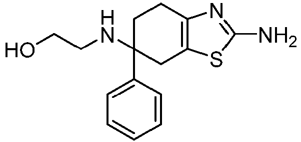 | ESI-MS m/z 290.4 [M+H]⁺. |
| **A395** | *N*⁶-(2-methoxyethyl)-6-phenyl-4,5,6,7-tetr ahydrobenzo[*d*]thiazole-2,6-diamine 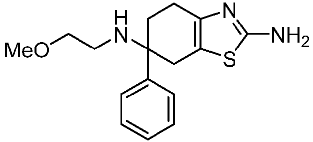 | ESI-MS m/z 304.4 [M+H]⁺. |
| **A396** | 3-(6-amino-2-((2,2,2-trifluoroethyl)amino | ESI-MS m/z 344.2 [M+H]⁺. |
| | )-4,5,6,7-tetrahydrobenzo[*d*]thiazol-6-yl)p henol 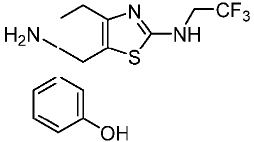 | ¹H NMR (400 MHz, DMSO-*d*₆) δ 9.29 (s, 1H), 7.11 (t, *J =* 7.9 Hz, 1H), 6.91 (d, *J* = 7.1 Hz, 2H), 6.64-6.62 (m, 1H), 4.45 (q, *J* = 9.1, 8.3 Hz, 2H), 2.72 (d, *J* = 16.1 Hz, 1H), 2.51-2.45 (m, 1H), 2.38 (d, *J =* 16.2 Hz, 1H), 2.15-2.06 (m, 2H), 1.87-1.84 (m, 1H). |

In the following pharmacological examples, when the compounds tested are racemic, they are designated as "rac", and when the compounds tested are pure enantiomers, they are designated as "R" or "S".

### Pharmacological Example 1: NMDA Receptor Antagonistic Activity Assay

The effect of the compounds on NMDA receptor (N-methyl-D-aspartate receptor, NR1/2A) channel currents was tested using the electrophysiological whole cell manual patch clamp method.

### Experimental instrument:

Patch clamp amplifier (Multiclamp 700B, Axopatch 200B, Axon, USA)
Digital-to-analog converter (Digidata 1440A, Digidata 1550B, Axon, USA)
Inverted microscope (IX71, IX51, Olympus, Japan)
Rapid administration system (RSC-200, Bio-Logic, France)
Micromanipulator (MX7600R, Syskiyou, USA)
Electrode puller (P-97, Sutter, USA)
Glass electrode (BF150-86-10, Sutter, USA)
Vibration isolation table and shielding mesh (63-534, TMC, USA)
Data acquisition and analysis software (pClamp, Axon, USA)
Carbon dioxide incubator (HERAcell 150i, Thermo, USA)
Biosafety cabinet (MODEL 1384, Thermo, USA)
Water purification system (Milli Q, Millipore, USA)

### Reagents:

Sodium chloride (NaCl) (Sigma, Cat: S7653)
Potassium chloride (KCl) (Sigma, Cat: P9333)
Cesium chloride (CsCl) (Sigma, Cat: V900481)
Cesium fluoride (CsF) (Sigma, Cat: 289345)
Calcium chloride (CaCl₂) (Sigma, Cat: 21115)
Glucose (Sigma, Cat: G7528)
4-(2-Hydroxyethyl)piperazine-1-ethanesulfonic acid, N-(2-hydroxyethyl)piperazine-N-(2-ethanesulfonic acid) (HEPES for short) (Sigma, Cat: H3375)
Ethylene glycol bis(2-aminoethyl ether)tetraacetic acid (EGTA for short) (Sigma, Cat: E3889) Lipofectamine 3000 transfection kit (Gibco, Cat: L3000015) (containing Lipofectamine 3000 and P3000 reagents)
DMEM (Gibco, Cat: C11995500BT)
Fetal bovine serum (FBS) (Gibco, Cat: 10099141)
Opti-MEM (Gibco, Cat: 31985070)
Sodium hydroxide (NaOH) (Sinopharm, Cat: 10019718)
Cesium hydroxide (CsOH) (Sigma, Cat: 232068)
Dimethyl sulfoxide (DMSO) (Sigma, Cat: 276855)
Glutamic acid (Sigma, Cat: G1626-100G)
Glycine (Amresco, Cat: 0167-1KG)
Extracellular fluid formulation (mM): 140 NaCl, 2.8 KCl, 1 CaCl₂, 10 HEPES and 20 Sucrose, adjusted to pH 7.4 with NaOH.
Intracellular fluid formulation (mM): 10 CsCl, 115 CsF, 10 EGTA and 10 HEPES, pH adjusted to 7.2 with CsOH.

### Specific operations:

### a. Cell culture and treatment

The HEK293 cell line was cultured in DMEM medium containing 10% fetal bovine serum at 37 °C with 5% carbon dioxide.

**Cell passage:** the old medium was removed, and the cells were washed once with PBS; 1 mL of 0.25% Trypsine-EDTA solution was then added, and the cells were incubated at room temperature for 1 min. When the cells detached from the dish bottom, 3 mL of complete medium (90% DMEM + 10% FBS) pre-warmed at 37 °C was added. The cell suspension was gently pipetted to separate the aggregated cells. The cell suspension was transferred to a sterile centrifuge tube and centrifuged at 800 rpm for 3 min to collect the cells. The cells were seeded in T25 cell culture flasks (final volume: 6 mL) at a ratio of 1:5 for expansion or maintenance culture.

**Transient transfection:** HEK-293 cells at a cell density of about 80% were replated to 35 mm² cell culture dishes 24 h before the transient transfection, with 3 × 10⁵ cells per cell culture dish. Transient transfection reagents were formulated per well at the following volumes:
1) 7.5 µL of Lipofectamine 3000 was added to 250 µL of Opti-MEM medium, gently pipetted to mix thoroughly, defined as component A, and incubated at room temperature for 5 min;
2) 3.6 µg of pCDNA5-FRT-TO-hNR1-T2A-2A plasmid, 0.4 µg of GFP plasmid, and 7.5 µL of P3000 were added to 250 µL of Opti-MEM medium, gently pipetted to mix thoroughly, defined as component B, and incubated at room temperature for 5 min;
3) component B was added to A, gently pipetted to mix thoroughly, and incubated at room temperature for 15 min to form the DNA-liposome mixture;
4) 500 µL of the DNA-liposome mixture was added to each well of 35 mm² cell culture dish, and the dishes were placed in an incubator for further culture;
5) after 6 h, the medium was replaced, the medium in the 35 mm² dishes was completely aspirated and replaced with 2 mL/well of complete medium (90% DMEM + 10% FBS), and the cells were further cultured for 18 h before patch clamp detection.

**b. Preparation of compound:** on the day of testing, the stock solution of the compound of the present disclosure was diluted with DMSO to an intermediate concentration, which was then further diluted with an extracellular solution (containing 100 µM glutamic acid + 100 µM glycine) to obtain the final concentration to be tested. The DMSO content in the final test concentration did not exceed 0.2%.

### c. Electrophysiological recording

Currents evoked by 100 µM glutamic acid (containing 100 µM glycine) were recorded at room temperature from HEK293 cells transiently expressing NMDA receptor channels using the whole-cell patch clamp technique. Glass microelectrodes were formed by pulling a glass electrode blank (BF150-86-10, Sutter) using a puller. The tip resistance after perfusion of electrode internal solution was about 2-5 MΩ. The glass microelectrodes could be connected to a patch clamp amplifier after being inserted into amplifier probes. The clamping voltage and data recording were controlled and recorded by a computer with a pClamp software, with a sampling frequency of 10 kHz and a filtering frequency of 2 kHz. After whole-cell recording was achieved, the cell was clamped at -70 mV. A gravity-driven rapid administration system was used to apply 100 µM glutamic acid (containing 100 µM glycine) to induce the channel current. After the current stabilized, the 100 µM glutamic acid (containing 100 µM glycine) was administered, and changes in current amplitude were observed. The compounds were administered sequentially from low to high concentration, followed by a final administration of 100 µM glutamic acid (containing 100 µM glycine). Each test concentration of the compound was applied for at least 20 s, and at least 2 cells (n ≥ 2) were tested for each concentration.

### d. Data processing

Data were analyzed using pClamp, GraphPad Prism 8, and Excel software. The inhibition degree of each compound at different concentrations on the channel current (evoked by 100 µM glutamic acid containing 100 µM glycine at -70 mV) was calculated using the following formula:$Inhibition % = \left[1-\left(I/Io\right)\right] \times 100 %$ where Inhibition% represents the percentage inhibition of the NMDA channel current by the compound, and *I* and *I*o are the current amplitudes evoked by 100 µM glutamic acid containing 100 µM glycine after and before administration, respectively.

The IC₅₀ of the compound was calculated using the GraphPad Prism 8 software by equation fitting as follows: $Y = Bottom + \left(Top-Bottom\right) / \left(1+{10}^{∧}\left(\left({LogIC}_{50}-X\right)\times HillSlope\right)\right)$ where X is the Log value of the test concentration of the test sample, Y is the inhibition percentage at the corresponding concentration, and Bottom and Top are the minimum and maximum inhibition percentages, respectively. The test results of some of the compounds are shown in Table 1.

**Table 1:**

| Compound | NMDAR antagonistic activity (IC₅₀, µM) |
|---|---|
| rac-A1 | 4.1 |
| rac-A3 | 16 |
| rac-A9 | 16 |
| rac-A10 | 15 |
| rac-A11 | 0.7 |
| rac-A12 | 3.4 |
| S-A12 | 0.18 |
| rac-A13 | 5.1 |
| S-A13 | 2.5 |
| rac-A14 | 3.8 |
| rac-A16 | 4.2 |
| rac-A18 | 5.7 |
| rac-A19 | 4.5 |
| rac-A21 | 4.1 |
| rac-A22 | 2.5 |
| rac-A25 | 1.2 |
| rac-A30 | 7.7 |
| rac-A32 | 8.6 |
| rac-A34 | 4.8 |
| rac-A3 5 | 5.5 |
| rac-A3 8 | 1.5 |
| rac-A3 9 | 12 |
| rac-A41 | 16 |
| rac-A43 | 6.5 |
| rac-A44 | 12.5 |
| rac-A46 | 10 |
| rac-A57 | 10 |
| rac-A62 | 6.5 |
| rac-A63 | 8.0 |
| rac-A64 | 5.7 |
| rac-A69 | 3.0 |
| rac-A70 | 1.4 |
| S-A70 | 0.84 |
| R-A70 | 16 |
| rac-A71 | 0.4 |
| rac-A82 | 4.6 |
| rac-A83 | 6.5 |
| rac-A85 | 4.7 |
| rac-A87 | 0.9 |
| S-A87 | 0.48 |
| rac-A88 | 13 |
| rac-A93 | 0.5 |
| rac-A95 | 3.1 |
| rac-A102 | 4.3 |
| rac-A104 | 1.6 |
| rac-A118 | 13 |
| rac-A132 | 15 |
| rac-A135 | 3.1 |
| rac-A141 | 3.1 |
| rac-A142 | 6.7 |
| rac-A146 | 3.2 |
| rac-A149 | 6.0 |
| rac-A150 | 3.5 |
| rac-A190 | 1.5 |
| rac-A223 | 2.9 |
| rac-A224 | 2.2 |
| rac-A227 | 1.2 |
| rac-A228 | 0.7 |
| rac-A229 | 0.67 |
| rac-A230 | 2.2 |
| rac-A231 | 1.4 |
| rac-A232 | 5.7 |
| rac-A233 | 10 |
| rac-A237 | 8.4 |
| rac-A238 | 4.3 |
| rac-A239 | 3.7 |
| rac-A240 | 13 |
| rac-A241 | 11 |
| rac-A242 | 9.6 |
| rac-A243 | 1.1 |
| rac-A245 | 2.9 |
| rac-A246 | 9.1 |
| rac-A250 | 3.5 |
| rac-A251 | 0.78 |
| rac-A253 | 5.1 |
| rac-A260 | 0.95 |
| rac-A265 | 2.3 |
| rac-A272 | 6.4 |
| rac-A283 | 14 |
| rac-A287 | 11 |
| rac-A288 | 6.8 |
| rac-A308 | 6.4 |
| rac-A313 | 4.4 |
| rac-A321 | 0.38 |
| rac-A322 | 0.75 |

The above data demonstrate that the compounds in the examples of the present disclosure exhibit certain antagonistic activity on NMDA receptors, indicating their therapeutic effects on central nervous system diseases associated with NMDA receptors.

### Pharmacological Example 2: Human Liver Microsome Assay

**Control and reagent:** NADPH, magnesium chloride, testosterone, phosphate buffer, etc. were supplied by Dalian Meilun Biotech Co., Ltd., etc.

**Liver microsome:** human liver microsomes were supplied by Corning.

**Experimental step:** the liver microsome incubations were performed in 96-well plates, each incubation system having a volume of 200 µL, in a medium of 0.1 M phosphate buffer (pH 7.4), including liver microsomes at a final concentration of 0.2 mg/mL, 1 µM of the test drug, 3.0 mM MgCl₂, 0.01% DMSO, 0.5% acetonitrile, and 2.0 mM NADPH. The incubation system described above without NADPH was pre-incubated at 37 °C for 5 min using a rotary water bath constant temperature oscillator. NADPH was added to initiate the reaction. At 0, 5, 15, 30, and 60 min, 20 µL of the sample was taken out from the incubation system after mixing well and added to 200 µL of acetonitrile containing an internal standard to terminate the reaction. Under the same conditions, 1 µM testosterone was used as a positive control to assess the reliability of the reaction system. For the negative control, phosphate buffer was used instead of NADPH, and all other incubation conditions remained the same. At 60 min, 20 µL of the sample was taken out after mixing well and added to 200 µL of termination solution containing an internal standard to terminate the reaction.

**Sample analysis:** After treatments such as precipitated protein extraction with an organic solvent, the incubated sample was analyzed using liquid chromatography-tandem mass spectrometry (LC-MS/MS). The concentrations of the test drug or the positive control drug in the sample were semi-quantified. The concentration in the sample was expressed using the ratio of the analyte peak area to the internal standard peak area.

**Data processing and analysis:** A standard curve of the ln residual rate of the drug in the incubation system with respect to the incubation time was plotted by using Excel software, linear regression was performed to obtain a slope k, and the half-life T_{1/2} (min) and the intrinsic clearance CLᵢₙₜ (mL/min/kg) were calculated according to the following formula: ${T}_{1 / 2} = - 0.693 / k$ ${CL}_{int} = \frac{0.693}{in vitro {T}_{1 / 2}} ⋅ \frac{incubation volume \left(mL\right)}{microsomes \left(mg\right)} ⋅ \frac{microsomes \left(mg\right)}{liver weight \left(g\right)} ⋅ \frac{liver weight \left(g\right)}{body weight \left(kg\right)}$

The experimental results are shown in Table 2 below:

**Table 2**

| Compound | Half-life (t_{1/2}, min) | Intrinsic clearance (Clᵢₙₜ, mL/min/kg) |
|---|---|---|
| rac-A5 | >120 | NA |
| rac-A7 | >120 | NA |
| rac-A12 | 327 | 12.4 |
| rac-A13 | 266 | 15.2 |
| rac-A14 | >120 | NA |
| rac-A16 | 42 | 95.5 |
| rac-A19 | 80 | 50.6 |
| rac-A32 | 196 | 20.7 |
| rac-A38 | 98 | 41.3 |
| rac-A41 | 128 | 31.6 |
| rac-A43 | 722 | 5.6 |
| rac-A48 | 547 | 7.4 |
| rac-A49 | 117 | 34.7 |
| rac-A55 | 62 | 65.8 |
| rac-A70 | >120 | NA |
| rac-A71 | 210 | 13.8 |
| Testosterone | 15.3 | 265 |

| | | |
|---|---|---|
| NA: not calculable. | | |

The above data demonstrate that the compounds in the examples of the present disclosure have good metabolic stability in human liver microsomes.

### Pharmacological Example 3: In Vivo Metabolism Experiment in Mice

**Animal:** male ICR mice. The animals were randomly assigned to treatment groups and fasted for 12 h prior to dosing.

**Drug:** the compound of the present disclosure was dissolved in a vehicle (5% DMSO + 5% Solutol HS15 + 90% saline). For intravenous (iv) administration, the dose was 2.5 mg/kg with a dosing volume of 5 mL/kg; for oral (po) administration, the dose was 10 mg/kg with a dosing volume of 10 mL/kg body weight. **Sample collection and bioanalysis:** blood was collected from the orbit of the mice and added into EP tubes containing heparin sodium at 0.083, 0.25, 0.5, 1, 2, 4, 6, and 8 h (n = 3 for each time point). Immediately after blood collection, the mice were euthanized by cervical dislocation, and mouse brains were collected at the corresponding time points. All samples were promptly collected, snap-frozen, and stored at -70 °C for later use. Plasma samples were obtained by centrifugation of whole blood. 10 µL of plasma was mixed with 190 µL of internal standard solution (20 ng/mL in acetonitrile containing 0.1% formic acid). After mixing, the samples were centrifuged at 13000 rpm for 10 min, and 120 µL of the supernatant was separately collected. Depending on the sensitivity of the compound, 0.5-10 µL of the supernatant was taken for drug analysis using the appropriate liquid chromatography-tandem mass spectrometry (LC-MS/MS). Each analyte was calibrated and identified using standards. According to brain tissue: the extract (50% acetonitrile/water) at a ratio of 1 g:8 mL was homogenized for 2 min using five 3 mm magnetic beads in an OMNI Bead Ruptor 24 Elite homogenizer at 3.2 m/s and centrifuged at 4 °C and 3000 g for 3 min. 50 µL of the supernatant was mixed with 200 µL of internal standard solution (20 ng/mL in 80% acetonitrile/water). After mixing, the samples were centrifuged at 13,000 rpm for 10 min, and 120 µL of the supernatant was separately collected. Depending on the sensitivity of the compound, 0.5-10 µL of the supernatant was taken for drug analysis using the appropriate LC-MS/MS method. Each analyte was calibrated and identified using standards.

**Data analysis:** drug concentrations below the lower limit of quantification (LLOQ) were considered zero. Pharmacokinetic parameters were analyzed using PK Solver with a non-compartmental, bolus injection or extravascular input analysis models. Data points below the LLOQ were excluded to improve the efficacy of t_{1/2} calculation. The experimental results are shown in Table 3 below:

**Table 3**

| Compound | Cmax (po) (ng/mL) | T_{1/2} (po) (h) | AUC_{0-inf(po)} (ng*h/mL) | F (%) |
|---|---|---|---|---|
| rac-A1 | 413 | 4.68 | 2832 | 132 |
| rac-A3 | 330 | 2.85 | 1162 | 56 |
| rac-A5 | 250 | 2.43 | 1012 | 63 |
| rac-A7 | 208 | 1.72 | 768 | 55 |
| rac-A 13 | 518 | 1.98 | 845 | 78 |
| rac-A14 | 1433 | 1.5 | 2904 | 105 |
| rac-A16 | 252 | 1.76 | 501 | 41 |
| rac-A19 | 284 | 2.84 | 797 | 48 |
| rac-A35 | 1136 | 2.66 | 5107 | / |
| rac-A38 | 360 | 1.8 | 1621 | 71 |
| rac-A41 | 808 | 1.5 | 1749 | 58 |
| rac-A43 | 869 | 1.9 | 2262 | 73 |
| rac-A46 | 2713 | 2.60 | 3905 | / |
| rac-A50 | 150 | 1.48 | 321 | 38 |
| rac-A69 | 456 | 0.69 | 602 | 132 |

The above data show that the compounds in the examples of the present disclosure have good oral bioavailability in mice.

### Pharmacological Example 4: Sigma Receptor Affinity Assay

### 4.1 Materials and reagents

**Cell membrane:** the membrane protein adopted in the experiment was extracted from an HEK293 cell strain stably expressing Sigma1 receptor and an HEK293 cell strain stably expressing Sigma2 receptor, which were constructed by WuXi AppTec.

### Reagents:

[3H]-DTG (PerkinElmer, Cat: NET986250UC)
Haloperidol (Sigma, Cat: Sigma-H1512-5G)
Tris-HCl (Sigma, Cat: T3038-1L)
NaCl (Sigma, Cat: S5150-1L)
PEI (Poly ethyleneimine) (Sigma, Cat: P3143)
Microscint 20 cocktail (PerkinElmer, Cat: 6013329)
Haloperidol (Sigma, Sigma-H1512-5G)

### Instruments and consumables:

Top Seal-A plate seal (Perkin Elmer, Cat# 6050185)
96 well conical polypropylene plates (Agilent, Cat# 50421385)
96-well cell collector (PerkinElmer, Cat# C961961)
Unifilter-96 GF/C filter plate (Perkin Elmer, Cat# 6005174)
Microbeta (PerkinElmer)

### 4.2 Methods and steps

1) Detection buffer: 50 mM Tris-HCl, pH = 7.4, stored at 4 °C.
2) Sigma 1R assay plate washing buffer: 50 mM Tris-HCl, pH = 7.4, stored at 4 °C; sigma2R assay plate washing buffer: 50 mM Tris-HCl, 100 mM NaCl, pH = 7.4, stored at 4 °C.
3) The compounds were diluted to each working concentration: 4-fold serial gradient dilutions for 8 concentrations.

The cell membrane solution and isotope solution were prepared with the pre-cooled detection buffer, with the following configuration information: the membrane concentration was 10 µg/well, and the final concentration of isotopic ligand [3H]-DTG was 5 nM.
5) After the test compound and the positive control were diluted to the working concentration, 1 µL was added to each well of the assay plate. For the Sigma 1R assay, 1 µL of DMSO was added to the high signal control well (high control), and 1 µL of 100 µM Haloperidol (final concentration of 500 nM) was added to the low signal control well (low control). For the Sigma 2R assay, 1 µL of DMSO was added to the high signal control well (high control), and 1 µL of 400 µM Haloperidol (final concentration of 2 µM) was added to the low signal control well (low control).
6) 100 µL of the cell membrane was added to the assay plate per well, followed by addition of 100 µL of the corresponding isotopic ligand.
7) The plate was sealed and incubated with shaking at 37 °C for 2 h. The GF/C filter plate was soaked with 0.3% PEI for at least 30 min.
8) After the incubation was completed, the cell membranes were collected on the GF/C filter plate using a cell collector, which was washed 4 times with the pre-cooled plate washing buffer, and then the GF/C filter plate was dried in an oven at 50 °C for 1 h.
9) The bottom of the dried GF/C filter plate was sealed, 50 µL of a scintillation solution was added to each well, and the well was sealed.
10) The plate was read using Microbeta.
11) Data were analyzed using GraphPad Prism, and IC₅₀ and Ki were calculated. The affinity inhibition constant Ki was calculated using the Cheng Prusoff equation: Ki=IC₅₀/(1+L/K_{D}). The experimental results are shown in Table 4 below:

**Table 4**

| Compound | Sigma1R (Ki, nM) | Sigma2R (Ki, nM) |
|---|---|---|
| rac-A3 | | 124 |
| S-A3 | | 990 |
| R-A3 | | 1762 |
| rac-A13 | | 308 |
| S-A13 | | 257 |
| R-A13 | | 289 |
| rac-A19 | | 523 |
| S-A19 | | 1124 |
| R-A19 | | 1272 |
| rac-A25 | 280 | |
| Positive control Haloperidol | 2.66 | 95 |

The above data in Table 4 demonstrate that the compounds in the examples of the present disclosure exhibit good affinity for sigma receptors, indicating their therapeutic effects on central nervous system diseases associated with sigma receptors.

### Pharmacological Example 5: Forced Swim Test

**Drug:** the compound of the present disclosure was first uniformly mixed with 5% DMSO and 5% solutol^{®} HS 15. hysiological saline was then added to make up 90% of the final volume. The mixture was prepared at an appropriate concentration immediately before use.

**Animal:** male C57 mice, about 22 g. The animals were randomly divided into a blank control group and test drug groups, 8 animals per group, and mice in each group were administered either the vehicle or the test compound via intraperitoneal injection.

**Experimental steps:** the mice were subjected to forced swim test 0.5 h after dosing. The water level in the forced swim device was 45 cm, and the water temperature was 25 °C. The mice were placed in an experimental room to adapt to the environment for 1 h before the experiment began. When the experiment began, the mice were placed in the device for 6 min, and the whole process was recorded by a camera. Only the motionless time of the mice in the last 4 min was counted when data were analyzed.

The experimental results are shown in Table 5 below:

**Table 5**

| Compound | Effective dose (mg/kg) |
|---|---|
| rac-A1 | 1 |
| rac-A3 | 3 |
| rac-A9 | 1 |
| rac-A12 | 1 |
| rac-A13 | 2.5 |
| rac-A14 | 3 |
| rac-A16 | 3 |
| rac-A3 5 | 3 |
| rac-A3 8 | 3 |
| rac-A41 | 3 |
| rac-A48 | 1 |
| rac-A70 | 1 |
| rac-A87 | 3 |
| S-A87 | 3 |
| rac-A132 | 3 |
| rac-A228 | 5 |
| rac-A255 | 5 |
| rac-A256 | 5 |
| rac-A259 | 5 |
| rac-A282 | 5 |

The data show that the drug groups show significant antidepressant-like effect at the low doses of 1-5 mg/kg.

### Pharmacological Example 6: Inhibitory Activity Assay against Monoamine Transporter

### (1) Inhibitory activity assay against 5-HT transporter:

Main reagents and instruments:
HEPES (Invitrogen, Cat: 15630-106)
HBSS (Invitrogen, Cat: 14025)
Bovine Serum Albumin (Sigma, Cat: B2064-100G)
Neurotransmitter transporter uptake assay kit (Molecular devices, Cat: R8174)
Incubator (Thermo, 240)
Envision (Perkin Elmer, envision2014)
384-well plate (Greiner, Cat: 784075)

In HEK-293 cells overexpressing human SERT, the transporter inhibitory effect of the test compound on human SERT was assayed using a Neurotransmitter transporter uptake assay kit (Molecular devices). The assay was performed as described in the kit instructions, using citalopram as a positive control. The specific procedures were as follows:
a) HEK-293-hSERT cells were seeded into a 384-well plate at 20000/well, and the 384-well plate was transferred to an incubator and incubated at 37 °C overnight;
b) the next day, in the 384-well plate, test solutions of citalopram and the compound of the present disclosure were prepared using an experimental buffer (HBSS solution containing 0.1% BSA and 20 mM HEPES). Citalopram was tested at an initial concentration of 1 µM in 3-fold dilution, and the test compound was tested at an initial concentration of 10 µM or 100 µM in 3-fold dilution, with 2 replicates for each concentration;
c) the 384-well plate with HEK-293-hSERT cells cultured therein was taken out from the incubator, the medium was removed from the wells, and 25 µL of the test compound solution was added to each well; the plate was incubated in an incubator at 37 °C for 30 min;
d) 25 µL of dye was added to each well, and the plate was incubated in an incubator at 37 °C for 30 min;
e) fluorescence values were read on Envision and data were analyzed using Graphpad Prism software with results shown in Table 6.

### (2) DAT and NET transporter inhibitory activity test:

Main reagents and instruments:

| **Name** | **Brand** | **Catalog No.** |
|---|---|---|
| Opti-MEM | Gibco | 51985-034 |
| Lipofectamine^{™} 3000 | Invitrogen | L3000015 |
| NET-pcDNA5/FRT plasmid | Genscript | / |
| DMEM | Corning | 10-013-CVR |
| Fetal Bovine Serum (FBS) | AusGeneX | FBS500-S |
| Fetal Bovine Serum, Dialyzed | Gibco | 30067334 |
| Hygromycin B | Solarbio | H8080-1g |
| DMSO | Sigma | D4540 |
| Neurotransmitter Transporter Uptake Assay Kit | Molecular devices | R8174 |
| HBSS | Gibco | 14025-092 |
| CORNING-384 FTC Plate | CORNING YIDA (Changsha Yida | CLS3764 |
| Low-speed centrifuge | Instrument) | TD25M |
| Carbon dioxide incubator | Esco (Singapore) | CCl-170B-8 |
| Multifunctional microplate reader | BioTek (USA) | Synergy 4 |

Transporter inhibition of human DAT and NET expressed in HEK-293 cells was assessed using a neurotransmitter transporter uptake assay kit. The assay was performed as described in the kit instructions, using centanafadine as a positive control. The specific procedures were as follows:

### Preparation of NET transiently transfected cells:

First day cell plating: HEK 293T cells were digested with trypsin, centrifuged, resuspended with a medium, counted, and seeded into a 6 cm culture dish at a density of 3 × 10⁶ cells/well. Second day cell transfection: HEK 293T cells were subjected to medium exchange, and then A, B tubes were prepared for NET-pcDNA5/FRT plasmid to be transfected. 200 µL of Opti-MEM was added to tube A, and 10 µL of Lipofectamine^{™} 3000 was added. The mixture was mixed well. 200 µL of Opti-MEM was added to tube B, and 5 µg of NET-pcDNA5/FRT plasmid was added. The mixture was mixed well, and then 10 µL of P3000^{™} was added to tube B. The mixture was mixed well (plasmid to transfection reagent ratio 1 µg: 2 µL). The diluted solution of tube A was added to the diluted solution of tube B. The mixture was mixed well, and incubated at room temperature for 15 min. Finally, the mixture was gently added to the cells that had been subjected to medium exchange, gently shaken to mix, and then incubated overnight in an incubator at 37 °C with a carbon dioxide concentration of 5%. The cells were used for compound functional activity assays 18-20 h after transfection.

### Preparation of DAT stably transfected cells:

DAT stably transfected culture: The DAT-HEK cell line was cultured in DMEM medium containing 10% fetal bovine serum and 0.2 mg/mL hygromycin B at 37 °C with 5% carbon dioxide.

DAT stably transfected cell passage: the old medium was removed, and the cells were washed once with PBS; 1 mL of TrypLE^{™} Express solution was then added, and the cells were incubated at 37 °C for about 2 min. When the cells were detached from the bottom of the dish, about 5 mL of complete medium pre-heated at 37 °C was added. The cell suspension was gently pipetted to separate the aggregated cells. The cell suspension was transferred to a sterile centrifuge tube and centrifuged at 1000 rpm for 5 min. In order to maintain the physiological activity of the cells, the experimental cell confluence was controlled at about 80%.

### Functional activity assay:

a) After overnight transfection of NET cells, the cells were digested with trypsin, resuspended with DMEM + 10% Dialyzed FBS medium, seeded into a 384-well plate at 20000 cells/well, and incubated overnight. Stably transfected DAT cells were digested with trypsin, resuspended with DMEM + 10% Dialyzed FBS medium, seeded into a 384-well plate at 2500 cells/well, and incubated overnight.
b) 1× Assay buffer was prepared according to the kit instruction for later use; the positive compound and the test compound were serially diluted with DMSO, and then diluted to 2× with 1× assay buffer.
c) The medium was removed from the 384-well plate by centrifugation.
d) 16 µL of the compound prepared in step b) was added to corresponding experimental wells, wherein an initial concentration of 2× positive control compound was added to the positive control well, and 0.2% DMSO buffer was added to the negative control well. After centrifuging, the plate was incubated at 37 °C for 30 min.
e) A detection reagent was prepared using 1× HBSS, and 16 µL of the detection reagent was added to each well. After centrifuging, the plate was incubated at 37 °C for 60 min.
f) After incubation, the reading at 510 nm under the excitation of the detection wavelength of 425 nm was measured using a microplate reader. Curve fitting and IC₅₀ calculation were performed using GraphPad Prism software nonlinear regression method. The results are shown below in Table 6.

**Table 6**

| Compound | DAT (IC₅₀, µM) | 5-HTT (IC₅₀, µM) | NET (IC₅₀, µM) |
|---|---|---|---|
| S-A1 | 6.5 | | |
| R-A1 | | 1.0 | |
| rac-A3 | 1.0 | 1.2 | |
| S-A3 | 1.6 | | |
| R-A3 | | 1.2 | |
| rac-A5 | 2.0 | | 3.3 |
| rac-A7 | 4.5 | | |
| rac-A8 | | 0.007 | |
| rac-A9 | | 0.8 | |
| rac-A10 | 6.6 | 1.3 | 4.2 |
| R-A12 | 2.0 | | 3.8 |
| S-A14 | 11.0 | | |
| R-A14 | | 0.067 | |
| S-A19 | 2.2 | | |
| R-A19 | | 1.2 | |
| R-A21 | | 0.91 | |
| S-A21 | 0.25 | | |
| R-A22 | | 0.71 | |
| S-A22 | 2.2 | | |
| R-A25 | | 0.9 | |
| S-A25 | 0.81 | | |
| rac-A41 | | 1.8 | |
| rac-A47 | 1.3 | | |
| rac-A48 | 0.76 | | 4.6 |
| rac-A49 | 0.33 | 1.2 | 2.5 |
| rac-A50 | 0.1 | 1.7 | 0.8 |
| R-A50 | | | 0.2 |
| S-A50 | 2.4 | | |
| rac-A51 | 0.2 | 1.2 | 0.3 |
| rac-A54 | 2.8 | 1.9 | |
| rac-A5 5 | 4.5 | 7.2 | |
| rac-A5 8 | 2.7 | | |
| rac-A65 | | 1.9 | 7.0 |
| rac-A67 | | | 3.0 |
| rac-A68 | | 0.7 | |
| R-A70 | 8.4 | | |
| rac-A81 | | 0.094 | |
| R-A87 | | 3.2 | |
| rac-A88 | | 0.9 | |
| rac-A89 | | 7.6 | |
| rac-A94 | | 4.3 | |
| rac-A96 | | | 14.0 |
| rac-A98 | | 16.0 | |
| rac-A99 | 11.0 | | |
| rac-A100 | | 17.0 | |
| rac-A114 | 1.0 | 1.0 | 3.5 |
| rac-A118 | | 2.0 | |
| rac-A131 | | 3.6 | |
| rac-A134 | | 17.0 | |
| rac-A145 | | 0.6 | |
| rac-A220 | | 8.9 | |
| rac-A222 | | 10 | |
| rac-A225 | | 1.0 | |
| rac-A226 | 2.0 | 2.0 | |
| S-A228 | 1.8 | | |
| R-A228 | | 2.9 | |
| rac-A233 | | 0.3 | |
| rac-A234 | 6.3 | 0.3 | |
| rac-A235 | | 5.9 | |
| rac-A236 | | 2.8 | |
| rac-A244 | | 1.5 | |
| rac-A247 | 1.5 | 4.5 | |
| rac-A249 | | 1.0 | |
| rac-A252 | 9.5 | | |
| rac-A254 | | 2.0 | |
| rac-A257 | | 0.3 | |
| rac-A262 | | 0.14 | |
| rac-A273 | | 1.0 | |
| rac-A279 | | 0.028 | |
| rac-A283 | 0.45 | | |
| rac-A294 | | 1.1 | |
| rac-A295 | | 0.11 | |
| rac-A299 | | 0.52 | |
| rac-A300 | | 2.1 | |
| rac-A301 | | 4.0 | |
| rac-A303 | 8.2 | | |
| rac-A304 | | 3.7 | |
| rac-A305 | 0.24 | | |
| rac-A306 | | 0.2 | |
| rac-A309 | 14.0 | | |
| rac-A310 | | 16.0 | |
| rac-A314 | | 5.6 | |
| rac-A315 | | 6.8 | |
| rac-A316 | | 4.0 | |
| rac-A317 | | 5.1 | |
| rac-A318 | | 4.6 | |
| rac-A319 | | 0.98 | |
| rac-A321 | 5.9 | | |
| rac-A332 | 3.2 | | |
| rac-A342 | 0.35 | | |
| rac-A374 | 3.8 | | |
| rac-A396 | 18.0 | | |
| Centanafadine | 0.08 | | 0.03 |
| Citalopram | | 0.008 | |

The above data demonstrate that the compounds of the present disclosure exhibit certain antagonistic activity on monoamine transporters, indicating their therapeutic effects on central nervous system diseases associated with monoamine transporters.

## Claims

1. A compound of formula (I) or a stereoisomer thereof, a geometric isomer thereof, a conformational isomer thereof, or a tautomer thereof, or a pharmaceutically acceptable salt thereof, a polymorph thereof, a solvate thereof, a hydrate thereof, or an isotopically labeled compound thereof: 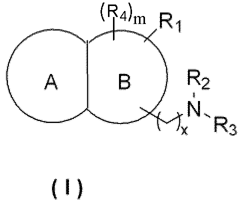 wherein:
ring A is selected from the group consisting of a 4- to 10-membered heterocyclic ring and a C6-C10 aromatic ring fused 4- to 10-membered heterocyclic ring, wherein the 4- to 10-membered heterocyclic ring comprises 1-3 heteroatoms selected from the group consisting of N, O, and S, and preferably, the 4- to 10-membered heterocyclic ring is a 5- to 8-membered heterocyclic ring; preferably, the heterocyclic ring in ring A comprises one N atom and one S atom, or two N atoms, or one S atom, or two N atoms and one S atom, or one N atom and one O atom;
ring A is optionally substituted with one or more R₅, wherein each R₅ is independently selected from the group consisting of halogen, hydroxy, amino, cyano, carboxy, oxo, C1-C6 alkyl, halogenated C1-C6 alkyl, C1-C6 alkoxy, C1-C6 alkanoyl, carbamoyl (-CONH₂), carbamoyl substituted with C1-C6 alkyl, amino substituted with one or two C1-C6 alkyl, amino substituted with one or two halogenated C1-C6 alkyl, amino substituted with one or two C1-C6 alkanoyl, C1-C6 alkoxycarbonyl, C3-C6 cycloalkyl, 4- to 10-membered heterocycloalkyl, C6-14 aryl, 5- to 10-membered heteroaryl, C6-C14 aryl C1-C6 alkyloxy, and 5- to 10-membered heteroaryl C1-C6 alkyloxy; preferably, each R₅ is independently selected from the group consisting of halogen, hydroxy, amino, cyano, carboxy, oxo, C1-C4 alkyl, halogenated C1-C4 alkyl, C1-C4 alkoxy, C1-C4 alkanoyl, carbamoyl (-CONH₂), carbamoyl substituted with C1-C4 alkyl, amino substituted with one or two C1-C4 alkyl, amino substituted with one or two halogenated C1-C4 alkyl, amino substituted with one or two C1-C4 alkanoyl, C1-C4 alkoxycarbonyl, C3-C6 cycloalkyl, 4- to 8-membered heterocycloalkyl, C6-10 aryl, 5- to 10-membered heteroaryl, C6-C10 aryl C1-C4 alkyloxy, and 5- to 10-membered heteroaryl C1-C4 alkyloxy; more preferably, each R₅ is independently selected from the group consisting of halogen (particularly bromine), amino, hydroxy, cyano, carboxy, C1-C3 alkyl (particularly methyl, ethyl, and isopropyl), halogenated C1-C3 alkyl (particularly trifluoromethyl), C1-C3 alkoxy (particularly methoxy and ethoxy), C1-C3 alkanoyl (particularly formyl and acetyl), carbamoyl (-CONH₂), formylamino, acetylamino, methylamino, ethylamino, N,N-dimethylamino, 2,2,2-trifluoroethylamino, C1-C3 alkoxycarbonyl (particularly methoxycarbonyl and ethoxycarbonyl), C3-C5 cycloalkyl (particularly cyclopropyl), phenyl, pyridinyl, pyrrolidinyl, piperidinyl, morpholinyl, and benzyloxy;
ring B is a 3- to 10-membered carbocyclic ring, preferably a 5- to 8-membered carbocyclic ring, such as a 5-, 6-, 7-, or 8-membered carbocyclic ring, and more preferably a 5-, 6-, or 7-membered carbocyclic ring;
R₁ and 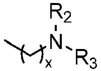 are attached to the same ring carbon atom in ring B;
x is an integer of 0 to 2;
R₂ and R₃ are each independently selected from the group consisting of hydrogen, C1-C6 alkyl, halogenated C1-C6 alkyl, C1-C6 alkoxy C1-C6 alkyl, hydroxy C1-C6 alkyl, C6-C14 aryl C1-C6 alkyl, C3-C6 cycloalkyl, C1-C6 alkanoyl, halogenated C1-C6 alkanoyl, C3-C6 cycloalkyl C1-C6 alkanoyl, C6-C14 aryl C1-C6 alkanoyl, C1-C6 alkylsulfonyl, C1-C6 alkylsulfinyl, and C3-C6 cycloalkyl C1-C6 alkyl; preferably, R₂ and R₃ are each independently selected from the group consisting of hydrogen, C1-C4 alkyl, halogenated C1-C4 alkyl, C1-C4 alkoxy C1-C4 alkyl, hydroxy C1-C4 alkyl, C6-C14 aryl C1-C4 alkyl, C3-C6 cycloalkyl, C1-C4 alkanoyl, halogenated C1-C4 alkanoyl, C3-C6 cycloalkyl C1-C4 alkanoyl, C6-14 aryl C1-C4 alkanoyl, C1-C4 alkylsulfonyl, C1-C4 alkylsulfinyl, and C3-C6 cycloalkyl C1-C4 alkyl; preferably, R₂ and R₃ are selected from the group consisting of hydrogen, C1-C4 alkyl (particularly methyl, ethyl, propyl, isopropyl, and *tert-butyl),* halogenated C1-C4 alkyl (particularly 1,1,1-trifluoroethyl), methoxyethyl, hydroxymethyl, hydroxyethyl, C3-C6 cycloalkylmethyl (particularly cyclopropylmethyl and cyclobutylmethyl), benzyl, C3-C6 cycloalkyl (particularly cyclopropyl), C1-C3 alkanoyl (particularly acetyl and propionyl), cyclopropylformyl, benzoyl, and tert-butylsulfinyl;
or, R₂ and R₅, together with the nitrogen atom to which they are attached, form a 3- to 9-membered heterocycloalkyl, whichoptionally comprises one or more additional nitrogen or oxygen atoms in the ring and is optionally substituted with one or more C1-C6 alkyl, preferably C1-C4 alkyl; preferably, R₂ and R₅, together with the nitrogen atom to which they are attached, form azetidinyl, pyrrolidinyl, piperidinyl, azepanyl, or morpholinyl;
R₁ is selected from the group consisting of C6-C14 aryl, 5- to 10-membered heteroaryl, 4- to 10-membered heterocyclyl, C6-C10 aryl fused 4- to 10-membered carbocyclic ring (e.g., indanyl), and C6-C10 aryl fused 4- to 10-membered heterocyclic ring (e.g., 1,2-methylenedioxyphenyl and 2,3-dihydrobenzofuranyl); preferably, R₁ is selected from the group consisting of C6-C14 aryl and 5- to 10-membered heteroaryl; more preferably, R₁ is phenyl, naphthyl, quinolyl, isoquinolyl, pyridinyl, pyrimidinyl, pyrazinyl, pyridazinyl, thienyl, or thiazolyl; still preferably, R₁ is phenyl, naphthyl, pyrimidinyl, pyridinyl, pyrazinyl, pyridazinyl, or quinolyl; further preferably, R₁ is phenyl;
the C6-C14 aryl, 5- to 10-membered heteroaryl, 4- to 10-membered heterocyclyl, C6-C10 aryl fused 4- to 10-membered carbocyclic ring, and C6-C10 aryl fused 4- to 10-membered heterocyclic ring are optionally substituted with one or more R₆;
R₆ are each independently selected from the group consisting of halogen, hydroxy, sulfhydryl, cyano, carbamoyl (NH₂CO-), sulfamoyl (NH₂SO₂-), C1-C6 alkyl, halogenated C1-C6 alkyl, hydroxy C1-C6 alkyl, C3-C6 cycloalkyl, C3-C6 cycloalkoxy, C3-C6 cycloalkyl substituted with C1-C3 alkyl, C3-C6 cycloalkoxy substituted with C1-C3 alkyl, C1-C6 alkoxy, C1-C6 alkylthio, halogenated C1-6 alkoxy, C3-C6 cycloalkyl C1-C6 alkoxy, C1-C6 alkanoyloxy, C6-C14 aryl, 5- to 10-membered heteroaryl, C6-C14 aryl C1-C6 alkoxy, and 5- to 10-membered heteroaryl C1-C6 alkoxy; preferably, R₆ are each independently selected from the group consisting of halogen, cyano, hydroxy, carbamoyl (NH₂CO-), sulfamoyl (NH₂SO₂-), C1-C4 alkyl, halogenated C1-C4 alkyl, hydroxy C1-C4 alkyl, C3-C6 cycloalkyl, C3-C6 cycloalkoxy, C3-C6 cycloalkyl substituted with C1-C3 alkyl, C3-C6 cycloalkoxy substituted with C1-C3 alkyl, C1-C4 alkoxy, C1-C4 alkylthio, halogenated C1-C4 alkoxy, C3-C6 cycloalkyl C1-C3 alkoxy, C1-C3 alkanoyloxy, C6-C10 aryl, 5- to 6-membered heteroaryl, C6-C10 aryl C1-C4 alkoxy, and 5- to 6-membered heteroaryl C1-C4 alkoxy; more preferably, R₆ are each independently selected from the group consisting of fluorine, chlorine, bromine, cyano, hydroxy, methyl, isopropyl, difluoromethyl, trifluoromethyl, hydroxymethyl, methylcyclopropyloxy, methoxy, ethoxy, isopropoxy, isobutoxy, methylthio, cyclopropyl, cyclopropoxy, carbamoyl (NH₂CO-), sulfamoyl (NH₂SO₂-), difluoromethoxy, trifluoromethoxy, 1,1,1-trifluoroethoxy, cyano, cyclopropylmethoxy, acetoxy, phenyl, pyridinyl, and benzyloxy;
m is an integer of 0 to 3; and
R₄ is attached to any carbon atom of ring B other than the carbon atom to which both R₁ and 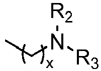 are attached, and R₄ are each independently selected from the group consisting of hydroxy, halogen, and C1-C6 alkyl.

2. The compound of formula (I) or the stereoisomer thereof, the geometric isomer thereof, the conformational isomer thereof, or the tautomer thereof, or the pharmaceutically acceptable salt thereof, the polymorph thereof, the solvate thereof, the hydrate thereof, or the isotopically labeled compound thereof according to claim 1, wherein,
ring A is selected from the group consisting of a 5- or 6-membered heterocyclic ring and a benzo 5- or 6-membered heterocyclic ring;
preferably, ring A is selected from the group consisting of 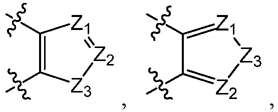 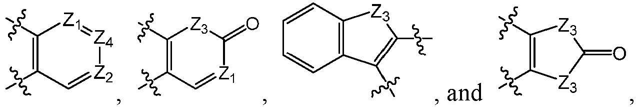
wherein Z₁, Z₂, and Z₄ are independently selected from the group consisting of N and CR₇, and Z₃ is selected from the group consisting of NR₇, O, and S; and Z₁, Z₂, and Z₄ are not simultaneously CR₇, wherein each R₇ is independently selected from the group consisting of hydrogen, halogen, cyano, hydroxy, amino, carbamoyl (-CONH₂), carbamoyl substituted with C1-C6 alkyl, carboxy, C1-C6 alkyl, C1-C6 alkoxy, halogenated C1-C6 alkyl, amino substituted with C1-C6 alkyl, amino substituted with halogenated C1-C6 alkyl, amino substituted with C1-C6 alkanoyl, C1-C6 alkanoyl, C1-C6 alkoxycarbonyl, C3-C6 cycloalkyl, 4- to 8-membered heterocycloalkyl, C6-10 aryl, 5- to 10-membered heteroaryl, C6-C10 aryl C1-C4 alkyloxy, and
5- to 10-membered heteroaryl C1-C4 alkyloxy; and more preferably, ring A is selected from the group consisting of 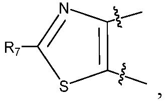 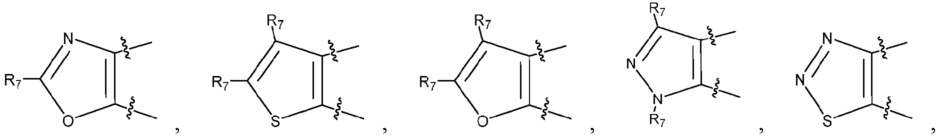 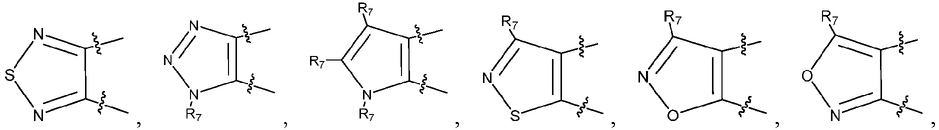 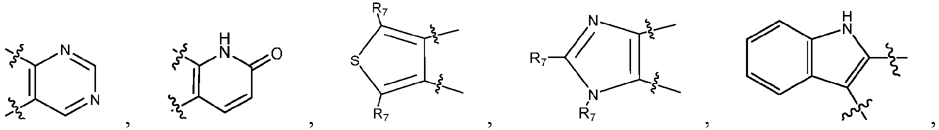 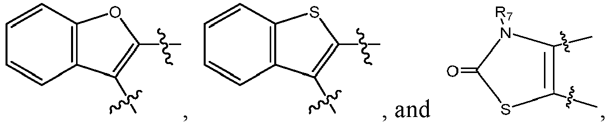 wherein each R₇ is independently selected from the group consisting of hydrogen, halogen (particularly bromine), amino, hydroxy, cyano, carboxy, C1-C3 alkyl (particularly methyl, ethyl, and isopropyl), halogenated C1-C3 alkyl (particularly trifluoromethyl), C1-C3 alkoxy (particularly methoxy and ethoxy), C1-C3 alkanoyl (particularly formyl and acetyl), carbamoyl (-CONH₂), formylamino, acetylamino, methylamino, ethylamino, N,N-dimethylamino, 2,2,2-trifluoroethylamino, C1-C3 alkoxycarbonyl (particularly methoxycarbonyl and ethoxycarbonyl), C3-C5 cycloalkyl (particularly cyclopropyl), morpholinyl, phenyl, pyridinyl, pyrrolidinyl, piperidinyl, and benzyloxy.

3. The compound of formula (I) or the stereoisomer thereof, the geometric isomer thereof, the conformational isomer thereof, or the tautomer thereof, or the pharmaceutically acceptable salt thereof, the polymorph thereof, the solvate thereof, the hydrate thereof, or the isotopically labeled compound thereof according to claim 1 or 2, wherein,
R₁ is phenyl or naphthyl optionally substituted with one or more R₆,
wherein R₆ is as defined in claim 1.

4. The compound of formula (I) or the stereoisomer thereof, the geometric isomer thereof, the conformational isomer thereof, or the tautomer thereof, or the pharmaceutically acceptable salt thereof, the polymorph thereof, the solvate thereof, the hydrate thereof, or the isotopically labeled compound thereof according to claim 3, wherein,
one of R₂ and R₃ is hydrogen, or
both R₂ and R₃ are hydrogen.

5. The compound of formula (I) or the stereoisomer thereof, the geometric isomer thereof, the conformational isomer thereof, or the tautomer thereof, or the pharmaceutically acceptable salt thereof, the polymorph thereof, the solvate thereof, the hydrate thereof, or the isotopically labeled compound thereof according to claim 2, wherein the compound of formula (I) is selected from the group consisting of: 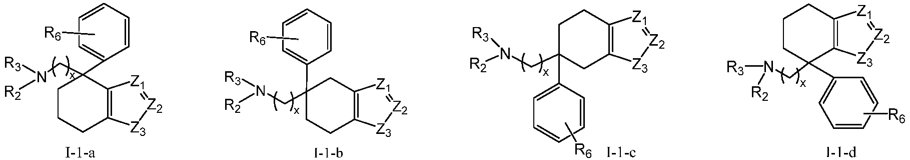 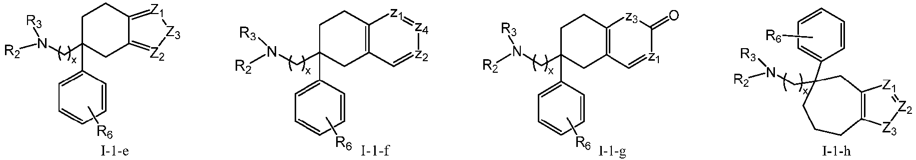 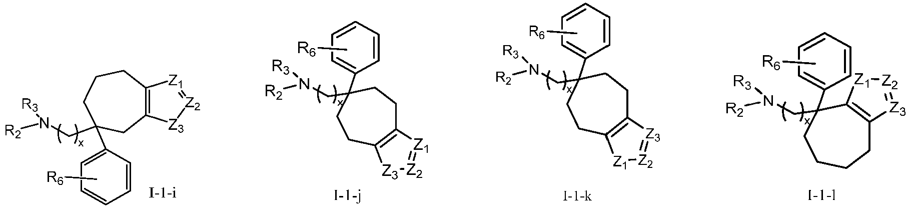 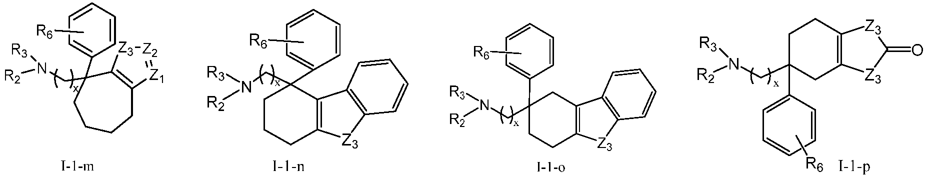 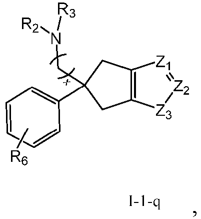 wherein x, Z₁, Z₂, Z₃, Z₄, R₂, R₅, and R₆ are as defined in claim 2.

6. The compound of formula (I) or the stereoisomer thereof, the geometric isomer thereof, the conformational isomer thereof, or the tautomer thereof, or the pharmaceutically acceptable salt thereof, the polymorph thereof, the solvate thereof, the hydrate thereof, or the isotopically labeled compound thereof according to claim 1, wherein the compound of formula (I) is selected from the group consisting of: 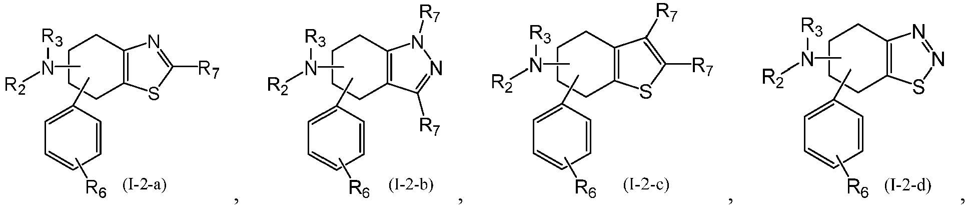 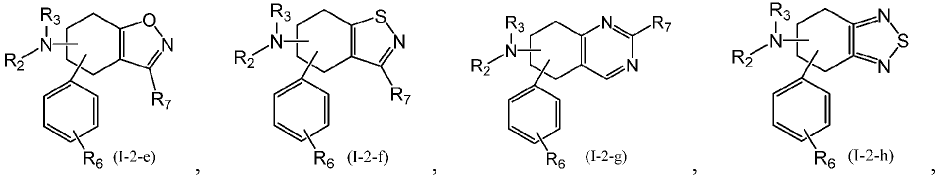 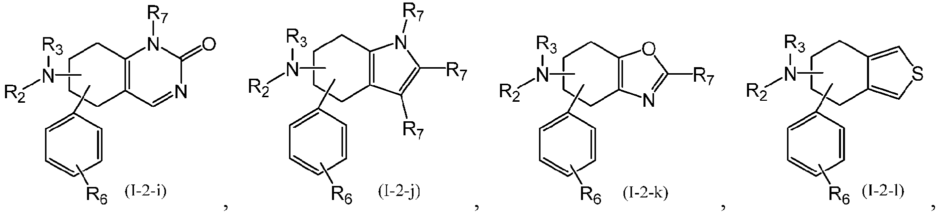 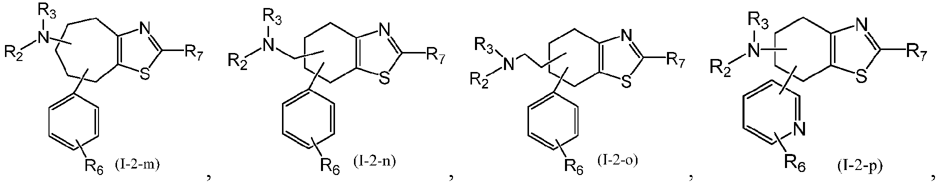 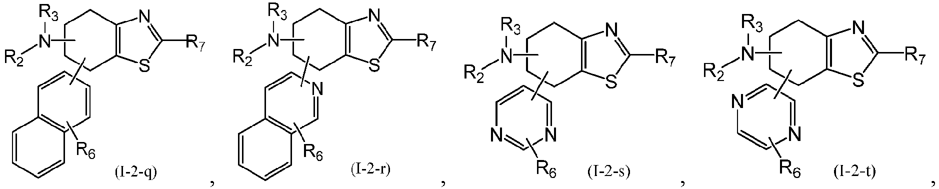 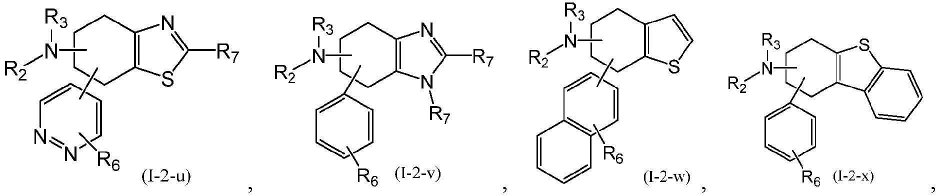 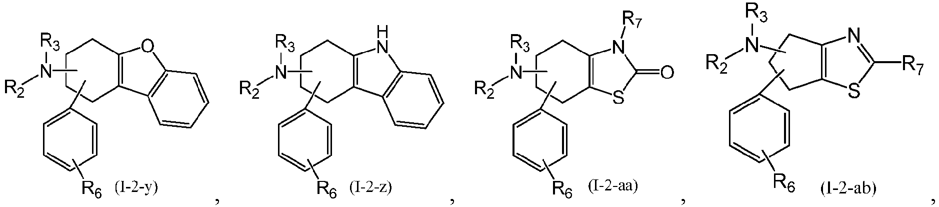 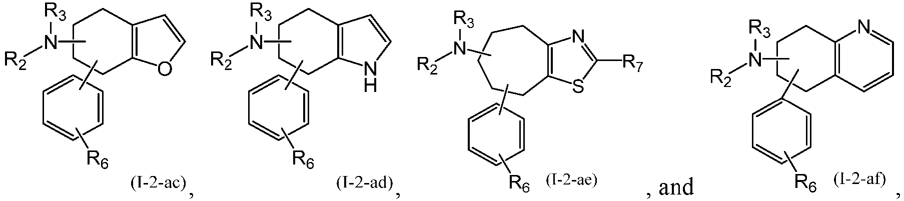
wherein R₂, R₅, R₆, and R₇ are as defined in claim 1;
and the phenyl, pyridinyl, naphthyl, quinolyl, pyrimidinyl, pyrazinyl, or pyridazinyl, and the -NR₂R₃, 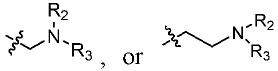 are attached to the same ring carbon atom of the carbocyclic ring to which they are attached.

7. The aryl-containing amine compound of formula (I), and the stereoisomer thereof, the geometric isomer thereof, the conformational isomer thereof, the tautomer thereof, the pharmaceutically acceptable salt thereof, the polymorph thereof, the solvate thereof, the hydrate thereof, and the isotopically labeled compound thereof according to any one of claims 1-6, wherein the compound of formula (I) is selected from the group consisting of:

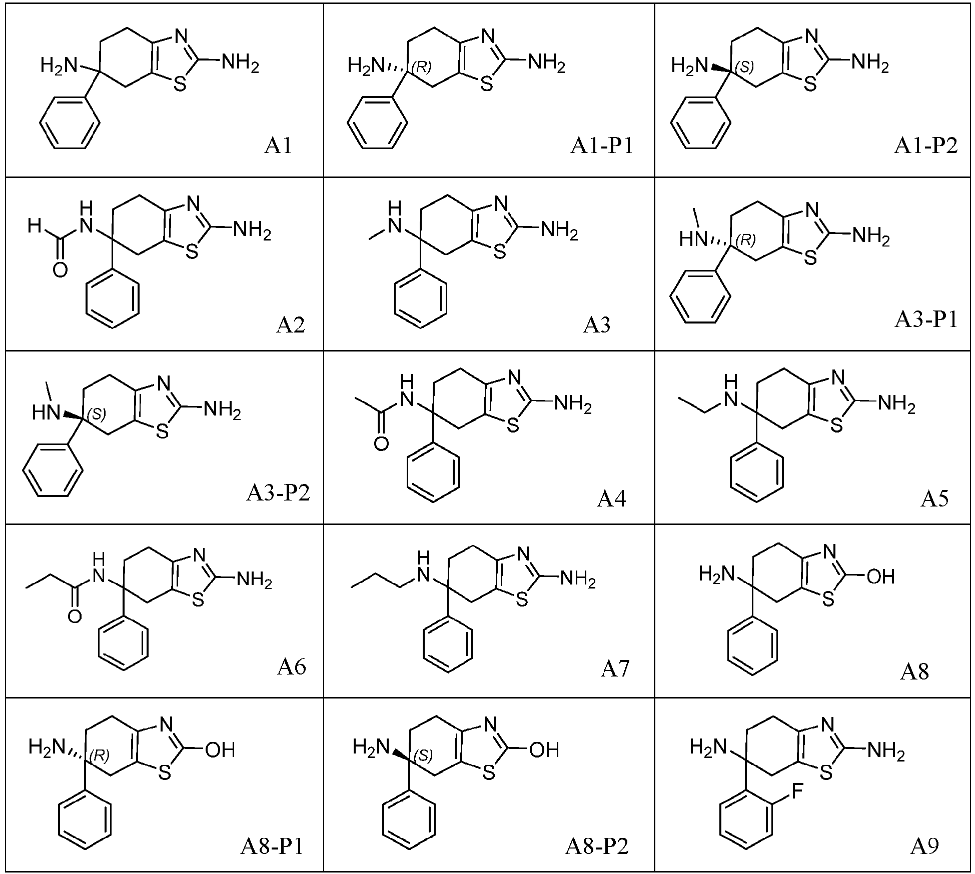

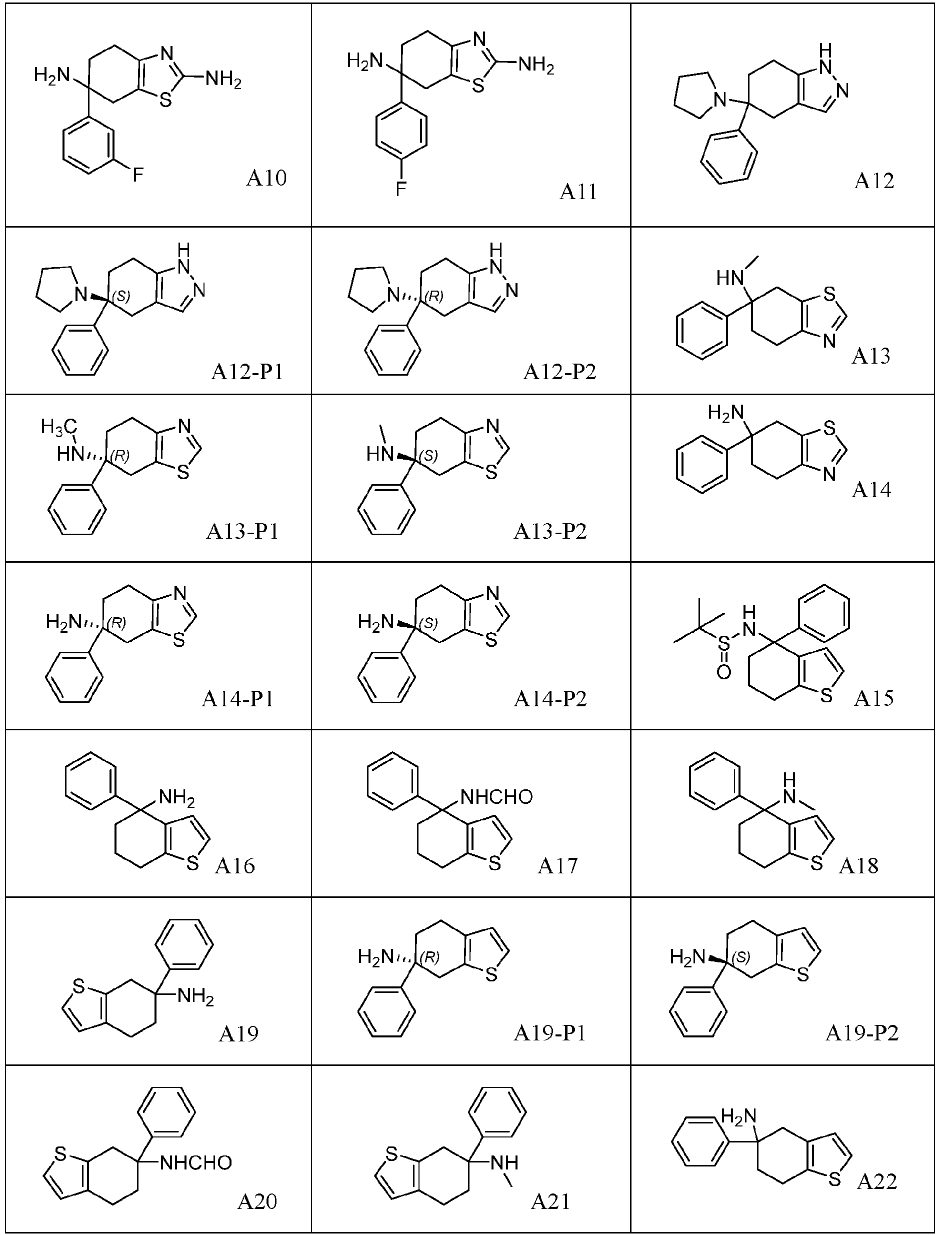

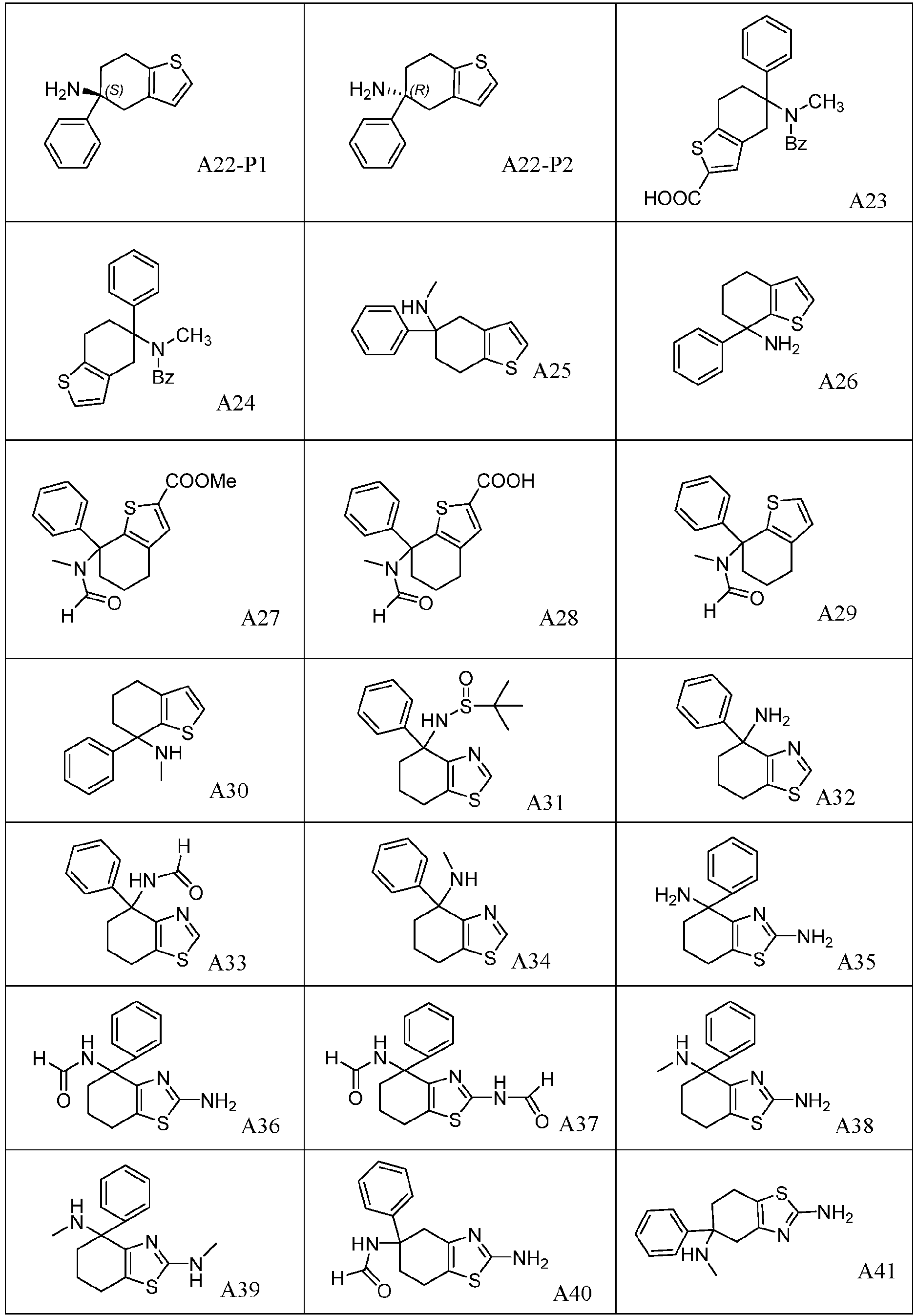

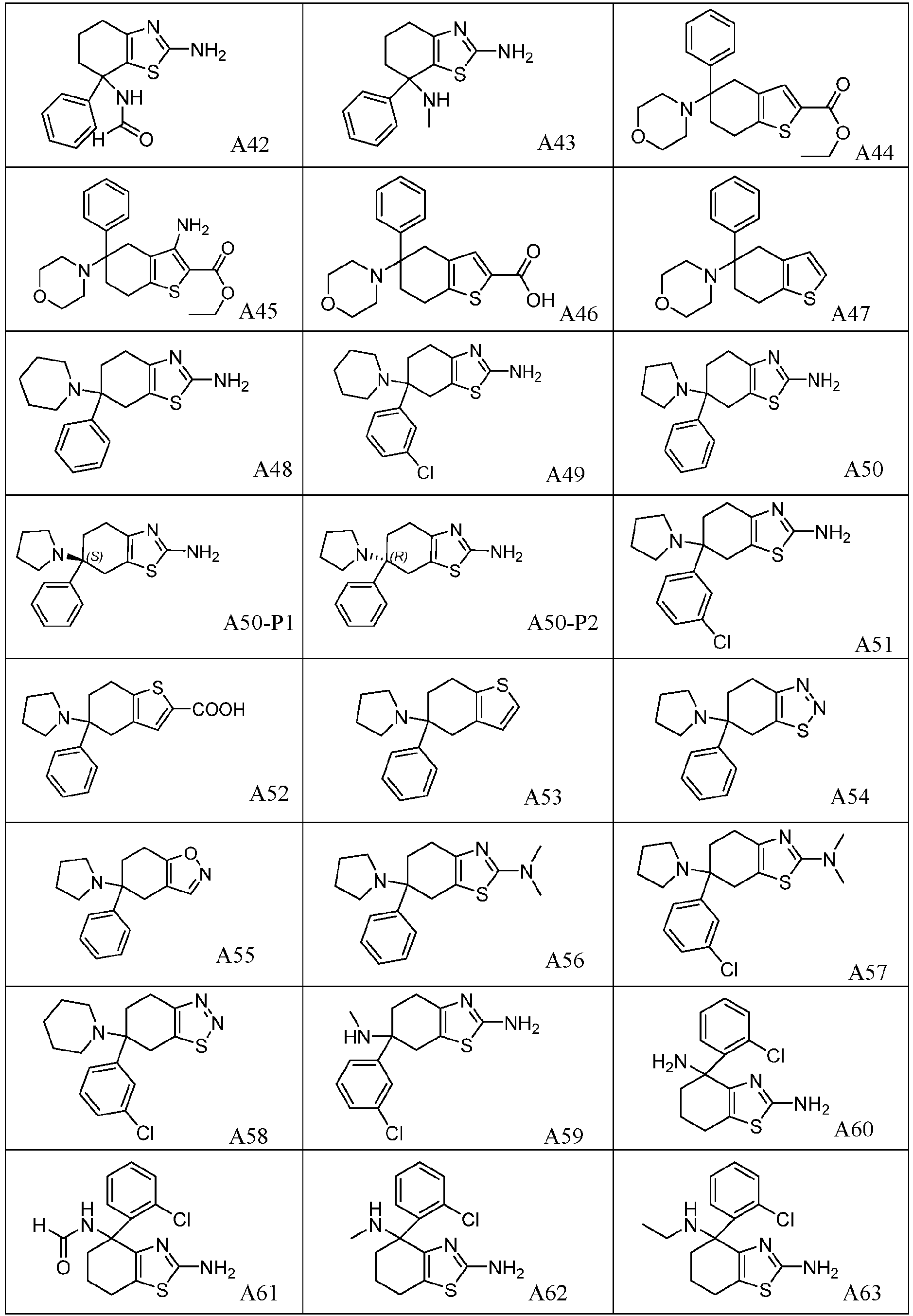

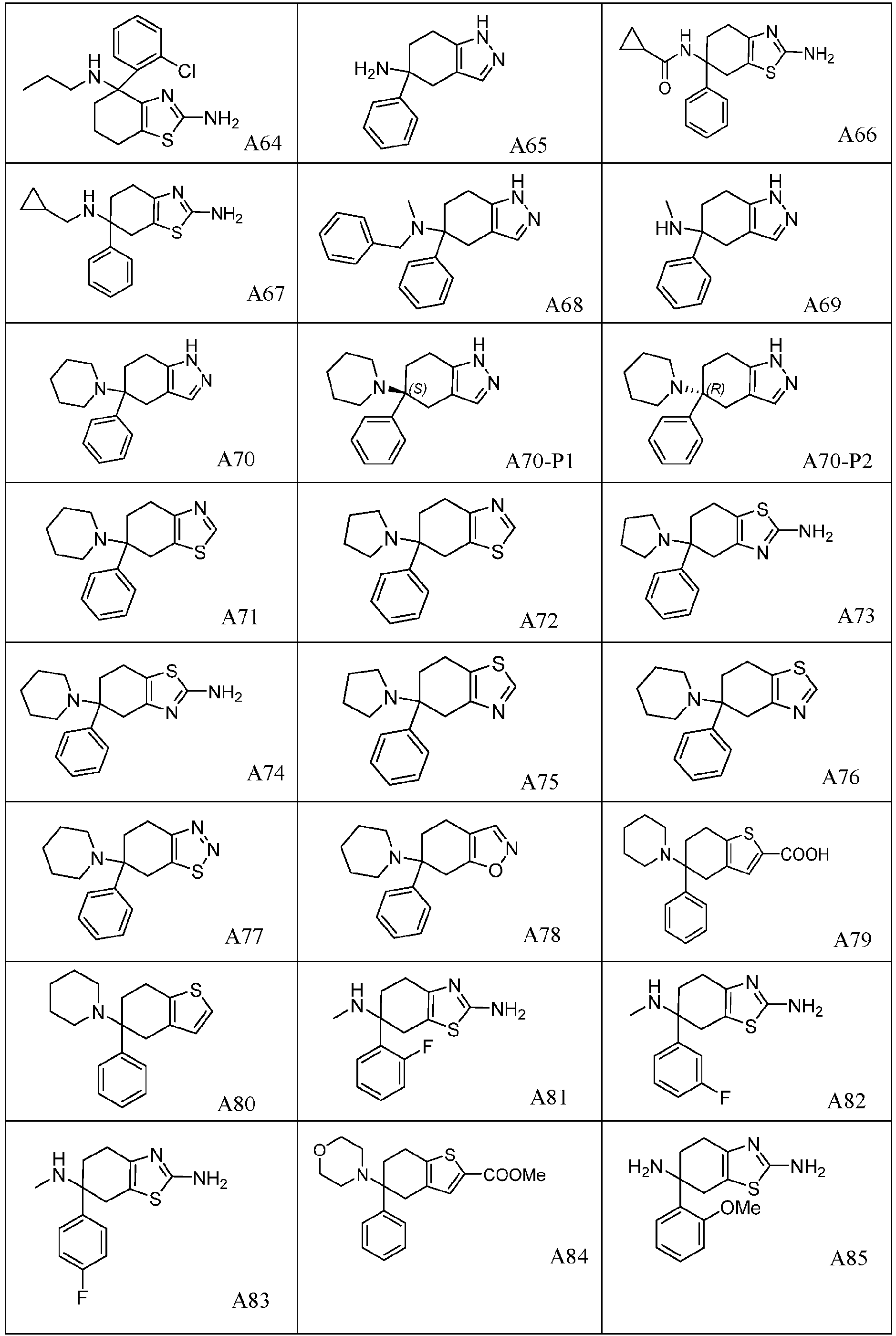

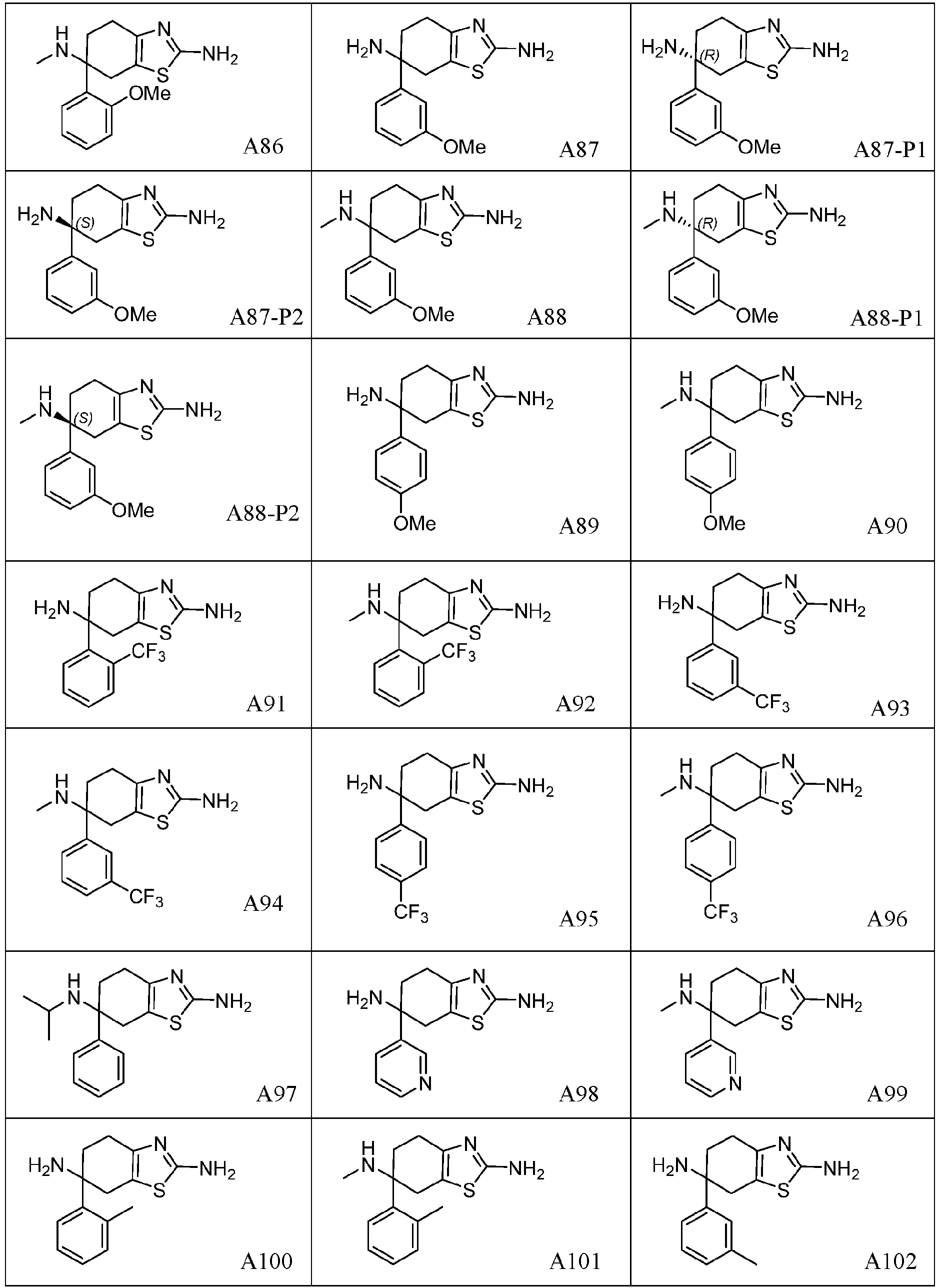

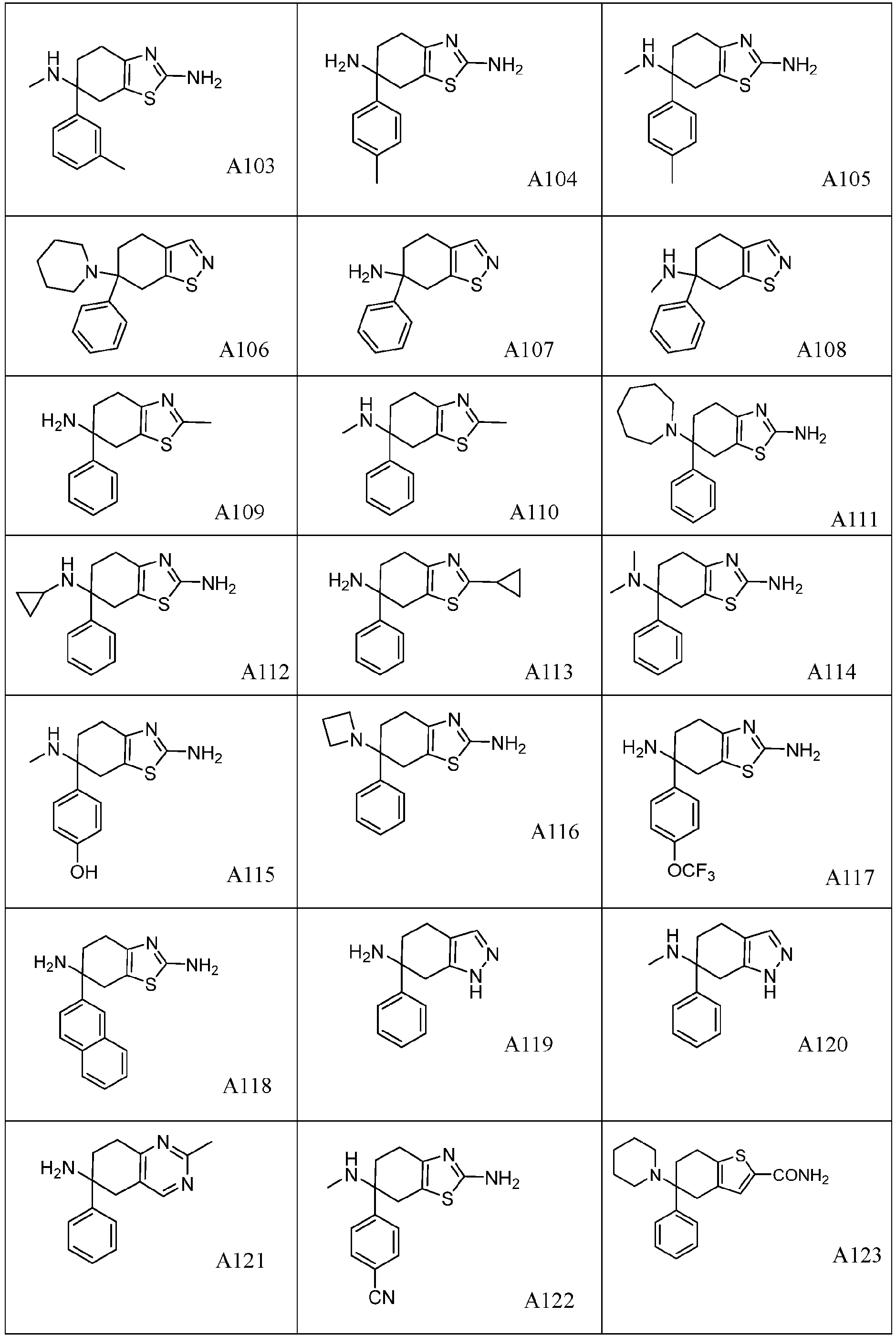

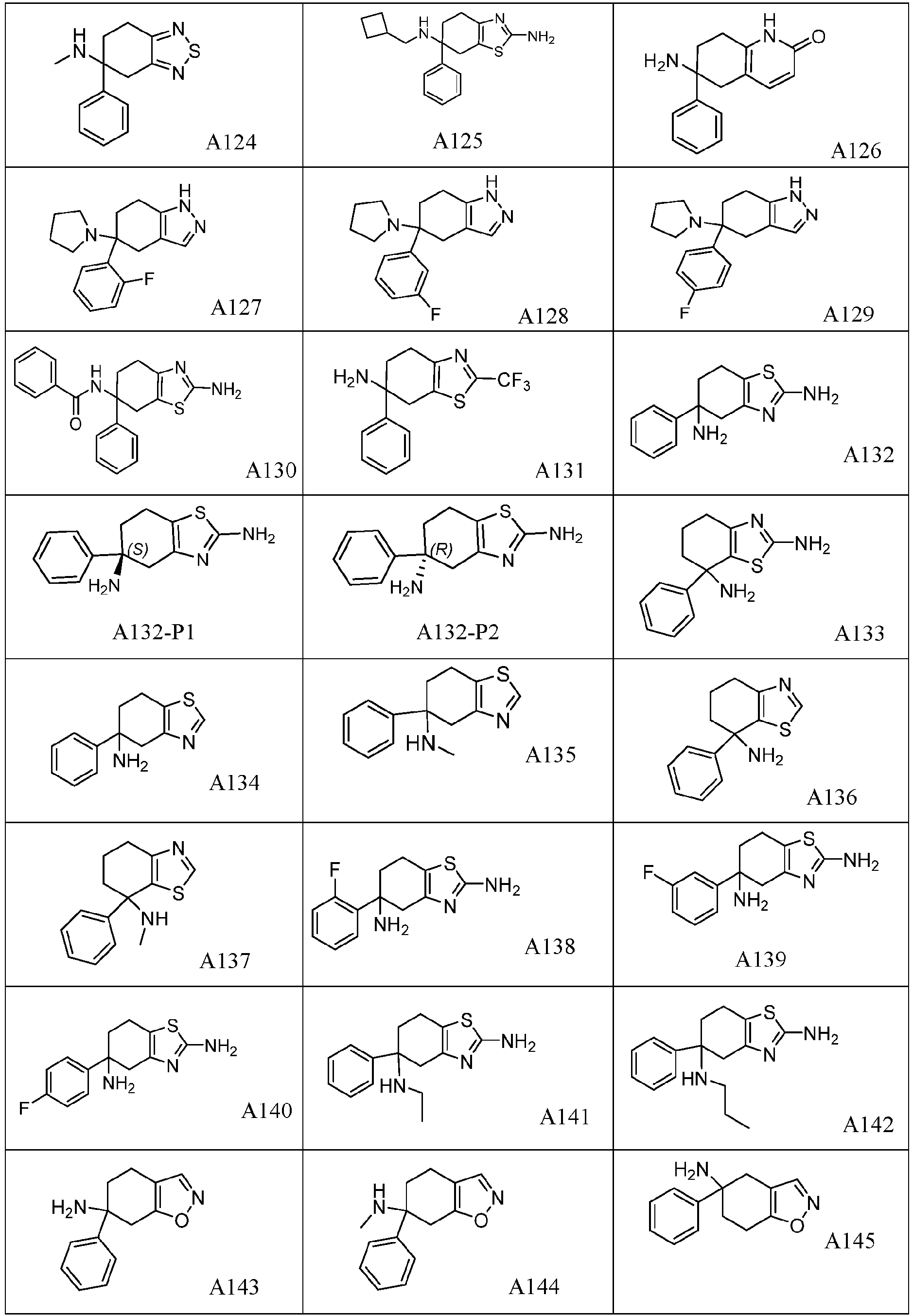

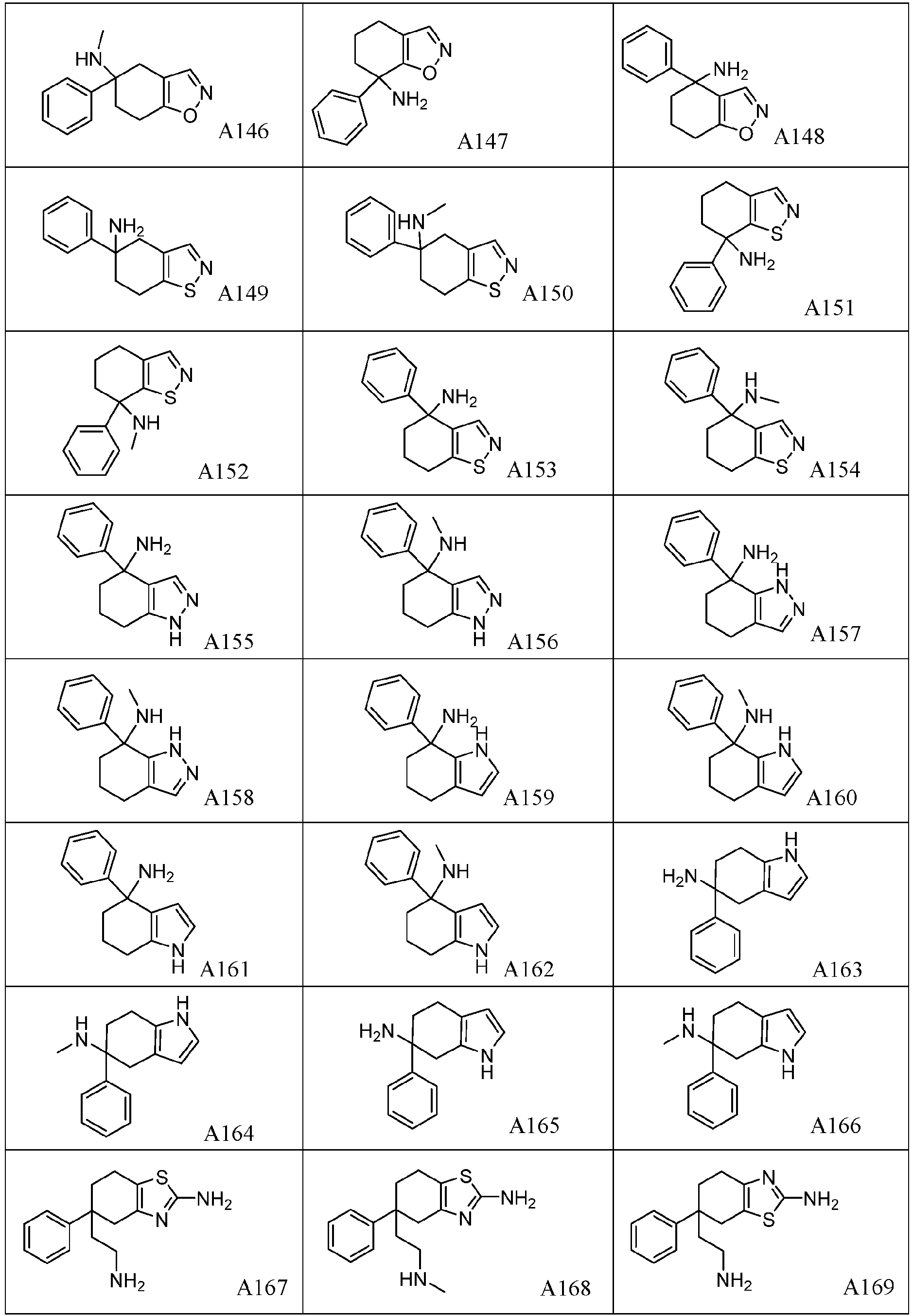

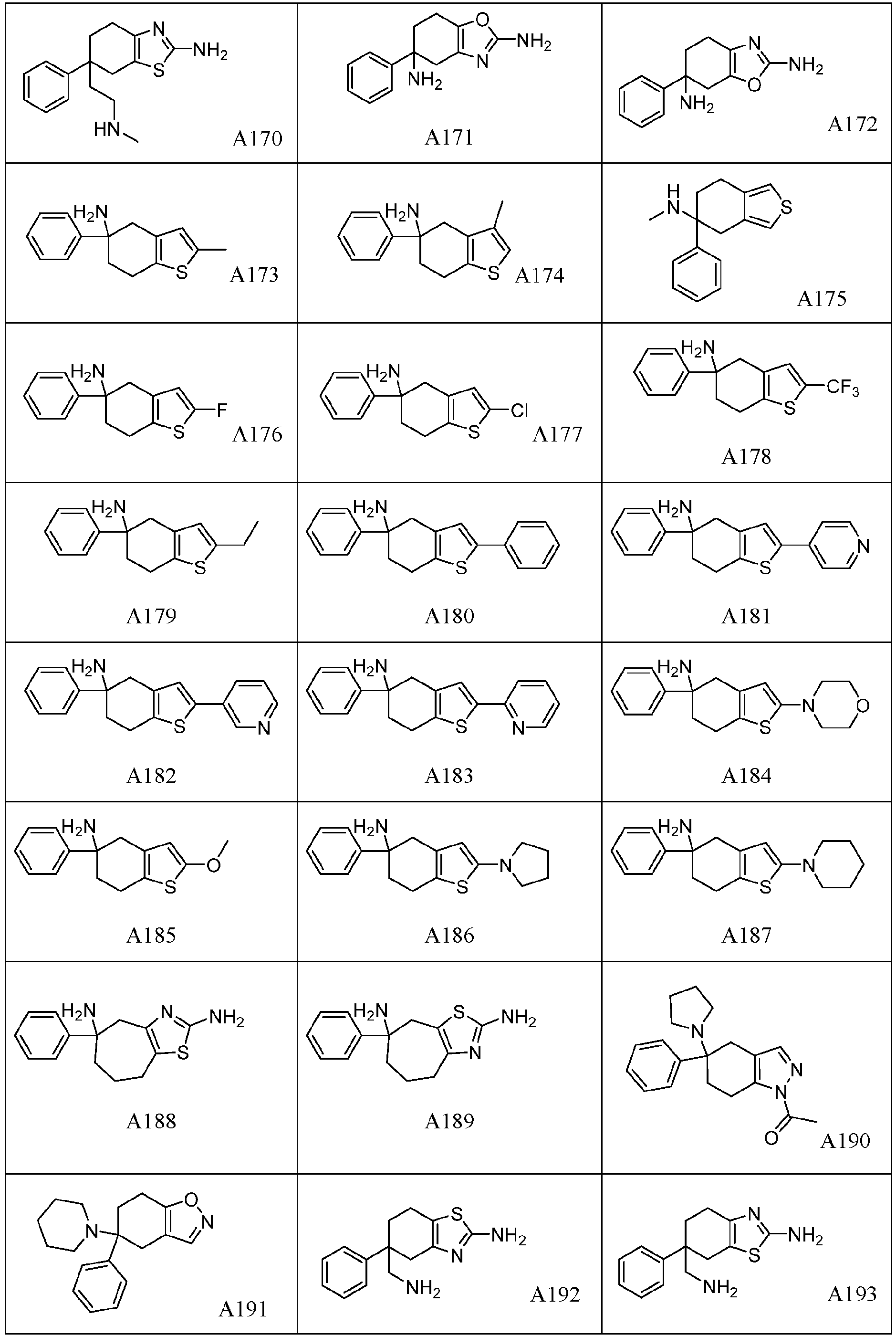

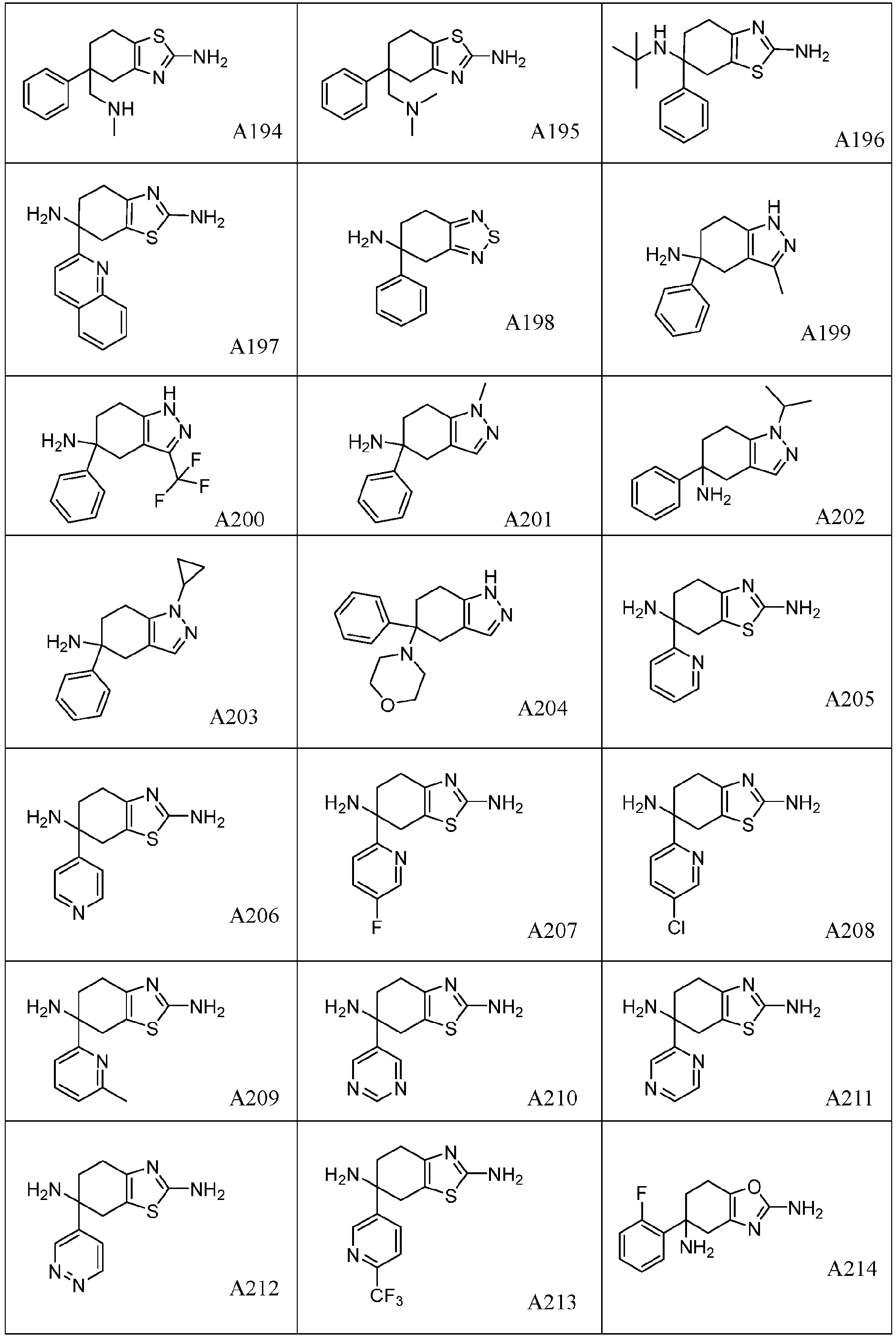

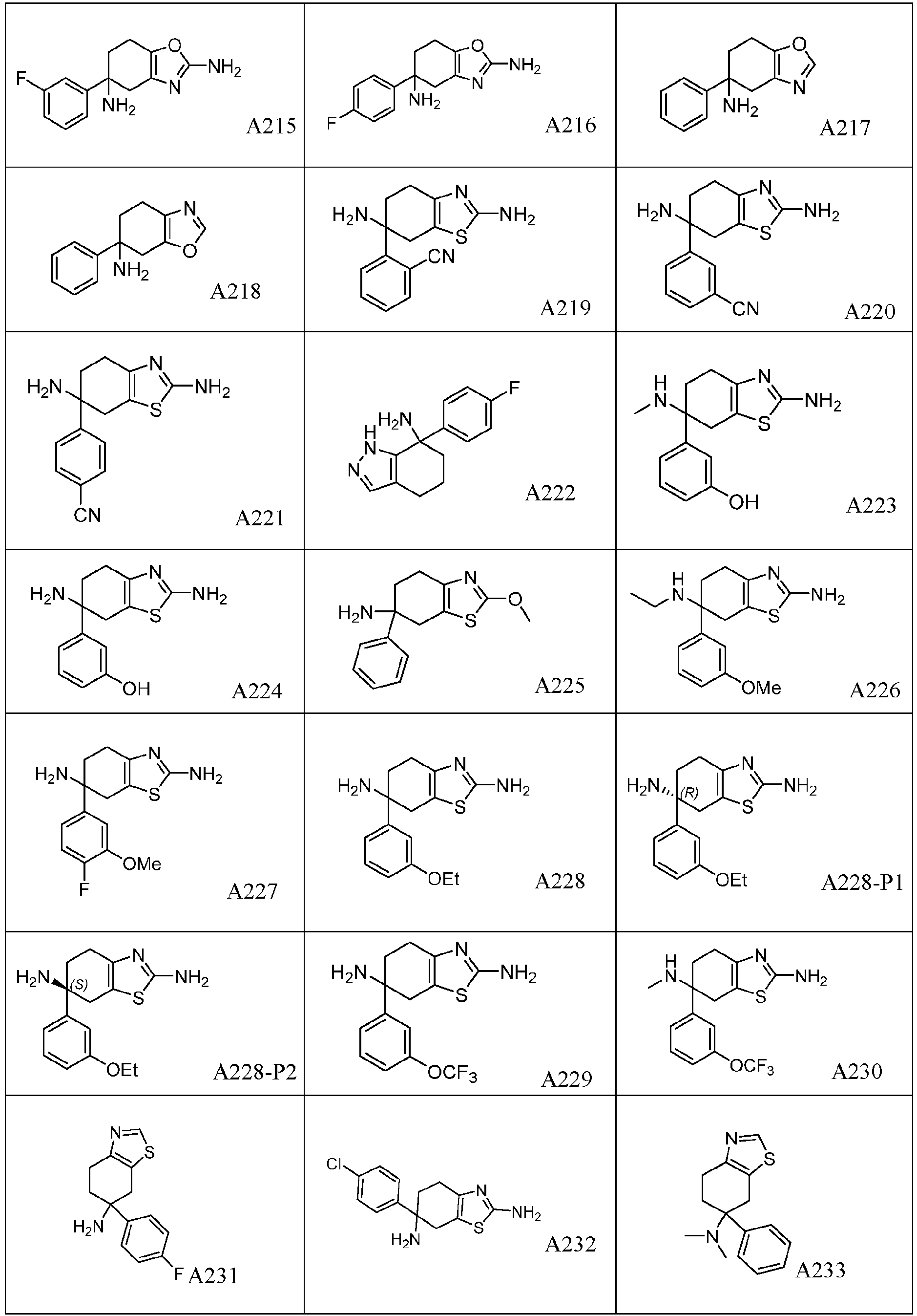

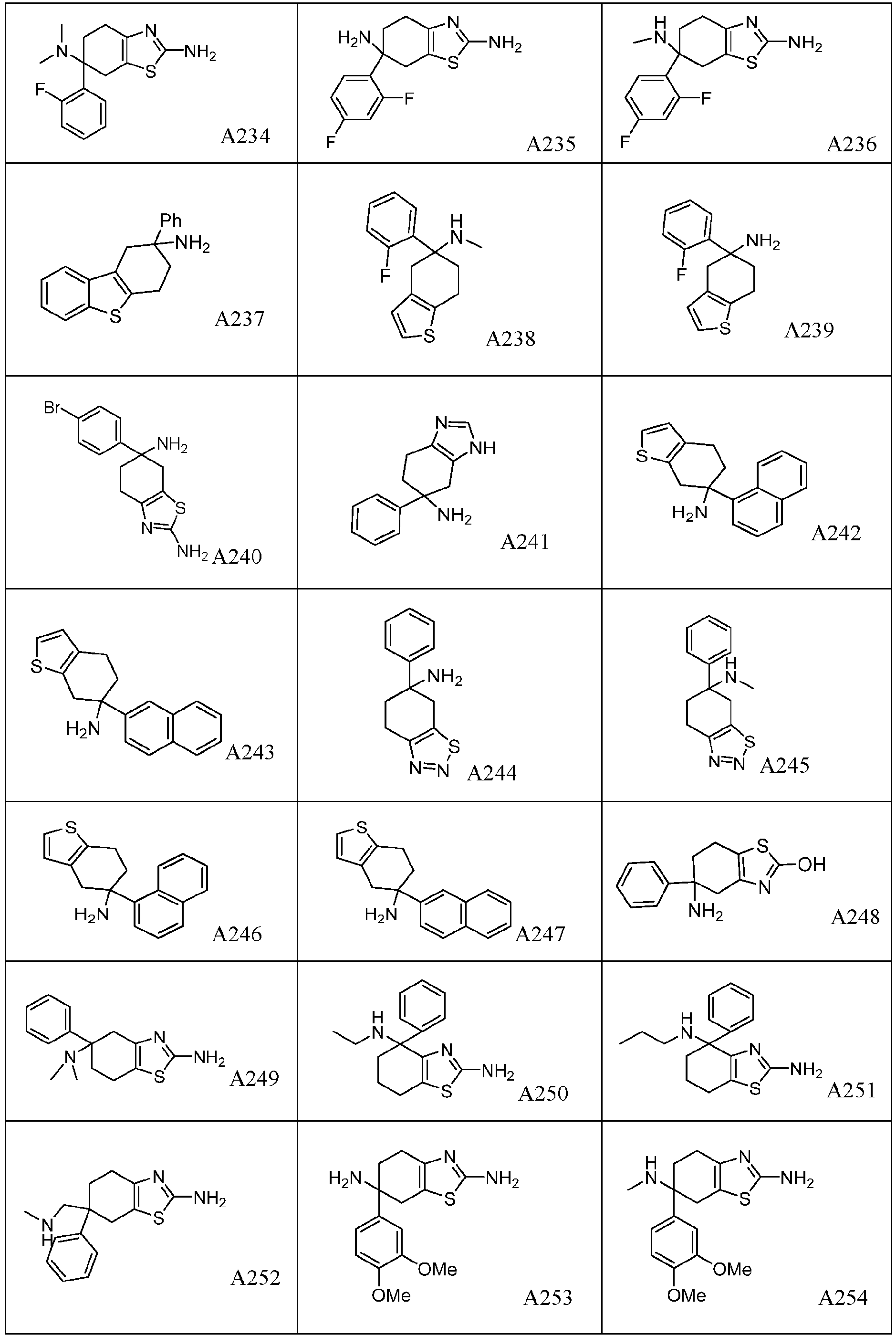

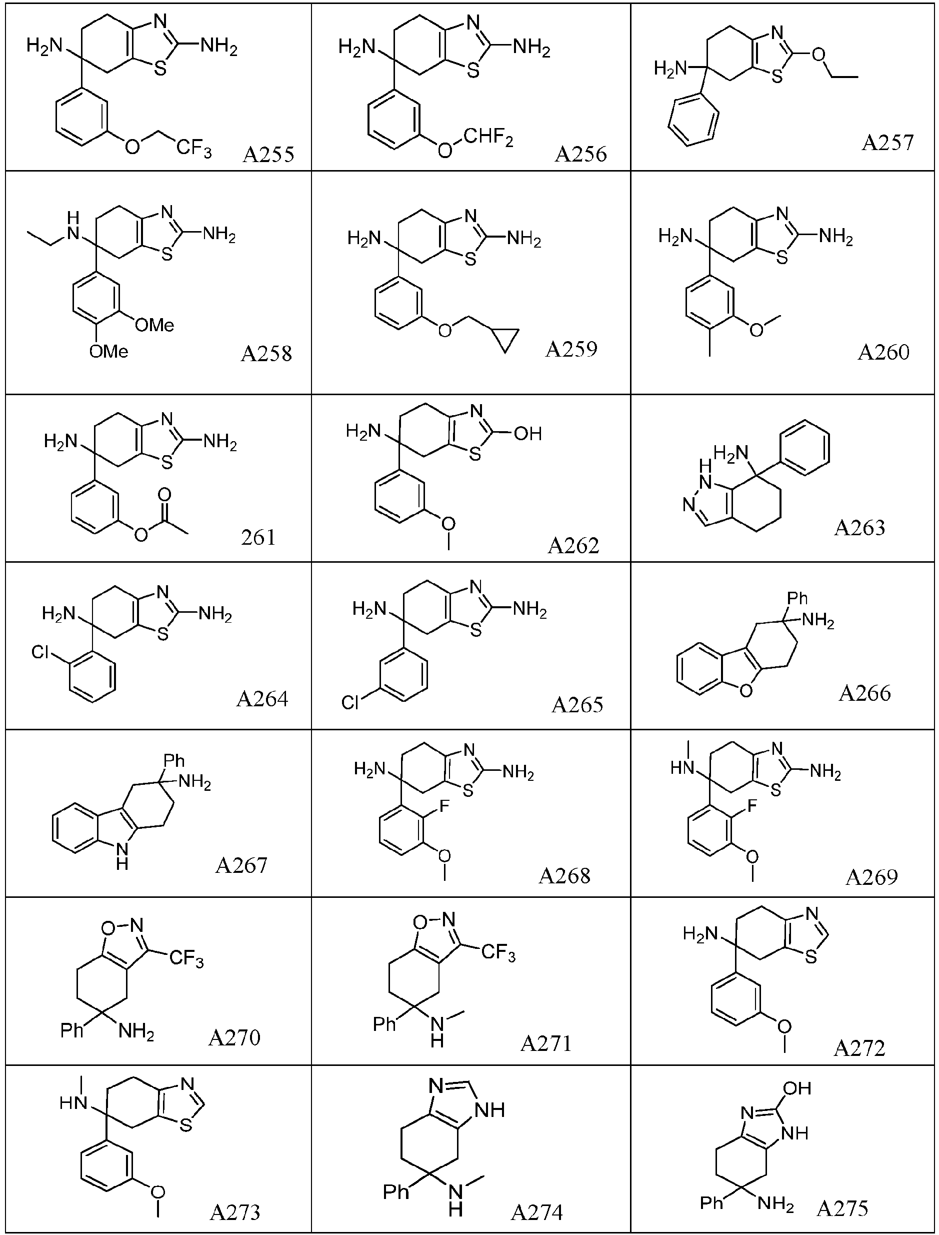

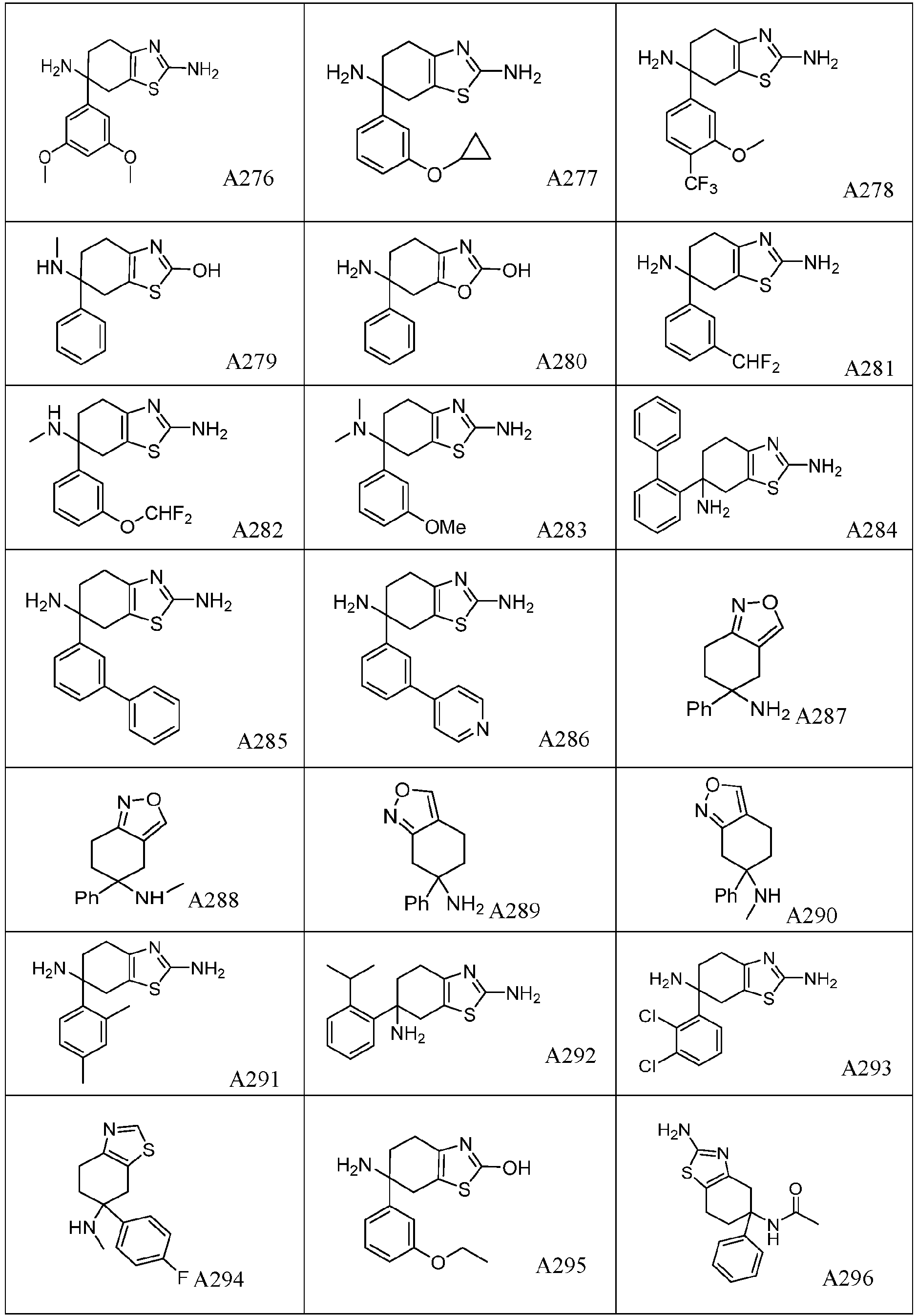

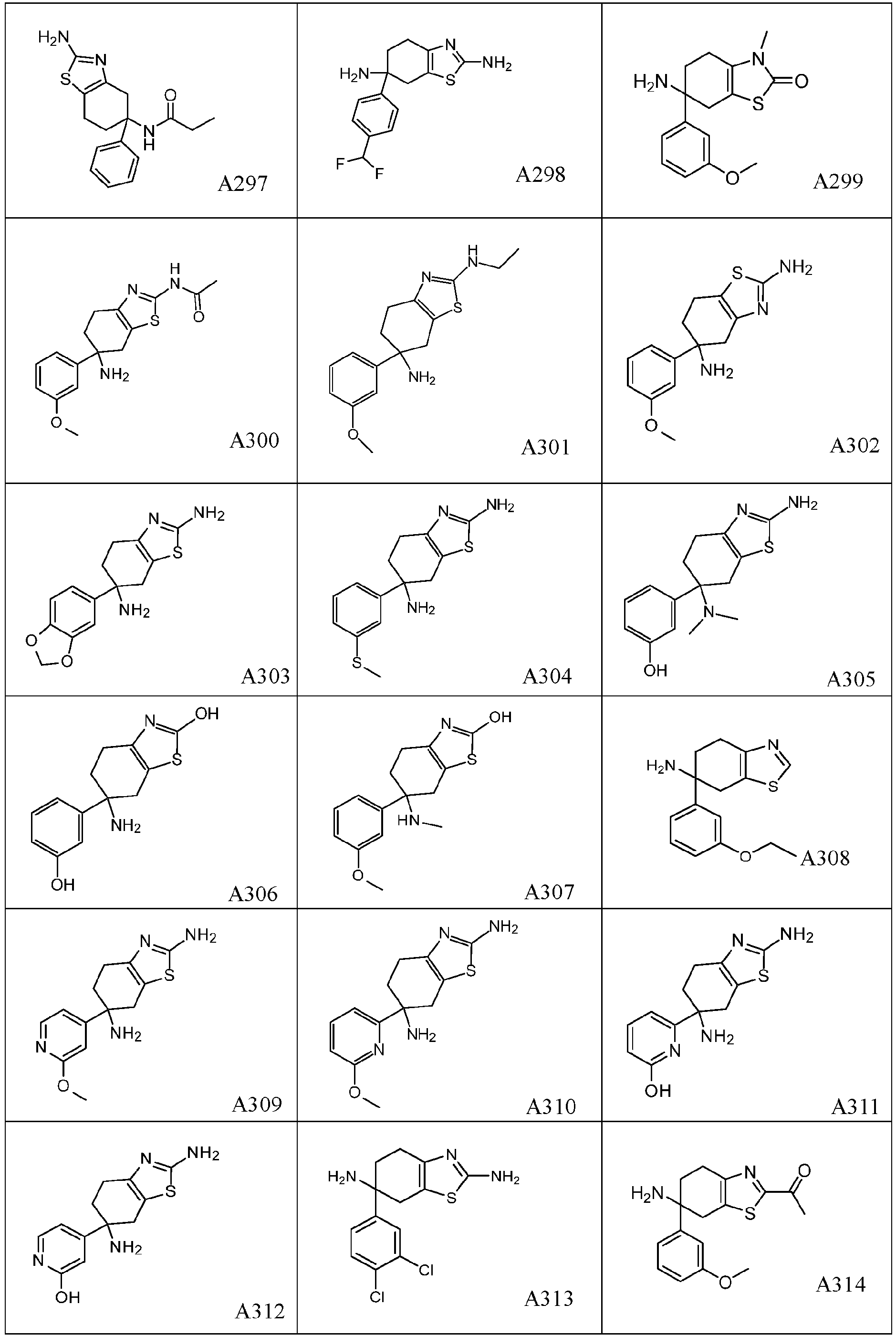

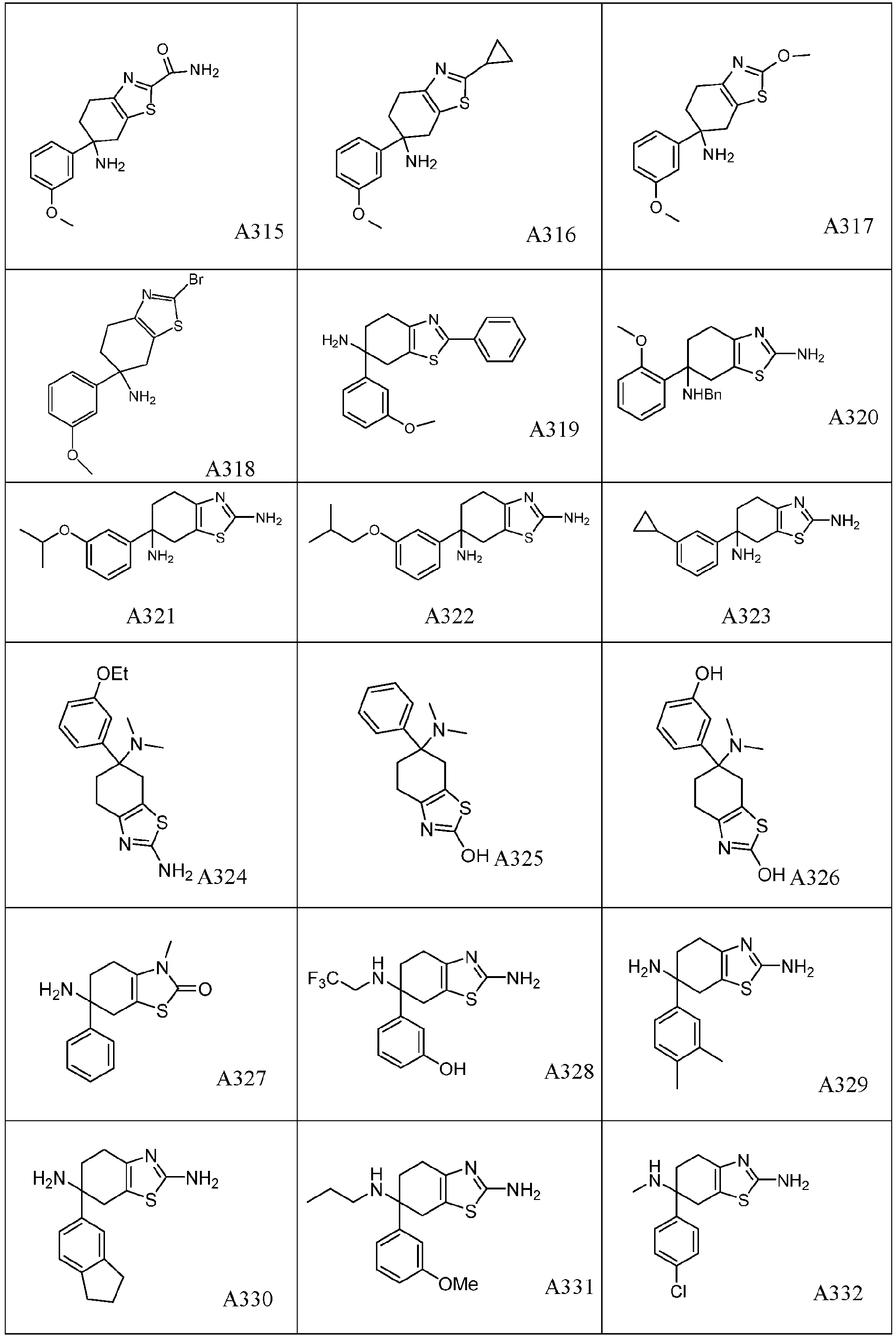

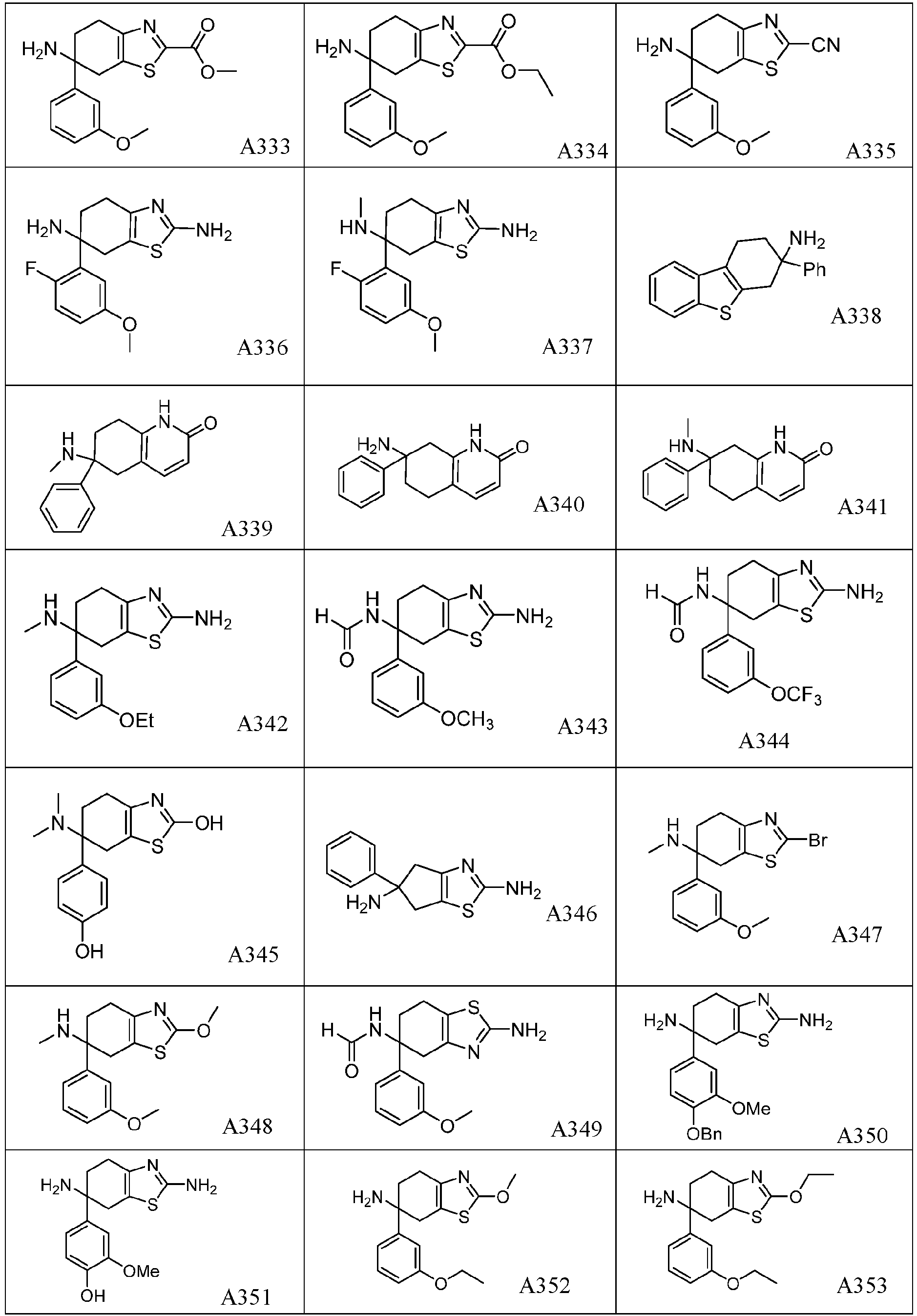

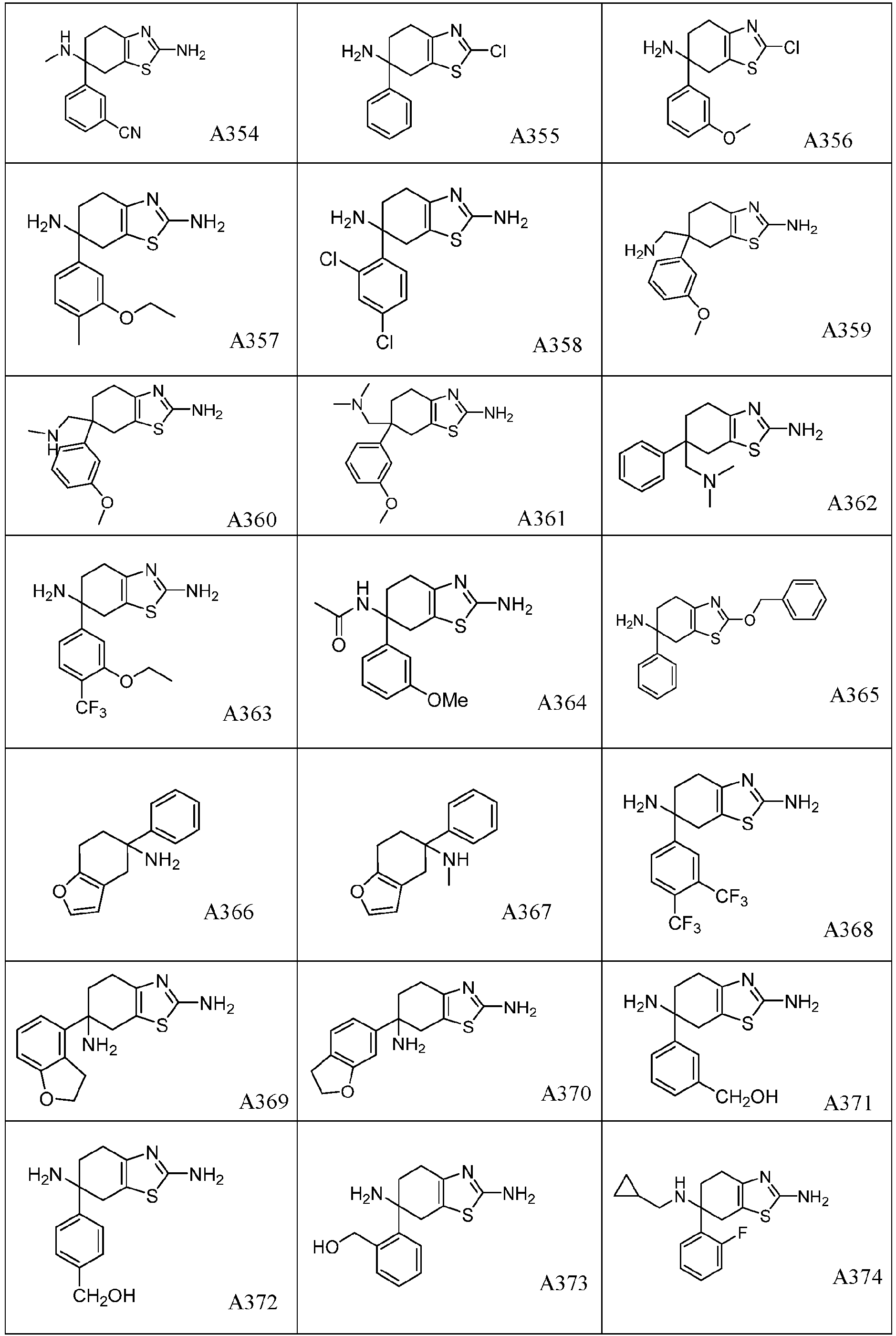

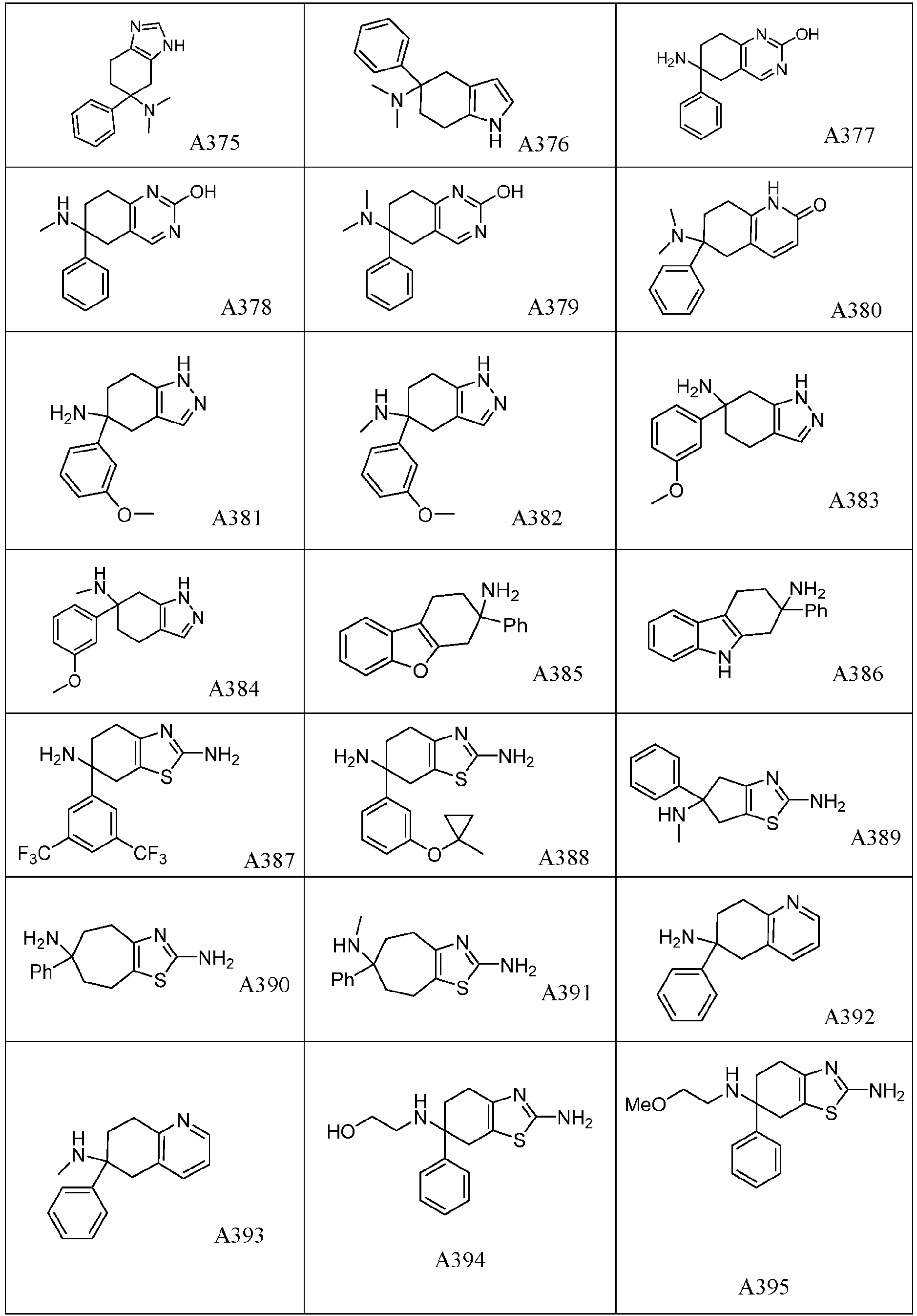

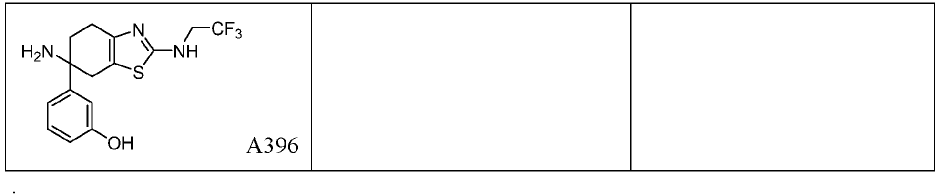

8. A method of preparing the aryl-containing amine compound of formula (I) according to any one of claims 1-7, wherein the method is carried out through one or a combination of the following methods 1-3:
Method 1:
as shown in reaction formula 1, the method comprises the steps of:
a) subjecting a compound of formula (II) and a compound of formula (III) to a condensation reaction to generate a compound of formula (IV);
b) subjecting the compound of formula (IV) and a compound of formula (V) to a nucleophilic addition reaction to generate a compound of formula (I-a); 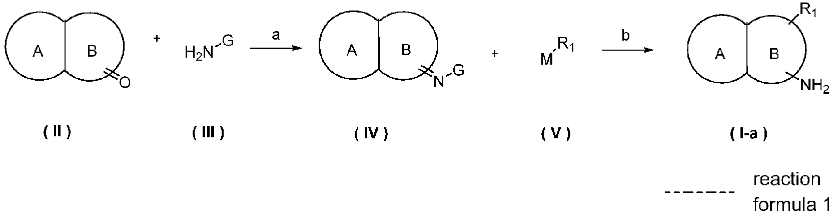
wherein ring A, ring B, and R₁ are as defined in the corresponding claims;
G represents a leaving group selected from the group consisting of C1-C6 alkylsulfinyl, benzenesulfinyl, naphthalenesulfinyl, and benzyl, wherein the C1-C6 alkylsulfinyl, benzenesulfinyl, naphthalenesulfinyl, and benzyl are optionally substituted with one or more groups selected from the group consisting of halogen, C1-C6 alkyl, nitro, hydroxy, amino, C1-C6 alkanoyl, C1-C6 alkoxy, and phenyl;
M represents a leaving group selected from the group consisting of a metal element, halogen, a metal complex, borane, silane, and a diazonium salt; preferably, M is -MgBr, -MgCl, or -Li;
Method 2:
as shown in reaction formula 2, the method comprises the steps of:
c) subjecting a compound of formula (II) and a compound of formula (V) to a nucleophilic addition reaction to generate a compound of formula (VI);
d) subjecting the compound of formula (VI) and azide to a substitution reaction to generate a compound of formula (VII);
e) subjecting the compound of formula (VII) to a reduction reaction to generate a compound of formula (I-a); 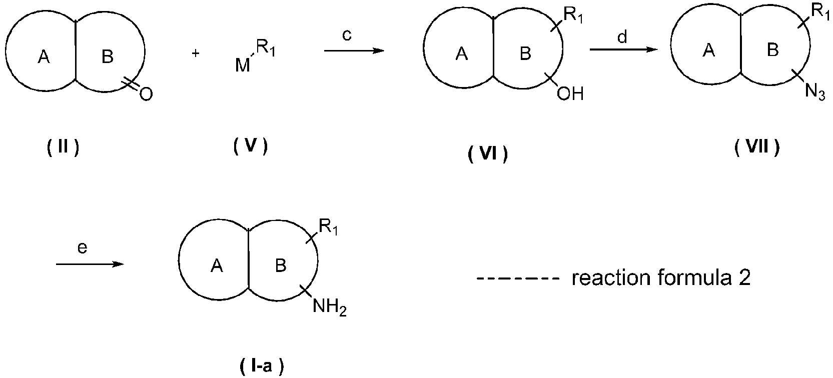
wherein ring A, ring B, and R₁ are as defined in the corresponding claims;
M represents a leaving group, such as a metal element, halogen, a metal complex, borane, silane, and a diazonium salt; preferably, M is -MgBr, -MgCl, or -Li; and
Method 3:
subjecting the compound of formula (I-a) obtained by the method 1 or 2 to a functional group transformation of amino to give other aryl-containing amine compounds of formula (I).

9. A pharmaceutical composition, comprising a therapeutically effective amount of one or more selected from the group consisting of the aryl-containing amine compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof according to any one of claims 1-7, and optionally one or more pharmaceutically acceptable carriers, diluents, or excipients.

10. Use of one or more of the aryl-containing amine compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof according to any one of claims 1-7, or the pharmaceutical composition according to claim 9, in preparing a medicament for regulating activity of an NMDA receptor and/or a monoamine transporter and/or a sigma receptor, particularly in preparing the following medicaments:
a) an NMDA receptor antagonist;
b) a monoamine transporter inhibitor; and
c) a sigma receptor agonist or antagonist.

11. Use of one or more of the aryl-containing amine compound of formula (I), the stereoisomer thereof, the geometric isomer thereof, the conformational isomer thereof, the tautomer thereof, and the pharmaceutically acceptable salt thereof, the polymorph thereof, the solvate thereof, the hydrate thereof, and the isotopically labeled compound thereof according to any one of claims 1-7, or the pharmaceutical composition according to claim 9, in preparing a medicament for preventing and/or treating a disease associated with an NMDA receptor and/or a monoamine transporter and/or a sigma receptor, particularly a central nervous system disease,
wherein preferably, the central nervous system disease is selected from the group consisting of: cerebral ischemia; stroke; cerebral infarction; traumatic brain injury; anti-NMDA receptor encephalitis; epilepsy; amyotrophic lateral sclerosis; schizophrenia; uncontrollable, intractable, or chronic schizophrenia; affective disorder; mental disorder; mood disorder; bipolar I disorder; bipolar II disorder; depressive disorder; endogenous depressive disorder; major depressive disorder; uncontrollable depressive disorder; dysthymic disorder; cyclothymic disorder; panic attack; panic disorder; social phobia; obsessive-compulsive disorder; impulse control disorder; post-traumatic stress disorder; anxiety disorder; acute stress disorder; hysteria; anorexia nervosa; sleep disorder; adjustment disorder; cognitive disorder; autism; neuropathic pain; mania; Parkinson's disease; Huntington's disease; Alzheimer's disease; dementia; memory disorder; hyperactivity disorder; attention deficit/hyperactivity disorder; tic disorder; other neurological events or neurodegeneration caused by NMDA receptor activation; preferably, the neuropathic pain comprises diabetic peripheral neuropathy, postherpetic neuralgia, complex regional pain syndrome, peripheral neuropathy, chemotherapy-induced neuropathic pain, cancer-related neuropathic pain, neuropathic low back pain, HIV-associated neuropathic pain, trigeminal neuralgia, and central post-stroke pain; and
further preferably, the central nervous system disease is selected from the group consisting of bipolar I disorder; bipolar II disorder; depressive disorder; endogenous depressive disorder; major depressive disorder; uncontrollable depressive disorder; dysthymic disorder; cyclothymic disorder; panic attack; panic disorder; social phobia; obsessive-compulsive disorder; impulse control disorder; post-traumatic stress disorder; anxiety disorder; acute stress disorder; Parkinson's disease; diabetic peripheral neuropathy; postherpetic neuralgia; complex regional pain syndrome.
